# EUROPEAN PATENT APPLICATION

(11) **EP 4 198 511 A1**
(43) Date of publication of application: **21.06.2023**
(21) Application number: 21215679.8
(22) Date of filing: 17.12.2021
(51) Int. Cl.: G01N 33/574, C12Q 1/6886, G01N 33/68, G16H 50/50

(54) **METHOD FOR DETERMINING AML PROGNOSIS**

(71) Applicant: Lehtio, Janne, 124 32 Bandhagen (SE)
(72) Inventor: LEHTIÖ, Janne, 124 32 Bandhagen (SE); VESTERLUND, Mattias, 113 35 Stockholm (SE)
(74) Representative: Aera A/S

(57) **Abstract**

The present invention relates to a method for stratifying an Acute Myeloid Leukemia ("AML") patient into a group of predicted outcome in relation to determinants such as, but not limited to, overall survival, event free survival and/or reaching complete remission, as well as predicted response to treatment and/or drugs. The method comprises generating a proteomic and/or a proteo-genomic profile of a patient suffering from Acute Myeloid Leukemia ("AML") and determining e.g., if said patient belongs to a high-risk or a low-risk group.

## Description

### FIELD OF THE INVENTION

The present invention relates to a biomarkers and methods for stratifying an Acute Myeloid Leukemia ("AML") patient into a group of predicted outcome in relation to determinants such as, but not limited to, overall survival, event free survival and/or reaching complete remission, as well as predicted response to treatment and/or drugs. The method comprises generating a proteomic profile to be used alone or together with a genomic profile of a patient suffering from Acute Myeloid Leukemia ("AML") and determining e.g., if said patient belongs to a high-risk, or a low-risk group, and/or a high-risk, an intermediate-risk or a low-risk group.

### BACKGROUND

Among patients with leukemia there can be a highly variable clinical course as reflected by varying survival times and resistance to therapy. Reliable individual prognostic tools are limited at present. However, advances in proteomic technologies provide new diagnostic and prognostic indicators for hematologic malignancies such as leukemia.

Acute myeloid leukemia (AML) is a genetically heterogeneous disease with frequent relapses and overall poor survival. Improving the understanding of molecular drivers and the genotypephenotype interplay in AML is needed to improve clinical decision making and prognostication.

Acute myeloid leukemia (AML) is an aggressive hematologic malignancy where immature blast cells arising from myeloid leukemic progenitors accumulate in the bone marrow and bloodstream. The incidence of AML increases with age with a median age of onset in Sweden of 71 years. It has been established that, like for many cancers, prevalence of AML is higher amongst males than females.

In the 1970's, cytarabine coupled to an anthracycline was first introduced as a treatment for AML and this has remained the mainstay treatment since. In recent years, new, targeted therapies focused on specific mutations (e.g. IDH1/2 or FLT3 mutations), genetic aberrations or key pathways have been introduced or advanced to late clinical trials. Although these drugs generally can improve clinical outcome, the vast genetic and phenotypic heterogeneity of AML patients leading to highly varying therapeutic responses remains a major clinical challenge. Hence, it is paramount to apply comprehensive proteomic profiling to identify AML patients that can benefit from novel targeted therapies.

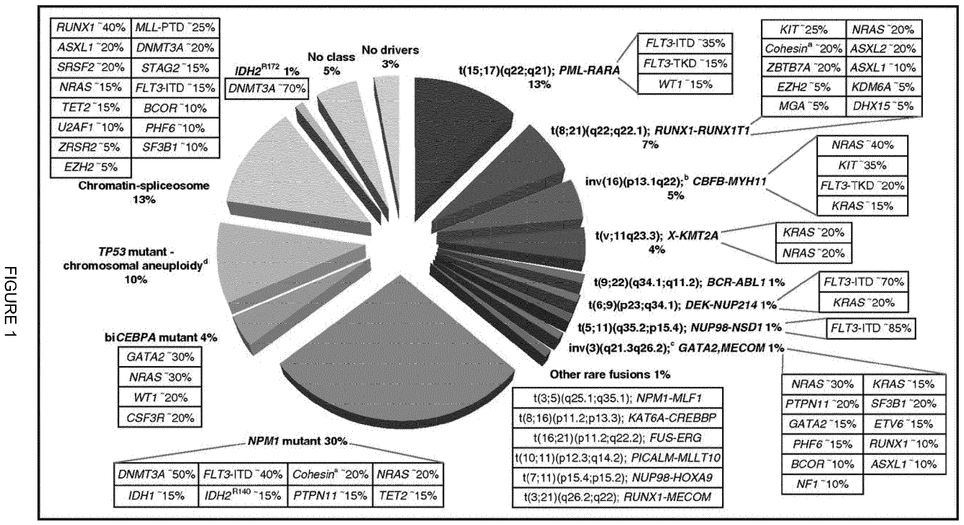

Several large cohort studies have helped elucidate the complex genomic and mutational landscape of AML [Ilyas, A.M., et al., Next generation sequencing of acute myeloid leukemia: influencing prognosis. BMC Genomics, 2015. 16 Suppl 1(Suppl 1): p. S5., Ley, T.J., et al., Genomic and epigenomic landscapes of adult de novo acute myeloid leukemia. N Engl J Med, 2013. 368(22): p. 2059-74.]. Recent publications have also described genomically defined subtypes of AML associated to chromosomal aberrations and mutations with clear links to patient survival [Papaemmanuil, E., H. Dohner, and P.J. Campbell, Genomic Classification in Acute Myeloid Leukemia. N Engl J Med, 2016. 375(9): p. 900-1.]. A summarizing illustration can be seen in figure 1 [Hartmut Döhner et al., Diagnosis and management of AML in adults: 2017 ELN recommendations from an international expert panel, BLOOD, 26 JANUARY 2017 x VOLUME 129, NUMBER 4].

Cancer is manifested by a number of genetic aberrations that alter the cellular phenotype and contribute to carcinogenesis. While these aberrations contribute to the evolution of cancer; cancer is predominantly a signalling disease [Yaffe, M.B., Why geneticists stole cancer research even though cancer is primarily a signalling disease. Sci Signal, 2019. 12(565).]. Global proteomics is an excellent way to study how genetic aberrations influence the molecular phenotype in cancer on a systems level. However, this approach has been hindered by the lack of analytical depth in standard proteomics methods.

Techniques to analyse the proteome have historically lagged behind those of DNA and RNA sequencing in terms of scale and analytical depth. Therefore, there are large gaps in knowledge on how genomic aberrations influence the functional proteome, including mutations found in prevalent diseases like cancer. While AML has been well characterized by sequencing methods, the proteome of AML has only been investigated to a shallow extent [Aasebø, E., et al., Global Cell Proteome Profiling, Phospho-signaling and Quantitative Proteomics for Identification of New Biomarkers in Acute Myeloid Leukemia Patients. Current pharmaceutical biotechnology, 2016. 17(1): p. 52-70.].

While there have been a few large proteogenomic studies published recently, none so far have focused on AML [Flores-Morales, A., et al., Proteogenomic Characterization of Patient-Derived Xenografts Highlights the Role of REST in Neuroendocrine Differentiation of Castration-Resistant Prostate Cancer. Clin Cancer Res, 2019. 25(2): p. 595-608., Mertins, P., et al., Proteogenomics connects somatic mutations to signalling in breast cancer. Nature, 2016. 534(7605): p. 55-62.].

Recent development of in-depth proteomics by the current inventors has for the first time allowed reaching the same analytical depth in proteomics as in transcriptomics [Arabi, A., et al., Proteomic screen reveals Fbw7 as a modulator of the NF-κB pathway, Nature Communications, volume 3, Article number: 976 (2012)]. The current disclosure thus for the first time describes an in-depth proteomic characterization of a treatment naïve AML patient cohort and applications of said analytical outcome to prognostically stratify individual AML patients as belonging to a either a high-risk, an intermediate-risk or a low-risk group or to a high-risk or a low-risk group.

### SUMMARY

The present invention is based on the herein for the first time disclosed in-depth mass spectrometry-based proteomic analysis of a clinically and molecularly well defined, treatment naïve, cohort of AML patients. It was found that altered expression levels of proteins involved in mRNA splicing correspond to shorter survival and a poor treatment response. It was determined that this is independent of currently used genetic risk stratifications and can on its own be used to improve prognostic estimates. It was shown that heterogenous genetic backgrounds contribute to this proteome phenotype and that it is related to epigenetic changes leading to altered splicing patterns which contribute to cancer progression. In addition to being a powerful tool on its own, the herein disclose proteomic analysis can also be used in combination with currently used genetic risk stratifications for an even more complex stratification of AML patients. Thus. the herein disclosed method relates to stratifying individual AML patients as belonging either to a high-risk or a low-risk group, or to a high-risk, an intermediate-risk or a low-risk group.

The present invention thus in its broadest aspect relates to a method for stratifying an AML patient into a high-risk or a low-risk group, or into a high-risk, an intermediate-risk or a low-risk group, in relation to overall survival and/or complete remission, based on proteomic profiling and/or on proteogenomic profiling, compared to an AML patient cohort of AML patients, wherein the method comprises,
a. isolating a blood and/or tissue sample from said patient,
b. processing said sample, wherein the processing comprises extracting expressed proteins from said sample,
c. analyzing the extracted proteins, including quantitatively determining their expression level,
d. determining a median expression level of at least 5 proteins of said extracted proteins, selected from the list of proteins with SEQ ID NOs: 1-17500 listed in table 1 for the intermediate samples of the cohort,
e. quantitatively determining the proteomic risk profile of said patient by identifying abnormal protein expression levels of at least 5 proteins selected from the list of proteins with SEQ ID NOs: 1-17500 listed in table 1, wherein the protein expression level is considered abnormal when
   i. the protein expression level of a protein with a SEQ ID NO listed in Table 2 is downregulated in such a way that the protein expression level of said protein is less than 95% of the median expression level of said protein for the intermediate samples of the cohort, or
   ii. the protein expression level of a protein with a SEQ ID NO listed in Table 3 is upregulated in such a way that the protein expression level of said protein is at least 5% above the median expression level of said protein for the intermediate samples of the cohort, and
f. wherein said AML patient is stratified as belonging to a high-risk group when abnormal protein levels are detected in step e).

In one embodiment, the method of the present invention comprises that the median expression level of the at least 5 proteins selected from the list of proteins with SEQ ID NOs: 1-17500 listed in table 1 for the intermediate samples of the cohort is predetermined.

In one method for stratifying an AML patient into a high-risk or a low-risk group, according to the present invention, said patient is classified as belonging to a high-risk group, when said patient's proteomic profile shows abnormal protein levels in at least 50% of the selected proteins.

In one method for stratifying an AML patient into a high-risk, an intermediate-risk or a low-risk group, according to the present invention, said patient is classified as belonging to a high-risk group, when said patient's proteomic profile shows abnormal protein levels in at least 50% of the selected proteins and said patient is classified as belonging to a median-risk group, when said patient's proteomic profile shows abnormal protein levels in at least 25% but less than 50% of the selected proteins and said patient is classified as belonging to a low-risk group, when said patient's proteomic profile shows abnormal protein levels in less than 25% of the selected proteins.

In another method for stratifying an AML patient into a high-risk or a low-risk group according to the present invention, one or more clustering algorithm(s) is/are used to cluster patients into at least a high-risk and a low-risk group based on abnormal protein levels of at least 5 proteins selected from the list of proteins with SEQ ID NOs: 1-17500.

The algorithm(s) used to cluster patients in a method according to the present invention is/are typically one or more algorithm(s) selected from the group consisting of Random Forest, k-Top Scoring Pairs, k-Nearest Neighbor, Support Vector Machines, oPLS-DA, PLS-DA, t-SNE, UMAP, PCA, lasso, Decision Trees, Naïve Bayes and Logistic Regression. Said one or more algorithm(s) can be used to cluster proteins of SEQ ID NO: 1-17500 into sub-groups, wherein one or more of the sub-groups is/are used to stratify patients into at least a high-risk and a low-risk group.

In one embodiment, a method for stratifying an AML patient into a high-risk or a low-risk group or into a high-risk, an intermediate-risk or a low-risk group according to the present invention comprises the use of less than 1000, 500, 100 or 50 protein sequences of any of SEQ ID NO: 1-17500.

In one method of the present invention, the proteomic risk profile of said patient is determined by identifying abnormal protein levels of at least 5 proteins selected from the list of proteins with a SEQ ID NO as provided in Table 4 and/or in Table 5.

In yet another aspect of the present invention, the method of the present invention further comprises extracting chromosomal DNA from said sample in step b. and analyzing the extracted DNA and determining genetic risk factors based on genetic variants in one or more genes listed in a genetic standard and providing a patient stratification into groups based on their overall, complete, combined, clinical and/or molecular profile. The standard can typically be selected from the group consisting of ELN2017 and NCCN Guidelines.

Typically, the genetic risk factors are determined based on genetic variants in at least 2, 4 or 5, such as in at least 10, 50 or 100 genes listed in a genetic standard and providing a patient stratification into groups based on their clinical and molecular profile. Thus, providing an additional aspect to the method of the present invention for a patient stratification into groups based on their overall, complete, combined, clinical and molecular profile.

Thus, in one method for stratifying an AML patient into a high-risk, an intermediate-risk or a low-risk group according to the present invention said patient is classified as belonging to a high-risk group, when said patient's proteomic profile shows abnormal protein levels in at least 50% of the selected proteins and said patient is classified as high-risk by determining genetic risk factors listed in a genetic standard. In one method for stratifying an AML patient into a high-risk, an intermediate-risk or a low-risk group according to the present invention, said patient is classified as belonging to a low-risk group, when said patient's proteomic profile shows abnormal protein levels in less than 50% of the selected proteins and said patient is classified as low-risk by determining genetic risk factors listed in a genetic standard.

In one method for stratifying an AML patient into a high-risk, an intermediate-risk or a low-risk group according to the present invention said patient is classified as belonging to a low-risk group, when said patient's proteomic profile shows abnormal protein levels in less than 25% of the selected proteins and said patient is classified as intermediate by determining genetic risk factors listed in a genetic standard. In one method for stratifying an AML patient into a high-risk, an intermediate-risk or a low-risk group according to the present invention said patient is classified as belonging to an intermediate-risk group, when said patient's proteomic profile shows abnormal protein levels in at least 25% and less than 50% of the selected proteins and said patient is classified as intermediate by determining genetic risk factors listed in a genetic standard.

A method for stratifying an AML patient according to the present invention comprises analyzing proteins and/or DNA extracted from blood and/or tissue samples from said patient e.g., selected from the group consisting of skin, mucosa, bone marrow, peripheral blood, isolated cells from blood and tumour cells. The currently described method thus enables the stratification of a patient by taking a single sample, or by taking a series and/or variety of samples from said same patient.

The extracted DNA and/or proteins are typically analysed using DNA hybridization, DNA sequencing, quantitative proteomic mass spectrometry, targeted mass spectrometry, ELISA, proximity ligation assay, proximity extension assay, aptamer-based assays, Analytical protein microarrays, reverse phase protein arrays, 2D-PAGE, Data-independent acquisition (DIA) mass spectrometry and/or Data-dependent acquisition (DDA) mass spectrometry.

The method of the present invention is particularly useful for stratifying a single AML patient as belonging to a high-risk, an intermediate-risk or a low-risk group according to the present invention wherein a median expression level of said extracted proteins has been predetermined. Thus, the single AML patient can routinely be checked against a predetermined standard provided to the clinician. Said predetermined standard can include both proteomic marker values as well as information from prevailing genetic standards.

Thus, a method for stratifying an AML patient according to the present invention also comprises providing a patient stratification into groups based on their overall, complete, combined, clinical and molecular profile.

The method of the present invention is in one embodiment used for predicting response to one or more apoptosis modulating drug(s) in an AML patient, wherein the patient is stratified as respondent when said patient is found to belong to the high-risk group or intermediate-risk group. An exemplary drug is selected from the group consisting of venetoclax, navitoclax and triciribine. The method of the present invention is in another embodiment used for predicting response to chemotheraphy in an AML patient, wherein the patient is stratified as respondent when said patient is found to belong to the low-risk group or the intermediate-risk group.

The method of the present invention is in one embodiment used for predicting response to therapy in a patient with acute myeloid leukemia (AML) comprising performing mass spectrometry on a plasma sample from a patient to generate a protein spectra comprising protein peaks, identifying a protein peak or group of protein peaks in the protein spectra corresponding to one or more proteins selected from the list of proteins with SEQ ID NOs: 1-17500, and predicting the patient's response to therapy based on the identification of one or more of the protein peaks.

### BRIEF DESCRIPTION OF THE FIGURES

*Figure 1*
   Hartmut Döhner et al., Diagnosis and management of AML in adults: 2017 ELN recommendations from an international expert panel, BLOOD, 26 JANUARY 2017 x VOLUME 129, NUMBER 4
*Figure 2*
   Patient outcomes by length of classifying list. Kaplan-meier curves for patients stratified by the different lists into two risk levels; high and low risk using DIA data for the discovery cohort. Log-rank tests were performed to evaluate significance levels and p-values are indicated in the figure. (A) Long List, (B) Medium list (C) Short list.
*Figure 3*
   Patient outcomes by length of classifying list. Kaplan-meier curves for patients stratified by the different lists into three risk levels; high, intermediate and low risk using DIA data for the discovery cohort. Log-rank tests were performed to evaluate significance levels and p-values are indicated in the figure. (A) Long List, (B) Medium list (C) Short list.
*Figure 4*
   Patient outcomes by risk assessment method for 10 prospectively collected patients. Kaplan-meier curves for patients stratified by the different risk assessment methods. Log-rank tests were performed to evaluate significance levels and p-values are indicated in the figure. (A) Patients stratified by ELN2017, (B) Same as (A) but Adverse and Intermediate was combined into a single group. (C) Patients stratified by proteomic risk assessment. (D) Patients stratified by Combined score 1 (see table 5). Patients with a score of 5 or 6 were grouped together and patients with a combined score of 2, 3 or 4 were grouped together. (E) Patients stratified by Combined score 2 (see table 11). Patients with a score of 5 or 6 were grouped together and patients with a combined score of 3 or 4 were grouped together.
*Figure 5*
   Proteomics analysis of AML. (A) Overview of the analysis workflow. (B) Relationship between peptide spectral matches and proteins identified. (C) Hieararchical clustering of patients by proteomic profiles, selected mutations and clinical features are shown. (D) Kaplan-Meier curves for patients belonging to cluster 1-4. (E) Heatmap of p-values for enrichment (Fisher test) for enrichment of genetic aberrations and mutations across proteomics clusters. (F) Sankayplot depicting how patients from different proteomic clusters align to the main genetic classifications from Papaemmanuil et al. (G) Same as E but to RNAseq classifications from TCGA(NEJM, 2013https://www.ncbi.nlm.nih.gov/pmc/articles/PMC3767041/). (H) Enrichment for proteomics clusters for 6 separate differentiation states, color denotes normalized enrichment score while adjusted p-values are visualized by the size of the circles. (I) UMAP of most variable proteins in the dataset, 8 protein clusters are highlighted and their enrichments and median levels in the 9 patient clusters are highlighted in the heatmap.
*Figure 6*
   Spliceosome levels and clinical features in AML. (A) Heatmap of spliceosomal proteins. Three separate clusters were identified high levels (n=45), intermediate levels (n=45 and low levels (n=28) (B) Kaplan-meier curves for patients belonging to the three groups. (C) Distribution of protein-mRNA correlations for individual genes for spliceosomal proteins and all protein-mRNA pairs in the dataset (D) Enrichment for the three groups for 6 separate differentiation states, color denotes normalized enrichment score while -log10(adjusted p-values) are visualized by the size of the circles. (E) Distribution of ELN2017 classifications in the three groups (F) Complete remission rates for the three groups (G) Survival curves for groups constructed by combining ELN2017 classifications with spliceosomal levels (Best: patients with lowest risk in at least one classification (Adverse, low levels) and not classified as highest risk in the other (Adverse, high levels); Intermediate: Intermediate in both, or high risk in one and low in the other; Worst: highest risk class in at least one classification and not classified as lowest risk in the other) (H) Waterfall plot of common AML mutations in the three groups. (I) Enrichment analysis for comparisons between the individual groups, color denotes normalized enrichment score while -log10(q-values) are visualized by the size of the circles.

### DEFINITIONS AND ABBREVIATIONS

In the present invention "AML" relates to "acute myeloid leukemia".
MS, Mass Spectrometry;
LC-MS/MS, liquid chromatography- tandem mass spectrometry;
LC-SRM-MS, liquid chromatography selected reaction monitoring mass spectrometry;
OGS, N-octyl glucoside;
MMTS, Methyl methanethiosulfonate;
TCEP, Tris-(2-carboxyethyl)-phosphine;
FA, Formic acid;
FAB, French-American-British (classification)
TPCK treated trypsin, Trypsin treated with L-(tosylamido-2-phenyl) ethyl chloromethyl ketone; beta -Gal, beta -galactosidase;
CV percent. Coefficient of variation;
LC-SRM-MS, Liquid chromatography-selected reaction monitoring mass spectrometry;
SIL peptide, Stable Isotope-Labeled Peptide;
DIA-MS, Data Independent acquisition mass spectrometry;
DDA-MS, Data Dependent acquisition mass spectrometry;
PRM, Parallell reaction monitoring
FDR, false discovery rate
OS, overall survival
SPE, solid phase extraction;
IQ panel, internal quality panel.

The term "proteome" refers to all the proteins expressed by a genome, and thus proteomics involves the identification of proteins in the body and the determination of their role in physiological and pathophysiological functions. The ^{~}30,000 genes defined by the Human Genome Project translate into 300,000 to 1 million proteins when alternate splicing and post-translational modifications are considered. While a genome remains unchanged to a large extent, the proteins in any particular cell change dramatically as genes are turned on and off in response to their environment.

As a reflection of the dynamic nature of the proteome, some researchers prefer to use the term "functional proteome" to describe all the proteins produced by a specific cell in a single time frame. Ultimately, it is believed that through proteomics, new disease markers and drug targets can be identified.

As used herein, the terms "centralized control system" or "centralized control network" refer to information and equipment management systems (e.g., a computer processor and computer memory) operably linked to multiple devices or apparatus (e.g., automated sample handling devices and separating apparatus). In preferred embodiments, the centralized control network is configured to control the operations of the apparatus and/or device linked to the network. For example, in some embodiments, the centralized control network controls the operation of multiple chromatography apparatuses, the transfer of sample between the apparatuses, and the analysis and presentation of data.

As used herein, the terms "computer memory" and "computer memory device" refer to any storage media readable by a computer processor. Examples of computer memory include, but are not limited to, RAM, ROM, computer chips, digital video disc (DVDs), compact discs (CDs), hard disk drives (HDD), and magnetic tape.

As used herein, the term "computer readable medium" refers to any device or system for storing and providing information (e.g., data and instructions) to a computer processor. Examples of computer readable media include, but are not limited to, DVDs, CDs, hard disk drives, magnetic tape and servers for streaming media over networks.

As used herein, the terms "processor" and "central processing unit" or "CPU" are used interchangeably and refers to a device that is able to read a program from a computer memory (e.g., ROM or other computer memory) and perform a set of steps according to the program.

It is to be understood that this invention is not limited to the particular methodology, protocols, and reagents described, as such may vary. It is also to be understood that the terminology used herein is for the purpose of describing particular embodiments only and is not intended to limit the scope of the present invention, which will be limited only by the appended claims.

As used herein the singular forms "a", "and", and "the" include plural referents unless the context clearly dictates otherwise. Thus, for example, reference to "a compound" includes a plurality of such compounds and reference to "the agent" includes reference to one or more agents and equivalents thereof known to those skilled in the art, and so forth. All technical and scientific terms used herein have the same meaning as commonly understood to one of ordinary skill in the art to which this invention belongs unless clearly indicated otherwise.

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

### DETAILED DESCRIPTION

The invention relates to the application of recently developed proteomic methods for identifying expression levels of AML-related proteins selected to correlate with AML progression and predicting disease outcome useful for diagnosis and prognosis, as well as to reagents, products and kits for performing said identification.

Defined lists of proteins specifically expressed in AML patients are presented herein. The AML-specific expression profiles of the proteins, disclosed herein for the first time, are associated with various properties of the disease AML. The invention therefore relates to the use of these protein panels or signature sets of such proteins for stratification, detection, diagnosis and treatment of AML, as well as for monitoring therapeutic progress in an AML patient. Thus, these proteins, both individually and as sets or "signatures" can be used to stratify the individual AML patient at least to belong to a high-risk or low-risk group of AML patients, thereby facilitating improved, optimized and/or individualized treatment of said AML patient displaying a specific expression profile of proteins identified herein for the first time as associated with the disease AML.

The disclosure presents applied in-depth proteomics to characterize the biology of AML. The inventors observed that certain genetically defined subtypes form well defined clusters or subclusters on the proteome level (e.g., inv(16), CEBPA) while other mutations and aberrations have little or varying impacts on protein levels. It could be observed that proteome profiles and clusters had clear relationships to survival. For the first time, a clear relationship between protein levels of particular spliceosome components and outcome was identified which could not be readily seen at the transcript level. Patients with high spliceosome levels demonstrated poorer survival, and this was independent of genetic risk factors and clinically used risk stratification models including ELN2017.

By combining the proteomic and genetic level information an improvement on existing models for patient stratification is herein provided, especially regarding short to mid-term outcomes. This highlights the clinical benefit of mass spectrometry screening of diagnostic patient samples. The invention presented herein can thus serve as an important tool for identifying patients with elevated risk and to adapt therapeutic strategies accordingly.

In the current context, the term "stratification" is used to describe the distribution of one or more patient(s) into subgroups, for instance a patient may be stratified by stage of AML, age, gender, response to certain drugs etc.

### Proteogenomic analysis of a treatment naïve Acute Myeloid Leukemia - study cohort

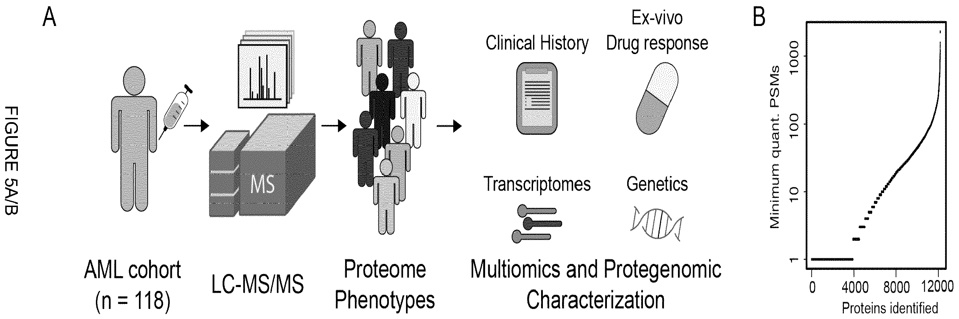

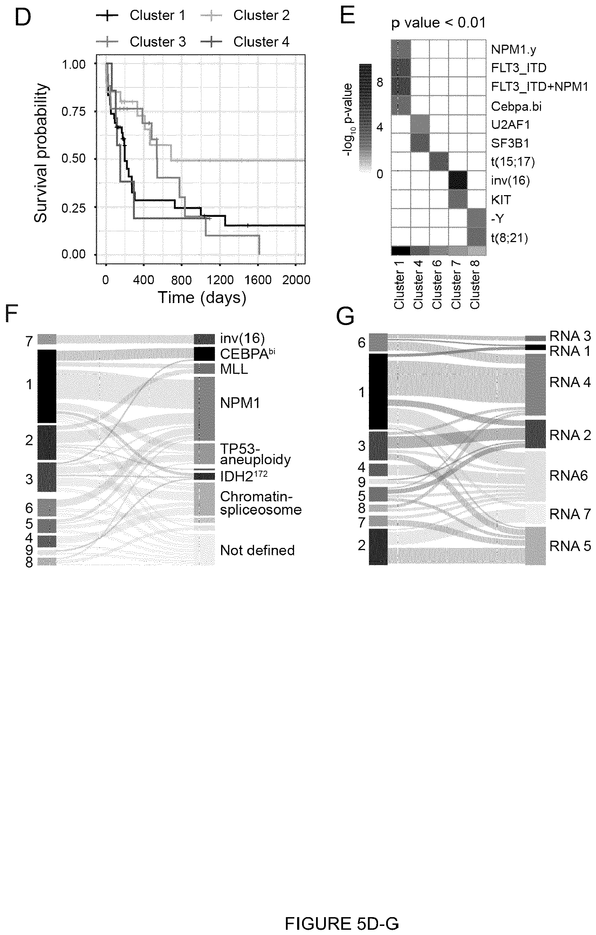

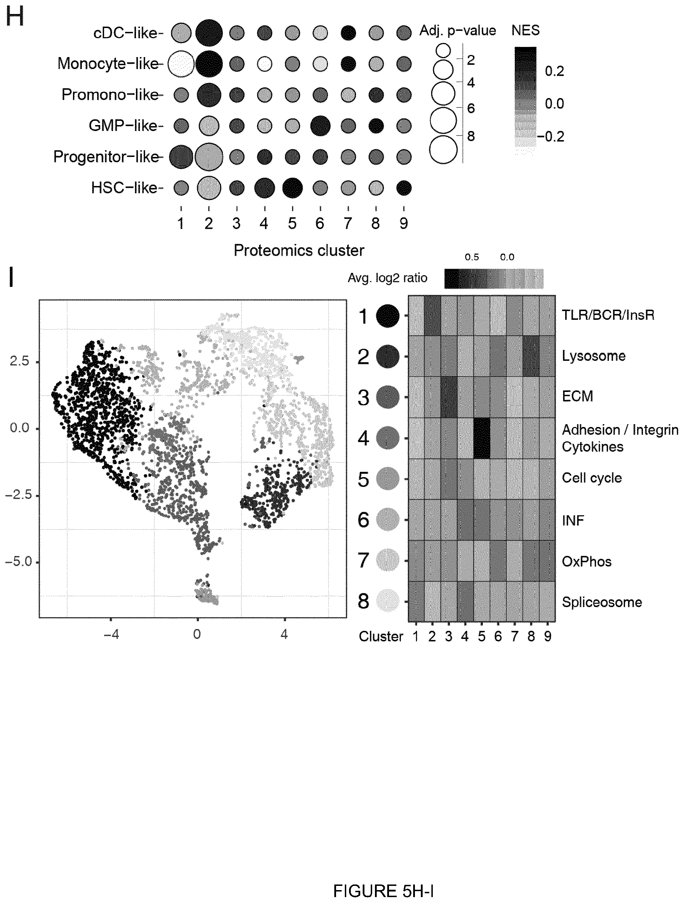

The experimental part shows a proteogenomic analysis of a treatment naïve Acute Myeloid Leukemia - study cohort selected from the larger Clinseq cohort and consisted of 118 Swedish, treatment naïve patients who all received first-line induction therapy for AML. Patients were characterized by mass-spectrometry based proteomics as well as RNA-sequencing, DNA-panel sequencing, Epic array and ex-vivo drug screening at baseline (Fig 5A). Longitudinal follow-up, survival outcome and clinical characteristics as well as treatment response was recorded for all patients (not shown). Mutational frequencies of commonly mutated genes were comparable to previous studies.

In-depth HiRIEF-LC-MS/MS was applied to the samples. The workflow identified and quantified more than 12000 protein products (gene centric, FDR<1%) across the entire cohort, with a full overlap of 8632 proteins (Fig 5B).

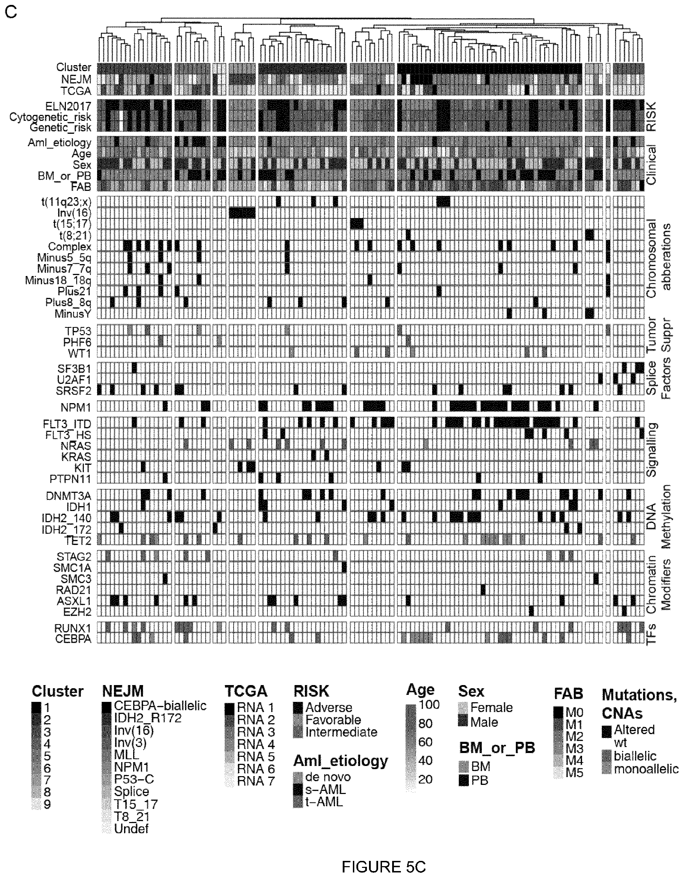

Using hierarchical clustering 9 clusters were identified in the cohort (Fig 5C). Survival outcomes differed between the distinct clusters (Fig 5D), with cluster 4 and 1 exhibiting poorer survival and patients in cluster 2 and 7 exhibiting longer survival. The experimental part also discloses observed treatment response, measured as whether patients achieved complete remission following induction therapy, and genetic risk classification (ELN2017) varied considerably between the clusters (not shown).

To investigate the relationship between mutational patterns and proteome phenotypes the panel sequencing data was utilized to investigate if there were enrichment in the clusters for specific genetic aberrations (Fig 5E).

Conversely, it was also investigated which mutations and genetic aberrations had the largest impact on protein levels and it was found that NPM1, FLT3^{ITD} led to the most prominent changes on the proteome level closely followed by transcription related changes due to biallelic CEBPA mutation and inv(16) (not shown). For 117 of the patients matching RNAseq and proteomics data was obtained and it was compared the correlation of the overlap (n=8971) on the gene symbol level. A median spearman correlation of 0.36 was found, which is within the range of what has been reported in previous studies of solid tumors, notably it is higher and a greater proportion of significant correlations was found (66%, adjusted P-value < 0.01) compared to what was found previously in acute lymphoblastic leukemia. Correlations of complex members was also significantly higher on the protein level compared to the mRNA level in line with previous reports.

It was also noted that the proteome level clusters also differed in terms of FAB-classification with stem-like or granulocytic differentiated subtypes (M0-M3) being more prevalent in cluster 1,4 and 6 while monocytic subtypes (M4-M5) were dominant in cluster 2 (not shown). In line with this, monocytic markers (CD14, and CD36) were elevated in samples from patients from cluster 2 and to a degree also in cluster 7 (inv(16)) (not shown). Patients in cluster 4 also exhibited increased levels of stemness markers CD34 and CD133. Additionally, it was also explored how signature gene sets for AML subtype differentiation derived from single cell RNA seq differed between the presently presented clusters (Fig 5H).

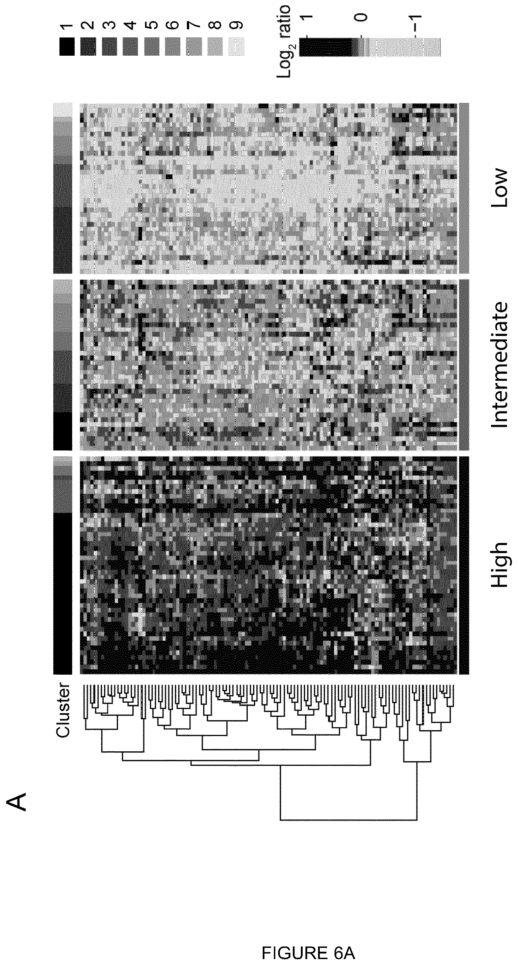

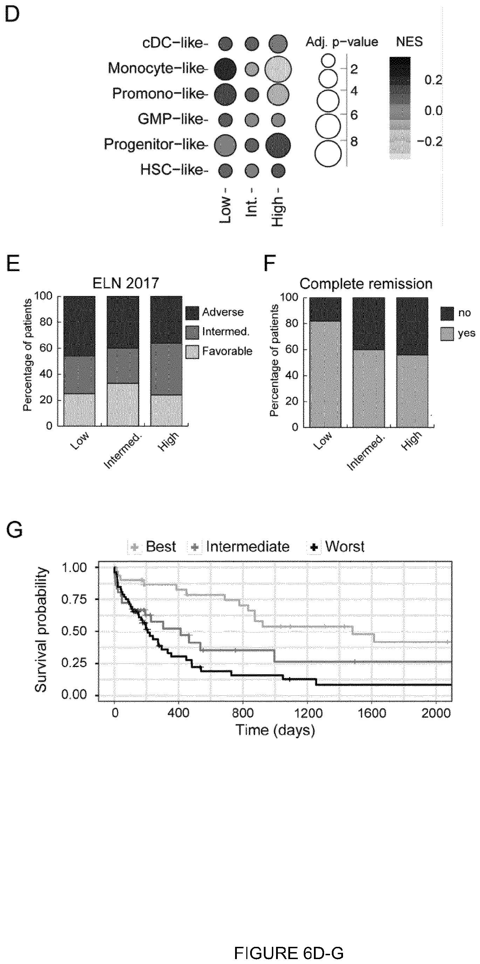

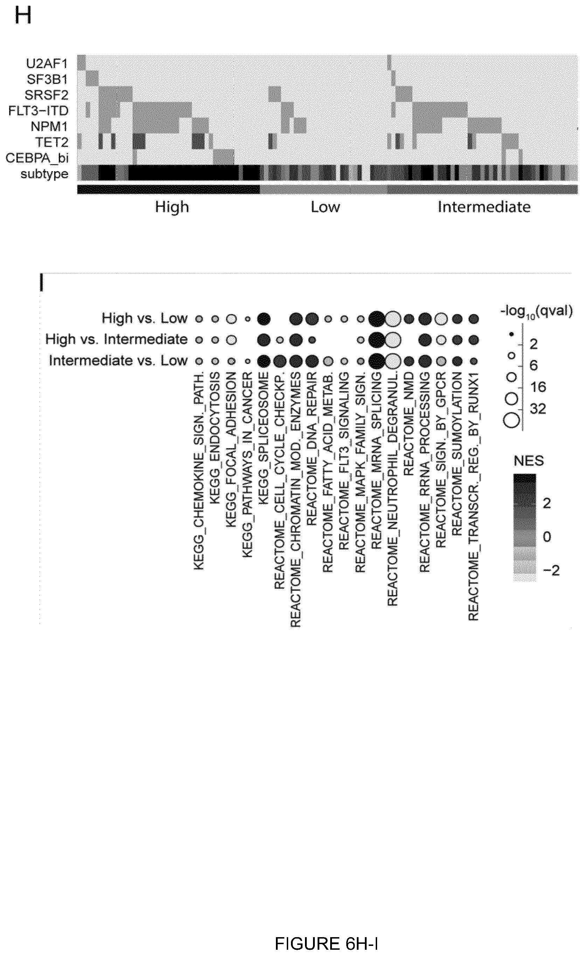

To more broadly characterize the proteomic phenotypes, the individual clusters were compared, and enrichment analysis was employed to investigate up and down regulated pathways (Fig 5l - enrichment). It was observed that monocyte related genes as well as genes associated to inflammation, TNF-signaling and IL10-signaling were upregulated in cluster 2. In clusters 3, 5 and 6 increased levels of integrin signalling, and integrin interaction related proteins was found. The inv(16) related cluster 7 exhibited decreased oxidative phosphorylation as well as increased chromosome maintenance. Clusters 1 and 4 exhibited increased levels of proteins related to transcription, mRNA processing and splicing. Specifically, spliceosome proteins were generally upregulated in these two clusters (Fig 6A).

*The present invention relates to the surprising finding that increased spliceosome levels define a*
*poor outcome population in AML patients.*

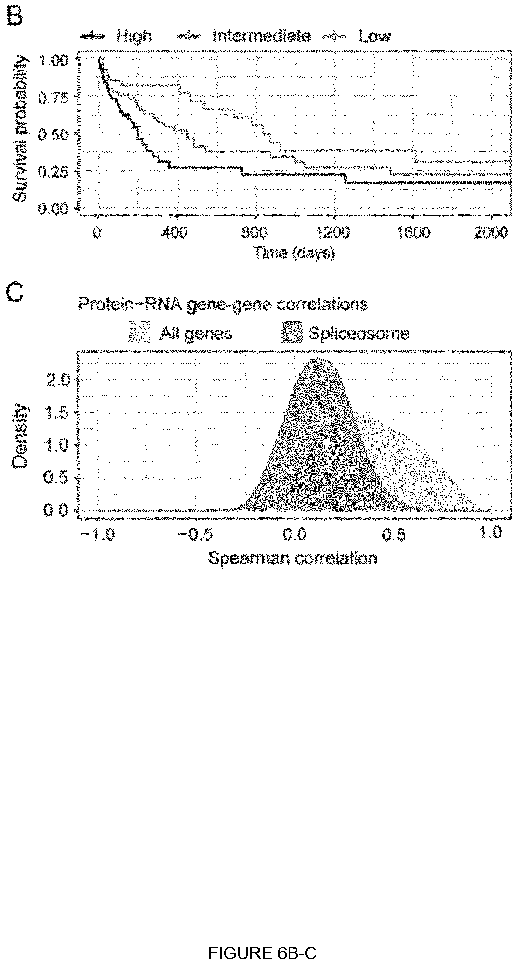

The patients were stratified into three groups based on clustering of spliceosomal proteins (Fig 6A) and investigated whether there is an association between outcome and spliceosome proteins. It was found that higher spliceosome levels were significantly associated with shorter overall survival (Fig 6B) and that lower levels were associated to a higher rate of complete remission (Chi-square High vs Low p-value = 0.02; Intermediate vs Low p-value = 0.05). Spliceosomal proteins were overall well correlated (not shown) while transcripts exhibited a lower level of correlation (Wilcoxon pval <1E-27). Additionally, the mRNA-protein correlations for the spliceosomal gene products were overall poor and the same phenotype could not be observed using the RNA sequencing data (fig 6C). No gender differences were observed between the groups, but patients with low levels of spliceosome proteins tended to be older.

There were no obvious differences in FAB classification between the three groups but using the scRNAseq derived gene sets described above it was observed that low spliceosome levels were more associated to a promono- or monocyte-like subtype and that the patients with high levels of spliceosome proteins also had increased levels of proteins associated to a progenitor-like state (Fig 6D).

As can be seen in the experimental part, there is no found association (X2-test, p = 0.62), between ELN2017 classification and spliceosome levels indicating that they are independent risk factors (Fig. 6E-F). Univariate cox-regression confirmed that both Adverse ELN-classification as well as high spliceosome levels were related to survival and both metrics retained their significant in a multivariable model indicating independent contributions (not shown). Indeed, combining the two metrics lead to improved stratification of the patient cohort in relation to overall survival (Fig 6G). To better elucidate the genetic contribution to the high spliceosome phenotype it was further investigated which mutations were found more frequently in the high and low spliceosome groups respectively (Fig 6H).

As can be seen in the experimental section, it was found that in addition to spliceosome and RNA processing genes being upregulated, genes involved in chromatin organization, sumoylation and DNA repair were also upregulated. Gene sets related to fatty acid metabolism, GPCR and chemokine signalling, hematopoietic lineage markers and cell adhesion/integrins/focal adhesion were comparatively downregulated (Fig 61).

### A method for stratifying

The surprising findings documented in the experimental section for the first time allow stratifying an AML patient by generating a proteomic profile of a patient and comparing said proteomic profile with the herein disclosed AML-specific expression profiles of the proteins selected from the list of proteins with SEQ ID NOs: 1-17500 (see sequence listing).

The stratification is based on a predicted outcome in relation to determinants such as but not limited to overall survival after 1 (one) year, event free survival, overall survival and/or reaching complete remission.

Thus, in one embodiment the AML patient stratification is done on the basis of predicted survival after 1 year. In another embodiment, the AML patient stratification is done on the basis of overall survival, event free survival and/or complete remission.

A method is herein disclosed for stratifying an AML patient into a high-risk or a low-risk group in relation to overall survival and/or complete remission, based on proteomic profiling, compared to an AML patient cohort of AML patients, wherein the method comprises,
a. isolating blood and/or tissue samples from said patient,
b. processing said sample(s), wherein the processing comprises
   i. extracting expressed proteins from said sample,
c. analyzing the extracted proteins,
d. determining a median expression level of said extracted proteins for the intermediate samples of the cohort, and
e. quantitatively determining the proteomic risk profile of said patient by identifying abnormal protein levels of at least 5 proteins selected from the list of proteins with SEQ ID NOs: 1-17500, wherein the protein level is considered abnormal when said protein level is downregulated or upregulated compared to the median expression level of the cohort, wherein
   i. downregulated refers to a protein level that is less than 95% of the median expression level of said protein for the intermediate samples of the cohort,
   ii. upregulated refers to a protein level that is at least 5% above the median expression level of said protein for the intermediate samples of the cohort,
   iii. proteins that are downregulated are proteins with a SEQ ID NO provided in Table 2, and
   iv. proteins that are upregulated are proteins with a SEQ ID NO provided in Table 3.

In one embodiment, said method is used for generating a proteomic profile of a patient suspected of suffering from high-risk Acute Myeloid Leukemia ("AML") or from low-risk Acute Myeloid Leukemia ("AML").

Thus, in one or more embodiments the AML patient is stratified as belonging to a high-risk group when abnormal protein levels are detected in step e) of the method.

### An AML patient

An AML patient is a patient, subject or individual diagnosed with AML.

As used herein, the term "patient" refers to an animal, preferably to a mammal, more preferably to a human. Depending on the embodiment in question, said patient may suffer from AML with or without diagnosis, be suspected to suffer from AML, be at risk of AML, or may have already been treated for AML. In one embodiment, the patient is suspected of suffering from high-risk Acute Myeloid Leukemia ("AML"). In another embodiment, the patient is suspected of suffering from low-risk Acute Myeloid Leukemia ("AML").

In the present context, the terms "human subject", "patient" and "individual" are interchangeable.

### A sample

A method for generating a proteomic profile of a patient suspected of suffering from high-risk Acute Myeloid Leukemia ("AML"), and/or for stratifying an AML patient into a high-risk or a low-risk group or into a high-risk, intermediate-risk or low-risk group in relation to overall survival and/or complete remission, based on proteomic profiling according to the present invention comprises isolating blood and/or tissue samples from said patient.

Thus, in one embodiment, the method is for generating a proteomic profile of a patient suspected of suffering from high-risk Acute Myeloid Leukemia ("AML") based on proteomic profiling according to the present invention comprises isolating blood and/or tissue samples from said patient.

In another embodiment, the method is for stratifying an AML patient into a high-risk or a low-risk group in relation to overall survival and/or complete remission, based on proteomic profiling according to the present invention comprises isolating blood and/or tissue samples from said patient.

In a further embodiment, the method is for stratifying an AML patient into a high-risk, intermediate-risk or low-risk group, in relation to overall survival and/or complete remission, based on proteomic profiling according to the present invention comprises isolating blood and/or tissue samples from said patient.

In yet another embodiment, the method is for stratifying an AML patient into one of at least three risk groups, such as but not limited to a high-risk group, an intermediate-risk group and a low-risk group, in relation to overall survival and/or complete remission, based on proteomic profiling according to the present invention comprises isolating blood and/or tissue samples from said patient.

As used herein, the term "sample" is used in its broadest sense. In one sense it can refer to a cell lysate. In another sense, it is meant to include a specimen or culture obtained from a patient. In particularly, the term "sample" refers to a biological sample, typically a clinical sample which may be, for example, a tissue sample such a skin sample, a cell sample such as a skin cell sample (e.g., a skin fibroblast sample), or a skin punch or skin shave, or a sample of a bodily fluid such as urine, blood, plasma, or serum, obtained from a patient. Preferred samples include biological samples encompassing fluids, solids, tissues, and gases. Presently, biological samples in particular include blood products (e.g., plasma and serum), saliva, urine, and the like. These examples are not to be construed as limiting the sample types applicable to the present disclosure.

In a presently preferred embodiment, bone marrow and/or peripheral blood samples are obtained/isolated at the time of diagnosis.

Generally, obtaining the sample to be analysed from a patient is not part of the present method, which may therefore be regarded as an *in vitro* method.

### AML patient cohort

A method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic profiling according to the present invention is based on proteomic profiling, compared to an AML patient cohort of AML patients.

The experimental part shows a proteogenomic analysis of a treatment naïve Acute Myeloid Leukemia - study cohort selected from the larger [Clinseq cohort Wang, M., et al., Validation of risk stratification models in acute myeloid leukemia using sequencing-based molecular profiling. Leukemia, 2017. 31(10): p. 2029-2036] and consisted of 118 Swedish, treatment naïve patients who all received first-line induction therapy for AML. In the present invention, the cohort is thus exemplified by 118 patients and the median expression level of said extracted proteins is determined for the intermediate samples of that cohort. Thus, the median expression level of the extractable proteins with SEQ ID NOs: 1-17500 is currently determined as shown in the experimental part.

The currently studied cohort is an exemplary cohort and based on predominantly Caucasian patients. It might be recommendable to use another cohort of treatment naïve Acute Myeloid Leukemia patients to determine the median expression level of the extractable proteins with SEQ ID NOs: 1-17500, such as defined by a certain age-group or genetic background. In certain aspects, it might be recommendable to use a cohort that is non-treatment naïve.

It is understood that the exact size of patient cohort can vary, as long as a median expression level of the extracted proteins can be determined and the quantitatively determined proteomic risk profile of said patient can be identifying as abnormal protein levels of at least 5 proteins selected from the list of proteins with SEQ ID NOs: 1-17500 (Table 1), wherein the protein level is considered abnormal when the level of any one of the proteins with a SEQ ID NO as provided in Table 3 are <95% of the median expression level for the intermediate samples of the cohort, or any one of the proteins with a SEQ ID NO as provided in Table 2 are up-regulated by at least >5% above the median expression level of the cohort.

### Processing said sample(s) and extracting expressed proteins

A method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic profiling according to the present invention comprises processing the patient's sample and extracting expressed proteins from said sample.

In one embodiment, at least one expressed protein selected from the list of proteins with SEQ ID NO: 1-1014, and/or at least one expressed protein selected from the list of proteins with SEQ ID NO: 1015-2519 are extracted from said sample. In one embodiment, at least five expressed proteins selected from the list of proteins with SEQ ID NO: 1-1014, and/or at least five expressed proteins selected from the list of proteins with SEQ ID NO: 1015-2519 are extracted from said sample.

The term "protein extraction" is in the current context used interchangeably with protein purification to describe a series of processes intended to isolate one or a few proteins from a complex mixture, such as cells or tissues. The purification process separates the protein and non-protein parts of the mixture. Separation steps usually exploit differences in protein size, physico-chemical properties, binding affinity and biological activity. The purified result may be termed protein isolate. The person skilled in the art will be able to choose from several standard laboratory proceedings to effect the protein extraction. Several preparative purifications steps are often deployed. The first step of each purification process is the disruption of the cells containing the protein. Non-limiting examples are for instance any one of the following methods: i) repeated freezing and thawing, ii) sonication, iii) homogenization by high pressure (French press), iv) homogenization by grinding (bead mill), and v) permeabilization by detergents (e.g., Triton X-100) and/or enzymes (e.g., lysozyme). Finally, the cell debris can be removed by centrifugation and/or filtration so that the proteins and other soluble compounds remain in the supernatant.

In some embodiments, the proteins are derived by proteolysis or chemical cleavage of a polypeptide. In an embodiment, a protease is utilized to cleave polypeptides into proteins. For example, the protease is trypsin. In additional embodiments, proteases or cleavage agents may be used including but not limited to trypsin, chymotrypsin, endoproteinase Lys-C, endoproteinase Asp-N, pepsin, thermolysin, papain, proteinase K, subtilisin, clostripain, exopeptidase, carboxypeptidase, cathepsin C, cyanogen bromide, formic acid, hydroxylamine, or NTCB, or a combination thereof. In some embodiments, the protease is trypsin.

In various embodiments, the proteins are derived by proteolysis or chemical cleavage of a protein. In an embodiment, a protease is utilized to cleave the protein into proteins or peptides. For example, the protease is trypsin. In additional embodiments, proteases or cleavage agents may be used including but not limited to trypsin, chymotrypsin, endoproteinase Lys-C, endoproteinase Asp-N, pepsin, thermolysin, papain, proteinase K, subtilisin, clostripain, exopeptidase, carboxypeptidase, cathepsin C, cyanogen bromide, formic acid, hydroxylamine, or NTCB, or a combination thereof.

Protein extraction and preparation is exemplified in the example section as dissolving cell pellets in a lysis buffer (4% SDS, 50 mM HEPES pH 7,6, 1 mM DTT) followed by heating to 95°C and sonication. The total protein amount was estimated (Bio-Rad DC). Thereafter samples were prepared for mass spectrometry analysis using a modified version of the SP3 protein clean-up and a digestion protocol [Moggridge, S., et al., Extending the Compatibility of the SP3 Paramagnetic Bead Processing Approach for Proteomics. J Proteome Res, 2018. 17(4): p. 1730-1740; Hughes, C.S., et al., Ultrasensitive proteome analysis using paramagnetic bead technology. Mol Syst Biol, 2014. 10: p. 757].

### Analyzing the extracted proteins and determining the expression levels of the extracted proteins

As used herein, the term "detection system capable of detecting proteins" refers to any detection apparatus, assay, or system that detects proteins derived from a protein separating apparatus (e.g., proteins in one or fractions collected from a separating apparatus). Such detection systems may detect properties of the protein itself (e.g., UV spectroscopy or mass spectrometry) or may detect labels (e.g., fluorescent labels) or other detectable signals associated with the protein. The detection system converts the detected criteria (e.g., absorbance, fluorescence, luminescence etc.) of the protein into a signal that can be processed or stored electronically or through similar means (e.g., detected through the use of a photomultiplier tube or similar system).

The extracted proteins are generally analysed by mass spectrometry. In a presently preferred embodiment, the mass spectrometry is data dependent acquisition (DDA) mass spectrometry and/or the mass spectrometry is data independent acquisition (DIA) mass spectrometry. In some embodiments, decreased or increased overall translation is determined quantitatively and/or qualitatively on the basis of signature molecules indicative of decreased or increased overall translation resulting from the use of alternative open reading frames or changes in post-translational modification, or signatures indicative of decreased or increased overall translation even though the precise mechanism of translation block or activation is not known. Suitable methods for measuring signatures include mass spectrometry, such as but not limited to selected from reaction monitoring (SRM) assays, multiple reaction monitoring (MRM) or parallel reaction monitoring (PRM) assays of translation products, as well as DDA shotgun and/or DIA approaches that provide diagnostic or predictive information. Additionally, antibody-based methods such as ELISA, proximity ligation assay, proximity extension assay, reverse phase protein arrays, aptamer-based assays, affibody based assays, and 2D-PAGE, for detection of decreased or increased overall translation can be used to measure of a single protein or group of proteins designating a "signature" that results from decreased or increased overall translation and is characteristic of or predictive. i.e., abnormal.

To determine whether a level of translation form a selected protein or group of proteins is abnormal, the median expression level of said extracted protein(s) for the intermediate samples of the cohort has to be determined. Once the median expression level is known, the determined translation levels can be compared therewith, and the significance of the difference can be assessed using standard statistical methods. Before to be compared with the median expression level, the translation levels are normalized using standard methods.

The detection methods particularly envisioned include detection by mass spectrometry, ELISA type antibody recognition or by measuring the enzymatic activity of unique translation products.

### Mass spectrometry

In various embodiments, the MS data is collected by a targeted acquisition method. Examples of the targeted acquisition method include but are not limited to Selective Reaction Monitoring (SRM) and/or Multiple Reaction Monitoring (MRM) methods and PRM methods. In various embodiments, acquiring MS data comprises acquiring Selective Reaction Monitoring (SRM) data and/or Multiple Reaction Monitoring (MRM) data.

In various embodiments, the MS data is collected by a data independent acquisition (DIA) method. In various embodiments, the MS data is collected by data dependent acquisition (DDA) method. Non-limiting examples of mass spectrometry techniques include collision-induced dissociation (CID), higher-energy collisional dissociation (HCD), electron-transfer dissociation (ETD), etc.

### Data Dependent Acquisition (DDA)

In various embodiments, the MS data is collected by a data dependent acquisition method. Thus, in some embodiments, the mass spectrometry is data dependent acquisition (DDA) mass spectrometry. In some embodiments, the one or more proteins are correlated to the one or more proteins with a SEQ ID NO as provided in the sequence listing. In some embodiments, the one or more proteins are correlated to the one or more proteins according with a sequence of SEQ ID NOs: 1-17500, wherein the protein level is considered abnormal when said protein level is downregulated or upregulated compared to the median expression level of the cohort. In embodiments downregulated refers to a protein level that is less than 95% of the median expression level of said protein for the intermediate samples of the cohort. In embodiments upregulated refers to a protein level that is at least 5% above the median expression level of said protein for the intermediate samples of the cohort. In that regards proteins that are downregulated are proteins with a SEQ ID NO provided in Table 2, and proteins that are upregulated are proteins with a SEQ ID NO provided in Table 3.

### Data Independent Acquisition (DIA)

In various embodiments, the MS data is collected by a data independent acquisition method. Thus, in some embodiments, the mass spectrometry is data independent acquisition (DIA) mass spectrometry. In some embodiments, the one or more proteins are correlated to the one or more proteins with a SEQ ID NO as provided in Table 4-5. In some embodiments, the one or more proteins are correlated to the one or more proteins according to one or more of SEQ ID NOs: 1-17500, wherein the protein level is considered abnormal when said protein level is of any one or more proteins is/are downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

Examples of the independent acquisition (DIA) method include but are not limited to Shotgun CID (see. e.g., Purvine et al. 2003), Original DIA (see e.g., Venable et al. 2004), MSE (see e.g., Silva et al. 2005), p2CID (see e.g., Ramos et al. 2006), PAcIFIC (see e.g., Panchaud et al. 2009), AIF (see e.g., Geiger et al. 2010), XDLA (see e.g., Carvalho et al. 2010), SWATH (see e.g., Gillet et al. 2012), and FT-ARM (see e.g., Weisbrod et al. 2012).

### Triple quadrupole mass spectrometer

In some embodiments, the mass spectrometer is a triple quadrupole mass spectrometer. In some embodiments the mass spectrometer is a Triple-Time Of Flight (Triple- TOF) mass spectrometer configured for SWATH or a Q-Exactive mass spectrometer (Thermo Scientific), or any instrument with sufficiently high scan speed and a quadrupole mass filter to perform data independent acquisition. Examples of triple quadrupole mass spectrometers (TQMS) that can perform MRM/SRM/SIM include but are not limited to: QTRAP(R) 6500 and 5500 System (Sciex); Triple QTriple Quad 6500 System (Sciex); Agilent 6400 Series Triple Quadrupole LC/MS systems; Thermo Scientific^{™} TSQ^{™} Triple Quadrupole system; quadrupole time-of-flight (QTOF) mass spectrometers, or hybrid quadrupole-orbitrap (QOrbitrap) mass spectrometers. Examples of quadrupole time-of-flight (QTOF) mass spectrometers include but are not limited to: TripleTOF(R) 6600 or 5600 System (Sciex); X500R QTOF System (Sciex); 6500 Series Accurate-Mass Quadrupole Time-of-Flight (Q-TOF) (Agilent); or Xevo G2-XS QTof Quadrupole Time-of-Flight Mass Spectrometry (Waters). Examples of hybrid quadrupole- orbitrap (QOrbitrap) mass spectrometers include but are not limited to: Q Exactive^{™} Hybrid Quadrupole-Orbitrap Mass Spectrometer (Thermo Scientific); or Orbitrap Fusion^{™} Tribrid^{™} (Thermo Scientific).

### Tandem mass spectrometry (MS/MS)

In some embodiments, the mass spectrometry technique is tandem mass spectrometry (MS/MS). In some embodiments, the mass spectrometry technique is liquid chromatography-tandem mass spectrometry (LC-MS/MS). In some embodiments, the mass spectrometry technique is liquid chromatography-selected reaction monitoring-mass spectrometry (LC-SRM-MS). In some embodiments, the mass spectrometry technique is liquid chromatography-multiple reaction monitoring-mass spectrometry (LC-MRM-MS). In some embodiments, the mass spectrometery technique is selected reaction monitoring (SRM). In some embodiments, the mass spectrometry technique is multiple reaction monitoring (MRM). In some embodiments, the mass spectrometry technique is parallel reaction monitoring (PRM). In some embodiments, the mass spectrometry technique is data-independent analysis (DIA). In some embodiments, the mass spectrometry technique is data-dependent analysis (DDA).

In various other embodiments, the method further comprises adding a stable isotope-labelled peptide or protein to the sample prior to mass spectrometry. In some embodiments, the absolute amount of a protein in the sample is determined by comparing the MS signals of natural and stable isotope-labelled peptides and/or proteins.

In various other embodiments, transitions for each protein with high and reproducible peak intensities are identified. In other embodiments, the collision energy for each transition is optimized. In other embodiments, mass spectrometry comprises selected reaction monitoring (SRM), or multiple reaction monitoring (MRM). In other embodiments, SRM or MRM is performed on a triple quadrapole mass spectrometer. In other embodiments, the proteins of interest are those with high correlations, strong signals, high signal/noise and/or sequences unique to the protein of interest.

### SRM/MRM/SIM

Selected-ion monitoring (SIM) or selected reaction monitoring (SRM) or multiple reaction monitoring (MRM) provide the simplest method set up and the most selective and sensitive quantification. SRM/MRM/SIM is a method used in tandem mass spectrometry in which an ion of a particular mass is selected in the first stage of a tandem mass spectrometer and an ion product of a fragmentation reaction of the precursor ion is selected in the second mass spectrometer stage for detection. Examples of triple quadrupole mass spectrometers (TQMS) that can perform MRM/SRM/SIM include but are not limited to: QTRAP(R) 6500 and 5500 System (Sciex); Triple QTriple Quad 6500 System (Sciex); Agilent 6400 Series Triple Quadrupole LC/MS systems; or Thermo Scientific^{™} TSQ^{™} Triple Quadrupole system.

### Parallel- Reaction Monitoring (PRM)

In addition to MRM, the protein(s) can also be quantified through Parallel- Reaction Monitoring (PRM). Parallel reaction monitoring (PRM) is the application of SRM with parallel detection of all transitions in a single analysis using a high-resolution mass spectrometer. PRM provides high selectivity, high sensitivity and high throughput to quantify selected peptide (Ql), hence quantifying proteins. Again, multiple peptides or proteins can be specifically selected for each protein. PRM methodology uses the quadrupole of a mass spectrometer to isolate a target precursor ion, fragments the targeted precursor ion in the collision cell, and then detects the resulting product ions in the Orbitrap mass analyser. Quantification is carried out after data acquisition by extracting one or more fragment ions with 5-10 ppm mass windows. PRM uses a quadrupole time-of-flight (QTOF) or hybrid quadrupole-orbitrap (QOrbitrap) mass spectrometer to carry out the peptide/protein quantitation. Examples of QTOF include but are not limited to: TripleTOF(R) 6600 or 5600 System (Sciex); X500R QTOF System (Sciex); 6500 Series Accurate-Mass Quadrupole Time-of-Flight (Q-TOF) (Agilent); or Xevo G2-XS QTof Quadrupole Time-of-Flight Mass Spectrometry (Waters). Examples of QOrbitrap include but are not limited to: Q Exactive^{™} Hybrid

Quadrupole-Orbitrap Mass Spectrometer (Thermo Scientific); or Orbitrap Fusion^{™} Tribrid^{™} (Thermo Scientific).

Non-limiting advantages of PRM include elimination of most interferences, provides more accuracy and attomole-level limits of detection and quantification, enables the confident confirmation of the peptide identity with spectral library matching, reduces assay development time since no target transitions need to be preselected, ensures UHPLC- compatible data acquisition speeds with spectrum multiplexing and advanced signal processing.

In various embodiments, stable isotope-labelled peptide or protein standards for absolute quantification are used. In other embodiments, the peptide or protein labelled with a stable isotope is used as an internal standard to obtain absolute quantification of the protein of interest. In other embodiments, the proteins are quantified and then the amount of the parent protein present is inferred before digesting the sample with trypsin. In other embodiments, MS responses are used to determine an upper limit of quantification (ULOQ) and a lower limit of quantification (LLOQ).

In various embodiments, the MS data comprises raw MS data obtained from a mass spectrometer and/or processed MS data in which proteins and their fragments (e.g., transitions and MS peaks) are already identified, analysed and/or quantified. In various embodiments, the MS data is Selective Reaction Monitoring (SRM) data or Parallel-Reaction Monitoring (PRM) data and/or Multiple Reaction Monitoring (MRM) data. In various embodiments, the MS data is Shotgun CID MS data, Original DIA MS Data, MSE MS data, p2CID MS Data, PAcIFIC MS Data, AIF MS Data, XDLA MS Data, SWATH MS data, or FT-ARM MS Data, or a combination thereof.

In various embodiments, acquiring MS data comprises operating a TripleTOF mass spectrometer, a triple quadrupole mass spectrometer, a liquid chromatography-mass spectrometry (LC-MS) system, a gas chromatography-mass spectrometry (GC-MS) system, or a tandem mass spectrometry (MS/MS) system, a dual time-of-flight (TOF-TOF) mass spectrometer, or a combination thereof.

In various embodiments, acquiring MS data comprises operating a mass spectrometer. Examples of the mass spectrometer include but are not limited to high-resolution instruments such as TripleTOF, Orbitrap, Fourier transform, and tandem time-of-flight (TOF/TOF) mass spectrometers; and high-sensitivity instruments such as triple quadrupole, ion trap, quadrupole TOF (QTOF), and Q trap mass spectrometers; and their hybrid and/or combination. High-resolution instruments are used to maximize the detection of proteins with minute mass-to-charge ratio (m/z) differences.

Conversely, because targeted proteomics emphasize sensitivity and throughput, high-sensitivity instruments are used. In some embodiments, the mass spectrometer is a TripleTOF mass spectrometer. In some embodiments, the mass spectrometer is a triple quadrupole mass spectrometer.

In some embodiments, acquiring MS data does not require operating a mass spectrometer. For examples, MS data can be acquired from MS experiments run previously and/or MS databases. In some embodiments, previously acquired SWATH MS data can be queried with a more comprehensive library to identify additional MS peaks derived from different and macromolecules.

Detection of a protein in a test sample involves routine methods. The skilled artisan can detect the presence or absence of a protein using well known methods. Such methods include diverse immunoassays. In general, immunoassays involve the binding of antibodies or similar probes to proteins in a sample such a histological section or binding of proteins in a sample to a solid phase support such as a plastic surface. Detectable antibodies are then added which selectively bind to the protein of interest. Detection of the antibody indicates the presence of the protein. The detectable antibody may be a labelled or an unlabelled antibody. Unlabelled antibody may be detected using a second, labelled antibody that specifically binds to the first antibody or a second, unlabelled antibody which can be detected using labelled protein A, a protein that complexes with antibodies. Various immunoassay procedures are described in Immunoassays for the 80's, A. Voller et al., Eds., University Park, 1981.

Detection of protein expression level is exemplified in the example section as determining the labelling efficiency by LC-MS/MS before pooling of the samples. For the sample clean-up step, a solid phase extraction (SPE strata-X-C, Phenomenex) was performed, and purified samples were dried in a SpeedVac. An aliquot of approximately 10 µg was suspended in LC mobile phase A and 1 µg was injected on the LC-MS/MS system.

Protein quantification by TMT10plex reporter ions was calculated using TMT PSM ratios to the entire sample set (all 10 TMT-channels) and normalized to the sample median. The median PSM TMT reporter ratio from peptides unique to a gene symbol was used for quantification. Protein false discovery rates were calculated using the picked-FDR method using gene symbols as protein groups and limited to 1% FDR.

### Quantitatively determining the proteomic risk profile by identifying abnormal protein levels

A method for generating a proteomic profile of a patient suspected of suffering from high-risk Acute Myeloid Leukemia ("AML"), and/or for stratifying an AML patient into a high-risk or a low-risk group or into a high risk, an intermediate risk or a low risk group in relation to overall survival, event-free survival and/or complete remission, based on proteomic profiling according to the present invention comprises quantitatively determining the proteomic risk profile of said patient by identifying abnormal protein expression levels of at least 5 proteins selected from the list of proteins with SEQ ID NOs: 1-17500, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3. Patients with abnormal protein expression levels are determined to belong in the high-risk group, i.e. suffering from high-risk Acute Myeloid Leukemia ("AML").

In one embodiment, the method comprises identifying abnormal protein levels of the extracted proteins, wherein an abnormal protein level of the at least 5 proteins selected from the list of proteins with a SEQ ID NO as provided in Table 3 is defined as < 95% of the median expression level for those proteins from a cohort of AML patients; and wherein an abnormal protein level of the at least one protein selected from the list of proteins with a SEQ ID NO as provided in Table 2 is defined as >5% above the median expression level for those proteins of the cohort. Patients with abnormal protein expression levels are determined to belong in the high-risk group, i.e., suffering from high-risk Acute Myeloid Leukemia ("AML").

Protein levels are generally tightly regulated by their expression, modification and degradation, all of which are in turn regulated directly or indirectly by other regulators of the specific pathway. In that sense, proteins that are maladapted in their expression level, i.e., displaying an abnormal expression level, may have a consequence for other proteins and *visa versa.* Thus, an abnormal expression level of a specific protein may be either up- or down regulated, relative to the normal protein level of that protein. In that regard, identifying abnormal protein expression levels, requires a knowledge about the normal level of the specific protein in question.

In one embodiment, identification of an abnormal protein level relates to a level of a protein which is at least 5 % upregulated or at least 5 % downregulated, in relation to the average or median protein level, of the protein in question, across the cohort.

Thus, in a furhter embodiment, the protein level is considered abnormal when the level of said protein is at least 105 % or below 95 % in relation to the average or median protein level, of the protein in question, across the cohort, such as a protein level of at least 106 %, 110 %, 150 %, 200 %, 300 %, 400 %, 500 % or 1000 %, or such as a protein level below 94 %, 90 %, 80 %, 50 %, 25 %, 10 %, 5 % or 1 %.

The median expresion level of a protein across the cohort, in the present disclosure, relates to the median expression level in the cohort. In the experimental section, the cohort is exemplified by 118 patients and the median expression level of said extracted proteins is determined for the intermediate samples of that cohort. Thus, the median expression level of the extractable proteins with SEQ ID NOs: 1-17500 is currently determined as shown in the experimental part.

In the current context the term "protein level" is used interchangably with the term "protein expression level".

### Proteomic profiling

A set of 17500 protein markers is provided herein whose expression level may be correlated to the prognosis of acute myeloid leukemia using cluster analysis. A series of subsets of these markers are identified as useful for disease prognosis are listed in Tables 4, 5, 6, 7, 8, 9, 10 and 11. The markers with SEQ ID NOs provided in Tables 6, 7 and 8 were found suitable for retrospective diagnosis and markers with SEQ ID NOs provided in Tables 9, 10 and 11 were found suitable for prospective diagnosis. Thus, the invention also relates to a method for clustering these markers into sub-groups, which may in turn be used to cluster patients making it possible to stratify patients based on the predicted disease prognosis.

Proteomic profiling relates to obtaining a profile describing the level of individual proteins in a sample obtained from a patient. In relation to the proteomic profiling a protein sample may be obtained from a tissue, blood, salvia, mucus and/or bone marrow sample, as described herein. In one embodiment, a proteomic profile relates to the individual protein level of every protein obtained in a sample. In another embodiment, a proteomic profile relates to a selected subset of proteins obtained from a sample.

The present disclosure describes a set of 17500 protein markers that can be used to distinguish between patients with a good AML prognosis ([treatment regiment, event free survival, overall survival or reaching complete remission]) and patients with a poor AML prognosis (disease relapse, non-responders to treatment etc.). These markers are listed with their gene symbol in table 1 and with their amino acid sequence in the sequence listing. Subsets are provided of at least 5, 10, 20, 30, 40, 50, 75, 100, 150, 200, 500, 1000, 2000 or 10000 markers, drawn from the set of 17500, which also distinguish between patients with good and poor prognosis.

The 17500 protein markers of the present invention, relates to a total of 2519 genes/coding regions, thus some genes are translated into more than one protein marker. As a consequence, some of the 17500 protein markers listed in Table 1 are encompassed in the sequence or sequences of other protein markers, which relates to the same gene.

In that regard, in one embodiment of the present disclosure a proteomic profile relates to the level of the 17500 protein markers, described in the present disclosure.

In another embodiment, a proteomic profile relates to a selected subset of proteins obtained from a sample, wherein the subset is selected amongst the 17500 proteins markers disclosed herein.

In a further embodiment, the subset is selected amongst the 17500 proteins markers disclosed herein, wherein the subset comprises at least 5, 10, 20, 30, 40, 50, 75, 100, 150, 200, 500, 1000 or 2000 of the protein markers.

### Signatures defined by mass spectrometry

Several methods are described based on application and exploitation of specific AML signatures and the proteins therein. Methods for determining these "signatures" from patient samples and thus providing evidence as to whether those patients are "high-risk," "intermediate-risk" or "low-risk". Such methods are based on the signatures defined by mass spectrometry (examples of which are listed herein) and can be determined using specific sets of antibodies or similar specific probe reagents to define which proteins of the signatures are present in the samples. Methods include immunohistochemistry, ELISA, protein arrays and similar methods known to the skilled person. Use of such methods provides diagnostic, prognostic and monitoring information allowing improved management of AML cancer patients' care and therapy.

### The list of proteins

All proteins with SEQ ID NOs: 1-17500 are listed in the sequence listing, wherein they are additionally provided with their respective Gene Symbols. The full protein sequences for each of the identified proteins are known in the art. Detailed information on each of these proteins including genomic information and listing of sources for nucleotide and protein sequences for each of these proteins is provided in the sequence listing and for specific proteins in the below tables in the form of list of Gene Symbols, for each gene symbol there are the associated ENSGs (Ensembl Gene IDs) and the associated ENSPs (Ensembl protein IDs) and a description. In some cases it is a 1:1 ratio i.e.: GENESYMBOL_A : ENSG_A : ENSP_A. For others, it is not a 1:1 relationship as several entries in the database (ENSGs/ENSPs) map to the same Gene Symbol, GENESYMBOL_B : ENSG_B1, ENSG_B2, ... : ENSP_B1, ENSP_B2.1, ENSP_2.2. All gene symbols and their correlating amino acid sequences are given in the sequence listing provided with the application. The full sequences can be identified by accessing any of these accession numbers in the https://www.ensembl.org search query. As these proteins are well known to the skilled artisan, the signatures of proteins and binding reagents described herein include variations and modifications to the sequences as well.

ENSEMBL references and gene names are provided for each SEQ ID NO in the sequence listing.

In total the present disclosure relates to a set of 2519 individual marker proteins, wherein each marker protein is also described by its Gene Symbol as provided in Table 1. The Gene Symbol in some cases covers a number of different sequence variants, thus the 2519 unique marker proteins of Table 1 results in a total of 17500 individual protein sequences identified as SEQ ID NOs: 1-17500, which are identified in the sequence listing provided with the application. Subsets of the marker proteins were generated, and these may be used for the stratification of AML patients into groups.

Upregulated protein markers are provided with SEQ ID NOs in Table 2 and downregulated protein markers are provided with SEQ ID NOs in Table 3.

Preferred sets of markers are provided in Table 4-5.

**Table 1 - Gene Symbols of selected 2519 markers**

| **Gene Symbol** | **Gene Symbol** | **Gene Symbol** | **Gene Symbol** | **Gene Symbol** | **Gene Symbol** | **Gene Symbol** | **Gene Symbol** | **Gene Symbol** |
|---|---|---|---|---|---|---|---|---|
| A2M | EDEM3 | MYO1C | SNX17 | BUD13 | FBXL12 | MEPCE | PRR12 | SYMPK |
| ABCB6 | EEF1D | MYO1E | SNX18 | BUD23 | FBXL20 | METTL14 | PSIP1 | SYNCRIP |
| ABCD1 | EEF1G | MYO5A | SNX27 | BUD31 | FBXO38 | METTL16 | PSMD5 | SZT2 |
| ABCD3 | EFCAB14 | MYOF | SNX3 | BYSL | FCF1 | METTL3 | PSPC1 | TADA1 |
| ABHD6 | EFHD2 | MZT1 | SNX30 | C11orf54 | FIP1L 1 | METTL7A | PTBP1 | TADA2B |
| ABR | EHBP1L 1 | NADK | SNX4 | C11orf58 | FIZ1 | MFAP1 | PTBP2 | TADA3 |
| ACAP2 | EHD4 | NAGA | SNX8 | C11orf68 | FKBP3 | MGA | PTEN | TAF1 |
| ACOT11 | EIF2S1 | NAGK | SORT1 | C11orf98 | FLI1 | MIER1 | PTGES3 | TAF10 |
| ACOT7 | EIF3C | NAGPA | SOWAHD | C12orf43 | FLYWCH 2 | MIOS | PUF60 | TAF15 |
| ACOT8 | EIF3L | NAIP | SPAG5 | C12orf45 | FMR1 | MIS12 | PUM2 | TAF1C |
| ACOX1 | EIF4EBP1 | NAPA | SPC24 | C12orf57 | FNBP1 | MLF2 | PUM3 | TAF1D |
| ACOX3 | EIF4EBP2 | NAPG | SPC25 | C17orf49 | FNBP4 | MLLT1 | PWP1 | TAF2 |
| ACPP | EIF4G1 | NBEAL1 | SPCS2 | C17orf80 | FOXJ2 | MLXIP | PWWP2A | TAF3 |
| ACSL1 | EIPR1 | NBEAL2 | SPCS3 | C18orf21 | FOXJ3 | MNAT1 | PWWP2B | TAF4 |
| ACSL3 | ELMO2 | NCAPD2 | SPPL2A | C19orf47 | FOXK2 | MNT | QRICH1 | TAF5 |
| ACTN1 | ELOB | NCAPG | SPR | C1orf131 | FOXP1 | MOCS3 | QTRT1 | TAF5L |
| ACTR1A | ELP3 | NCAPG2 | SPRYD7 | C1orf174 | FPGS | MORC2 | QTRT2 | TAF6 |
| ACTR2 | EMB | NCAPH | SPTA1 | C1orf35 | FRA10AC 1 | MORC3 | RABL3 | TAF6L |
| ACTR3 | EMILIN1 | NCDN | SPTB | C2orf49 | FRG1 | MORF4L1 | RAD17 | TAF7 |
| ADA2 | EMILIN2 | NCEH1 | SQOR | C5orf22 | FTO | MORF4L2 | RAD21 | TAF8 |
| ADAM10 | EML2 | NCF1 | SRGAP2 | C5orf24 | FTSJ3 | MPG | RAD50 | TALDO1 |
| ADAM15 | EML4 | NCF2 | SRGN | C7orf26 | FUBP1 | MPHOSP H10 | RAD54L2 | TATDN1 |
| ADAP1 | ENTPD1 | NCF4 | SSR1 | C7orf50 | FUBP3 | MPHOSP H6 | RAD9A | TBL1XR1 |
| ADD2 | EPB41L3 | NCKIPSD | SSR4 | C9orf78 | FUNDC1 | MPHOSP H8 | RAE1 | TBL3 |
| AGPAT3 | EPHX1 | NCLN | STARD3 | CA5B | FUS | MPI | RAl1 | TCEA1 |
| AGTPBP1 | ERBIN | NCSTN | STARD5 | CAAP1 | FXR1 | MPND | RALY | TCEA2 |
| AGTRAP | ERCC6L | NDC80 | STBD1 | CABIN1 | FXR2 | MRE11 | RANBP2 | TCEAL1 |
| AIF1 | ERGIC2 | NDST1 | STEAP3 | CACTIN | FYTTD1 | MRGBP | RANBP3 | TCEAL4 |
| AK1 | ERGIC3 | NECAP1 | STIM2 | CAMLG | G3BP1 | MRTO4 | RASSF1 | TCEANC2 |
| AK6 | ERO1A | NECAP2 | STK24 | CAND1 | GABPA | MSH2 | RAVER1 | TCERG1 |
| AKAP10 | ERP29 | NEDD9 | STK25 | CARMIL2 | GABPB1 | MSH3 | RAVER2 | TCF20 |
| AKAP13 | ERP44 | NHLRC2 | STOM | CASP2 | GAR1 | MSH6 | RBBP4 | TCF3 |
| AKT2 | ESAM | NLRC4 | STS | CASP3 | GATAD1 | MSI2 | RBBP5 | TCOF1 |
| ALAD | EVI2B | NLRP3 | STX10 | CASP6 | GATAD2A | MSL1 | RBBP6 | TDG |
| ALB | EVI5 | NOCT | STX11 | CASP7 | GATAD2B | MSL2 | RBBP9 | TDP2 |
| ALDH16A 1 | EVI5L | NPTN | STX12 | CBFA2T2 | GCSH | MSL3 | RBL2 | TEC |
| ALDH3B1 | F5 | NRBF2 | STX2 | CBFA2T3 | GDPD1 | MTA1 | RBM10 | TERF1 |
| ALG1 | FABP4 | NSF | STX6 | CBFB | GEMIN2 | MTA2 | RBM12 | TERF2 |
| ALOX5 | FAM107B | NTAN1 | STX8 | CBLL1 | GLE1 | MTF2 | RBM12B | TERF2IP |
| ALOX5AP | FAM114A 1 | NUDT4 | STXBP2 | CBX2 | GLOD4 | MTIF3 | RBM14 | TEX10 |
| AMFR | FAM151B | NUF2 | SULT1B1 | CBX3 | GLYR1 | MTOR | RBM15 | TFAP4 |
| ANK1 | FAM160A 2 | OAT | SUMF1 | CBX4 | GMEB1 | MTREX | RBM15B | TFIP11 |
| ANKFY1 | FAM177A 1 | OCRL | SYK | CBX5 | GMEB2 | MVB12A | RBM17 | THAP11 |
| ANKIB1 | FAM49B | OGFRL1 | SYNGR2 | CBX8 | GNA15 | MYBBP1 A | RBM19 | THAP12 |
| ANO10 | FAM98C | OPLAH | SYNJ1 | CCAR1 | GNL2 | MYL6B | RBM22 | THOC1 |
| ANO6 | FANCB | ORM1 | TACC1 | CCAR2 | GNL3 | MYO18A | RBM25 | THOC2 |
| ANXA2 | FANCD2 | OSBPL1A | TANGO6 | CCDC102 A | GON7 | MYSM1 | RBM26 | THOC3 |
| ANXA3 | FANCI | OTULIN | TAOK1 | CCDC117 | GPALPP1 | N4BP2 | RBM27 | THOC5 |
| ANXA5 | FAR1 | OXR1 | TBC1D1 | CCDC12 | GPATCH 1 | N4BP2L2 | RBM28 | THOC6 |
| ANXA6 | FAS | P4HB | TBCD | CCDC130 | GPATCH 11 | NAB2 | RBM3 | THOC7 |
| AOAH | FASN | PADI2 | TBK1 | CCDC137 | GPATCH 4 | NACC1 | RBM33 | THOP1 |
| AP1M1 | FBP1 | PADI4 | TCIRG1 | CCDC174 | GPATCH 8 | NAE1 | RBM34 | THRAP3 |
| AP2A1 | FCAR | PANK4 | TELO2 | CCDC59 | GPKOW | NAT10 | RBM39 | THUMPD 1 |
| AP2M1 | FCER1G | PAPSS2 | TESC | CCDC77 | GPS2 | NAXE | RBM42 | THYN1 |
| AP2S1 | FCGR1A | PARVB | TEX2 | CCDC82 | GPSM1 | NBN | RBM45 | TIA1 |
| AP3B1 | FCGR2A | PATL1 | TFEB | CCDC86 | GRAMD4 | NCBP1 | RBM4B | TIAL1 |
| AP3D1 | FCGRT | PBK | TGFBI | CCDC9 | GRAP | NCBP2 | RBM5 | TIMM13 |
| AP4B1 | FCN1 | PC | TGM2 | CCDC97 | GRHPR | NCBP3 | RBM6 | TIMM17B |
| AP4E1 | FERMT3 | PDE4D | THEMIS2 | CCNH | GSE1 | NCL | RBM8A | TIMM8A |
| APAF1 | FGD2 | PDIA3 | TIMP1 | CCNL1 | GSPT2 | NCOA2 | RBMX | TIMM8B |
| APH1A | FGD4 | PDIA4 | TIMP2 | CCNL2 | GTF2B | NCOA3 | RBMX2 | TIPRL |
| APLP2 | FGL2 | PDIA5 | TLN1 | CCNT2 | GTF2E1 | NCOA5 | RBMXL1 | TLE3 |
| APOA1 | FGR | PDIA6 | TLN2 | CD2BP2 | GTF2E2 | NCOR1 | RCC1 | TLK1 |
| APOB | FHOD1 | PDLIM7 | TMBIM1 | CD3EAP | GTF2F1 | NCOR2 | RCC2 | TLK2 |
| APOBR | FKBP15 | PDPK1 | TMBIM6 | CD99 | GTF2F2 | NELFA | RCCD1 | TMA16 |
| APOC3 | FKBP9 | PDSS1 | TMC8 | CDC40 | GTF2H1 | NELFB | RCL1 | TMEM209 |
| APOE | FLNA | PDXK | TMED4 | CDC5L | GTF2H3 | NELFCD | RCN2 | TMEM222 |
| ARAP1 | FLOT1 | PEA15 | TMED8 | CDC73 | GTF2I | NELFE | RCOR1 | TMEM263 |
| ARAP3 | FLOT2 | PECAM1 | TMEM120 A | CDK11B | GTF3C1 | NEPRO | RCOR3 | TMPO |
| ARF4 | FN1 | PECR | TMEM165 | CDK12 | GTF3C2 | NFATC21 P | RDH14 | TNPO2 |
| ARFIP1 | FN3K | PFKFB4 | TMEM167 A | CDK13 | GTF3C3 | NFIC | RECQL | TNRC18 |
| ARG1 | FNDC3B | PGAM1 | TMEM199 | CDK19 | GTF3C4 | NFRKB | RECQL5 | TOMM70 |
| ARHGAP1 | FRYL | PGGT1B | TMEM214 | CDK6 | GTF3C5 | NFYA | RERE | TOP1 |
| ARHGAP1 0 | FTH1 | PGM1 | TMEM30 A | CDK9 | GTPBP3 | NFYB | REST | TOP2B |
| ARHGAP1 2 | FTL | PHACTR2 | TMEM62 | CDKN2AI P | GTPBP4 | NFYC | REXO4 | TP53BP1 |
| ARHGAP1 8 | FYN | PHKA1 | TMEM94 | CDYL | HCFC1 | NGDN | RFC1 | TP53RK |
| ARHGAP2 6 | GAA | PHKA2 | TMOD1 | CEBPZ | HDAC1 | NHEJ1 | RFX1 | TPR |
| ARHGDIA | GABARA P | PHKB | TMPPE | CELF2 | HDAC2 | NHP2 | RFX3 | TPRKB |
| ARHGEF1 | GABARA PL1 | PHKG2 | TNFAIP2 | CENPB | HDAC3 | NIFK | RFX5 | TRA2A |
| ARHGEF1 1 | GABARA PL2 | PI4K2B | TNFAIP3 | CENPC | HDAC5 | NIP7 | RFX7 | TRA2B |
| ARL15 | GALM | PIP4P2 | TNFAIP8 | CENPV | HDDC3 | NIPBL | RFXAP | TRAF3IP3 |
| ARPC2 | GALNT10 | PITPNC1 | TNFSF13 B | CEP128 | HDGF | NIPSNAP 1 | RING1 | TRIM13 |
| ARPC5 | GALNT3 | PKLR | TOLLIP | CEP170 | HDGFL2 | NKAPD1 | RLF | TRIM24 |
| ASAP1 | GAPVD1 | PKM | TOM1 | CEP41 | HDHD2 | NKRF | RNASEH 2A | TRIM28 |
| ASB6 | GBF1 | PLAUR | TOM1L2 | CEP57 | HEATR1 | NKTR | RNASEH 2B | TRIM33 |
| ASCC3 | GC | PLBD1 | TOP2A | CEP63 | HHEX | NLE1 | RNASEH 2C | TRIM65 |
| ASL | GCA | PLCB3 | TOR1AIP 2 | CETN2 | HINT1 | NMD3 | RNF113A | TRIR |
| ASNS | GDAP2 | PLCH1 | TOR3A | CFAP20 | HIRA | NMNAT1 | RNF114 | TRMT1 |
| ASPM | GET4 | PLCL2 | TP53I11 | CFDP1 | HMCES | NOC2L | RNF169 | TRMT1L |
| ATG12 | GFPT1 | PLD3 | TPD52L2 | CGGBP1 | HMG20A | NOC3L | RNF2 | TRMT2A |
| ATG3 | GGA2 | PLEC | TPM1 | CHAMP1 | HMG20B | NOC4L | RNF220 | TRMT6 |
| ATG4A | GGA3 | PLEKHO2 | TPM3 | CHD1 | HMGB1 | NOL10 | RNMT | TRMT61A |
| ATG7 | GGCX | PLIN2 | TPP1 | CHD2 | HMGN1 | NOL11 | RNPC3 | TRNT1 |
| ATG9A | GGT1 | PLK1 | TPRG1L | CHD4 | HMGN3 | NOL4L | RNPS1 | TRRAP |
| ATL3 | GLA | PLOD1 | TPT1 | CHD6 | HMGN4 | NOL6 | RP9 | TRUB1 |
| ATP11A | GLIPR2 | PLOD3 | TRAF3 | CHD8 | HMGXB4 | NOL7 | RPA1 | TSC22D1 |
| ATP11B | GLRX | PLP2 | TRAF7 | CHD9 | HNRNPA 0 | NOL8 | RPA2 | TSN |
| ATP13A3 | GLRX3 | PLS3 | TRAP1 | CHERP | HNRNPA 1 | NOL9 | RPA3 | TSNAX |
| ATP1A1 | GLTP | PLSCR1 | TRAPPC1 1 | CHMP1B | HNRNPA 2B1 | NOLC1 | RPAP2 | TSSC4 |
| ATP1B3 | GMIP | PLSCR3 | TRAPPC1 2 | CHTOP | HNRNPA 3 | NONO | RPF1 | TTC14 |
| ATP2A2 | GMPPA | PLTP | TRAPPC1 3 | CIR1 | HNRNPA B | NOP10 | RPF2 | TTC19 |
| ATP6AP1 | GMPPB | POR | TRAPPC5 | CIRBP | HNRNPC | NOP14 | RPL7L1 | TTC33 |
| A TP6AP2 | GNAI2 | PPFIBP2 | TRAPPC8 | CIZ1 | HNRNPD | NOP16 | RPP14 | TTC5 |
| ATP6V0A 1 | GNAI3 | PPIB | TRIOBP | CLASRP | HNRNPD L | NOP2 | RPP25 | TTF1 |
| A TP6V0D 1 | GNG5 | PPM1B | TRIP13 | CLIP2 | HNRNPF | NOP53 | RPP30 | TWISTNB |
| ATP6V1A | GNPDA1 | PPP1R12 C | TSPAN14 | CMSS1 | HNRNPH 2 | NOP56 | RPP38 | TXNL4A |
| ATP6V1B 2 | GOLGA1 | PPP2R1B | TSPO | CMTR1 | HNRNPH 3 | NOP58 | RPP40 | U2AF2 |
| ATP6V1C 1 | GOLGA7 | PPP3CB | TTK | CNBP | HNRNPK | NOP9 | RPRD1A | U2SURP |
| ATP6V1D | GPAT4 | PPP4R1 | TTYH3 | CNOT1 | HNRNPL | NOSIP | RPRD1B | UBA2 |
| ATP6V1E 1 | GPCPD1 | PRC1 | TUBA4A | CNOT11 | HNRNPM | NR2C2 | RPRD2 | UBA3 |
| ATP6V1F | GPD1L | PRDX4 | TXN2 | CNOT2 | HNRNPR | NR3C1 | RPS19BP 1 | UBE2E1 |
| ATP9B | GPI | PRKACA | TXNDC11 | CNOT3 | HNRNPU | NRDE2 | RPS6KA5 | UBE21 |
| ATXN1 | GPR108 | PRKAG1 | TXNDC12 | CNOT7 | HNRNPU L1 | NRF1 | RPTOR | UBFD1 |
| AUP1 | GPSM3 | PRKAR2B | TXNDC5 | CNOT8 | HNRNPU L2 | NSA2 | RPUSD2 | UBLCP1 |
| AZGP1 | GRB2 | PRKCA | TYROBP | CNOT9 | HP1BP3 | NSL1 | RRAGB | UBN1 |
| AZI2 | GRN | PRKCD | TYSND1 | CNTRL | HSF1 | NSRP1 | RREB1 | UBQLN4 |
| AZU1 | GSN | PRMT7 | UAP1 | COPS7B | HSH2D | NSUN2 | RRP1 | UBR5 |
| B4GALT1 | GSPT1 | PRPS2 | UBAC1 | CPSF1 | HSPA1L | NTHL1 | RRP12 | UBTF |
| BAG3 | GSTM3 | PRPSAP1 | UBE2H | CPSF2 | HSPA8 | NUB1 | RRP15 | UBXN7 |
| BAG6 | GSTT1 | PRTN3 | UBE2K | CPSF3 | HSPBAP1 | NUBPL | RRP1B | UCKL1 |
| BASP1 | GYG1 | PSAT1 | UBE2S | CPSF4 | HTATSF1 | NUCKS1 | RRP36 | UFL1 |
| BECN1 | GYS1 | PSEN1 | UBE3C | CPSF6 | HUS1 | NUDCD1 | RRP7A | UHRF2 |
| BIN3 | HBB | PSMD11 | UBL3 | CPSF7 | IAH1 | NUDCD2 | RRP8 | UIMC1 |
| BLVRA | HBD | PSMD12 | UBR2 | CRBN | IFT140 | NUDT16L 1 | RRP9 | UNC119B |
| BLVRB | HCK | PSMD13 | UNC119 | CRIP2 | IFT20 | NUDT21 | RRS1 | UNK |
| BPI | HDAC4 | PSMD6 | UNC13D | CRNKL1 | IFT81 | NUMA1 | RSBN1 | UPF2 |
| BSG | HECTD1 | PSMD7 | UPP1 | CSTF1 | IK | NUP107 | RSBN1L | UPF3A |
| BST1 | HERC1 | PSMD8 | URM1 | CSTF2 | IKZF1 | NUP133 | RSF1 | UPF3B |
| BST2 | HEXA | PSMD9 | USP15 | CSTF2T | IL3RA | NUP153 | RSL1D1 | UPRT |
| C18orf32 | HK1 | PSTPIP1 | USP25 | CSTF3 | ILF2 | NUP214 | RSRC1 | URB1 |
| C19orf38 | HK3 | PTGS1 | USP32 | CTBP1 | ILF3 | NUP50 | RSRC2 | URB2 |
| C1GALT1 | HLA-B | PTK2B | USP8 | CTBP2 | ILKAP | NUP58 | RTCB | URI1 |
| C1GALT1 C1 | HM13 | PTPN12 | UTS2 | CTCF | IMP3 | NUP85 | RTF1 | USF1 |
| C20orf27 | HMMR | PTPN18 | VAMP3 | CTDP1 | IMP4 | NUP88 | RTF2 | USF2 |
| C2CD2L | HMOX1 | PTPN22 | VAMP8 | CTNNBL1 | ING1 | NUP98 | RTRAF | USP11 |
| C3 | HP | PTPRE | VASP | CTR9 | ING3 | NUTF2 | RUNX1 | USP34 |
| C5AR1 | HPSE | PTPRJ | VMP1 | CUL4B | ING5 | NXF1 | RUVBL1 | USP39 |
| C9 | HSP90B1 | PTX3 | VPS26B | CUL5 | INO80 | NXT1 | RUVBL2 | USP48 |
| C9orf40 | HSPA13 | PUDP | VPS29 | CUTC | INO80C | OARD1 | SAE1 | USP5 |
| C9orf72 | HSPA2 | PYROXD 1 | VPS39 | CUX1 | INO80E | OCIAD2 | SAFB | USP7 |
| CA1 | HSPA4L | QSOX1 | VPS41 | CWC15 | INPP5B | ODF2 | SAFB2 | UTP11 |
| CA2 | HSPA5 | RAB10 | VPS50 | CWC22 | INTS1 | OFD1 | SAMD4B | UTP14A |
| CALD1 | HSPA6 | RAB11A | VPS52 | CWC25 | INTS10 | ORC2 | SAP130 | UTP15 |
| CAMK1 | HSPB1 | RAB11FI P1 | VPS53 | CWC27 | INTS12 | ORC3 | SAP18 | UTP18 |
| CAMK2D | HSPB11 | RAB15 | VPS54 | CWF19L2 | INTS13 | ORC4 | SAP30BP | UTP20 |
| CAMKK2 | HSPG2 | RAB18 | VSTM1 | CXorf56 | INTS14 | ORC5 | SARDH | UTP23 |
| CANT1 | HVCN1 | RAB1A | VTI1A | CXXC1 | INTS2 | OSGEP | SART1 | UTP3 |
| CAPN2 | HYOU1 | RAB20 | VTI1B | DAXX | INTS4 | PAF1 | SART3 | UTP4 |
| CAPNS1 | ICAM1 | RAB21 | VTN | DAZAP1 | INTS5 | PAK1IP1 | SATB1 | UTP6 |
| CAPZA1 | ICAM3 | RAB27A | WBP2 | DBR1 | INTS6 | PAN3 | SCAF1 | UXT |
| CAPZB | IFNGR1 | RAB29 | WDFY1 | DCAF1 | INTS7 | PAPOLA | SCAF11 | VEZF1 |
| CARD16 | IGF2R | RAB31 | WDFY2 | DCAF13 | INTS8 | PAPOLG | SCAF4 | VEZT |
| CARHSP1 | IGFBP7 | RAB32 | WDFY3 | DCAF16 | INTS9 | PARN | SCAF8 | VIRMA |
| CAST | IGHG1 | RAB33A | WDR1 | DCAF7 | IRF2BP1 | PARP1 | SCAI | VPS37B |
| CBL | IGKC | RAB33B | WDR41 | DCK | ISG20L2 | PARP16 | SCLY | VPS72 |
| CC2D1A | IKBIP | RAB35 | WDR62 | DCPS | ISYNA1 | PARS2 | SCMH1 | WAC |
| CCDC167 | IKBKE | RAB3GA P2 | WDR7 | DCTD | ITPA | PATZ1 | SCML2 | WAPL |
| CCDC69 | IKBKG | RAB43 | WNK1 | DCUN1D 4 | ITPR2 | PAXBP1 | SDAD1 | WBP11 |
| CCDC88B | IL17RA | RAB4A | XYLB | DDA1 | IVNS1AB P | PAXIP1 | SDE2 | WDR12 |
| CCNA2 | IL1RN | RAB5A | YES1 | DDAH2 | IWS1 | PAXX | SDR39U1 | WDR18 |
| CCNB1 | ILK | RAB5B | YKT6 | DDX1 | JADE2 | PBRM1 | SENP6 | WDR19 |
| CCNB2 | IMPDH1 | RAB5C | YOD1 | DDX10 | JADE3 | PBX2 | SENP7 | WDR24 |
| CCNY | INTS6L | RAB6B | YTHDC2 | DDX17 | JRKL | PBXIP1 | SEPHS1 | WDR3 |
| CCNYL1 | IPO9 | RAB7A | YWHAH | DDX18 | JUND | PCBP1 | SEPHS2 | WDR33 |
| CD101 | IQSEC1 | RAB8B | ZBTB7B | DDX20 | KANSL1 | PCBP2 | SET | WDR36 |
| CD151 | IRAK3 | RABGGT A | ZBTB8OS | DDX23 | KANSL2 | PCF11 | SETD1A | WDR43 |
| CD300A | IRS2 | RABGGT B | ZC3H7A | DDX27 | KANSL3 | PCGF1 | SETD1B | WDR46 |
| CD300LF | ITFG1 | RAC2 | ZDHHC20 | DDX31 | KAT14 | PCGF5 | SETD2 | WDR48 |
| CD33 | ITGAL | RACK1 | ZFYVE16 | DDX39B | KAT2A | PCIF1 | SETDB1 | WDR5 |
| CD36 | ITGAM | RALB | ZFYVE21 | DDX41 | KAT5 | PCNT | SETMAR | WDR59 |
| CD4 | ITGAX | RAP1A | ZWINT | DDX42 | KAT6B | PCYT1A | SF1 | WDR70 |
| CD58 | ITGB1 | RAP1B | ZYG11B | DDX46 | KAT7 | PDCD11 | SF3A1 | WDR75 |
| CD63 | ITGB2 | RAP2B | ZYX | DDX49 | KAT8 | PDCD4 | SF3A2 | WDR77 |
| CD9 | ITGB5 | RBM47 | ZZEF1 | DDX5 | KCTD18 | PDCD7 | SF3A3 | WDR82 |
| CD99L2 | ITIH2 | REEP5 | AAR2 | DDX50 | KDM1A | PDRG1 | SF3B1 | WDR89 |
| CDA | ITIH3 | RENBP | AATF | DDX51 | KDM2B | PDS5A | SF3B2 | WDR92 |
| CDC27 | ITIH4 | RGS18 | ABL2 | DDX52 | KDM3A | PDS5B | SF3B3 | WIZ |
| CDC42BP A | ITPK1 | RGS19 | ABRAXA S1 | DDX54 | KDM3B | PEBP1 | SF3B4 | WRAP53 |
| CDC42BP B | ITSN1 | RHEB | ABT1 | DDX55 | KDM5A | PELP1 | SF3B5 | WRNIP1 |
| CDK1 | JAK2 | RHOF | ACIN1 | DDX56 | KDM5B | PES1 | SF3B6 | WTAP |
| CEBPE | JDP2 | RIPOR1 | ACTL6A | DEAF1 | KDM8 | PHAX | SFPQ | XAB2 |
| CENPM | JPT1 | RIPOR2 | ACTR5 | DEK | KDSR | PHC1 | SFR1 | XPC |
| CEP44 | KBTBD11 | RMDN2 | ACTR8 | DENR | KHDC4 | PHC2 | SFSWAP | XPO1 |
| CES1 | KBTBD2 | RNASE2 | ADA | DEPTOR | KHDRBS 1 | PHC3 | SIN3A | XPO5 |
| CFD | KCTD10 | RNASE3 | ADAR | DFFA | KHSRP | PHF10 | SIN3B | XRCC1 |
| CFLAR | KCTD12 | RNF123 | ADH5 | DHRS11 | KIAA1143 | PHF11 | SLC25A3 6 | XRCC5 |
| CGAS | KCTD9 | RNF141 | ADK | DHX15 | KIAA1191 | PHF12 | SLC39A1 0 | XRCC6 |
| CHAC2 | KIAA0319 L | RNH1 | ADNP | DHX16 | KLC4 | PHF14 | SLC9A7 | XRN2 |
| CHM | KIAA0513 | ROCK2 | AFF1 | DHX33 | KLHL36 | PHF2 | SLTM | YEATS2 |
| CHMP6 | KIAA0930 | ROGDI | AFF4 | DHX37 | KMT2A | PHF20 | SLU7 | YEATS4 |
| CHP1 | KIDINS22 0 | RP2 | AGO1 | DHX38 | KMT2B | PHF20L1 | SMAD2 | YLPM1 |
| CIB1 | KIF13B | RPS14 | AGO3 | DHX40 | KMT2D | PHF21A | SMAD4 | YTHDC1 |
| CIP2A | KIF15 | RPS20 | AGPAT5 | DHX8 | KMT2E | PHF23 | SMARCA 2 | YTHDF2 |
| CIT | KIF21B | RPS27 | AHCTF1 | DHX9 | KNOP1 | PHF3 | SMARCA 4 | YWHAQ |
| CKAP4 | KIF2C | RRAS | AHDC1 | DIDO1 | KRI1 | PHF5A | SMARCA 5 | YY1 |
| CKS2 | KLHL22 | RSU1 | AKAP11 | DIMT1 | KRR1 | PHF6 | SMARCA D1 | ZBED1 |
| CLCC1 | KNL1 | RTN1 | AKAP17A | DIS3 | L3MBTL2 | PHF8 | SMARCA L1 | ZBTB1 |
| CLCN3 | KNTC1 | RTN3 | AKAP8L | DKC1 | L3MBTL3 | PHIP | SMARCB 1 | ZBTB11 |
| CLDND1 | KRAS | S100A11 | AKR1C3 | DMAP1 | LANCL1 | PHRF1 | SMARCC 1 | ZBTB14 |
| CLEC16A | LAMP1 | S100A12 | ALDH5A1 | DNAJB6 | LARP7 | PIAS4 | SMARCC 2 | ZBTB17 |
| CLPB | LAPTM5 | S100A6 | ALKBH5 | DNAJC17 | LAS1L | PIK3C2B | SMARCD 1 | ZBTB2 |
| CLPTM1 | LCP1 | S100A8 | ALYREF | DNAJC8 | LCOR | PIKFYVE | SMARCD 2 | ZBTB21 |
| CLTA | LDLRAP1 | S100A9 | ANKMY2 | DNAJC9 | LDB1 | PIN4 | SMARCE 1 | ZBTB24 |
| CLTB | LGALS3 | S100P | ANKRD11 | DNMT3A | LEMD3 | PIP4K2B | SMC1A | ZBTB33 |
| CLTC | LGALS8 | SAMHD1 | ANKRD12 | DNMT3B | LENG8 | PLEKHA5 | SMC3 | ZBTB40 |
| CLU | LILRB4 | SAR1A | ANKRD28 | DNPEP | LEO1 | PLIN3 | SMIM24 | ZBTB44 |
| CMIP | LIN7C | SAR1B | ANKRD54 | DNTTIP1 | LGALS9 | PLPBP | SMNDC1 | ZC3H10 |
| CNIH4 | LMAN2 | SCAMP2 | ANP32A | DNTTIP2 | LIG3 | PLRG1 | SMU1 | ZC3H13 |
| CNPY3 | LMBRD2 | SCP2 | ANP32B | DPF2 | LIN37 | PM20D2 | SNIP1 | ZC3H14 |
| COASY | LMTK2 | SCPEP1 | APEX1 | DPH2 | LIN9 | PML | SNRNP20 0 | ZC3H18 |
| COL6A1 | LNPK | SDCBP | API5 | DR1 | LLPH | PMS2 | SNRNP27 | ZC3H4 |
| COL6A2 | LPCAT1 | SDF2L1 | APOBEC 3F | DRAP1 | LMNB1 | PNISR | SNRNP40 | ZC3HC1 |
| COL6A3 | LRP1 | SEC11A | APOO | DSN1 | LMNB2 | PNN | SNRNP48 | ZC4H2 |
| COLGALT 1 | LRPAP1 | SEC11C | APTX | DUS3L | LPIN1 | PNO1 | SNRNP70 | ZCCHC17 |
| COPA | LRRC25 | SEC23B | AQR | DUSP11 | LRIF1 | POC1B | SNRPA | ZCCHC3 |
| COPB1 | LRRC8C | SEC24A | ARGLU1 | DYNLL1 | LRRC41 | POC5 | SNRPA1 | ZCCHC7 |
| COPB2 | LRRC8D | SEC24D | ARHGAP 22 | DYNLL2 | LRRC47 | POGK | SNRPB2 | ZCCHC8 |
| COPE | LRRK1 | SEC61B | ARHGAP 25 | E2F3 | LRWD1 | POGLUT1 | SNRPC | ZCCHC9 |
| COPS7A | LST1 | SEC63 | ARHGEF 2 | EBNA1BP 2 | LSG1 | POGZ | SNRPD1 | ZCRB1 |
| COPZ1 | LTA4H | SELENBP 1 | ARID1A | EDC3 | LSM2 | POLB | SNRPD2 | ZFAT |
| CORO2A | LTN1 | SELENO N | ARID1B | EDF1 | LSM3 | POLDIP3 | SNRPD3 | ZFC3H1 |
| COTL1 | LUM | SELENO O | ARID2 | EFTUD2 | LSM4 | POLI | SNRPE | ZFP64 |
| CP | LYST | SELENOT | ARID4A | EHMT1 | LSM5 | POLR1A | SNRPF | ZFR |
| CPM | LYZ | SEMA4A | ARID4B | EHMT2 | LSM6 | POLR1B | SNRPG | ZHX1 |
| CPNE2 | M6PR | SERPINB 10 | ARL14EP | EIF1AX | LSM7 | POLR1C | SNU13 | ZHX2 |
| CPNE3 | MAN2A1 | SERPINB 2 | ARL2BP | EIF2AK4 | LSM8 | POLR1D | SNW1 | ZKSCAN1 |
| CPPED1 | MAN2B1 | SERPINB 8 | ARL6IP4 | EIF4A3 | LUC7L2 | POLR1E | SON | ZKSCAN8 |
| CPQ | MANF | SERPINC 1 | ARRB1 | EIF4E | LUC7L3 | POLR2B | SORD | ZMAT2 |
| CR1 | MAP2K1 | SERPINF 1 | ARSK | ELAC2 | LYL1 | POLR2C | SP140L | ZMAT5 |
| CRELD2 | MAP2K2 | SERPING 1 | ASH2L | ELAVL1 | LYSMD2 | POLR2D | SP3 | ZMIZ1 |
| CSGALNA CT2 | MAP3K4 | SESN2 | ASMTL | ELF1 | LYSMD3 | POLR2E | SPEN | ZMYM2 |
| CSRP1 | MAP3K5 | SETD7 | ATAD2B | ELF2 | LZTFL1 | POLR2G | SPIN1 | ZMYM3 |
| CST3 | MAP3K6 | SFT2D2 | ATE1 | ELL | MACF1 | POLR2I | SPOUT1 | ZMYM4 |
| CST7 | MAP4K4 | SGSH | ATF2 | ELMO1 | MAD1L1 | POLR2L | SPTAN1 | ZMYND8 |
| CSTA | MAPK1 | SGTB | ATF7IP | ELMSAN1 | MAD2L1B P | POLR3A | SPTBN1 | ZNF131 |
| CSTB | MAPK3 | SH3BP2 | ATRX | ELOA | MAF1 | POLR3B | SREK1 | ZNF143 |
| CTDNEP1 | MARCKS | SH3BP5 | ATXN7L3 | EMSY | MAFK | POLR3C | SRF | ZNF148 |
| CTSA | MAST3 | SHARPIN | BABAM1 | ENGASE | MAK16 | POLR3D | SRFBP1 | ZNF207 |
| CTSG | MBOAT2 | SHC1 | BABAM2 | ENO3 | MAP1A | POLR3E | SRP14 | ZNF22 |
| CTSH | MCL1 | SHCBP1 | BAG1 | ENY2 | MAP2K6 | POLR3F | SRP9 | ZNF24 |
| CTSL | MCTP1 | SHMT1 | BAHCC1 | EP400 | MAP7 | POLR3K | SRRM1 | ZNF276 |
| CTSZ | MCU | SHMT2 | BANF1 | EPC1 | MAST4 | POP1 | SRRT | ZNF280C |
| CTTN | MEFV | SIDT2 | BANP | EPC2 | MAT2B | POP4 | SRSF1 | ZNF280D |
| CYBA | MEGF9 | SIGMAR1 | BAZ1B | EPM2AIP 1 | MAU2 | POU2F1 | SRSF10 | ZNF292 |
| CYBB | METTL9 | SIK2 | BAZ2A | ERCC2 | MAX | PPHLN1 | SRSF11 | ZNF3 |
| CYBRD1 | MFSD1 | SIRPA | BAZ2B | ERCC3 | MAZ | PPIE | SRSF2 | ZNF326 |
| CYP27A1 | MGAM | SIRPB2 | BBX | ERCC5 | MBD1 | PPIG | SRSF3 | ZNF346 |
| CYSTM1 | MGAT1 | SLC12A9 | BCAS2 | ERCC6 | MBD2 | PPIH | SRSF4 | ZNF384 |
| DAAM1 | MGAT2 | SLC15A4 | BCKDHB | ERG | MBD3 | PPIL1 | SRSF5 | ZNF407 |
| DAD1 | MGAT4B | SLC16A1 | BCL11A | ERGIC1 | MBIP | PPIL2 | SRSF6 | ZNF445 |
| DAGLB | MGST2 | SLC16A3 | BCL2 | ERH | MBNL1 | PPIL3 | SRSF7 | ZNF451 |
| DAPP1 | MICAL1 | SLC16A7 | BCL7A | ERI1 | MBOAT7 | PPIL4 | SRSF9 | ZNF512 |
| DBNL | MICU1 | SLC17A5 | BCL7C | ERI3 | MCM3AP | PPP1R10 | SS18 | ZNF512B |
| DCTN3 | MICU2 | SLC1A4 | BCLAF1 | ESD | MCRS1 | PPP1R14 B | SSB | ZNF574 |
| DEGS1 | MILR1 | SLC1A5 | BCORL1 | ESF1 | MDC1 | PPP1R8 | SSU72 | ZNF579 |
| DENND1A | MINDY1 | SLC27A3 | BCR | ESS2 | MDM1 | PPP4R2 | STAG1 | ZNF592 |
| DENND2D | MINK1 | SLC29A1 | BDH2 | ETV6 | MEAF6 | PPP4R3A | STAT5A | ZNF593 |
| DENND4A | MITD1 | SLC2A1 | BEND3 | EWSR1 | MECP2 | PPWD1 | STK19 | ZNF609 |
| DENND4B | MLKL | SLC2A3 | BIRC2 | EXD2 | MED1 | PQBP1 | STK26 | ZNF616 |
| DGAT1 | MMRN1 | SLC2A6 | BMS1 | EXOSC1 | MED10 | PRCC | STK38L | ZNF618 |
| DGKG | MNDA | SLC30A1 | BOD1 | EXOSC10 | MED11 | PRDM10 | STN1 | ZNF622 |
| DHX32 | MON2 | SLC38A1 0 | BOD1L1 | EXOSC2 | MED12 | PRKACB | STRBP | ZNF629 |
| DIAPH1 | MOSPD2 | SLC39A1 1 | BOP1 | EXOSC3 | MED13L | PRKDC | STRIP1 | ZNF638 |
| DIAPH3 | MPP6 | SLC39A1 4 | BPNT1 | EXOSC4 | MED14 | PRKRA | STRN3 | ZNF639 |
| DLG1 | MRC1 | SLC3A2 | BPTF | EXOSC5 | MED15 | PRKRIP1 | STRN4 | ZNF644 |
| DLGAP5 | MROH1 | SLC43A3 | BRCC3 | EXOSC6 | MED16 | PRMT1 | STX17 | ZNF646 |
| DNAJA4 | MSTO1 | SLC44A1 | BRD1 | EXOSC7 | MED18 | PRMT6 | STYX | ZNF654 |
| DNAJC13 | MTFP1 | SLC44A2 | BRD2 | EXOSC8 | MED21 | PRPF18 | SUDS3 | ZNF668 |
| DNAJC3 | MTFR2 | SLC4A2 | BRD3 | EXOSC9 | MED22 | PRPF19 | SUGP1 | ZNF687 |
| DNAJC5 | MTHFD2 | SLC52A2 | BRD4 | EZH1 | MED23 | PRPF3 | SUGP2 | ZNF688 |
| DNASE1L 1 | MTHFR | SLC5A6 | BRD7 | FAM117A | MED24 | PRPF31 | SUMO2 | ZNF691 |
| DOCK10 | MTMR3 | SLC7A1 | BRD8 | FAM136A | MED25 | PRPF38A | SUPT16H | ZNF740 |
| DOCK5 | MTMR6 | SLMAP | BRF1 | FAM172A | MED27 | PRPF38B | SUPT20H | ZNF768 |
| DOK2 | MYDGF | SMC2 | BRIX1 | FAM204A | MED29 | PRPF39 | SUPT3H | ZNF787 |
| DPH6 | MYH10 | SMC4 | BRMS1 | FAM50B | MED4 | PRPF4 | SUPT5H | ZNF830 |
| DPYSL2 | MYH14 | SMCR8 | BRMS1L | FAM76A | MED6 | PRPF40A | SUPT6H | ZNHIT1 |
| DYSF | MYH9 | SNAP23 | BRPF1 | FAM76B | MED7 | PRPF40B | SUPT7L | ZRANB2 |
| ECE1 | MYL4 | SNTB2 | BRWD1 | FAM98B | MED8 | PRPF4B | SURF6 | ZRSR2 |
| ECHDC1 | MYL6 | SNX1 | BTF3L4 | FBL | MED9 | PRPF6 | SWAP70 | ZSCAN26 |
| EDEM2 | MYLK | SNX11 | BUB3 | FBLL1 | MEN1 | PRPF8 | SYF2 | |

**Table 2 - upregulated proteins**

| **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** |
|---|---|---|---|---|---|---|---|---|---|---|---|
| 8 | 1745 | 3025 | 4539 | 5966 | 7281 | 8505 | 9966 | 11508 | 13046 | 14652 | 15985 |
| 9 | 1746 | 3026 | 4540 | 5967 | 7289 | 8506 | 9967 | 11509 | 13047 | 14653 | 15986 |
| 10 | 1747 | 3027 | 4541 | 5968 | 7290 | 8507 | 9968 | 11510 | 13048 | 14654 | 15987 |
| 11 | 1748 | 3038 | 4542 | 5969 | 7291 | 8508 | 9969 | 11511 | 13066 | 14655 | 15988 |
| 12 | 1749 | 3039 | 4543 | 5970 | 7292 | 8509 | 9970 | 11512 | 13067 | 14656 | 15989 |
| 13 | 1750 | 3040 | 4544 | 5971 | 7293 | 8510 | 9971 | 11513 | 13068 | 14657 | 15990 |
| 14 | 1751 | 3041 | 4545 | 5972 | 7294 | 8511 | 9972 | 11514 | 13069 | 14658 | 15991 |
| 15 | 1752 | 3042 | 4546 | 5973 | 7295 | 8512 | 9973 | 11515 | 13070 | 14659 | 15992 |
| 16 | 1753 | 3043 | 4547 | 5981 | 7296 | 8513 | 9974 | 11516 | 13071 | 14660 | 15993 |
| 17 | 1754 | 3044 | 4548 | 5982 | 7297 | 8532 | 9975 | 11517 | 13072 | 14661 | 15994 |
| 18 | 1755 | 3045 | 4549 | 5983 | 7298 | 8533 | 9976 | 11518 | 13073 | 14662 | 15995 |
| 19 | 1756 | 3046 | 4550 | 5984 | 7299 | 8534 | 9977 | 11519 | 13083 | 14671 | 15996 |
| 20 | 1763 | 3047 | 4551 | 5985 | 7300 | 8535 | 9978 | 11520 | 13084 | 14672 | 15997 |
| 21 | 1764 | 3048 | 4552 | 5986 | 7301 | 8536 | 9979 | 11521 | 13085 | 14673 | 15998 |
| 22 | 1765 | 3049 | 4572 | 5987 | 7302 | 8537 | 9991 | 11522 | 13086 | 14674 | 15999 |
| 23 | 1766 | 3050 | 4573 | 5988 | 7303 | 8538 | 9992 | 11523 | 13087 | 14675 | 16000 |
| 24 | 1767 | 3051 | 4574 | 5989 | 7304 | 8539 | 9993 | 11524 | 13088 | 14676 | 16001 |
| 25 | 1768 | 3052 | 4575 | 5990 | 7305 | 8540 | 9994 | 11525 | 13089 | 14677 | 16002 |
| 26 | 1769 | 3053 | 4576 | 5991 | 7306 | 8541 | 9995 | 11526 | 13090 | 14678 | 16003 |
| 27 | 1798 | 3054 | 4590 | 5992 | 7307 | 8542 | 9996 | 11527 | 13091 | 14679 | 16004 |
| 38 | 1799 | 3055 | 4591 | 5993 | 7308 | 8543 | 9997 | 11528 | 13092 | 14687 | 16005 |
| 39 | 1800 | 3056 | 4592 | 5994 | 7309 | 8544 | 9998 | 11531 | 13093 | 14688 | 16006 |
| 40 | 1801 | 3057 | 4593 | 5995 | 7310 | 8545 | 10007 | 11532 | 13094 | 14689 | 16007 |
| 41 | 1802 | 3058 | 4594 | 5996 | 7311 | 8546 | 10008 | 11533 | 13131 | 14690 | 16014 |
| 42 | 1803 | 3059 | 4595 | 5997 | 7312 | 8547 | 10009 | 11534 | 13132 | 14691 | 16015 |
| 43 | 1804 | 3060 | 4596 | 5998 | 7313 | 8548 | 10010 | 11535 | 13133 | 14692 | 16021 |
| 44 | 1805 | 3061 | 4597 | 5999 | 7314 | 8549 | 10011 | 11536 | 13134 | 14693 | 16022 |
| 45 | 1806 | 3062 | 4598 | 6000 | 7315 | 8550 | 10012 | 11537 | 13135 | 14694 | 16023 |
| 46 | 1807 | 3073 | 4599 | 6001 | 7316 | 8551 | 10013 | 11538 | 13136 | 14695 | 16024 |
| 47 | 1808 | 3074 | 4600 | 6002 | 7317 | 8552 | 10014 | 11539 | 13137 | 14696 | 16025 |
| 48 | 1809 | 3075 | 4601 | 6003 | 7318 | 8553 | 10015 | 11540 | 13138 | 14697 | 16026 |
| 50 | 1810 | 3076 | 4602 | 6004 | 7319 | 8554 | 10016 | 11541 | 13139 | 14698 | 16027 |
| 51 | 1811 | 3077 | 4603 | 6005 | 7320 | 8555 | 10017 | 11542 | 13140 | 14699 | 16028 |
| 52 | 1812 | 3078 | 4604 | 6006 | 7321 | 8556 | 10018 | 11543 | 13141 | 14700 | 16029 |
| 53 | 1813 | 3079 | 4605 | 6007 | 7322 | 8557 | 10019 | 11544 | 13142 | 14701 | 16030 |
| 54 | 1814 | 3080 | 4606 | 6008 | 7323 | 8558 | 10020 | 11545 | 13143 | 14702 | 16031 |
| 55 | 1815 | 3081 | 4607 | 6009 | 7324 | 8559 | 10021 | 11546 | 13144 | 14703 | 16032 |
| 56 | 1816 | 3082 | 4608 | 6010 | 7325 | 8560 | 10022 | 11547 | 13145 | 14704 | 16033 |
| 57 | 1817 | 3083 | 4609 | 6011 | 7326 | 8561 | 10023 | 11548 | 13146 | 14705 | 16034 |
| 58 | 1822 | 3084 | 4610 | 6012 | 7327 | 8562 | 10024 | 11549 | 13147 | 14706 | 16035 |
| 59 | 1823 | 3085 | 4611 | 6013 | 7328 | 8563 | 10025 | 11550 | 13148 | 14707 | 16036 |
| 60 | 1824 | 3086 | 4612 | 6014 | 7329 | 8564 | 10026 | 11559 | 13149 | 14732 | 16037 |
| 61 | 1825 | 3087 | 4613 | 6015 | 7330 | 8565 | 10027 | 11560 | 13150 | 14733 | 16045 |
| 62 | 1826 | 3088 | 4614 | 6016 | 7331 | 8597 | 10028 | 11561 | 13151 | 14734 | 16046 |
| 63 | 1827 | 3089 | 4615 | 6017 | 7332 | 8598 | 10029 | 11562 | 13152 | 14735 | 16047 |
| 64 | 1828 | 3090 | 4616 | 6018 | 7333 | 8599 | 10030 | 11563 | 13153 | 14736 | 16048 |
| 65 | 1830 | 3091 | 4624 | 6019 | 7334 | 8600 | 10031 | 11564 | 13154 | 14772 | 16049 |
| 82 | 1831 | 3092 | 4625 | 6020 | 7335 | 8601 | 10032 | 11565 | 13155 | 14773 | 16050 |
| 83 | 1832 | 3093 | 4626 | 6021 | 7336 | 8602 | 10033 | 11566 | 13156 | 14774 | 16051 |
| 84 | 1833 | 3094 | 4627 | 6022 | 7337 | 8603 | 10034 | 11567 | 13157 | 14775 | 16052 |
| 85 | 1834 | 3095 | 4628 | 6023 | 7354 | 8604 | 10035 | 11568 | 13158 | 14776 | 16053 |
| 86 | 1835 | 3096 | 4629 | 6024 | 7355 | 8605 | 10036 | 11569 | 13159 | 14777 | 16054 |
| 87 | 1836 | 3097 | 4630 | 6025 | 7356 | 8606 | 10046 | 11570 | 13160 | 14778 | 16055 |
| 88 | 1837 | 3098 | 4631 | 6026 | 7357 | 8607 | 10089 | 11571 | 13161 | 14779 | 16056 |
| 89 | 1838 | 3099 | 4632 | 6027 | 7358 | 8608 | 10090 | 11572 | 13162 | 14780 | 16057 |
| 90 | 1839 | 3100 | 4633 | 6028 | 7359 | 8609 | 10091 | 11573 | 13163 | 14781 | 16058 |
| 91 | 1840 | 3101 | 4634 | 6029 | 7360 | 8617 | 10092 | 11574 | 13164 | 14782 | 16059 |
| 92 | 1841 | 3102 | 4635 | 6030 | 7361 | 8632 | 10093 | 11575 | 13165 | 14783 | 16060 |
| 93 | 1842 | 3107 | 4636 | 6031 | 7362 | 8633 | 10094 | 11576 | 13166 | 14784 | 16061 |
| 94 | 1843 | 3108 | 4637 | 6032 | 7363 | 8634 | 10095 | 11577 | 13167 | 14785 | 16062 |
| 95 | 1844 | 3134 | 4638 | 6033 | 7364 | 8635 | 10096 | 11578 | 13168 | 14786 | 16063 |
| 96 | 1845 | 3135 | 4654 | 6038 | 7365 | 8636 | 10097 | 11579 | 13169 | 14787 | 16064 |
| 97 | 1846 | 3136 | 4655 | 6039 | 7366 | 8637 | 10098 | 11580 | 13170 | 14788 | 16065 |
| 98 | 1847 | 3166 | 4656 | 6040 | 7367 | 8638 | 10099 | 11581 | 13171 | 14789 | 16066 |
| 99 | 1848 | 3167 | 4657 | 6041 | 7368 | 8639 | 10100 | 11582 | 13172 | 14790 | 16067 |
| 100 | 1849 | 3168 | 4658 | 6042 | 7369 | 8640 | 10101 | 11583 | 13173 | 14791 | 16068 |
| 101 | 1850 | 3169 | 4674 | 6043 | 7370 | 8641 | 10104 | 11584 | 13208 | 14792 | 16069 |
| 102 | 1851 | 3175 | 4675 | 6044 | 7371 | 8642 | 10105 | 11585 | 13209 | 14793 | 16070 |
| 103 | 1852 | 3176 | 4676 | 6045 | 7372 | 8643 | 10106 | 11586 | 13210 | 14794 | 16071 |
| 110 | 1853 | 3177 | 4677 | 6046 | 7373 | 8644 | 10107 | 11587 | 13211 | 14795 | 16072 |
| 111 | 1854 | 3178 | 4678 | 6047 | 7374 | 8645 | 10108 | 11588 | 13212 | 14796 | 16073 |
| 112 | 1855 | 3179 | 4679 | 6056 | 7375 | 8646 | 10109 | 11589 | 13223 | 14797 | 16074 |
| 113 | 1865 | 3180 | 4680 | 6057 | 7376 | 8647 | 10110 | 11590 | 13224 | 14798 | 16075 |
| 114 | 1866 | 3181 | 4681 | 6058 | 7377 | 8648 | 10111 | 11591 | 13225 | 14799 | 16076 |
| 115 | 1867 | 3182 | 4682 | 6059 | 7378 | 8649 | 10112 | 11592 | 13226 | 14800 | 16077 |
| 116 | 1868 | 3183 | 4683 | 6060 | 7379 | 8650 | 10113 | 11604 | 13227 | 14801 | 16078 |
| 117 | 1869 | 3184 | 4684 | 6061 | 7395 | 8651 | 10114 | 11605 | 13228 | 14802 | 16084 |
| 118 | 1870 | 3185 | 4685 | 6062 | 7396 | 8652 | 10115 | 11606 | 13229 | 14803 | 16085 |
| 120 | 1871 | 3186 | 4691 | 6063 | 7397 | 8653 | 10116 | 11607 | 13235 | 14804 | 16086 |
| 121 | 1872 | 3187 | 4692 | 6072 | 7398 | 8666 | 10117 | 11629 | 13236 | 14805 | 16087 |
| 122 | 1873 | 3188 | 4693 | 6073 | 7399 | 8667 | 10118 | 11630 | 13237 | 14807 | 16088 |
| 123 | 1874 | 3196 | 4694 | 6074 | 7400 | 8668 | 10119 | 11631 | 13238 | 14808 | 16089 |
| 124 | 1875 | 3197 | 4695 | 6075 | 7401 | 8669 | 10120 | 11632 | 13239 | 14809 | 16090 |
| 125 | 1876 | 3198 | 4696 | 6076 | 7402 | 8670 | 10121 | 11633 | 13240 | 14810 | 16091 |
| 126 | 1877 | 3199 | 4697 | 6077 | 7403 | 8671 | 10122 | 11634 | 13241 | 14811 | 16092 |
| 127 | 1878 | 3200 | 4698 | 6078 | 7404 | 8672 | 10142 | 11635 | 13242 | 14812 | 16093 |
| 128 | 1879 | 3208 | 4699 | 6079 | 7405 | 8673 | 10143 | 11638 | 13243 | 14813 | 16094 |
| 129 | 1880 | 3209 | 4700 | 6080 | 7406 | 8674 | 10144 | 11639 | 13244 | 14814 | 16095 |
| 130 | 1881 | 3210 | 4701 | 6081 | 7407 | 8675 | 10145 | 11640 | 13245 | 14815 | 16096 |
| 131 | 1920 | 3211 | 4702 | 6082 | 7408 | 8676 | 10146 | 11641 | 13246 | 14822 | 16097 |
| 132 | 1921 | 3212 | 4703 | 6083 | 7409 | 8677 | 10147 | 11644 | 13247 | 14823 | 16098 |
| 133 | 1922 | 3213 | 4704 | 6084 | 7410 | 8678 | 10148 | 11645 | 13257 | 14824 | 16099 |
| 134 | 1923 | 3214 | 4705 | 6085 | 7417 | 8679 | 10149 | 11646 | 13258 | 14825 | 16100 |
| 135 | 1924 | 3215 | 4706 | 6086 | 7418 | 8680 | 10150 | 11647 | 13259 | 14826 | 16101 |
| 136 | 1925 | 3216 | 4721 | 6087 | 7419 | 8681 | 10151 | 11648 | 13260 | 14827 | 16102 |
| 137 | 1926 | 3217 | 4722 | 6088 | 7420 | 8682 | 10152 | 11649 | 13261 | 14828 | 16103 |
| 138 | 1927 | 3218 | 4723 | 6089 | 7421 | 8683 | 10153 | 11650 | 13262 | 14829 | 16104 |
| 139 | 1928 | 3219 | 4724 | 6090 | 7422 | 8684 | 10154 | 11651 | 13273 | 14839 | 16105 |
| 140 | 1929 | 3220 | 4725 | 6091 | 7423 | 8685 | 10155 | 11652 | 13274 | 14840 | 16106 |
| 141 | 1930 | 3221 | 4726 | 6092 | 7424 | 8686 | 10156 | 11653 | 13275 | 14841 | 16107 |
| 142 | 1931 | 3222 | 4727 | 6093 | 7425 | 8687 | 10157 | 11654 | 13276 | 14842 | 16108 |
| 143 | 1932 | 3237 | 4738 | 6094 | 7426 | 8688 | 10158 | 11655 | 13277 | 14843 | 16109 |
| 144 | 1933 | 3241 | 4739 | 6095 | 7427 | 8689 | 10159 | 11656 | 13278 | 14844 | 16110 |
| 145 | 1934 | 3242 | 4740 | 6096 | 7428 | 8690 | 10160 | 11657 | 13279 | 14845 | 16111 |
| 146 | 1935 | 3243 | 4741 | 6097 | 7429 | 8691 | 10169 | 11658 | 13280 | 14846 | 16112 |
| 147 | 1936 | 3244 | 4742 | 6098 | 7430 | 8692 | 10170 | 11659 | 13281 | 14847 | 16113 |
| 148 | 1937 | 3245 | 4743 | 6099 | 7431 | 8693 | 10171 | 11660 | 13289 | 14848 | 16114 |
| 149 | 1938 | 3246 | 4744 | 6108 | 7432 | 8694 | 10172 | 11661 | 13290 | 14849 | 16115 |
| 150 | 1939 | 3252 | 4745 | 6109 | 7433 | 8695 | 10173 | 11662 | 13291 | 14850 | 16116 |
| 151 | 1940 | 3253 | 4746 | 6110 | 7434 | 8696 | 10174 | 11663 | 13292 | 14851 | 16117 |
| 152 | 1941 | 3254 | 4747 | 6111 | 7435 | 8697 | 10175 | 11664 | 13293 | 14852 | 16118 |
| 179 | 1942 | 3255 | 4748 | 6112 | 7436 | 8698 | 10176 | 11665 | 13294 | 14868 | 16119 |
| 180 | 1943 | 3256 | 4749 | 6113 | 7437 | 8699 | 10177 | 11666 | 13295 | 14869 | 16120 |
| 181 | 1944 | 3257 | 4750 | 6114 | 7438 | 8706 | 10178 | 11702 | 13296 | 14870 | 16123 |
| 182 | 1945 | 3258 | 4751 | 6115 | 7439 | 8707 | 10179 | 11703 | 13297 | 14871 | 16124 |
| 183 | 1946 | 3259 | 4752 | 6116 | 7440 | 8708 | 10180 | 11704 | 13298 | 14872 | 16125 |
| 184 | 1947 | 3293 | 4753 | 6117 | 7441 | 8709 | 10181 | 11705 | 13299 | 14873 | 16126 |
| 185 | 1948 | 3294 | 4754 | 6118 | 7442 | 8710 | 10192 | 11706 | 13300 | 14874 | 16127 |
| 186 | 1949 | 3295 | 4755 | 6119 | 7443 | 8711 | 10193 | 11707 | 13301 | 14875 | 16128 |
| 187 | 1950 | 3296 | 4756 | 6120 | 7444 | 8712 | 10200 | 11708 | 13302 | 14876 | 16160 |
| 188 | 1951 | 3297 | 4757 | 6121 | 7445 | 8713 | 10201 | 11709 | 13303 | 14877 | 16161 |
| 189 | 1952 | 3298 | 4758 | 6135 | 7446 | 8714 | 10202 | 11710 | 13304 | 14878 | 16162 |
| 190 | 1953 | 3299 | 4759 | 6136 | 7447 | 8715 | 10203 | 11711 | 13328 | 14879 | 16163 |
| 191 | 1954 | 3300 | 4760 | 6147 | 7448 | 8716 | 10204 | 11712 | 13329 | 14880 | 16164 |
| 192 | 1955 | 3301 | 4761 | 6148 | 7449 | 8717 | 10205 | 11713 | 13330 | 14881 | 16165 |
| 193 | 1956 | 3302 | 4762 | 6149 | 7450 | 8718 | 10206 | 11714 | 13331 | 14882 | 16166 |
| 194 | 1957 | 3303 | 4768 | 6150 | 7451 | 8719 | 10207 | 11715 | 13332 | 14883 | 16167 |
| 204 | 1958 | 3304 | 4769 | 6151 | 7452 | 8720 | 10208 | 11716 | 13333 | 14884 | 16168 |
| 205 | 1990 | 3305 | 4770 | 6152 | 7453 | 8725 | 10209 | 11717 | 13334 | 14885 | 16169 |
| 206 | 1991 | 3306 | 4771 | 6153 | 7454 | 8733 | 10210 | 11718 | 13335 | 14886 | 16170 |
| 207 | 1992 | 3307 | 4772 | 6154 | 7455 | 8734 | 10211 | 11719 | 13336 | 14887 | 16171 |
| 208 | 1993 | 3308 | 4773 | 6155 | 7456 | 8735 | 10212 | 11720 | 13337 | 14888 | 16172 |
| 209 | 1994 | 3309 | 4774 | 6156 | 7457 | 8736 | 10213 | 11721 | 13338 | 14889 | 16173 |
| 210 | 1995 | 3310 | 4775 | 6157 | 7458 | 8737 | 10214 | 11722 | 13345 | 14890 | 16174 |
| 219 | 1996 | 3321 | 4776 | 6158 | 7459 | 8738 | 10215 | 11723 | 13346 | 14891 | 16175 |
| 220 | 1997 | 3322 | 4777 | 6159 | 7460 | 8739 | 10216 | 11738 | 13347 | 14892 | 16176 |
| 221 | 1998 | 3323 | 4778 | 6160 | 7461 | 8740 | 10217 | 11739 | 13385 | 14893 | 16177 |
| 222 | 1999 | 3324 | 4779 | 6161 | 7462 | 8741 | 10218 | 11740 | 13386 | 14894 | 16178 |
| 223 | 2000 | 3325 | 4780 | 6162 | 7463 | 8742 | 10219 | 11741 | 13387 | 14895 | 16179 |
| 224 | 2001 | 3326 | 4781 | 6166 | 7473 | 8751 | 10220 | 11742 | 13388 | 14896 | 16180 |
| 225 | 2002 | 3327 | 4782 | 6167 | 7474 | 8752 | 10221 | 11743 | 13389 | 14897 | 16181 |
| 226 | 2003 | 3328 | 4783 | 6174 | 7475 | 8753 | 10231 | 11744 | 13395 | 14898 | 16182 |
| 227 | 2004 | 3329 | 4784 | 6175 | 7476 | 8754 | 10232 | 11745 | 13396 | 14899 | 16189 |
| 228 | 2005 | 3330 | 4785 | 6176 | 7477 | 8755 | 10233 | 11746 | 13397 | 14900 | 16190 |
| 229 | 2006 | 3331 | 4786 | 6177 | 7494 | 8768 | 10234 | 11747 | 13398 | 14901 | 16191 |
| 230 | 2007 | 3332 | 4787 | 6178 | 7495 | 8769 | 10235 | 11748 | 13399 | 14902 | 16192 |
| 231 | 2008 | 3333 | 4788 | 6179 | 7496 | 8770 | 10236 | 11749 | 13400 | 14903 | 16193 |
| 232 | 2009 | 3334 | 4789 | 6180 | 7497 | 8771 | 10237 | 11750 | 13401 | 14904 | 16252 |
| 233 | 2010 | 3335 | 4790 | 6181 | 7498 | 8772 | 10238 | 11751 | 13402 | 14905 | 16253 |
| 234 | 2011 | 3336 | 4791 | 6182 | 7499 | 8773 | 10239 | 11752 | 13403 | 14906 | 16254 |
| 235 | 2012 | 3337 | 4792 | 6183 | 7500 | 8774 | 10240 | 11753 | 13404 | 14907 | 16255 |
| 236 | 2013 | 3338 | 4793 | 6184 | 7501 | 8775 | 10253 | 11754 | 13405 | 14908 | 16256 |
| 237 | 2014 | 3339 | 4794 | 6195 | 7502 | 8776 | 10254 | 11755 | 13406 | 14909 | 16257 |
| 238 | 2015 | 3340 | 4795 | 6196 | 7503 | 8777 | 10255 | 11756 | 13407 | 14910 | 16258 |
| 239 | 2019 | 3341 | 4796 | 6197 | 7504 | 8778 | 10256 | 11757 | 13408 | 14911 | 16259 |
| 240 | 2020 | 3342 | 4797 | 6198 | 7538 | 8779 | 10257 | 11758 | 13409 | 14912 | 16260 |
| 241 | 2021 | 3343 | 4798 | 6199 | 7539 | 8780 | 10258 | 11759 | 13410 | 14913 | 16261 |
| 266 | 2022 | 3344 | 4799 | 6200 | 7540 | 8781 | 10259 | 11760 | 13411 | 14914 | 16262 |
| 267 | 2023 | 3354 | 4800 | 6201 | 7541 | 8782 | 10260 | 11761 | 13412 | 14915 | 16263 |
| 268 | 2024 | 3355 | 4801 | 6202 | 7542 | 8783 | 10261 | 11762 | 13413 | 14916 | 16264 |
| 269 | 2025 | 3356 | 4802 | 6203 | 7543 | 8784 | 10265 | 11763 | 13414 | 14917 | 16265 |
| 270 | 2026 | 3357 | 4803 | 6204 | 7544 | 8785 | 10266 | 11764 | 13415 | 14918 | 16266 |
| 271 | 2027 | 3358 | 4804 | 6205 | 7545 | 8786 | 10267 | 11765 | 13416 | 14919 | 16270 |
| 272 | 2028 | 3359 | 4805 | 6206 | 7546 | 8788 | 10268 | 11766 | 13417 | 14920 | 16271 |
| 292 | 2029 | 3360 | 4806 | 6207 | 7547 | 8789 | 10269 | 11767 | 13418 | 14921 | 16272 |
| 293 | 2030 | 3361 | 4807 | 6208 | 7548 | 8790 | 10270 | 11768 | 13419 | 14922 | 16273 |
| 294 | 2031 | 3362 | 4808 | 6209 | 7549 | 8791 | 10271 | 11769 | 13420 | 14923 | 16274 |
| 340 | 2032 | 3363 | 4809 | 6210 | 7550 | 8792 | 10272 | 11770 | 13421 | 14924 | 16275 |
| 341 | 2033 | 3364 | 4819 | 6211 | 7551 | 8793 | 10273 | 11771 | 13422 | 14925 | 16276 |
| 342 | 2034 | 3365 | 4820 | 6212 | 7552 | 8794 | 10274 | 11772 | 13423 | 14926 | 16277 |
| 344 | 2035 | 3366 | 4821 | 6213 | 7553 | 8795 | 10275 | 11773 | 13424 | 14927 | 16278 |
| 345 | 2036 | 3367 | 4822 | 6214 | 7554 | 8796 | 10276 | 11774 | 13425 | 14928 | 16279 |
| 346 | 2037 | 3368 | 4823 | 6215 | 7555 | 8797 | 10277 | 11775 | 13436 | 14929 | 16280 |
| 347 | 2038 | 3369 | 4824 | 6216 | 7556 | 8798 | 10278 | 11776 | 13437 | 14930 | 16281 |
| 348 | 2039 | 3370 | 4825 | 6217 | 7557 | 8799 | 10283 | 11777 | 13438 | 14931 | 16282 |
| 349 | 2040 | 3371 | 4826 | 6218 | 7558 | 8800 | 10292 | 11778 | 13439 | 14932 | 16283 |
| 350 | 2041 | 3372 | 4827 | 6219 | 7559 | 8801 | 10293 | 11779 | 13440 | 14933 | 16284 |
| 351 | 2042 | 3373 | 4828 | 6220 | 7560 | 8802 | 10294 | 11780 | 13441 | 14934 | 16285 |
| 352 | 2043 | 3374 | 4829 | 6221 | 7561 | 8803 | 10295 | 11781 | 13442 | 14935 | 16295 |
| 353 | 2044 | 3375 | 4830 | 6222 | 7562 | 8804 | 10296 | 11782 | 13443 | 14936 | 16296 |
| 354 | 2055 | 3376 | 4831 | 6223 | 7563 | 8805 | 10297 | 11783 | 13444 | 14937 | 16297 |
| 355 | 2056 | 3377 | 4832 | 6224 | 7564 | 8806 | 10305 | 11784 | 13445 | 14938 | 16298 |
| 356 | 2057 | 3378 | 4833 | 6225 | 7565 | 8807 | 10306 | 11785 | 13454 | 14939 | 16299 |
| 357 | 2058 | 3379 | 4834 | 6226 | 7566 | 8808 | 10307 | 11786 | 13455 | 14940 | 16300 |
| 358 | 2059 | 3380 | 4835 | 6227 | 7567 | 8809 | 10308 | 11787 | 13456 | 14941 | 16306 |
| 359 | 2060 | 3381 | 4836 | 6228 | 7568 | 8810 | 10309 | 11788 | 13457 | 14942 | 16307 |
| 360 | 2061 | 3382 | 4837 | 6229 | 7569 | 8811 | 10310 | 11789 | 13458 | 14943 | 16308 |
| 361 | 2068 | 3383 | 4864 | 6230 | 7570 | 8812 | 10311 | 11790 | 13459 | 14944 | 16309 |
| 362 | 2069 | 3384 | 4865 | 6231 | 7571 | 8813 | 10312 | 11791 | 13460 | 14945 | 16310 |
| 363 | 2070 | 3385 | 4866 | 6232 | 7572 | 8814 | 10313 | 11802 | 13461 | 14946 | 16311 |
| 364 | 2071 | 3386 | 4867 | 6233 | 7573 | 8815 | 10314 | 11803 | 13462 | 14947 | 16312 |
| 365 | 2072 | 3387 | 4873 | 6234 | 7574 | 8816 | 10315 | 11804 | 13463 | 14948 | 16313 |
| 370 | 2075 | 3388 | 4874 | 6235 | 7575 | 8818 | 10316 | 11811 | 13464 | 14949 | 16314 |
| 371 | 2076 | 3389 | 4875 | 6236 | 7576 | 8819 | 10317 | 11812 | 13465 | 14950 | 16315 |
| 372 | 2077 | 3390 | 4876 | 6237 | 7577 | 8820 | 10318 | 11813 | 13466 | 14951 | 16316 |
| 373 | 2078 | 3391 | 4877 | 6238 | 7578 | 8821 | 10319 | 11814 | 13467 | 14952 | 16317 |
| 374 | 2079 | 3392 | 4878 | 6239 | 7579 | 8832 | 10320 | 11815 | 13501 | 14953 | 16318 |
| 375 | 2080 | 3393 | 4879 | 6240 | 7580 | 8835 | 10321 | 11816 | 13502 | 14954 | 16319 |
| 376 | 2081 | 3394 | 4880 | 6242 | 7581 | 8836 | 10322 | 11817 | 13503 | 14955 | 16320 |
| 377 | 2082 | 3395 | 4881 | 6243 | 7582 | 8837 | 10323 | 11818 | 13504 | 14956 | 16321 |
| 378 | 2083 | 3396 | 4882 | 6244 | 7595 | 8838 | 10324 | 11819 | 13505 | 14957 | 16322 |
| 379 | 2084 | 3397 | 4883 | 6245 | 7596 | 8839 | 10325 | 11820 | 13506 | 14958 | 16323 |
| 380 | 2085 | 3398 | 4884 | 6246 | 7607 | 8840 | 10326 | 11821 | 13507 | 14959 | 16324 |
| 381 | 2086 | 3399 | 4885 | 6247 | 7608 | 8841 | 10329 | 11822 | 13508 | 14960 | 16325 |
| 382 | 2087 | 3400 | 4886 | 6248 | 7609 | 8842 | 10330 | 11823 | 13509 | 14961 | 16326 |
| 383 | 2088 | 3401 | 4887 | 6249 | 7610 | 8843 | 10331 | 11824 | 13510 | 14962 | 16327 |
| 384 | 2089 | 3402 | 4888 | 6250 | 7611 | 8844 | 10332 | 11825 | 13511 | 14963 | 16328 |
| 385 | 2090 | 3403 | 4889 | 6251 | 7612 | 8845 | 10333 | 11826 | 13512 | 14964 | 16329 |
| 386 | 2091 | 3404 | 4890 | 6252 | 7613 | 8846 | 10334 | 11829 | 13513 | 14965 | 16330 |
| 387 | 2092 | 3405 | 4891 | 6253 | 7614 | 8847 | 10335 | 11830 | 13514 | 14966 | 16331 |
| 388 | 2093 | 3406 | 4892 | 6254 | 7615 | 8848 | 10350 | 11831 | 13515 | 14967 | 16332 |
| 389 | 2094 | 3407 | 4893 | 6255 | 7623 | 8849 | 10351 | 11832 | 13516 | 14982 | 16333 |
| 390 | 2095 | 3408 | 4894 | 6256 | 7624 | 8850 | 10352 | 11833 | 13517 | 14983 | 16334 |
| 391 | 2105 | 3410 | 4895 | 6257 | 7625 | 8851 | 10353 | 11834 | 13518 | 14984 | 16335 |
| 392 | 2106 | 3411 | 4896 | 6258 | 7626 | 8852 | 10354 | 11835 | 13519 | 14985 | 16336 |
| 393 | 2107 | 3412 | 4897 | 6259 | 7627 | 8853 | 10355 | 11836 | 13520 | 14986 | 16361 |
| 394 | 2108 | 3413 | 4898 | 6260 | 7628 | 8854 | 10356 | 11837 | 13521 | 14987 | 16362 |
| 395 | 2109 | 3414 | 4899 | 6261 | 7629 | 8855 | 10357 | 11838 | 13522 | 14988 | 16363 |
| 396 | 2110 | 3415 | 4900 | 6262 | 7630 | 8881 | 10358 | 11839 | 13523 | 14989 | 16364 |
| 397 | 2111 | 3416 | 4901 | 6263 | 7631 | 8882 | 10359 | 11840 | 13524 | 14990 | 16365 |
| 398 | 2112 | 3417 | 4902 | 6264 | 7632 | 8883 | 10360 | 11841 | 13525 | 14991 | 16366 |
| 399 | 2113 | 3418 | 4903 | 6265 | 7633 | 8884 | 10361 | 11842 | 13526 | 14992 | 16371 |
| 400 | 2114 | 3419 | 4904 | 6266 | 7634 | 8885 | 10362 | 11843 | 13527 | 14993 | 16372 |
| 401 | 2115 | 3420 | 4905 | 6267 | 7635 | 8886 | 10363 | 11844 | 13528 | 14994 | 16373 |
| 402 | 2116 | 3421 | 4906 | 6268 | 7636 | 8887 | 10364 | 11851 | 13529 | 14995 | 16374 |
| 403 | 2117 | 3422 | 4907 | 6269 | 7637 | 8888 | 10365 | 11852 | 13530 | 14996 | 16375 |
| 405 | 2118 | 3423 | 4908 | 6270 | 7638 | 8889 | 10366 | 11853 | 13531 | 14997 | 16376 |
| 406 | 2119 | 3424 | 4909 | 6271 | 7639 | 8890 | 10367 | 11854 | 13532 | 14998 | 16377 |
| 407 | 2120 | 3439 | 4928 | 6272 | 7640 | 8891 | 10368 | 11855 | 13533 | 14999 | 16378 |
| 408 | 2121 | 3440 | 4929 | 6273 | 7641 | 8892 | 10369 | 11863 | 13534 | 15000 | 16379 |
| 409 | 2122 | 3441 | 4930 | 6274 | 7643 | 8893 | 10370 | 11864 | 13546 | 15001 | 16380 |
| 410 | 2123 | 3442 | 4931 | 6275 | 7644 | 8894 | 10371 | 11865 | 13547 | 15002 | 16381 |
| 411 | 2124 | 3443 | 4932 | 6276 | 7645 | 8895 | 10372 | 11866 | 13548 | 15003 | 16385 |
| 412 | 2125 | 3444 | 4933 | 6277 | 7646 | 8896 | 10373 | 11867 | 13549 | 15004 | 16386 |
| 413 | 2126 | 3445 | 4934 | 6278 | 7647 | 8897 | 10374 | 11868 | 13550 | 15005 | 16387 |
| 414 | 2127 | 3446 | 4935 | 6279 | 7648 | 8898 | 10375 | 11869 | 13551 | 15006 | 16388 |
| 415 | 2128 | 3447 | 4936 | 6280 | 7649 | 8899 | 10376 | 11870 | 13552 | 15007 | 16393 |
| 431 | 2149 | 3448 | 4937 | 6281 | 7650 | 8900 | 10377 | 11871 | 13553 | 15008 | 16394 |
| 432 | 2150 | 3449 | 4938 | 6282 | 7651 | 8901 | 10378 | 11872 | 13554 | 15009 | 16395 |
| 433 | 2151 | 3450 | 4939 | 6283 | 7652 | 8902 | 10379 | 11873 | 13555 | 15010 | 16396 |
| 434 | 2152 | 3451 | 4940 | 6284 | 7653 | 8903 | 10380 | 11874 | 13556 | 15011 | 16408 |
| 435 | 2153 | 3452 | 4941 | 6285 | 7654 | 8904 | 10381 | 11875 | 13557 | 15012 | 16409 |
| 436 | 2154 | 3453 | 4942 | 6286 | 7655 | 8905 | 10382 | 11876 | 13558 | 15013 | 16453 |
| 437 | 2155 | 3454 | 4943 | 6287 | 7656 | 8906 | 10383 | 11877 | 13559 | 15026 | 16454 |
| 438 | 2156 | 3455 | 4944 | 6288 | 7657 | 8914 | 10384 | 11878 | 13560 | 15027 | 16455 |
| 439 | 2157 | 3456 | 4951 | 6289 | 7658 | 8915 | 10385 | 11879 | 13561 | 15028 | 16456 |
| 440 | 2158 | 3460 | 4952 | 6290 | 7659 | 8916 | 10386 | 11880 | 13562 | 15029 | 16457 |
| 441 | 2159 | 3461 | 4953 | 6291 | 7660 | 8917 | 10387 | 11881 | 13563 | 15030 | 16461 |
| 442 | 2160 | 3462 | 4954 | 6292 | 7661 | 8918 | 10388 | 11882 | 13564 | 15031 | 16462 |
| 443 | 2161 | 3463 | 4955 | 6293 | 7662 | 8919 | 10389 | 11883 | 13565 | 15032 | 16463 |
| 444 | 2162 | 3464 | 4956 | 6294 | 7663 | 8920 | 10390 | 11884 | 13566 | 15033 | 16464 |
| 445 | 2163 | 3465 | 4957 | 6295 | 7664 | 8926 | 10391 | 11885 | 13567 | 15034 | 16465 |
| 446 | 2164 | 3466 | 4958 | 6296 | 7665 | 8927 | 10392 | 11886 | 13568 | 15035 | 16466 |
| 447 | 2165 | 3467 | 4959 | 6297 | 7666 | 8928 | 10393 | 11887 | 13569 | 15036 | 16467 |
| 448 | 2166 | 3468 | 4960 | 6307 | 7667 | 8929 | 10394 | 11888 | 13570 | 15037 | 16468 |
| 449 | 2167 | 3469 | 4961 | 6308 | 7668 | 8930 | 10395 | 11889 | 13571 | 15038 | 16469 |
| 450 | 2187 | 3470 | 4962 | 6309 | 7669 | 8931 | 10396 | 11890 | 13572 | 15039 | 16470 |
| 451 | 2188 | 3471 | 4963 | 6323 | 7670 | 8932 | 10397 | 11891 | 13573 | 15049 | 16471 |
| 452 | 2189 | 3480 | 4964 | 6324 | 7671 | 8933 | 10398 | 11892 | 13574 | 15050 | 16472 |
| 453 | 2190 | 3481 | 4965 | 6325 | 7672 | 8934 | 10399 | 11893 | 13575 | 15051 | 16473 |
| 454 | 2191 | 3482 | 4966 | 6326 | 7673 | 8935 | 10400 | 11894 | 13576 | 15052 | 16474 |
| 455 | 2192 | 3483 | 4967 | 6327 | 7674 | 8936 | 10401 | 11900 | 13577 | 15053 | 16475 |
| 456 | 2193 | 3484 | 4968 | 6339 | 7675 | 8949 | 10402 | 11901 | 13578 | 15054 | 16476 |
| 457 | 2194 | 3485 | 4969 | 6340 | 7676 | 8950 | 10403 | 11902 | 13579 | 15055 | 16477 |
| 458 | 2195 | 3497 | 4970 | 6341 | 7677 | 8951 | 10404 | 11903 | 13580 | 15056 | 16478 |
| 459 | 2196 | 3498 | 4971 | 6342 | 7678 | 8952 | 10405 | 11904 | 13581 | 15057 | 16479 |
| 460 | 2197 | 3499 | 4972 | 6343 | 7679 | 8953 | 10406 | 11939 | 13587 | 15058 | 16480 |
| 461 | 2198 | 3500 | 4973 | 6344 | 7680 | 8954 | 10407 | 11940 | 13588 | 15059 | 16481 |
| 462 | 2199 | 3501 | 4974 | 6345 | 7681 | 8955 | 10408 | 11941 | 13589 | 15060 | 16482 |
| 478 | 2200 | 3502 | 4975 | 6346 | 7682 | 8956 | 10409 | 11942 | 13590 | 15061 | 16483 |
| 479 | 2201 | 3503 | 4976 | 6347 | 7683 | 8957 | 10410 | 11943 | 13591 | 15062 | 16484 |
| 480 | 2202 | 3504 | 4977 | 6348 | 7684 | 8958 | 10411 | 11944 | 13592 | 15063 | 16485 |
| 481 | 2203 | 3505 | 4979 | 6349 | 7685 | 8959 | 10412 | 11945 | 13593 | 15064 | 16486 |
| 482 | 2204 | 3506 | 4980 | 6350 | 7686 | 8960 | 10413 | 11946 | 13594 | 15065 | 16487 |
| 483 | 2205 | 3507 | 4981 | 6351 | 7687 | 8961 | 10414 | 11975 | 13595 | 15066 | 16488 |
| 490 | 2206 | 3508 | 4982 | 6352 | 7688 | 8972 | 10415 | 11976 | 13596 | 15067 | 16489 |
| 491 | 2207 | 3509 | 4983 | 6353 | 7689 | 8979 | 10416 | 11977 | 13597 | 15068 | 16490 |
| 492 | 2208 | 3510 | 4984 | 6354 | 7690 | 8980 | 10420 | 11978 | 13601 | 15069 | 16491 |
| 493 | 2209 | 3511 | 4985 | 6355 | 7707 | 8981 | 10421 | 11979 | 13602 | 15070 | 16492 |
| 494 | 2210 | 3512 | 4986 | 6356 | 7708 | 8982 | 10422 | 11980 | 13603 | 15071 | 16493 |
| 495 | 2211 | 3513 | 4987 | 6357 | 7709 | 8983 | 10423 | 11981 | 13604 | 15072 | 16494 |
| 496 | 2212 | 3514 | 4988 | 6358 | 7710 | 8990 | 10424 | 11982 | 13605 | 15073 | 16495 |
| 497 | 2213 | 3518 | 4989 | 6361 | 7714 | 8991 | 10425 | 11988 | 13615 | 15074 | 16496 |
| 498 | 2214 | 3519 | 4990 | 6362 | 7715 | 8992 | 10426 | 11989 | 13616 | 15075 | 16497 |
| 499 | 2215 | 3520 | 4991 | 6363 | 7716 | 8993 | 10427 | 11990 | 13617 | 15076 | 16498 |
| 500 | 2216 | 3521 | 4992 | 6364 | 7717 | 8994 | 10428 | 11991 | 13618 | 15077 | 16504 |
| 501 | 2217 | 3524 | 4993 | 6365 | 7718 | 8995 | 10429 | 11992 | 13619 | 15078 | 16505 |
| 502 | 2222 | 3525 | 4994 | 6367 | 7719 | 8996 | 10444 | 11993 | 13626 | 15079 | 16506 |
| 503 | 2223 | 3526 | 4999 | 6368 | 7720 | 8997 | 10445 | 11994 | 13627 | 15080 | 16507 |
| 504 | 2224 | 3527 | 5000 | 6369 | 7721 | 8998 | 10446 | 11995 | 13628 | 15081 | 16508 |
| 505 | 2225 | 3528 | 5001 | 6370 | 7722 | 8999 | 10447 | 11996 | 13629 | 15082 | 16509 |
| 514 | 2226 | 3529 | 5002 | 6371 | 7723 | 9018 | 10448 | 11997 | 13633 | 15083 | 16510 |
| 515 | 2227 | 3530 | 5003 | 6372 | 7724 | 9019 | 10455 | 12002 | 13634 | 15084 | 16511 |
| 516 | 2239 | 3531 | 5004 | 6373 | 7725 | 9026 | 10456 | 12003 | 13635 | 15085 | 16512 |
| 517 | 2240 | 3553 | 5005 | 6374 | 7726 | 9036 | 10457 | 12004 | 13636 | 15086 | 16513 |
| 518 | 2241 | 3558 | 5006 | 6375 | 7727 | 9037 | 10458 | 12005 | 13637 | 15087 | 16514 |
| 519 | 2242 | 3559 | 5007 | 6376 | 7728 | 9043 | 10459 | 12006 | 13638 | 15088 | 16515 |
| 520 | 2243 | 3563 | 5008 | 6377 | 7729 | 9044 | 10460 | 12010 | 13639 | 15089 | 16516 |
| 521 | 2244 | 3564 | 5009 | 6378 | 7730 | 9045 | 10461 | 12011 | 13640 | 15090 | 16517 |
| 522 | 2245 | 3565 | 5010 | 6397 | 7731 | 9054 | 10462 | 12012 | 13641 | 15132 | 16518 |
| 534 | 2246 | 3566 | 5011 | 6398 | 7732 | 9055 | 10463 | 12017 | 13642 | 15133 | 16519 |
| 535 | 2247 | 3567 | 5012 | 6399 | 7733 | 9056 | 10464 | 12018 | 13648 | 15134 | 16520 |
| 536 | 2248 | 3568 | 5013 | 6400 | 7734 | 9057 | 10465 | 12034 | 13649 | 15135 | 16521 |
| 537 | 2249 | 3569 | 5014 | 6401 | 7735 | 9058 | 10466 | 12061 | 13650 | 15136 | 16525 |
| 538 | 2250 | 3570 | 5015 | 6402 | 7736 | 9059 | 10467 | 12062 | 13651 | 15137 | 16526 |
| 539 | 2253 | 3581 | 5016 | 6403 | 7737 | 9083 | 10468 | 12063 | 13652 | 15138 | 16527 |
| 540 | 2254 | 3582 | 5017 | 6404 | 7738 | 9084 | 10469 | 12064 | 13653 | 15139 | 16528 |
| 541 | 2255 | 3583 | 5031 | 6405 | 7739 | 9085 | 10470 | 12065 | 13654 | 15140 | 16529 |
| 542 | 2256 | 3584 | 5032 | 6406 | 7740 | 9086 | 10471 | 12066 | 13655 | 15141 | 16530 |
| 543 | 2257 | 3585 | 5033 | 6407 | 7741 | 9087 | 10472 | 12067 | 13656 | 15142 | 16531 |
| 544 | 2258 | 3586 | 5034 | 6408 | 7742 | 9088 | 10473 | 12068 | 13657 | 15143 | 16532 |
| 545 | 2259 | 3587 | 5035 | 6412 | 7743 | 9089 | 10474 | 12069 | 13658 | 15144 | 16533 |
| 546 | 2260 | 3588 | 5036 | 6413 | 7744 | 9090 | 10475 | 12070 | 13659 | 15145 | 16537 |
| 547 | 2261 | 3589 | 5037 | 6414 | 7745 | 9091 | 10476 | 12071 | 13660 | 15146 | 16538 |
| 548 | 2262 | 3590 | 5040 | 6415 | 7746 | 9092 | 10477 | 12072 | 13661 | 15147 | 16539 |
| 549 | 2263 | 3591 | 5041 | 6416 | 7747 | 9093 | 10478 | 12073 | 13662 | 15148 | 16540 |
| 550 | 2264 | 3592 | 5042 | 6417 | 7748 | 9143 | 10479 | 12074 | 13663 | 15149 | 16541 |
| 551 | 2265 | 3593 | 5048 | 6418 | 7749 | 9144 | 10480 | 12075 | 13664 | 15150 | 16542 |
| 552 | 2272 | 3594 | 5049 | 6419 | 7750 | 9151 | 10481 | 12076 | 13665 | 15151 | 16543 |
| 553 | 2273 | 3595 | 5050 | 6420 | 7751 | 9152 | 10482 | 12077 | 13666 | 15157 | 16544 |
| 554 | 2274 | 3596 | 5051 | 6421 | 7752 | 9153 | 10483 | 12078 | 13667 | 15158 | 16545 |
| 555 | 2275 | 3597 | 5052 | 6422 | 7753 | 9154 | 10484 | 12079 | 13668 | 15159 | 16546 |
| 556 | 2276 | 3598 | 5053 | 6423 | 7754 | 9155 | 10485 | 12080 | 13669 | 15160 | 16547 |
| 557 | 2277 | 3599 | 5054 | 6424 | 7755 | 9156 | 10486 | 12081 | 13670 | 15161 | 16548 |
| 558 | 2278 | 3600 | 5055 | 6425 | 7756 | 9157 | 10487 | 12085 | 13671 | 15162 | 16549 |
| 559 | 2279 | 3601 | 5056 | 6426 | 7757 | 9158 | 10488 | 12086 | 13672 | 15163 | 16550 |
| 573 | 2282 | 3602 | 5057 | 6427 | 7758 | 9159 | 10489 | 12087 | 13673 | 15164 | 16551 |
| 574 | 2283 | 3603 | 5058 | 6428 | 7759 | 9160 | 10490 | 12088 | 13674 | 15165 | 16552 |
| 575 | 2284 | 3604 | 5059 | 6429 | 7760 | 9161 | 10491 | 12098 | 13675 | 15166 | 16553 |
| 576 | 2285 | 3605 | 5060 | 6430 | 7761 | 9162 | 10492 | 12099 | 13676 | 15167 | 16554 |
| 577 | 2286 | 3606 | 5061 | 6431 | 7762 | 9163 | 10493 | 12105 | 13677 | 15168 | 16555 |
| 578 | 2287 | 3607 | 5062 | 6432 | 7763 | 9164 | 10494 | 12106 | 13678 | 15169 | 16556 |
| 579 | 2288 | 3608 | 5063 | 6433 | 7764 | 9165 | 10502 | 12107 | 13679 | 15170 | 16557 |
| 580 | 2289 | 3609 | 5064 | 6434 | 7765 | 9166 | 10503 | 12108 | 13680 | 15171 | 16558 |
| 590 | 2290 | 3610 | 5065 | 6435 | 7766 | 9169 | 10504 | 12109 | 13681 | 15172 | 16559 |
| 591 | 2291 | 3611 | 5081 | 6436 | 7767 | 9170 | 10505 | 12110 | 13691 | 15173 | 16560 |
| 592 | 2292 | 3612 | 5082 | 6437 | 7768 | 9171 | 10506 | 12111 | 13692 | 15174 | 16561 |
| 593 | 2293 | 3613 | 5083 | 6438 | 7769 | 9172 | 10507 | 12112 | 13693 | 15175 | 16562 |
| 594 | 2294 | 3614 | 5084 | 6439 | 7770 | 9173 | 10508 | 12113 | 13694 | 15176 | 16577 |
| 595 | 2295 | 3615 | 5085 | 6440 | 7771 | 9183 | 10509 | 12114 | 13695 | 15177 | 16578 |
| 596 | 2296 | 3616 | 5086 | 6441 | 7772 | 9184 | 10510 | 12123 | 13696 | 15178 | 16579 |
| 597 | 2297 | 3617 | 5088 | 6442 | 7773 | 9185 | 10511 | 12124 | 13697 | 15179 | 16580 |
| 598 | 2298 | 3618 | 5089 | 6443 | 7774 | 9186 | 10512 | 12177 | 13698 | 15180 | 16581 |
| 599 | 2299 | 3619 | 5090 | 6444 | 7775 | 9187 | 10513 | 12178 | 13699 | 15181 | 16582 |
| 600 | 2300 | 3620 | 5105 | 6445 | 7812 | 9188 | 10518 | 12195 | 13700 | 15182 | 16583 |
| 601 | 2329 | 3621 | 5106 | 6446 | 7813 | 9189 | 10519 | 12196 | 13701 | 15183 | 16584 |
| 614 | 2330 | 3622 | 5107 | 6479 | 7814 | 9190 | 10520 | 12197 | 13702 | 15184 | 16585 |
| 615 | 2331 | 3623 | 5108 | 6480 | 7815 | 9191 | 10521 | 12198 | 13703 | 15185 | 16586 |
| 616 | 2332 | 3624 | 5109 | 6481 | 7816 | 9192 | 10522 | 12199 | 13704 | 15186 | 16587 |
| 617 | 2333 | 3625 | 5110 | 6482 | 7817 | 9193 | 10523 | 12211 | 13705 | 15187 | 16588 |
| 618 | 2334 | 3626 | 5111 | 6497 | 7818 | 9194 | 10524 | 12212 | 13712 | 15188 | 16589 |
| 619 | 2335 | 3627 | 5112 | 6498 | 7819 | 9195 | 10525 | 12213 | 13713 | 15189 | 16590 |
| 640 | 2336 | 3628 | 5113 | 6499 | 7820 | 9196 | 10526 | 12214 | 13714 | 15190 | 16591 |
| 641 | 2337 | 3629 | 5114 | 6500 | 7821 | 9197 | 10527 | 12215 | 13715 | 15191 | 16592 |
| 642 | 2338 | 3630 | 5115 | 6501 | 7822 | 9198 | 10528 | 12216 | 13716 | 15192 | 16593 |
| 643 | 2339 | 3631 | 5116 | 6502 | 7823 | 9199 | 10535 | 12217 | 13717 | 15193 | 16594 |
| 644 | 2340 | 3632 | 5117 | 6515 | 7824 | 9200 | 10536 | 12218 | 13718 | 15194 | 16595 |
| 645 | 2341 | 3633 | 5118 | 6516 | 7825 | 9201 | 10537 | 12219 | 13752 | 15195 | 16596 |
| 646 | 2342 | 3638 | 5119 | 6517 | 7826 | 9202 | 10538 | 12220 | 13753 | 15196 | 16597 |
| 647 | 2343 | 3639 | 5120 | 6518 | 7827 | 9203 | 10539 | 12221 | 13754 | 15197 | 16602 |
| 648 | 2344 | 3640 | 5121 | 6519 | 7828 | 9204 | 10540 | 12222 | 13755 | 15198 | 16607 |
| 649 | 2345 | 3641 | 5122 | 6520 | 7829 | 9205 | 10541 | 12223 | 13756 | 15199 | 16608 |
| 650 | 2346 | 3642 | 5123 | 6521 | 7830 | 9206 | 10542 | 12224 | 13757 | 15207 | 16609 |
| 651 | 2347 | 3643 | 5124 | 6522 | 7831 | 9207 | 10543 | 12225 | 13758 | 15208 | 16610 |
| 652 | 2348 | 3644 | 5125 | 6552 | 7832 | 9218 | 10544 | 12226 | 13759 | 15209 | 16611 |
| 653 | 2349 | 3669 | 5126 | 6553 | 7833 | 9219 | 10545 | 12227 | 13760 | 15210 | 16612 |
| 654 | 2350 | 3670 | 5127 | 6554 | 7834 | 9237 | 10546 | 12228 | 13761 | 15211 | 16613 |
| 655 | 2351 | 3671 | 5128 | 6555 | 7835 | 9238 | 10547 | 12229 | 13762 | 15212 | 16614 |
| 656 | 2352 | 3672 | 5129 | 6556 | 7836 | 9239 | 10548 | 12230 | 13763 | 15213 | 16615 |
| 657 | 2353 | 3673 | 5130 | 6557 | 7837 | 9240 | 10549 | 12231 | 13764 | 15214 | 16616 |
| 658 | 2359 | 3674 | 5131 | 6558 | 7838 | 9241 | 10550 | 12243 | 13765 | 15215 | 16617 |
| 659 | 2360 | 3675 | 5132 | 6559 | 7839 | 9242 | 10551 | 12244 | 13766 | 15216 | 16618 |
| 660 | 2361 | 3676 | 5133 | 6562 | 7840 | 9255 | 10552 | 12245 | 13767 | 15217 | 16619 |
| 661 | 2362 | 3677 | 5134 | 6563 | 7841 | 9256 | 10557 | 12246 | 13768 | 15218 | 16620 |
| 662 | 2363 | 3678 | 5135 | 6564 | 7842 | 9257 | 10558 | 12269 | 13769 | 15219 | 16621 |
| 663 | 2364 | 3679 | 5144 | 6565 | 7843 | 9258 | 10559 | 12270 | 13770 | 15220 | 16622 |
| 664 | 2365 | 3680 | 5145 | 6566 | 7844 | 9259 | 10560 | 12271 | 13771 | 15221 | 16623 |
| 665 | 2366 | 3681 | 5148 | 6567 | 7845 | 9260 | 10561 | 12272 | 13772 | 15222 | 16631 |
| 666 | 2389 | 3682 | 5149 | 6568 | 7846 | 9261 | 10562 | 12273 | 13773 | 15223 | 16632 |
| 667 | 2390 | 3683 | 5150 | 6592 | 7847 | 9262 | 10563 | 12274 | 13774 | 15224 | 16633 |
| 668 | 2391 | 3684 | 5151 | 6593 | 7848 | 9263 | 10564 | 12275 | 13775 | 15225 | 16634 |
| 669 | 2392 | 3685 | 5152 | 6594 | 7849 | 9264 | 10574 | 12276 | 13776 | 15226 | 16635 |
| 670 | 2393 | 3686 | 5153 | 6595 | 7850 | 9265 | 10575 | 12277 | 13777 | 15227 | 16636 |
| 671 | 2394 | 3687 | 5154 | 6596 | 7851 | 9266 | 10608 | 12278 | 13778 | 15228 | 16637 |
| 672 | 2395 | 3688 | 5155 | 6597 | 7852 | 9267 | 10609 | 12279 | 13779 | 15229 | 16638 |
| 673 | 2396 | 3689 | 5156 | 6598 | 7853 | 9268 | 10610 | 12280 | 13780 | 15230 | 16639 |
| 674 | 2397 | 3690 | 5157 | 6599 | 7854 | 9269 | 10611 | 12281 | 13781 | 15231 | 16640 |
| 677 | 2398 | 3691 | 5158 | 6600 | 7855 | 9270 | 10619 | 12282 | 13798 | 15232 | 16641 |
| 678 | 2399 | 3692 | 5159 | 6601 | 7856 | 9271 | 10648 | 12283 | 13799 | 15233 | 16661 |
| 682 | 2400 | 3693 | 5160 | 6602 | 7857 | 9272 | 10649 | 12284 | 13800 | 15234 | 16662 |
| 683 | 2401 | 3694 | 5173 | 6603 | 7858 | 9273 | 10650 | 12285 | 13801 | 15235 | 16663 |
| 684 | 2402 | 3695 | 5174 | 6604 | 7859 | 9274 | 10651 | 12286 | 13802 | 15236 | 16664 |
| 685 | 2403 | 3696 | 5175 | 6605 | 7860 | 9275 | 10652 | 12287 | 13803 | 15238 | 16665 |
| 686 | 2404 | 3697 | 5176 | 6606 | 7861 | 9276 | 10653 | 12288 | 13804 | 15239 | 16666 |
| 687 | 2405 | 3701 | 5177 | 6607 | 7862 | 9277 | 10654 | 12290 | 13805 | 15240 | 16667 |
| 688 | 2406 | 3702 | 5201 | 6608 | 7863 | 9278 | 10660 | 12291 | 13806 | 15246 | 16668 |
| 689 | 2407 | 3703 | 5202 | 6609 | 7864 | 9279 | 10661 | 12292 | 13807 | 15255 | 16669 |
| 690 | 2408 | 3704 | 5203 | 6610 | 7865 | 9280 | 10662 | 12293 | 13808 | 15256 | 16670 |
| 691 | 2409 | 3705 | 5204 | 6611 | 7866 | 9281 | 10663 | 12294 | 13809 | 15257 | 16671 |
| 692 | 2410 | 3706 | 5205 | 6612 | 7867 | 9282 | 10664 | 12295 | 13810 | 15258 | 16672 |
| 693 | 2411 | 3707 | 5206 | 6613 | 7868 | 9283 | 10665 | 12296 | 13811 | 15270 | 16673 |
| 703 | 2412 | 3708 | 5207 | 6614 | 7869 | 9284 | 10666 | 12297 | 13812 | 15271 | 16674 |
| 704 | 2413 | 3709 | 5227 | 6615 | 7870 | 9285 | 10684 | 12298 | 13813 | 15272 | 16675 |
| 705 | 2414 | 3710 | 5228 | 6616 | 7871 | 9286 | 10685 | 12299 | 13814 | 15273 | 16676 |
| 724 | 2415 | 3711 | 5229 | 6617 | 7872 | 9287 | 10686 | 12300 | 13815 | 15274 | 16694 |
| 725 | 2416 | 3712 | 5230 | 6618 | 7873 | 9288 | 10687 | 12301 | 13816 | 15275 | 16695 |
| 726 | 2417 | 3713 | 5249 | 6619 | 7874 | 9289 | 10688 | 12302 | 13817 | 15276 | 16696 |
| 727 | 2418 | 3714 | 5250 | 6620 | 7875 | 9290 | 10689 | 12318 | 13818 | 15277 | 16697 |
| 728 | 2419 | 3715 | 5252 | 6621 | 7876 | 9291 | 10690 | 12319 | 13819 | 15278 | 16698 |
| 735 | 2420 | 3716 | 5253 | 6622 | 7877 | 9292 | 10691 | 12320 | 13820 | 15279 | 16699 |
| 736 | 2421 | 3717 | 5254 | 6623 | 7878 | 9293 | 10692 | 12321 | 13821 | 15280 | 16700 |
| 737 | 2422 | 3718 | 5255 | 6624 | 7879 | 9294 | 10693 | 12322 | 13822 | 15281 | 16701 |
| 738 | 2423 | 3719 | 5256 | 6625 | 7880 | 9295 | 10694 | 12323 | 13823 | 15282 | 16702 |
| 739 | 2424 | 3720 | 5257 | 6626 | 7881 | 9296 | 10695 | 12324 | 13824 | 15283 | 16703 |
| 740 | 2425 | 3721 | 5258 | 6627 | 7882 | 9297 | 10696 | 12325 | 13825 | 15284 | 16704 |
| 776 | 2426 | 3722 | 5259 | 6628 | 7883 | 9298 | 10697 | 12326 | 13826 | 15285 | 16719 |
| 777 | 2427 | 3723 | 5260 | 6629 | 7884 | 9299 | 10698 | 12327 | 13827 | 15286 | 16726 |
| 778 | 2428 | 3724 | 5261 | 6630 | 7885 | 9300 | 10699 | 12328 | 13828 | 15287 | 16727 |
| 779 | 2429 | 3725 | 5262 | 6631 | 7886 | 9301 | 10706 | 12329 | 13829 | 15288 | 16728 |
| 780 | 2430 | 3726 | 5263 | 6632 | 7900 | 9302 | 10707 | 12330 | 13830 | 15289 | 16729 |
| 781 | 2431 | 3727 | 5264 | 6633 | 7901 | 9303 | 10708 | 12331 | 13831 | 15290 | 16730 |
| 782 | 2432 | 3728 | 5265 | 6634 | 7902 | 9304 | 10709 | 12332 | 13832 | 15291 | 16731 |
| 783 | 2433 | 3729 | 5266 | 6635 | 7903 | 9305 | 10710 | 12333 | 13833 | 15292 | 16732 |
| 784 | 2439 | 3730 | 5267 | 6636 | 7904 | 9306 | 10711 | 12334 | 13834 | 15293 | 16733 |
| 785 | 2440 | 3731 | 5268 | 6637 | 7905 | 9307 | 10712 | 12335 | 13837 | 15294 | 16734 |
| 786 | 2441 | 3732 | 5269 | 6638 | 7906 | 9312 | 10713 | 12341 | 13838 | 15295 | 16735 |
| 787 | 2442 | 3733 | 5270 | 6639 | 7907 | 9313 | 10714 | 12342 | 13839 | 15296 | 16736 |
| 788 | 2443 | 3739 | 5271 | 6640 | 7908 | 9314 | 10741 | 12343 | 13843 | 15297 | 16737 |
| 789 | 2444 | 3740 | 5272 | 6641 | 7909 | 9315 | 10742 | 12344 | 13844 | 15298 | 16738 |
| 790 | 2445 | 3741 | 5273 | 6642 | 7910 | 9316 | 10743 | 12370 | 13845 | 15299 | 16739 |
| 791 | 2446 | 3742 | 5274 | 6643 | 7911 | 9317 | 10744 | 12371 | 13846 | 15300 | 16740 |
| 792 | 2447 | 3743 | 5275 | 6644 | 7912 | 9318 | 10745 | 12372 | 13847 | 15306 | 16741 |
| 793 | 2448 | 3744 | 5286 | 6649 | 7913 | 9319 | 10746 | 12373 | 13848 | 15307 | 16742 |
| 794 | 2449 | 3745 | 5287 | 6650 | 7914 | 9320 | 10747 | 12374 | 13849 | 15308 | 16743 |
| 795 | 2450 | 3746 | 5288 | 6651 | 7915 | 9321 | 10748 | 12375 | 13850 | 15309 | 16744 |
| 796 | 2451 | 3747 | 5289 | 6652 | 7916 | 9322 | 10749 | 12376 | 13851 | 15310 | 16762 |
| 810 | 2452 | 3748 | 5290 | 6653 | 7917 | 9323 | 10750 | 12377 | 13852 | 15311 | 16763 |
| 811 | 2453 | 3749 | 5291 | 6654 | 7918 | 9324 | 10751 | 12378 | 13853 | 15312 | 16764 |
| 812 | 2454 | 3750 | 5292 | 6655 | 7919 | 9325 | 10752 | 12379 | 13854 | 15313 | 16765 |
| 813 | 2455 | 3751 | 5293 | 6656 | 7920 | 9326 | 10753 | 12380 | 13855 | 15314 | 16766 |
| 814 | 2456 | 3752 | 5294 | 6657 | 7921 | 9327 | 10764 | 12381 | 13856 | 15315 | 16767 |
| 815 | 2457 | 3753 | 5309 | 6658 | 7922 | 9328 | 10765 | 12382 | 13857 | 15316 | 16768 |
| 816 | 2458 | 3754 | 5310 | 6659 | 7923 | 9329 | 10766 | 12383 | 13858 | 15317 | 16769 |
| 817 | 2459 | 3771 | 5311 | 6660 | 7924 | 9330 | 10767 | 12384 | 13859 | 15324 | 16770 |
| 818 | 2460 | 3772 | 5312 | 6661 | 7925 | 9331 | 10768 | 12385 | 13860 | 15325 | 16771 |
| 819 | 2473 | 3773 | 5313 | 6662 | 7926 | 9332 | 10769 | 12386 | 13861 | 15326 | 16772 |
| 838 | 2494 | 3774 | 5314 | 6663 | 7927 | 9333 | 10770 | 12387 | 13862 | 15327 | 16773 |
| 839 | 2495 | 3775 | 5315 | 6669 | 7928 | 9334 | 10771 | 12388 | 13863 | 15328 | 16774 |
| 840 | 2496 | 3776 | 5316 | 6670 | 7929 | 9335 | 10772 | 12389 | 13864 | 15329 | 16775 |
| 841 | 2497 | 3777 | 5318 | 6671 | 7930 | 9336 | 10773 | 12390 | 13865 | 15330 | 16776 |
| 842 | 2498 | 3778 | 5319 | 6672 | 7931 | 9337 | 10774 | 12391 | 13866 | 15331 | 16777 |
| 843 | 2499 | 3779 | 5320 | 6673 | 7932 | 9338 | 10775 | 12392 | 13867 | 15332 | 16778 |
| 844 | 2500 | 3780 | 5321 | 6674 | 7933 | 9339 | 10776 | 12393 | 13868 | 15333 | 16779 |
| 845 | 2501 | 3781 | 5322 | 6675 | 7934 | 9340 | 10777 | 12409 | 13869 | 15334 | 16780 |
| 846 | 2502 | 3782 | 5323 | 6676 | 7935 | 9341 | 10778 | 12410 | 13870 | 15335 | 16782 |
| 847 | 2503 | 3783 | 5324 | 6677 | 7936 | 9342 | 10779 | 12411 | 13871 | 15336 | 16783 |
| 848 | 2504 | 3784 | 5325 | 6678 | 7937 | 9343 | 10780 | 12412 | 13872 | 15337 | 16784 |
| 849 | 2505 | 3785 | 5326 | 6679 | 7938 | 9344 | 10795 | 12413 | 13873 | 15338 | 16785 |
| 850 | 2506 | 3786 | 5327 | 6680 | 7939 | 9345 | 10796 | 12414 | 13874 | 15339 | 16786 |
| 851 | 2507 | 3787 | 5328 | 6681 | 7940 | 9346 | 10797 | 12415 | 13875 | 15340 | 16787 |
| 852 | 2508 | 3788 | 5329 | 6682 | 7941 | 9347 | 10798 | 12416 | 13876 | 15341 | 16788 |
| 853 | 2509 | 3789 | 5330 | 6683 | 7942 | 9348 | 10801 | 12417 | 13891 | 15342 | 16789 |
| 854 | 2510 | 3790 | 5331 | 6684 | 7943 | 9349 | 10802 | 12418 | 13892 | 15343 | 16796 |
| 855 | 2511 | 3791 | 5336 | 6685 | 7944 | 9350 | 10803 | 12419 | 13893 | 15344 | 16797 |
| 856 | 2512 | 3792 | 5337 | 6686 | 7945 | 9351 | 10804 | 12420 | 13894 | 15345 | 16798 |
| 858 | 2513 | 3793 | 5338 | 6687 | 7946 | 9352 | 10805 | 12421 | 13895 | 15346 | 16799 |
| 859 | 2514 | 3794 | 5339 | 6688 | 7947 | 9353 | 10806 | 12422 | 13896 | 15347 | 16800 |
| 860 | 2515 | 3795 | 5340 | 6689 | 7948 | 9354 | 10807 | 12423 | 13897 | 15348 | 16801 |
| 861 | 2516 | 3796 | 5341 | 6690 | 7949 | 9355 | 10841 | 12424 | 13898 | 15349 | 16802 |
| 862 | 2517 | 3822 | 5342 | 6691 | 7950 | 9356 | 10842 | 12425 | 13899 | 15350 | 16803 |
| 863 | 2518 | 3823 | 5347 | 6692 | 7951 | 9358 | 10843 | 12426 | 13900 | 15351 | 16804 |
| 864 | 2519 | 3824 | 5348 | 6693 | 7952 | 9359 | 10854 | 12438 | 13901 | 15352 | 16805 |
| 865 | 2520 | 3825 | 5349 | 6694 | 7953 | 9360 | 10855 | 12439 | 13902 | 15356 | 16815 |
| 866 | 2521 | 3826 | 5350 | 6695 | 7954 | 9361 | 10856 | 12440 | 13903 | 15357 | 16840 |
| 867 | 2522 | 3827 | 5351 | 6696 | 7955 | 9362 | 10857 | 12441 | 13904 | 15358 | 16841 |
| 868 | 2528 | 3828 | 5352 | 6697 | 7956 | 9363 | 10868 | 12442 | 13905 | 15359 | 16842 |
| 869 | 2529 | 3829 | 5353 | 6721 | 7957 | 9364 | 10869 | 12443 | 13906 | 15360 | 16843 |
| 876 | 2530 | 3830 | 5354 | 6722 | 7958 | 9365 | 10870 | 12444 | 13907 | 15361 | 16844 |
| 877 | 2531 | 3831 | 5355 | 6723 | 7959 | 9366 | 10871 | 12445 | 13908 | 15362 | 16845 |
| 878 | 2532 | 3832 | 5356 | 6724 | 7960 | 9367 | 10872 | 12446 | 13909 | 15363 | 16846 |
| 882 | 2533 | 3833 | 5357 | 6725 | 7961 | 9368 | 10873 | 12447 | 13910 | 15364 | 16847 |
| 883 | 2534 | 3834 | 5358 | 6728 | 7962 | 9369 | 10874 | 12448 | 13911 | 15365 | 16848 |
| 884 | 2535 | 3835 | 5359 | 6729 | 7963 | 9370 | 10875 | 12449 | 13912 | 15366 | 16849 |
| 885 | 2536 | 3836 | 5360 | 6730 | 7964 | 9371 | 10876 | 12450 | 13913 | 15367 | 16850 |
| 886 | 2537 | 3837 | 5361 | 6739 | 7965 | 9372 | 10877 | 12451 | 13914 | 15368 | 16851 |
| 887 | 2538 | 3838 | 5362 | 6740 | 7966 | 9373 | 10878 | 12452 | 13915 | 15369 | 16852 |
| 888 | 2539 | 3839 | 5363 | 6741 | 7967 | 9374 | 10879 | 12453 | 13916 | 15370 | 16853 |
| 889 | 2540 | 3840 | 5364 | 6742 | 7968 | 9375 | 10880 | 12454 | 13917 | 15371 | 16854 |
| 895 | 2541 | 3841 | 5365 | 6743 | 7969 | 9376 | 10881 | 12455 | 13918 | 15372 | 16855 |
| 896 | 2542 | 3842 | 5366 | 6744 | 7970 | 9377 | 10882 | 12456 | 13919 | 15373 | 16856 |
| 897 | 2543 | 3843 | 5367 | 6745 | 7971 | 9378 | 10883 | 12457 | 13920 | 15374 | 16857 |
| 898 | 2544 | 3847 | 5368 | 6746 | 7972 | 9379 | 10884 | 12458 | 13921 | 15375 | 16858 |
| 899 | 2545 | 3848 | 5369 | 6747 | 7973 | 9380 | 10885 | 12459 | 13922 | 15376 | 16859 |
| 900 | 2546 | 3849 | 5370 | 6748 | 7983 | 9381 | 10886 | 12460 | 13923 | 15377 | 16860 |
| 901 | 2547 | 3850 | 5371 | 6749 | 7984 | 9382 | 10887 | 12461 | 13924 | 15378 | 16861 |
| 902 | 2550 | 3851 | 5372 | 6790 | 7985 | 9383 | 10888 | 12462 | 13925 | 15379 | 16862 |
| 903 | 2551 | 3852 | 5373 | 6791 | 7986 | 9384 | 10889 | 12463 | 13926 | 15380 | 16863 |
| 904 | 2552 | 3853 | 5374 | 6792 | 7987 | 9385 | 10890 | 12464 | 13927 | 15381 | 16864 |
| 905 | 2553 | 3854 | 5375 | 6793 | 7988 | 9386 | 10891 | 12465 | 13928 | 15382 | 16865 |
| 911 | 2554 | 3855 | 5376 | 6794 | 7989 | 9387 | 10892 | 12466 | 13929 | 15383 | 16875 |
| 912 | 2581 | 3856 | 5377 | 6795 | 7990 | 9388 | 10893 | 12467 | 13930 | 15384 | 16876 |
| 913 | 2582 | 3911 | 5378 | 6796 | 7991 | 9389 | 10894 | 12468 | 13934 | 15385 | 16877 |
| 914 | 2583 | 3912 | 5379 | 6797 | 7992 | 9390 | 10895 | 12469 | 13935 | 15386 | 16878 |
| 915 | 2584 | 3913 | 5380 | 6798 | 7993 | 9391 | 10896 | 12470 | 13936 | 15387 | 16879 |
| 916 | 2585 | 3914 | 5381 | 6799 | 7994 | 9392 | 10897 | 12471 | 13937 | 15388 | 16880 |
| 917 | 2586 | 3915 | 5382 | 6800 | 7995 | 9393 | 10898 | 12472 | 13938 | 15389 | 16882 |
| 918 | 2587 | 3916 | 5383 | 6810 | 7996 | 9394 | 10899 | 12473 | 13957 | 15390 | 16883 |
| 919 | 2588 | 3917 | 5384 | 6811 | 7997 | 9395 | 10900 | 12474 | 13958 | 15391 | 16884 |
| 920 | 2589 | 3918 | 5385 | 6812 | 7998 | 9396 | 10910 | 12475 | 13962 | 15392 | 16885 |
| 921 | 2590 | 3919 | 5386 | 6813 | 7999 | 9397 | 10911 | 12476 | 13963 | 15393 | 16886 |
| 922 | 2591 | 3920 | 5387 | 6814 | 8000 | 9398 | 10912 | 12477 | 13964 | 15394 | 16887 |
| 923 | 2592 | 3921 | 5388 | 6815 | 8001 | 9399 | 10913 | 12478 | 13965 | 15395 | 16888 |
| 924 | 2593 | 3922 | 5389 | 6823 | 8002 | 9400 | 10914 | 12479 | 13966 | 15396 | 16889 |
| 925 | 2594 | 3923 | 5390 | 6824 | 8003 | 9401 | 10915 | 12480 | 13967 | 15397 | 16890 |
| 926 | 2595 | 3924 | 5391 | 6825 | 8004 | 9402 | 10919 | 12481 | 13968 | 15398 | 16891 |
| 927 | 2596 | 3925 | 5392 | 6826 | 8005 | 9403 | 10920 | 12482 | 13969 | 15399 | 16892 |
| 941 | 2597 | 3926 | 5393 | 6827 | 8006 | 9404 | 10921 | 12486 | 13970 | 15400 | 16893 |
| 942 | 2598 | 3927 | 5394 | 6828 | 8007 | 9405 | 10922 | 12487 | 13971 | 15401 | 16894 |
| 953 | 2599 | 3928 | 5395 | 6829 | 8008 | 9406 | 10923 | 12488 | 13972 | 15402 | 16895 |
| 954 | 2600 | 3941 | 5396 | 6830 | 8009 | 9407 | 10924 | 12513 | 13973 | 15403 | 16896 |
| 955 | 2601 | 3942 | 5397 | 6831 | 8010 | 9408 | 10929 | 12514 | 13974 | 15414 | 16897 |
| 956 | 2625 | 3943 | 5398 | 6832 | 8011 | 9409 | 10930 | 12515 | 13975 | 15415 | 16898 |
| 957 | 2626 | 3944 | 5399 | 6833 | 8012 | 9410 | 10931 | 12516 | 13976 | 15416 | 16899 |
| 958 | 2627 | 3945 | 5400 | 6834 | 8013 | 9411 | 10932 | 12517 | 13977 | 15417 | 16900 |
| 959 | 2628 | 3946 | 5401 | 6835 | 8014 | 9412 | 10933 | 12518 | 13978 | 15418 | 16901 |
| 991 | 2629 | 3947 | 5418 | 6836 | 8015 | 9413 | 10934 | 12519 | 13979 | 15419 | 16902 |
| 999 | 2630 | 3948 | 5419 | 6837 | 8016 | 9414 | 10935 | 12520 | 13980 | 15420 | 16903 |
| 1000 | 2631 | 3949 | 5420 | 6838 | 8017 | 9415 | 10936 | 12521 | 13981 | 15444 | 16904 |
| 1001 | 2632 | 3950 | 5421 | 6839 | 8018 | 9416 | 10937 | 12522 | 13982 | 15445 | 16905 |
| 1002 | 2633 | 3951 | 5422 | 6840 | 8019 | 9417 | 10938 | 12523 | 13983 | 15446 | 16906 |
| 1035 | 2634 | 3952 | 5423 | 6841 | 8023 | 9418 | 10939 | 12524 | 13984 | 15447 | 16907 |
| 1036 | 2635 | 3953 | 5424 | 6842 | 8024 | 9443 | 10940 | 12525 | 13985 | 15448 | 16908 |
| 1037 | 2636 | 3954 | 5425 | 6843 | 8025 | 9444 | 10941 | 12526 | 13986 | 15449 | 16909 |
| 1038 | 2637 | 3955 | 5426 | 6844 | 8026 | 9445 | 10942 | 12527 | 13987 | 15450 | 16910 |
| 1039 | 2638 | 3956 | 5427 | 6845 | 8027 | 9446 | 10943 | 12528 | 13994 | 15451 | 16911 |
| 1040 | 2639 | 3971 | 5428 | 6859 | 8028 | 9447 | 10944 | 12529 | 13995 | 15452 | 16912 |
| 1041 | 2640 | 3972 | 5429 | 6860 | 8036 | 9448 | 10945 | 12530 | 13996 | 15453 | 16913 |
| 1069 | 2641 | 3973 | 5430 | 6861 | 8037 | 9449 | 10946 | 12531 | 13997 | 15454 | 16914 |
| 1070 | 2642 | 3974 | 5431 | 6862 | 8038 | 9450 | 10947 | 12532 | 13998 | 15455 | 16915 |
| 1071 | 2643 | 3975 | 5432 | 6863 | 8039 | 9451 | 10948 | 12533 | 13999 | 15456 | 16916 |
| 1072 | 2644 | 3976 | 5433 | 6864 | 8040 | 9452 | 10949 | 12534 | 14000 | 15457 | 16917 |
| 1073 | 2645 | 3977 | 5434 | 6865 | 8041 | 9453 | 10950 | 12535 | 14001 | 15458 | 16918 |
| 1074 | 2646 | 3978 | 5435 | 6866 | 8042 | 9454 | 10951 | 12554 | 14022 | 15459 | 16919 |
| 1075 | 2647 | 3979 | 5436 | 6867 | 8043 | 9455 | 10952 | 12555 | 14023 | 15460 | 16922 |
| 1076 | 2648 | 3980 | 5437 | 6868 | 8044 | 9456 | 10953 | 12556 | 14024 | 15461 | 16923 |
| 1077 | 2649 | 3981 | 5438 | 6869 | 8045 | 9468 | 10954 | 12557 | 14025 | 15462 | 16934 |
| 1078 | 2650 | 3982 | 5439 | 6871 | 8046 | 9469 | 10955 | 12558 | 14026 | 15463 | 16935 |
| 1079 | 2651 | 3983 | 5440 | 6872 | 8047 | 9470 | 10956 | 12559 | 14027 | 15464 | 16936 |
| 1080 | 2652 | 3984 | 5441 | 6873 | 8048 | 9471 | 10957 | 12560 | 14028 | 15465 | 16937 |
| 1081 | 2653 | 3985 | 5442 | 6874 | 8049 | 9472 | 10958 | 12561 | 14029 | 15466 | 16943 |
| 1082 | 2654 | 3986 | 5443 | 6875 | 8050 | 9473 | 10959 | 12562 | 14030 | 15467 | 16944 |
| 1083 | 2655 | 3987 | 5449 | 6876 | 8051 | 9474 | 10976 | 12563 | 14035 | 15468 | 16945 |
| 1084 | 2656 | 3988 | 5450 | 6877 | 8052 | 9475 | 10981 | 12564 | 14036 | 15469 | 16946 |
| 1085 | 2666 | 3989 | 5451 | 6878 | 8053 | 9506 | 10982 | 12565 | 14037 | 15470 | 16947 |
| 1086 | 2667 | 3990 | 5452 | 6879 | 8054 | 9507 | 10983 | 12566 | 14040 | 15471 | 16948 |
| 1087 | 2668 | 3991 | 5453 | 6880 | 8055 | 9508 | 10984 | 12567 | 14044 | 15472 | 16949 |
| 1088 | 2669 | 3992 | 5454 | 6881 | 8056 | 9509 | 10985 | 12568 | 14045 | 15473 | 16950 |
| 1089 | 2670 | 3993 | 5455 | 6882 | 8057 | 9510 | 10986 | 12569 | 14046 | 15474 | 16951 |
| 1090 | 2671 | 3994 | 5456 | 6883 | 8058 | 9511 | 10987 | 12570 | 14047 | 15475 | 16952 |
| 1091 | 2672 | 3995 | 5457 | 6884 | 8059 | 9512 | 10988 | 12571 | 14048 | 15476 | 16953 |
| 1092 | 2673 | 3996 | 5458 | 6885 | 8063 | 9513 | 10989 | 12572 | 14049 | 15477 | 16954 |
| 1093 | 2674 | 3997 | 5459 | 6886 | 8064 | 9514 | 10990 | 12573 | 14050 | 15478 | 16955 |
| 1094 | 2675 | 3998 | 5460 | 6887 | 8065 | 9515 | 10991 | 12574 | 14051 | 15479 | 16956 |
| 1095 | 2676 | 3999 | 5461 | 6888 | 8066 | 9516 | 10992 | 12575 | 14056 | 15480 | 16957 |
| 1096 | 2677 | 4006 | 5462 | 6889 | 8093 | 9517 | 10993 | 12576 | 14057 | 15481 | 16958 |
| 1104 | 2678 | 4007 | 5463 | 6890 | 8094 | 9518 | 10994 | 12577 | 14058 | 15482 | 16959 |
| 1105 | 2679 | 4008 | 5464 | 6891 | 8095 | 9519 | 10995 | 12578 | 14059 | 15483 | 16970 |
| 1106 | 2680 | 4021 | 5465 | 6892 | 8096 | 9520 | 10996 | 12579 | 14060 | 15484 | 16971 |
| 1107 | 2681 | 4022 | 5466 | 6893 | 8097 | 9521 | 10997 | 12580 | 14061 | 15485 | 16972 |
| 1108 | 2682 | 4023 | 5471 | 6894 | 8098 | 9522 | 10998 | 12581 | 14062 | 15486 | 16973 |
| 1109 | 2683 | 4024 | 5472 | 6895 | 8099 | 9523 | 10999 | 12582 | 14063 | 15487 | 16974 |
| 1110 | 2684 | 4025 | 5473 | 6896 | 8100 | 9524 | 11000 | 12583 | 14064 | 15488 | 16975 |
| 1111 | 2685 | 4026 | 5474 | 6897 | 8101 | 9525 | 11001 | 12584 | 14081 | 15489 | 16976 |
| 1133 | 2686 | 4027 | 5475 | 6898 | 8102 | 9526 | 11002 | 12592 | 14082 | 15490 | 16977 |
| 1134 | 2687 | 4028 | 5476 | 6899 | 8103 | 9527 | 11003 | 12593 | 14083 | 15491 | 16978 |
| 1135 | 2688 | 4029 | 5477 | 6900 | 8104 | 9528 | 11004 | 12594 | 14084 | 15492 | 16979 |
| 1136 | 2689 | 4030 | 5478 | 6901 | 8105 | 9529 | 11005 | 12614 | 14085 | 15493 | 16980 |
| 1137 | 2690 | 4031 | 5479 | 6902 | 8106 | 9530 | 11006 | 12615 | 14086 | 15494 | 16981 |
| 1138 | 2691 | 4032 | 5486 | 6903 | 8107 | 9531 | 11007 | 12616 | 14087 | 15495 | 16982 |
| 1139 | 2692 | 4033 | 5487 | 6904 | 8108 | 9532 | 11015 | 12617 | 14088 | 15496 | 16983 |
| 1140 | 2693 | 4034 | 5488 | 6905 | 8109 | 9533 | 11016 | 12618 | 14089 | 15502 | 16984 |
| 1141 | 2694 | 4035 | 5489 | 6906 | 8110 | 9534 | 11017 | 12619 | 14091 | 15503 | 16985 |
| 1142 | 2695 | 4036 | 5490 | 6907 | 8111 | 9535 | 11018 | 12620 | 14092 | 15504 | 16986 |
| 1143 | 2696 | 4037 | 5491 | 6908 | 8112 | 9536 | 11019 | 12621 | 14093 | 15505 | 16987 |
| 1144 | 2697 | 4038 | 5492 | 6909 | 8113 | 9537 | 11020 | 12622 | 14094 | 15506 | 16988 |
| 1145 | 2698 | 4039 | 5493 | 6910 | 8114 | 9538 | 11021 | 12630 | 14095 | 15507 | 16989 |
| 1146 | 2699 | 4040 | 5494 | 6911 | 8115 | 9539 | 11050 | 12631 | 14096 | 15508 | 16990 |
| 1147 | 2700 | 4041 | 5495 | 6912 | 8124 | 9540 | 11051 | 12638 | 14097 | 15509 | 16991 |
| 1148 | 2701 | 4042 | 5496 | 6913 | 8125 | 9541 | 11052 | 12639 | 14098 | 15510 | 16992 |
| 1149 | 2702 | 4043 | 5497 | 6915 | 8126 | 9542 | 11053 | 12640 | 14099 | 15511 | 16993 |
| 1150 | 2703 | 4044 | 5498 | 6916 | 8127 | 9543 | 11054 | 12641 | 14125 | 15512 | 16994 |
| 1151 | 2704 | 4104 | 5499 | 6917 | 8128 | 9544 | 11055 | 12642 | 14126 | 15513 | 16995 |
| 1152 | 2705 | 4105 | 5500 | 6918 | 8129 | 9545 | 11056 | 12643 | 14127 | 15514 | 16996 |
| 1153 | 2706 | 4106 | 5501 | 6919 | 8130 | 9546 | 11057 | 12644 | 14128 | 15515 | 16997 |
| 1154 | 2707 | 4107 | 5502 | 6920 | 8131 | 9551 | 11058 | 12645 | 14129 | 15516 | 16998 |
| 1155 | 2708 | 4108 | 5519 | 6921 | 8132 | 9552 | 11059 | 12646 | 14130 | 15517 | 17008 |
| 1156 | 2709 | 4109 | 5520 | 6922 | 8133 | 9553 | 11076 | 12647 | 14131 | 15532 | 17009 |
| 1180 | 2710 | 4110 | 5521 | 6923 | 8134 | 9554 | 11077 | 12648 | 14132 | 15533 | 17010 |
| 1181 | 2711 | 4111 | 5522 | 6924 | 8135 | 9555 | 11078 | 12649 | 14135 | 15534 | 17011 |
| 1182 | 2712 | 4112 | 5523 | 6925 | 8136 | 9556 | 11079 | 12650 | 14136 | 15535 | 17012 |
| 1183 | 2713 | 4113 | 5524 | 6926 | 8137 | 9568 | 11080 | 12651 | 14137 | 15536 | 17013 |
| 1184 | 2714 | 4114 | 5525 | 6927 | 8138 | 9569 | 11081 | 12652 | 14138 | 15537 | 17014 |
| 1185 | 2718 | 4115 | 5526 | 6928 | 8143 | 9570 | 11082 | 12653 | 14158 | 15538 | 17015 |
| 1186 | 2719 | 4116 | 5527 | 6929 | 8144 | 9571 | 11083 | 12654 | 14159 | 15539 | 17016 |
| 1187 | 2720 | 4117 | 5528 | 6933 | 8145 | 9572 | 11084 | 12655 | 14160 | 15540 | 17017 |
| 1188 | 2721 | 4120 | 5529 | 6934 | 8146 | 9573 | 11085 | 12656 | 14161 | 15541 | 17018 |
| 1189 | 2722 | 4121 | 5530 | 6935 | 8147 | 9574 | 11086 | 12657 | 14162 | 15542 | 17019 |
| 1190 | 2723 | 4122 | 5531 | 6936 | 8148 | 9575 | 11087 | 12658 | 14163 | 15543 | 17020 |
| 1191 | 2724 | 4123 | 5532 | 6937 | 8149 | 9576 | 11088 | 12659 | 14164 | 15544 | 17021 |
| 1192 | 2725 | 4124 | 5533 | 6942 | 8163 | 9577 | 11089 | 12660 | 14165 | 15545 | 17022 |
| 1193 | 2726 | 4125 | 5534 | 6943 | 8164 | 9578 | 11090 | 12661 | 14166 | 15546 | 17023 |
| 1194 | 2727 | 4126 | 5535 | 6944 | 8165 | 9579 | 11091 | 12662 | 14167 | 15547 | 17024 |
| 1195 | 2728 | 4127 | 5536 | 6950 | 8166 | 9601 | 11092 | 12663 | 14168 | 15548 | 17025 |
| 1212 | 2729 | 4128 | 5537 | 6951 | 8167 | 9602 | 11093 | 12664 | 14169 | 15549 | 17026 |
| 1213 | 2730 | 4129 | 5538 | 6952 | 8168 | 9603 | 11094 | 12665 | 14170 | 15550 | 17027 |
| 1214 | 2731 | 4130 | 5561 | 6953 | 8169 | 9604 | 11095 | 12666 | 14195 | 15551 | 17028 |
| 1215 | 2732 | 4131 | 5562 | 6954 | 8170 | 9605 | 11096 | 12667 | 14196 | 15552 | 17029 |
| 1216 | 2737 | 4132 | 5563 | 6955 | 8171 | 9606 | 11097 | 12668 | 14197 | 15553 | 17030 |
| 1217 | 2738 | 4133 | 5564 | 6956 | 8172 | 9607 | 11098 | 12685 | 14198 | 15554 | 17031 |
| 1218 | 2739 | 4134 | 5565 | 6957 | 8173 | 9608 | 11099 | 12686 | 14199 | 15555 | 17037 |
| 1219 | 2740 | 4135 | 5566 | 6958 | 8174 | 9609 | 11106 | 12687 | 14200 | 15556 | 17038 |
| 1220 | 2741 | 4136 | 5567 | 6959 | 8175 | 9610 | 11107 | 12688 | 14201 | 15557 | 17039 |
| 1221 | 2742 | 4137 | 5568 | 6960 | 8176 | 9611 | 11108 | 12692 | 14202 | 15558 | 17040 |
| 1222 | 2743 | 4161 | 5571 | 6961 | 8177 | 9612 | 11109 | 12693 | 14203 | 15559 | 17041 |
| 1223 | 2744 | 4162 | 5572 | 6962 | 8178 | 9613 | 11110 | 12694 | 14204 | 15560 | 17088 |
| 1224 | 2745 | 4163 | 5573 | 6963 | 8179 | 9614 | 11111 | 12695 | 14205 | 15561 | 17089 |
| 1225 | 2746 | 4164 | 5574 | 6964 | 8180 | 9615 | 11112 | 12696 | 14206 | 15562 | 17090 |
| 1226 | 2747 | 4165 | 5575 | 6965 | 8181 | 9616 | 11113 | 12697 | 14207 | 15563 | 17091 |
| 1254 | 2748 | 4166 | 5576 | 6966 | 8182 | 9617 | 11114 | 12698 | 14208 | 15564 | 17092 |
| 1255 | 2749 | 4167 | 5577 | 6967 | 8183 | 9618 | 11115 | 12699 | 14209 | 15565 | 17093 |
| 1256 | 2750 | 4168 | 5578 | 6968 | 8187 | 9619 | 11116 | 12700 | 14214 | 15566 | 17094 |
| 1269 | 2751 | 4169 | 5579 | 6969 | 8188 | 9620 | 11117 | 12701 | 14231 | 15567 | 17099 |
| 1281 | 2752 | 4170 | 5580 | 6970 | 8189 | 9621 | 11118 | 12702 | 14232 | 15568 | 17100 |
| 1282 | 2753 | 4171 | 5581 | 6971 | 8190 | 9632 | 11119 | 12703 | 14233 | 15569 | 17101 |
| 1288 | 2754 | 4172 | 5582 | 6972 | 8191 | 9633 | 11120 | 12704 | 14234 | 15570 | 17102 |
| 1313 | 2779 | 4173 | 5583 | 6973 | 8192 | 9634 | 11121 | 12705 | 14235 | 15571 | 17103 |
| 1314 | 2780 | 4174 | 5584 | 6974 | 8193 | 9635 | 11122 | 12706 | 14236 | 15572 | 17104 |
| 1315 | 2781 | 4175 | 5585 | 6975 | 8194 | 9636 | 11123 | 12739 | 14237 | 15573 | 17105 |
| 1316 | 2782 | 4176 | 5591 | 6977 | 8195 | 9637 | 11124 | 12740 | 14245 | 15575 | 17106 |
| 1317 | 2783 | 4177 | 5592 | 6982 | 8196 | 9638 | 11125 | 12741 | 14246 | 15576 | 17107 |
| 1318 | 2784 | 4178 | 5593 | 6983 | 8197 | 9639 | 11126 | 12742 | 14247 | 15577 | 17108 |
| 1319 | 2785 | 4182 | 5594 | 6984 | 8198 | 9640 | 11127 | 12743 | 14248 | 15578 | 17109 |
| 1320 | 2789 | 4183 | 5595 | 6985 | 8199 | 9641 | 11128 | 12753 | 14251 | 15579 | 17110 |
| 1321 | 2790 | 4184 | 5596 | 6986 | 8200 | 9642 | 11129 | 12754 | 14252 | 15580 | 17111 |
| 1322 | 2791 | 4185 | 5597 | 6987 | 8201 | 9643 | 11130 | 12755 | 14253 | 15581 | 17112 |
| 1323 | 2792 | 4186 | 5598 | 6988 | 8202 | 9644 | 11131 | 12756 | 14254 | 15582 | 17113 |
| 1324 | 2793 | 4187 | 5599 | 6989 | 8203 | 9645 | 11132 | 12757 | 14255 | 15583 | 17114 |
| 1325 | 2794 | 4188 | 5600 | 6990 | 8204 | 9646 | 11133 | 12768 | 14277 | 15584 | 17115 |
| 1326 | 2795 | 4191 | 5601 | 6991 | 8205 | 9647 | 11134 | 12769 | 14278 | 15585 | 17134 |
| 1327 | 2796 | 4192 | 5602 | 6992 | 8206 | 9648 | 11137 | 12770 | 14279 | 15586 | 17135 |
| 1328 | 2797 | 4193 | 5608 | 6993 | 8207 | 9649 | 11138 | 12772 | 14280 | 15587 | 17136 |
| 1329 | 2800 | 4194 | 5626 | 6994 | 8208 | 9650 | 11139 | 12773 | 14281 | 15588 | 17137 |
| 1330 | 2801 | 4195 | 5638 | 6995 | 8209 | 9651 | 11140 | 12774 | 14282 | 15589 | 17138 |
| 1331 | 2802 | 4196 | 5639 | 6996 | 8210 | 9652 | 11141 | 12775 | 14283 | 15590 | 17139 |
| 1332 | 2803 | 4197 | 5640 | 6997 | 8211 | 9653 | 11142 | 12776 | 14295 | 15591 | 17140 |
| 1340 | 2804 | 4198 | 5641 | 6998 | 8212 | 9654 | 11143 | 12777 | 14296 | 15592 | 17141 |
| 1341 | 2805 | 4199 | 5642 | 6999 | 8213 | 9655 | 11144 | 12778 | 14297 | 15593 | 17142 |
| 1342 | 2806 | 4200 | 5643 | 7005 | 8214 | 9656 | 11145 | 12779 | 14298 | 15594 | 17143 |
| 1343 | 2807 | 4201 | 5644 | 7006 | 8215 | 9657 | 11146 | 12780 | 14299 | 15595 | 17144 |
| 1344 | 2808 | 4202 | 5645 | 7007 | 8216 | 9660 | 11147 | 12781 | 14300 | 15596 | 17145 |
| 1345 | 2809 | 4203 | 5646 | 7008 | 8217 | 9661 | 11148 | 12782 | 14301 | 15597 | 17146 |
| 1346 | 2810 | 4204 | 5647 | 7009 | 8218 | 9662 | 11149 | 12783 | 14302 | 15598 | 17147 |
| 1347 | 2811 | 4205 | 5668 | 7010 | 8219 | 9663 | 11150 | 12784 | 14303 | 15599 | 17148 |
| 1348 | 2812 | 4206 | 5669 | 7020 | 8220 | 9664 | 11151 | 12785 | 14304 | 15600 | 17149 |
| 1349 | 2813 | 4212 | 5670 | 7021 | 8221 | 9665 | 11152 | 12786 | 14305 | 15602 | 17150 |
| 1350 | 2814 | 4213 | 5671 | 7022 | 8222 | 9666 | 11158 | 12787 | 14306 | 15603 | 17151 |
| 1351 | 2815 | 4214 | 5672 | 7023 | 8223 | 9681 | 11159 | 12788 | 14307 | 15604 | 17152 |
| 1352 | 2816 | 4215 | 5673 | 7024 | 8224 | 9682 | 11160 | 12789 | 14308 | 15605 | 17153 |
| 1353 | 2817 | 4216 | 5674 | 7025 | 8225 | 9683 | 11161 | 12790 | 14309 | 15606 | 17154 |
| 1354 | 2818 | 4217 | 5675 | 7026 | 8226 | 9684 | 11162 | 12791 | 14310 | 15607 | 17155 |
| 1355 | 2819 | 4224 | 5676 | 7027 | 8227 | 9685 | 11163 | 12792 | 14311 | 15608 | 17161 |
| 1356 | 2820 | 4225 | 5677 | 7028 | 8228 | 9686 | 11164 | 12793 | 14312 | 15609 | 17162 |
| 1357 | 2821 | 4226 | 5678 | 7029 | 8229 | 9687 | 11165 | 12794 | 14313 | 15610 | 17163 |
| 1358 | 2822 | 4227 | 5679 | 7030 | 8230 | 9688 | 11166 | 12795 | 14314 | 15611 | 17164 |
| 1359 | 2823 | 4228 | 5680 | 7031 | 8231 | 9689 | 11167 | 12796 | 14315 | 15612 | 17165 |
| 1360 | 2824 | 4229 | 5681 | 7032 | 8232 | 9690 | 11168 | 12797 | 14316 | 15613 | 17166 |
| 1361 | 2825 | 4230 | 5682 | 7033 | 8233 | 9691 | 11169 | 12798 | 14317 | 15614 | 17167 |
| 1362 | 2833 | 4231 | 5683 | 7034 | 8234 | 9692 | 11170 | 12799 | 14318 | 15615 | 17168 |
| 1363 | 2834 | 4232 | 5684 | 7035 | 8241 | 9693 | 11171 | 12800 | 14326 | 15616 | 17181 |
| 1384 | 2835 | 4233 | 5685 | 7036 | 8249 | 9694 | 11172 | 12801 | 14327 | 15617 | 17182 |
| 1385 | 2836 | 4234 | 5686 | 7037 | 8250 | 9695 | 11173 | 12802 | 14328 | 15618 | 17183 |
| 1386 | 2837 | 4235 | 5687 | 7038 | 8251 | 9696 | 11174 | 12803 | 14329 | 15619 | 17184 |
| 1387 | 2838 | 4236 | 5688 | 7039 | 8252 | 9697 | 11181 | 12804 | 14330 | 15620 | 17185 |
| 1388 | 2839 | 4237 | 5689 | 7040 | 8253 | 9698 | 11182 | 12805 | 14331 | 15621 | 17186 |
| 1390 | 2840 | 4238 | 5699 | 7041 | 8254 | 9699 | 11183 | 12806 | 14334 | 15660 | 17188 |
| 1391 | 2841 | 4239 | 5700 | 7052 | 8255 | 9700 | 11184 | 12807 | 14335 | 15661 | 17189 |
| 1392 | 2842 | 4240 | 5701 | 7053 | 8256 | 9701 | 11185 | 12808 | 14336 | 15662 | 17190 |
| 1393 | 2843 | 4283 | 5702 | 7054 | 8257 | 9702 | 11186 | 12809 | 14337 | 15663 | 17191 |
| 1394 | 2844 | 4284 | 5703 | 7055 | 8258 | 9703 | 11187 | 12810 | 14338 | 15664 | 17192 |
| 1395 | 2845 | 4285 | 5704 | 7056 | 8259 | 9704 | 11188 | 12811 | 14339 | 15665 | 17193 |
| 1396 | 2846 | 4286 | 5714 | 7057 | 8260 | 9705 | 11189 | 12812 | 14340 | 15666 | 17194 |
| 1397 | 2847 | 4287 | 5715 | 7058 | 8261 | 9706 | 11190 | 12813 | 14341 | 15667 | 17195 |
| 1398 | 2848 | 4288 | 5716 | 7059 | 8262 | 9707 | 11191 | 12814 | 14344 | 15668 | 17196 |
| 1399 | 2849 | 4295 | 5717 | 7060 | 8278 | 9708 | 11192 | 12815 | 14345 | 15669 | 17197 |
| 1400 | 2850 | 4297 | 5718 | 7061 | 8279 | 9709 | 11193 | 12816 | 14346 | 15670 | 17203 |
| 1401 | 2851 | 4303 | 5725 | 7062 | 8280 | 9710 | 11194 | 12817 | 14347 | 15671 | 17204 |
| 1412 | 2852 | 4304 | 5726 | 7063 | 8281 | 9711 | 11195 | 12818 | 14348 | 15672 | 17205 |
| 1413 | 2853 | 4305 | 5727 | 7064 | 8282 | 9712 | 11196 | 12819 | 14349 | 15673 | 17206 |
| 1414 | 2854 | 4306 | 5728 | 7065 | 8283 | 9713 | 11197 | 12820 | 14350 | 15674 | 17207 |
| 1415 | 2855 | 4307 | 5729 | 7066 | 8284 | 9714 | 11198 | 12821 | 14351 | 15675 | 17208 |
| 1416 | 2856 | 4308 | 5730 | 7067 | 8285 | 9715 | 11199 | 12822 | 14352 | 15690 | 17209 |
| 1417 | 2860 | 4309 | 5731 | 7068 | 8286 | 9716 | 11200 | 12841 | 14353 | 15691 | 17210 |
| 1429 | 2864 | 4310 | 5732 | 7069 | 8287 | 9717 | 11201 | 12842 | 14354 | 15692 | 17211 |
| 1430 | 2865 | 4311 | 5733 | 7070 | 8288 | 9718 | 11202 | 12843 | 14355 | 15693 | 17212 |
| 1431 | 2866 | 4312 | 5734 | 7071 | 8289 | 9719 | 11203 | 12844 | 14356 | 15694 | 17213 |
| 1432 | 2867 | 4313 | 5735 | 7072 | 8290 | 9762 | 11204 | 12845 | 14357 | 15695 | 17214 |
| 1433 | 2868 | 4328 | 5736 | 7073 | 8291 | 9763 | 11205 | 12846 | 14358 | 15696 | 17215 |
| 1434 | 2869 | 4329 | 5737 | 7074 | 8292 | 9764 | 11206 | 12847 | 14359 | 15697 | 17216 |
| 1435 | 2870 | 4330 | 5738 | 7075 | 8293 | 9765 | 11207 | 12848 | 14360 | 15698 | 17217 |
| 1436 | 2871 | 4331 | 5739 | 7076 | 8294 | 9766 | 11208 | 12849 | 14361 | 15699 | 17218 |
| 1437 | 2872 | 4332 | 5740 | 7078 | 8295 | 9767 | 11209 | 12850 | 14362 | 15700 | 17219 |
| 1438 | 2873 | 4333 | 5741 | 7079 | 8296 | 9772 | 11210 | 12851 | 14363 | 15701 | 17228 |
| 1456 | 2874 | 4334 | 5742 | 7080 | 8297 | 9773 | 11211 | 12852 | 14364 | 15702 | 17229 |
| 1457 | 2875 | 4335 | 5743 | 7081 | 8298 | 9774 | 11222 | 12853 | 14365 | 15703 | 17235 |
| 1458 | 2876 | 4336 | 5744 | 7082 | 8299 | 9789 | 11228 | 12854 | 14366 | 15704 | 17236 |
| 1459 | 2877 | 4337 | 5745 | 7083 | 8300 | 9790 | 11229 | 12862 | 14367 | 15744 | 17237 |
| 1460 | 2878 | 4338 | 5746 | 7089 | 8301 | 9791 | 11230 | 12863 | 14368 | 15745 | 17238 |
| 1461 | 2879 | 4339 | 5747 | 7090 | 8302 | 9792 | 11231 | 12864 | 14369 | 15746 | 17239 |
| 1462 | 2880 | 4340 | 5748 | 7091 | 8303 | 9793 | 11232 | 12865 | 14370 | 15747 | 17240 |
| 1463 | 2889 | 4341 | 5749 | 7092 | 8304 | 9794 | 11233 | 12866 | 14391 | 15748 | 17241 |
| 1468 | 2890 | 4342 | 5750 | 7097 | 8305 | 9795 | 11234 | 12867 | 14392 | 15749 | 17242 |
| 1469 | 2891 | 4343 | 5751 | 7098 | 8306 | 9796 | 11235 | 12868 | 14393 | 15750 | 17243 |
| 1470 | 2892 | 4344 | 5752 | 7099 | 8331 | 9797 | 11236 | 12869 | 14394 | 15751 | 17248 |
| 1471 | 2893 | 4345 | 5753 | 7100 | 8332 | 9798 | 11237 | 12870 | 14395 | 15752 | 17249 |
| 1482 | 2894 | 4348 | 5754 | 7101 | 8336 | 9799 | 11238 | 12871 | 14396 | 15753 | 17250 |
| 1483 | 2895 | 4349 | 5755 | 7102 | 8337 | 9800 | 11239 | 12872 | 14397 | 15754 | 17251 |
| 1484 | 2896 | 4350 | 5756 | 7121 | 8351 | 9801 | 11240 | 12873 | 14398 | 15755 | 17252 |
| 1485 | 2897 | 4351 | 5757 | 7122 | 8352 | 9802 | 11241 | 12874 | 14399 | 15756 | 17268 |
| 1486 | 2898 | 4352 | 5758 | 7123 | 8353 | 9803 | 11242 | 12881 | 14400 | 15757 | 17269 |
| 1487 | 2899 | 4353 | 5759 | 7124 | 8354 | 9804 | 11243 | 12882 | 14401 | 15758 | 17270 |
| 1488 | 2900 | 4354 | 5761 | 7125 | 8358 | 9805 | 11244 | 12883 | 14402 | 15759 | 17271 |
| 1489 | 2901 | 4355 | 5762 | 7126 | 8359 | 9806 | 11245 | 12884 | 14459 | 15760 | 17272 |
| 1490 | 2902 | 4356 | 5763 | 7127 | 8360 | 9807 | 11246 | 12885 | 14460 | 15761 | 17273 |
| 1500 | 2903 | 4357 | 5805 | 7128 | 8361 | 9808 | 11247 | 12886 | 14461 | 15762 | 17274 |
| 1501 | 2904 | 4358 | 5806 | 7129 | 8362 | 9809 | 11248 | 12887 | 14462 | 15763 | 17275 |
| 1502 | 2905 | 4359 | 5807 | 7130 | 8366 | 9810 | 11249 | 12888 | 14463 | 15764 | 17276 |
| 1505 | 2906 | 4360 | 5808 | 7131 | 8367 | 9811 | 11250 | 12889 | 14464 | 15765 | 17277 |
| 1506 | 2907 | 4361 | 5809 | 7132 | 8368 | 9812 | 11266 | 12890 | 14465 | 15766 | 17278 |
| 1507 | 2908 | 4362 | 5810 | 7133 | 8369 | 9813 | 11267 | 12891 | 14475 | 15767 | 17279 |
| 1508 | 2909 | 4363 | 5811 | 7134 | 8370 | 9820 | 11268 | 12892 | 14498 | 15768 | 17280 |
| 1509 | 2910 | 4364 | 5812 | 7135 | 8371 | 9821 | 11269 | 12893 | 14499 | 15769 | 17281 |
| 1510 | 2911 | 4365 | 5813 | 7136 | 8372 | 9822 | 11270 | 12894 | 14500 | 15770 | 17282 |
| 1511 | 2912 | 4366 | 5814 | 7137 | 8373 | 9823 | 11271 | 12896 | 14501 | 15771 | 17283 |
| 1512 | 2913 | 4367 | 5815 | 7138 | 8374 | 9824 | 11272 | 12897 | 14502 | 15772 | 17284 |
| 1513 | 2914 | 4368 | 5816 | 7139 | 8375 | 9825 | 11273 | 12898 | 14503 | 15773 | 17285 |
| 1514 | 2915 | 4369 | 5817 | 7140 | 8376 | 9826 | 11274 | 12899 | 14504 | 15774 | 17286 |
| 1515 | 2916 | 4370 | 5818 | 7141 | 8377 | 9827 | 11275 | 12900 | 14505 | 15775 | 17287 |
| 1516 | 2917 | 4371 | 5819 | 7142 | 8378 | 9828 | 11276 | 12901 | 14506 | 15776 | 17288 |
| 1517 | 2918 | 4372 | 5820 | 7143 | 8379 | 9829 | 11277 | 12902 | 14507 | 15777 | 17324 |
| 1518 | 2919 | 4373 | 5821 | 7144 | 8380 | 9830 | 11278 | 12903 | 14508 | 15794 | 17325 |
| 1519 | 2920 | 4374 | 5822 | 7145 | 8381 | 9831 | 11279 | 12904 | 14509 | 15795 | 17326 |
| 1520 | 2921 | 4375 | 5823 | 7146 | 8382 | 9832 | 11280 | 12905 | 14510 | 15796 | 17327 |
| 1521 | 2924 | 4376 | 5824 | 7147 | 8383 | 9842 | 11281 | 12906 | 14511 | 15797 | 17328 |
| 1522 | 2925 | 4386 | 5833 | 7148 | 8384 | 9843 | 11282 | 12907 | 14512 | 15798 | 17329 |
| 1523 | 2926 | 4398 | 5834 | 7149 | 8385 | 9844 | 11283 | 12908 | 14513 | 15799 | 17330 |
| 1524 | 2927 | 4399 | 5835 | 7150 | 8386 | 9845 | 11284 | 12909 | 14517 | 15800 | 17331 |
| 1525 | 2928 | 4400 | 5836 | 7151 | 8387 | 9846 | 11285 | 12910 | 14529 | 15801 | 17332 |
| 1526 | 2929 | 4401 | 5837 | 7152 | 8388 | 9847 | 11286 | 12911 | 14530 | 15802 | 17333 |
| 1527 | 2930 | 4402 | 5838 | 7153 | 8389 | 9848 | 11287 | 12912 | 14539 | 15803 | 17334 |
| 1528 | 2931 | 4403 | 5839 | 7154 | 8390 | 9862 | 11288 | 12913 | 14540 | 15855 | 17335 |
| 1529 | 2932 | 4404 | 5840 | 7155 | 8391 | 9863 | 11289 | 12914 | 14541 | 15856 | 17345 |
| 1530 | 2933 | 4405 | 5841 | 7156 | 8402 | 9864 | 11361 | 12916 | 14542 | 15857 | 17353 |
| 1531 | 2934 | 4406 | 5842 | 7157 | 8403 | 9870 | 11362 | 12917 | 14543 | 15858 | 17354 |
| 1532 | 2935 | 4407 | 5843 | 7158 | 8404 | 9871 | 11363 | 12918 | 14544 | 15859 | 17355 |
| 1533 | 2936 | 4408 | 5844 | 7159 | 8405 | 9872 | 11364 | 12919 | 14545 | 15870 | 17356 |
| 1534 | 2937 | 4409 | 5855 | 7160 | 8406 | 9873 | 11365 | 12934 | 14546 | 15871 | 17357 |
| 1535 | 2938 | 4410 | 5856 | 7161 | 8407 | 9874 | 11366 | 12935 | 14547 | 15872 | 17358 |
| 1536 | 2939 | 4411 | 5857 | 7162 | 8408 | 9875 | 11367 | 12936 | 14548 | 15873 | 17359 |
| 1537 | 2940 | 4412 | 5858 | 7163 | 8409 | 9876 | 11368 | 12937 | 14549 | 15874 | 17360 |
| 1538 | 2941 | 4413 | 5859 | 7164 | 8410 | 9877 | 11369 | 12938 | 14550 | 15875 | 17361 |
| 1539 | 2942 | 4414 | 5860 | 7172 | 8411 | 9878 | 11381 | 12939 | 14551 | 15888 | 17362 |
| 1540 | 2943 | 4415 | 5861 | 7173 | 8430 | 9879 | 11382 | 12940 | 14552 | 15889 | 17363 |
| 1541 | 2944 | 4416 | 5862 | 7174 | 8431 | 9880 | 11383 | 12941 | 14553 | 15890 | 17364 |
| 1542 | 2945 | 4417 | 5863 | 7175 | 8432 | 9881 | 11384 | 12942 | 14554 | 15891 | 17378 |
| 1543 | 2946 | 4418 | 5864 | 7176 | 8433 | 9882 | 11385 | 12943 | 14564 | 15892 | 17379 |
| 1544 | 2947 | 4419 | 5865 | 7177 | 8434 | 9883 | 11386 | 12944 | 14565 | 15893 | 17380 |
| 1545 | 2948 | 4420 | 5866 | 7178 | 8435 | 9884 | 11387 | 12945 | 14566 | 15894 | 17381 |
| 1546 | 2949 | 4421 | 5867 | 7179 | 8436 | 9885 | 11388 | 12946 | 14569 | 15895 | 17382 |
| 1574 | 2950 | 4422 | 5868 | 7180 | 8437 | 9886 | 11389 | 12947 | 14570 | 15896 | 17391 |
| 1575 | 2951 | 4423 | 5869 | 7181 | 8438 | 9887 | 11390 | 12948 | 14571 | 15897 | 17392 |
| 1576 | 2952 | 4424 | 5870 | 7182 | 8439 | 9888 | 11391 | 12963 | 14572 | 15898 | 17393 |
| 1594 | 2953 | 4447 | 5871 | 7183 | 8440 | 9889 | 11404 | 12964 | 14573 | 15899 | 17394 |
| 1595 | 2954 | 4448 | 5872 | 7184 | 8441 | 9890 | 11428 | 12965 | 14574 | 15900 | 17395 |
| 1596 | 2955 | 4449 | 5873 | 7185 | 8442 | 9891 | 11429 | 12966 | 14575 | 15901 | 17396 |
| 1597 | 2956 | 4450 | 5912 | 7219 | 8443 | 9892 | 11430 | 12967 | 14576 | 15902 | 17397 |
| 1615 | 2957 | 4451 | 5913 | 7220 | 8444 | 9893 | 11431 | 12968 | 14577 | 15903 | 17398 |
| 1616 | 2958 | 4452 | 5914 | 7221 | 8445 | 9894 | 11432 | 12969 | 14578 | 15904 | 17399 |
| 1617 | 2959 | 4453 | 5915 | 7222 | 8446 | 9895 | 11433 | 12970 | 14579 | 15918 | 17400 |
| 1618 | 2960 | 4454 | 5916 | 7223 | 8447 | 9896 | 11442 | 12971 | 14580 | 15919 | 17401 |
| 1630 | 2961 | 4455 | 5917 | 7224 | 8448 | 9897 | 11443 | 12972 | 14581 | 15920 | 17402 |
| 1631 | 2962 | 4456 | 5918 | 7225 | 8449 | 9898 | 11444 | 12973 | 14582 | 15921 | 17403 |
| 1632 | 2963 | 4457 | 5919 | 7226 | 8450 | 9899 | 11445 | 12974 | 14583 | 15922 | 17404 |
| 1633 | 2964 | 4458 | 5920 | 7227 | 8451 | 9900 | 11446 | 12975 | 14584 | 15923 | 17405 |
| 1634 | 2965 | 4459 | 5921 | 7228 | 8452 | 9901 | 11447 | 12976 | 14585 | 15926 | 17406 |
| 1635 | 2966 | 4460 | 5922 | 7229 | 8453 | 9902 | 11448 | 12977 | 14586 | 15927 | 17407 |
| 1636 | 2967 | 4461 | 5923 | 7230 | 8454 | 9903 | 11449 | 12978 | 14587 | 15928 | 17408 |
| 1643 | 2968 | 4462 | 5924 | 7231 | 8455 | 9904 | 11450 | 12979 | 14588 | 15929 | 17409 |
| 1644 | 2969 | 4463 | 5925 | 7232 | 8469 | 9914 | 11451 | 12996 | 14589 | 15930 | 17410 |
| 1645 | 2970 | 4464 | 5926 | 7233 | 8470 | 9915 | 11452 | 12997 | 14590 | 15931 | 17411 |
| 1646 | 2971 | 4488 | 5927 | 7234 | 8471 | 9916 | 11453 | 12998 | 14591 | 15932 | 17412 |
| 1672 | 2972 | 4489 | 5928 | 7235 | 8472 | 9917 | 11454 | 12999 | 14592 | 15933 | 17413 |
| 1673 | 2973 | 4490 | 5929 | 7236 | 8473 | 9918 | 11455 | 13000 | 14616 | 15934 | 17414 |
| 1674 | 2974 | 4491 | 5930 | 7237 | 8474 | 9919 | 11456 | 13001 | 14617 | 15935 | 17415 |
| 1675 | 2975 | 4492 | 5936 | 7246 | 8475 | 9920 | 11460 | 13002 | 14618 | 15936 | 17416 |
| 1676 | 2976 | 4493 | 5937 | 7247 | 8476 | 9921 | 11461 | 13003 | 14619 | 15937 | 17417 |
| 1682 | 2977 | 4494 | 5938 | 7248 | 8477 | 9922 | 11462 | 13004 | 14620 | 15938 | 17418 |
| 1683 | 2978 | 4495 | 5939 | 7249 | 8478 | 9923 | 11463 | 13005 | 14621 | 15939 | 17419 |
| 1684 | 2979 | 4496 | 5940 | 7250 | 8479 | 9924 | 11464 | 13006 | 14622 | 15940 | 17420 |
| 1685 | 2980 | 4497 | 5941 | 7251 | 8480 | 9925 | 11465 | 13007 | 14623 | 15941 | 17421 |
| 1686 | 2981 | 4498 | 5942 | 7252 | 8481 | 9926 | 11466 | 13008 | 14624 | 15942 | 17422 |
| 1689 | 2982 | 4499 | 5943 | 7253 | 8482 | 9927 | 11467 | 13009 | 14625 | 15943 | 17423 |
| 1691 | 2983 | 4500 | 5944 | 7254 | 8483 | 9928 | 11472 | 13010 | 14626 | 15944 | 17425 |
| 1692 | 2984 | 4501 | 5945 | 7255 | 8484 | 9929 | 11473 | 13011 | 14627 | 15945 | 17426 |
| 1693 | 2985 | 4502 | 5946 | 7256 | 8485 | 9931 | 11478 | 13012 | 14628 | 15946 | 17427 |
| 1694 | 3006 | 4503 | 5947 | 7257 | 8486 | 9932 | 11479 | 13013 | 14629 | 15947 | 17428 |
| 1695 | 3007 | 4504 | 5948 | 7258 | 8487 | 9933 | 11480 | 13014 | 14630 | 15948 | 17479 |
| 1696 | 3008 | 4505 | 5949 | 7259 | 8488 | 9934 | 11481 | 13015 | 14631 | 15949 | 17480 |
| 1697 | 3009 | 4506 | 5950 | 7260 | 8489 | 9935 | 11482 | 13030 | 14632 | 15950 | 17482 |
| 1698 | 3010 | 4507 | 5951 | 7266 | 8490 | 9936 | 11483 | 13031 | 14633 | 15951 | 17483 |
| 1699 | 3011 | 4513 | 5952 | 7267 | 8491 | 9937 | 11484 | 13032 | 14634 | 15952 | 17496 |
| 1720 | 3012 | 4514 | 5953 | 7268 | 8492 | 9938 | 11485 | 13033 | 14635 | 15953 | 17497 |
| 1721 | 3013 | 4515 | 5954 | 7269 | 8493 | 9939 | 11486 | 13034 | 14636 | 15954 | 17498 |
| 1734 | 3014 | 4516 | 5955 | 7270 | 8494 | 9940 | 11487 | 13035 | 14637 | 15955 | 17499 |
| 1735 | 3015 | 4517 | 5956 | 7271 | 8495 | 9941 | 11488 | 13036 | 14638 | 15956 | 17500 |
| 1736 | 3016 | 4518 | 5957 | 7272 | 8496 | 9942 | 11499 | 13037 | 14639 | 15957 | |
| 1737 | 3017 | 4519 | 5958 | 7273 | 8497 | 9943 | 11500 | 13038 | 14640 | 15958 | |
| 1738 | 3018 | 4520 | 5959 | 7274 | 8498 | 9944 | 11501 | 13039 | 14641 | 15978 | |
| 1739 | 3019 | 4521 | 5960 | 7275 | 8499 | 9945 | 11502 | 13040 | 14646 | 15979 | |
| 1740 | 3020 | 4522 | 5961 | 7276 | 8500 | 9946 | 11503 | 13041 | 14647 | 15980 | |
| 1741 | 3021 | 4523 | 5962 | 7277 | 8501 | 9947 | 11504 | 13042 | 14648 | 15981 | |
| 1742 | 3022 | 4524 | 5963 | 7278 | 8502 | 9951 | 11505 | 13043 | 14649 | 15982 | |
| 1743 | 3023 | 4537 | 5964 | 7279 | 8503 | 9952 | 11506 | 13044 | 14650 | 15983 | |
| 1744 | 3024 | 4538 | 5965 | 7280 | 8504 | 9965 | 11507 | 13045 | 14651 | 15984 | |

**Table 3 - downregulated proteins**

| **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** |
|---|---|---|---|---|---|---|---|---|---|---|---|
| 1 | 1258 | 2607 | 4209 | 5666 | 7346 | 9060 | 10597 | 11947 | 13198 | 14497 | 16197 |
| 2 | 1259 | 2608 | 4210 | 5667 | 7347 | 9061 | 10598 | 11948 | 13199 | 14514 | 16198 |
| 3 | 1260 | 2609 | 4211 | 5690 | 7348 | 9062 | 10599 | 11949 | 13200 | 14515 | 16199 |
| 4 | 1261 | 2610 | 4218 | 5691 | 7349 | 9063 | 10600 | 11950 | 13201 | 14516 | 16200 |
| 5 | 1262 | 2611 | 4219 | 5692 | 7350 | 9064 | 10601 | 11951 | 13202 | 14518 | 16201 |
| 6 | 1263 | 2612 | 4220 | 5693 | 7351 | 9065 | 10602 | 11952 | 13203 | 14519 | 16202 |
| 7 | 1264 | 2613 | 4221 | 5694 | 7352 | 9066 | 10603 | 11953 | 13204 | 14520 | 16203 |
| 28 | 1265 | 2614 | 4222 | 5695 | 7353 | 9067 | 10604 | 11954 | 13205 | 14521 | 16204 |
| 29 | 1266 | 2615 | 4223 | 5696 | 7380 | 9068 | 10605 | 11955 | 13206 | 14522 | 16205 |
| 30 | 1267 | 2616 | 4241 | 5697 | 7381 | 9069 | 10606 | 11956 | 13207 | 14523 | 16206 |
| 31 | 1268 | 2617 | 4242 | 5698 | 7382 | 9070 | 10607 | 11957 | 13213 | 14524 | 16207 |
| 32 | 1270 | 2618 | 4243 | 5705 | 7383 | 9071 | 10612 | 11958 | 13214 | 14525 | 16208 |
| 33 | 1271 | 2619 | 4244 | 5706 | 7384 | 9072 | 10613 | 11959 | 13215 | 14526 | 16209 |
| 34 | 1272 | 2620 | 4245 | 5707 | 7385 | 9073 | 10614 | 11960 | 13216 | 14527 | 16210 |
| 35 | 1273 | 2621 | 4246 | 5708 | 7386 | 9074 | 10615 | 11961 | 13217 | 14528 | 16211 |
| 36 | 1274 | 2622 | 4247 | 5709 | 7387 | 9075 | 10616 | 11962 | 13218 | 14531 | 16212 |
| 37 | 1275 | 2623 | 4248 | 5710 | 7388 | 9076 | 10617 | 11963 | 13219 | 14532 | 16213 |
| 49 | 1276 | 2624 | 4249 | 5711 | 7389 | 9077 | 10618 | 11964 | 13220 | 14533 | 16214 |
| 66 | 1277 | 2657 | 4250 | 5712 | 7390 | 9078 | 10620 | 11965 | 13221 | 14534 | 16215 |
| 67 | 1278 | 2658 | 4251 | 5713 | 7391 | 9079 | 10621 | 11966 | 13222 | 14535 | 16216 |
| 68 | 1279 | 2659 | 4252 | 5719 | 7392 | 9080 | 10622 | 11967 | 13230 | 14536 | 16217 |
| 69 | 1280 | 2660 | 4253 | 5720 | 7393 | 9081 | 10623 | 11968 | 13231 | 14537 | 16218 |
| 70 | 1283 | 2661 | 4254 | 5721 | 7394 | 9082 | 10624 | 11969 | 13232 | 14538 | 16219 |
| 71 | 1284 | 2662 | 4255 | 5722 | 7411 | 9094 | 10625 | 11970 | 13233 | 14555 | 16220 |
| 72 | 1285 | 2663 | 4256 | 5723 | 7412 | 9095 | 10626 | 11971 | 13234 | 14556 | 16221 |
| 73 | 1286 | 2664 | 4257 | 5724 | 7413 | 9096 | 10627 | 11972 | 13248 | 14557 | 16222 |
| 74 | 1287 | 2665 | 4258 | 5760 | 7414 | 9097 | 10628 | 11973 | 13249 | 14558 | 16223 |
| 75 | 1289 | 2715 | 4259 | 5764 | 7415 | 9098 | 10629 | 11974 | 13250 | 14559 | 16224 |
| 76 | 1290 | 2716 | 4260 | 5765 | 7416 | 9099 | 10630 | 11983 | 13251 | 14560 | 16225 |
| 77 | 1291 | 2717 | 4261 | 5766 | 7464 | 9100 | 10631 | 11984 | 13252 | 14561 | 16226 |
| 78 | 1292 | 2733 | 4262 | 5767 | 7465 | 9101 | 10632 | 11985 | 13253 | 14562 | 16227 |
| 79 | 1293 | 2734 | 4263 | 5768 | 7466 | 9102 | 10633 | 11986 | 13254 | 14563 | 16228 |
| 80 | 1294 | 2735 | 4264 | 5769 | 7467 | 9103 | 10634 | 11987 | 13255 | 14567 | 16229 |
| 81 | 1295 | 2736 | 4265 | 5770 | 7468 | 9104 | 10635 | 11998 | 13256 | 14568 | 16230 |
| 104 | 1296 | 2755 | 4266 | 5771 | 7469 | 9105 | 10636 | 11999 | 13263 | 14593 | 16231 |
| 105 | 1297 | 2756 | 4267 | 5772 | 7470 | 9106 | 10637 | 12000 | 13264 | 14594 | 16232 |
| 106 | 1298 | 2757 | 4268 | 5773 | 7471 | 9107 | 10638 | 12001 | 13265 | 14595 | 16233 |
| 107 | 1299 | 2758 | 4269 | 5774 | 7472 | 9108 | 10639 | 12007 | 13266 | 14596 | 16234 |
| 108 | 1300 | 2759 | 4270 | 5775 | 7478 | 9109 | 10640 | 12008 | 13267 | 14597 | 16235 |
| 109 | 1301 | 2760 | 4271 | 5776 | 7479 | 9110 | 10641 | 12009 | 13268 | 14598 | 16236 |
| 119 | 1302 | 2761 | 4272 | 5777 | 7480 | 9111 | 10642 | 12013 | 13269 | 14599 | 16237 |
| 153 | 1303 | 2762 | 4273 | 5778 | 7481 | 9112 | 10643 | 12014 | 13270 | 14600 | 16238 |
| 154 | 1304 | 2763 | 4274 | 5779 | 7482 | 9113 | 10644 | 12015 | 13271 | 14601 | 16239 |
| 155 | 1305 | 2764 | 4275 | 5780 | 7483 | 9114 | 10645 | 12016 | 13272 | 14602 | 16240 |
| 156 | 1306 | 2765 | 4276 | 5781 | 7484 | 9115 | 10646 | 12019 | 13282 | 14603 | 16241 |
| 157 | 1307 | 2766 | 4277 | 5782 | 7485 | 9116 | 10647 | 12020 | 13283 | 14604 | 16242 |
| 158 | 1308 | 2767 | 4278 | 5783 | 7486 | 9117 | 10655 | 12021 | 13284 | 14605 | 16243 |
| 159 | 1309 | 2768 | 4279 | 5784 | 7487 | 9118 | 10656 | 12022 | 13285 | 14606 | 16244 |
| 160 | 1310 | 2769 | 4280 | 5785 | 7488 | 9119 | 10657 | 12023 | 13286 | 14607 | 16245 |
| 161 | 1311 | 2770 | 4281 | 5786 | 7489 | 9120 | 10658 | 12024 | 13287 | 14608 | 16246 |
| 162 | 1312 | 2771 | 4282 | 5787 | 7490 | 9121 | 10659 | 12025 | 13288 | 14609 | 16247 |
| 163 | 1333 | 2772 | 4289 | 5788 | 7491 | 9122 | 10667 | 12026 | 13305 | 14610 | 16248 |
| 164 | 1334 | 2773 | 4290 | 5789 | 7492 | 9123 | 10668 | 12027 | 13306 | 14611 | 16249 |
| 165 | 1335 | 2774 | 4291 | 5790 | 7493 | 9124 | 10669 | 12028 | 13307 | 14612 | 16250 |
| 166 | 1336 | 2775 | 4292 | 5791 | 7505 | 9125 | 10670 | 12029 | 13308 | 14613 | 16251 |
| 167 | 1337 | 2776 | 4293 | 5792 | 7506 | 9126 | 10671 | 12030 | 13309 | 14614 | 16267 |
| 168 | 1338 | 2777 | 4294 | 5793 | 7507 | 9127 | 10672 | 12031 | 13310 | 14615 | 16268 |
| 169 | 1339 | 2778 | 4296 | 5794 | 7508 | 9128 | 10673 | 12032 | 13311 | 14642 | 16269 |
| 170 | 1364 | 2786 | 4298 | 5795 | 7509 | 9129 | 10674 | 12033 | 13312 | 14643 | 16286 |
| 171 | 1365 | 2787 | 4299 | 5796 | 7510 | 9130 | 10675 | 12035 | 13313 | 14644 | 16287 |
| 172 | 1366 | 2788 | 4300 | 5797 | 7511 | 9131 | 10676 | 12036 | 13314 | 14645 | 16288 |
| 173 | 1367 | 2798 | 4301 | 5798 | 7512 | 9132 | 10677 | 12037 | 13315 | 14663 | 16289 |
| 174 | 1368 | 2799 | 4302 | 5799 | 7513 | 9133 | 10678 | 12038 | 13316 | 14664 | 16290 |
| 175 | 1369 | 2826 | 4314 | 5800 | 7514 | 9134 | 10679 | 12039 | 13317 | 14665 | 16291 |
| 176 | 1370 | 2827 | 4315 | 5801 | 7515 | 9135 | 10680 | 12040 | 13318 | 14666 | 16292 |
| 177 | 1371 | 2828 | 4316 | 5802 | 7516 | 9136 | 10681 | 12041 | 13319 | 14667 | 16293 |
| 178 | 1372 | 2829 | 4317 | 5803 | 7517 | 9137 | 10682 | 12042 | 13320 | 14668 | 16294 |
| 195 | 1373 | 2830 | 4318 | 5804 | 7518 | 9138 | 10683 | 12043 | 13321 | 14669 | 16301 |
| 196 | 1374 | 2831 | 4319 | 5825 | 7519 | 9139 | 10700 | 12044 | 13322 | 14670 | 16302 |
| 197 | 1375 | 2832 | 4320 | 5826 | 7520 | 9140 | 10701 | 12045 | 13323 | 14680 | 16303 |
| 198 | 1376 | 2857 | 4321 | 5827 | 7521 | 9141 | 10702 | 12046 | 13324 | 14681 | 16304 |
| 199 | 1377 | 2858 | 4322 | 5828 | 7522 | 9142 | 10703 | 12047 | 13325 | 14682 | 16305 |
| 200 | 1378 | 2859 | 4323 | 5829 | 7523 | 9145 | 10704 | 12048 | 13326 | 14683 | 16337 |
| 201 | 1379 | 2861 | 4324 | 5830 | 7524 | 9146 | 10705 | 12049 | 13327 | 14684 | 16338 |
| 202 | 1380 | 2862 | 4325 | 5831 | 7525 | 9147 | 10715 | 12050 | 13339 | 14685 | 16339 |
| 203 | 1381 | 2863 | 4326 | 5832 | 7526 | 9148 | 10716 | 12051 | 13340 | 14686 | 16340 |
| 211 | 1382 | 2881 | 4327 | 5845 | 7527 | 9149 | 10717 | 12052 | 13341 | 14708 | 16341 |
| 212 | 1383 | 2882 | 4346 | 5846 | 7528 | 9150 | 10718 | 12053 | 13342 | 14709 | 16342 |
| 213 | 1389 | 2883 | 4347 | 5847 | 7529 | 9167 | 10719 | 12054 | 13343 | 14710 | 16343 |
| 214 | 1402 | 2884 | 4377 | 5848 | 7530 | 9168 | 10720 | 12055 | 13344 | 14711 | 16344 |
| 215 | 1403 | 2885 | 4378 | 5849 | 7531 | 9174 | 10721 | 12056 | 13348 | 14712 | 16345 |
| 216 | 1404 | 2886 | 4379 | 5850 | 7532 | 9175 | 10722 | 12057 | 13349 | 14713 | 16346 |
| 217 | 1405 | 2887 | 4380 | 5851 | 7533 | 9176 | 10723 | 12058 | 13350 | 14714 | 16347 |
| 218 | 1406 | 2888 | 4381 | 5852 | 7534 | 9177 | 10724 | 12059 | 13351 | 14715 | 16348 |
| 242 | 1407 | 2922 | 4382 | 5853 | 7535 | 9178 | 10725 | 12060 | 13352 | 14716 | 16349 |
| 243 | 1408 | 2923 | 4383 | 5854 | 7536 | 9179 | 10726 | 12082 | 13353 | 14717 | 16350 |
| 244 | 1409 | 2986 | 4384 | 5874 | 7537 | 9180 | 10727 | 12083 | 13354 | 14718 | 16351 |
| 245 | 1410 | 2987 | 4385 | 5875 | 7583 | 9181 | 10728 | 12084 | 13355 | 14719 | 16352 |
| 246 | 1411 | 2988 | 4387 | 5876 | 7584 | 9182 | 10729 | 12089 | 13356 | 14720 | 16353 |
| 247 | 1418 | 2989 | 4388 | 5877 | 7585 | 9208 | 10730 | 12090 | 13357 | 14721 | 16354 |
| 248 | 1419 | 2990 | 4389 | 5878 | 7586 | 9209 | 10731 | 12091 | 13358 | 14722 | 16355 |
| 249 | 1420 | 2991 | 4390 | 5879 | 7587 | 9210 | 10732 | 12092 | 13359 | 14723 | 16356 |
| 250 | 1421 | 2992 | 4391 | 5880 | 7588 | 9211 | 10733 | 12093 | 13360 | 14724 | 16357 |
| 251 | 1422 | 2993 | 4392 | 5881 | 7589 | 9212 | 10734 | 12094 | 13361 | 14725 | 16358 |
| 252 | 1423 | 2994 | 4393 | 5882 | 7590 | 9213 | 10735 | 12095 | 13362 | 14726 | 16359 |
| 253 | 1424 | 2995 | 4394 | 5883 | 7591 | 9214 | 10736 | 12096 | 13363 | 14727 | 16360 |
| 254 | 1425 | 2996 | 4395 | 5884 | 7592 | 9215 | 10737 | 12097 | 13364 | 14728 | 16367 |
| 255 | 1426 | 2997 | 4396 | 5885 | 7593 | 9216 | 10738 | 12100 | 13365 | 14729 | 16368 |
| 256 | 1427 | 2998 | 4397 | 5886 | 7594 | 9217 | 10739 | 12101 | 13366 | 14730 | 16369 |
| 257 | 1428 | 2999 | 4425 | 5887 | 7597 | 9220 | 10740 | 12102 | 13367 | 14731 | 16370 |
| 258 | 1439 | 3000 | 4426 | 5888 | 7598 | 9221 | 10754 | 12103 | 13368 | 14737 | 16382 |
| 259 | 1440 | 3001 | 4427 | 5889 | 7599 | 9222 | 10755 | 12104 | 13369 | 14738 | 16383 |
| 260 | 1441 | 3002 | 4428 | 5890 | 7600 | 9223 | 10756 | 12115 | 13370 | 14739 | 16384 |
| 261 | 1442 | 3003 | 4429 | 5891 | 7601 | 9224 | 10757 | 12116 | 13371 | 14740 | 16389 |
| 262 | 1443 | 3004 | 4430 | 5892 | 7602 | 9225 | 10758 | 12117 | 13372 | 14741 | 16390 |
| 263 | 1444 | 3005 | 4431 | 5893 | 7603 | 9226 | 10759 | 12118 | 13373 | 14742 | 16391 |
| 264 | 1445 | 3028 | 4432 | 5894 | 7604 | 9227 | 10760 | 12119 | 13374 | 14743 | 16392 |
| 265 | 1446 | 3029 | 4433 | 5895 | 7605 | 9228 | 10761 | 12120 | 13375 | 14744 | 16397 |
| 273 | 1447 | 3030 | 4434 | 5896 | 7606 | 9229 | 10762 | 12121 | 13376 | 14745 | 16398 |
| 274 | 1448 | 3031 | 4435 | 5897 | 7616 | 9230 | 10763 | 12122 | 13377 | 14746 | 16399 |
| 275 | 1449 | 3032 | 4436 | 5898 | 7617 | 9231 | 10781 | 12125 | 13378 | 14747 | 16400 |
| 276 | 1450 | 3033 | 4437 | 5899 | 7618 | 9232 | 10782 | 12126 | 13379 | 14748 | 16401 |
| 277 | 1451 | 3034 | 4438 | 5900 | 7619 | 9233 | 10783 | 12127 | 13380 | 14749 | 16402 |
| 278 | 1452 | 3035 | 4439 | 5901 | 7620 | 9234 | 10784 | 12128 | 13381 | 14750 | 16403 |
| 279 | 1453 | 3036 | 4440 | 5902 | 7621 | 9235 | 10785 | 12129 | 13382 | 14751 | 16404 |
| 280 | 1454 | 3037 | 4441 | 5903 | 7622 | 9236 | 10786 | 12130 | 13383 | 14752 | 16405 |
| 281 | 1455 | 3063 | 4442 | 5904 | 7642 | 9243 | 10787 | 12131 | 13384 | 14753 | 16406 |
| 282 | 1464 | 3064 | 4443 | 5905 | 7691 | 9244 | 10788 | 12132 | 13390 | 14754 | 16407 |
| 283 | 1465 | 3065 | 4444 | 5906 | 7692 | 9245 | 10789 | 12133 | 13391 | 14755 | 16410 |
| 284 | 1466 | 3066 | 4445 | 5907 | 7693 | 9246 | 10790 | 12134 | 13392 | 14756 | 16411 |
| 285 | 1467 | 3067 | 4446 | 5908 | 7694 | 9247 | 10791 | 12135 | 13393 | 14757 | 16412 |
| 286 | 1472 | 3068 | 4465 | 5909 | 7695 | 9248 | 10792 | 12136 | 13394 | 14758 | 16413 |
| 287 | 1473 | 3069 | 4466 | 5910 | 7696 | 9249 | 10793 | 12137 | 13426 | 14759 | 16414 |
| 288 | 1474 | 3070 | 4467 | 5911 | 7697 | 9250 | 10794 | 12138 | 13427 | 14760 | 16415 |
| 289 | 1475 | 3071 | 4468 | 5931 | 7698 | 9251 | 10799 | 12139 | 13428 | 14761 | 16416 |
| 290 | 1476 | 3072 | 4469 | 5932 | 7699 | 9252 | 10800 | 12140 | 13429 | 14762 | 16417 |
| 291 | 1477 | 3103 | 4470 | 5933 | 7700 | 9253 | 10808 | 12141 | 13430 | 14763 | 16418 |
| 295 | 1478 | 3104 | 4471 | 5934 | 7701 | 9254 | 10809 | 12142 | 13431 | 14764 | 16419 |
| 296 | 1479 | 3105 | 4472 | 5935 | 7702 | 9308 | 10810 | 12143 | 13432 | 14765 | 16420 |
| 297 | 1480 | 3106 | 4473 | 5974 | 7703 | 9309 | 10811 | 12144 | 13433 | 14766 | 16421 |
| 298 | 1481 | 3109 | 4474 | 5975 | 7704 | 9310 | 10812 | 12145 | 13434 | 14767 | 16422 |
| 299 | 1491 | 3110 | 4475 | 5976 | 7705 | 9311 | 10813 | 12146 | 13435 | 14768 | 16423 |
| 300 | 1492 | 3111 | 4476 | 5977 | 7706 | 9357 | 10814 | 12147 | 13446 | 14769 | 16424 |
| 301 | 1493 | 3112 | 4477 | 5978 | 7711 | 9419 | 10815 | 12148 | 13447 | 14770 | 16425 |
| 302 | 1494 | 3113 | 4478 | 5979 | 7712 | 9420 | 10816 | 12149 | 13448 | 14771 | 16426 |
| 303 | 1495 | 3114 | 4479 | 5980 | 7713 | 9421 | 10817 | 12150 | 13449 | 14806 | 16427 |
| 304 | 1496 | 3115 | 4480 | 6034 | 7776 | 9422 | 10818 | 12151 | 13450 | 14816 | 16428 |
| 305 | 1497 | 3116 | 4481 | 6035 | 7777 | 9423 | 10819 | 12152 | 13451 | 14817 | 16429 |
| 306 | 1498 | 3117 | 4482 | 6036 | 7778 | 9424 | 10820 | 12153 | 13452 | 14818 | 16430 |
| 307 | 1499 | 3118 | 4483 | 6037 | 7779 | 9425 | 10821 | 12154 | 13453 | 14819 | 16431 |
| 308 | 1503 | 3119 | 4484 | 6048 | 7780 | 9426 | 10822 | 12155 | 13468 | 14820 | 16432 |
| 309 | 1504 | 3120 | 4485 | 6049 | 7781 | 9427 | 10823 | 12156 | 13469 | 14821 | 16433 |
| 310 | 1547 | 3121 | 4486 | 6050 | 7782 | 9428 | 10824 | 12157 | 13470 | 14830 | 16434 |
| 311 | 1548 | 3122 | 4487 | 6051 | 7783 | 9429 | 10825 | 12158 | 13471 | 14831 | 16435 |
| 312 | 1549 | 3123 | 4508 | 6052 | 7784 | 9430 | 10826 | 12159 | 13472 | 14832 | 16436 |
| 313 | 1550 | 3124 | 4509 | 6053 | 7785 | 9431 | 10827 | 12160 | 13473 | 14833 | 16437 |
| 314 | 1551 | 3125 | 4510 | 6054 | 7786 | 9432 | 10828 | 12161 | 13474 | 14834 | 16438 |
| 315 | 1552 | 3126 | 4511 | 6055 | 7787 | 9433 | 10829 | 12162 | 13475 | 14835 | 16439 |
| 316 | 1553 | 3127 | 4512 | 6064 | 7788 | 9434 | 10830 | 12163 | 13476 | 14836 | 16440 |
| 317 | 1554 | 3128 | 4525 | 6065 | 7789 | 9435 | 10831 | 12164 | 13477 | 14837 | 16441 |
| 318 | 1555 | 3129 | 4526 | 6066 | 7790 | 9436 | 10832 | 12165 | 13478 | 14838 | 16442 |
| 319 | 1556 | 3130 | 4527 | 6067 | 7791 | 9437 | 10833 | 12166 | 13479 | 14853 | 16443 |
| 320 | 1557 | 3131 | 4528 | 6068 | 7792 | 9438 | 10834 | 12167 | 13480 | 14854 | 16444 |
| 321 | 1558 | 3132 | 4529 | 6069 | 7793 | 9439 | 10835 | 12168 | 13481 | 14855 | 16445 |
| 322 | 1559 | 3133 | 4530 | 6070 | 7794 | 9440 | 10836 | 12169 | 13482 | 14856 | 16446 |
| 323 | 1560 | 3137 | 4531 | 6071 | 7795 | 9441 | 10837 | 12170 | 13483 | 14857 | 16447 |
| 324 | 1561 | 3138 | 4532 | 6100 | 7796 | 9442 | 10838 | 12171 | 13484 | 14858 | 16448 |
| 325 | 1562 | 3139 | 4533 | 6101 | 7797 | 9457 | 10839 | 12172 | 13485 | 14859 | 16449 |
| 326 | 1563 | 3140 | 4534 | 6102 | 7798 | 9458 | 10840 | 12173 | 13486 | 14860 | 16450 |
| 327 | 1564 | 3141 | 4535 | 6103 | 7799 | 9459 | 10844 | 12174 | 13487 | 14861 | 16451 |
| 328 | 1565 | 3142 | 4536 | 6104 | 7800 | 9460 | 10845 | 12175 | 13488 | 14862 | 16452 |
| 329 | 1566 | 3143 | 4553 | 6105 | 7801 | 9461 | 10846 | 12176 | 13489 | 14863 | 16458 |
| 330 | 1567 | 3144 | 4554 | 6106 | 7802 | 9462 | 10847 | 12179 | 13490 | 14864 | 16459 |
| 331 | 1568 | 3145 | 4555 | 6107 | 7803 | 9463 | 10848 | 12180 | 13491 | 14865 | 16460 |
| 332 | 1569 | 3146 | 4556 | 6122 | 7804 | 9464 | 10849 | 12181 | 13492 | 14866 | 16499 |
| 333 | 1570 | 3147 | 4557 | 6123 | 7805 | 9465 | 10850 | 12182 | 13493 | 14867 | 16500 |
| 334 | 1571 | 3148 | 4558 | 6124 | 7806 | 9466 | 10851 | 12183 | 13494 | 14968 | 16501 |
| 335 | 1572 | 3149 | 4559 | 6125 | 7807 | 9467 | 10852 | 12184 | 13495 | 14969 | 16502 |
| 336 | 1573 | 3150 | 4560 | 6126 | 7808 | 9476 | 10853 | 12185 | 13496 | 14970 | 16503 |
| 337 | 1577 | 3151 | 4561 | 6127 | 7809 | 9477 | 10858 | 12186 | 13497 | 14971 | 16522 |
| 338 | 1578 | 3152 | 4562 | 6128 | 7810 | 9478 | 10859 | 12187 | 13498 | 14972 | 16523 |
| 339 | 1579 | 3153 | 4563 | 6129 | 7811 | 9479 | 10860 | 12188 | 13499 | 14973 | 16524 |
| 343 | 1580 | 3154 | 4564 | 6130 | 7887 | 9480 | 10861 | 12189 | 13500 | 14974 | 16534 |
| 366 | 1581 | 3155 | 4565 | 6131 | 7888 | 9481 | 10862 | 12190 | 13535 | 14975 | 16535 |
| 367 | 1582 | 3156 | 4566 | 6132 | 7889 | 9482 | 10863 | 12191 | 13536 | 14976 | 16536 |
| 368 | 1583 | 3157 | 4567 | 6133 | 7890 | 9483 | 10864 | 12192 | 13537 | 14977 | 16563 |
| 369 | 1584 | 3158 | 4568 | 6134 | 7891 | 9484 | 10865 | 12193 | 13538 | 14978 | 16564 |
| 404 | 1585 | 3159 | 4569 | 6137 | 7892 | 9485 | 10866 | 12194 | 13539 | 14979 | 16565 |
| 416 | 1586 | 3160 | 4570 | 6138 | 7893 | 9486 | 10867 | 12200 | 13540 | 14980 | 16566 |
| 417 | 1587 | 3161 | 4571 | 6139 | 7894 | 9487 | 10901 | 12201 | 13541 | 14981 | 16567 |
| 418 | 1588 | 3162 | 4577 | 6140 | 7895 | 9488 | 10902 | 12202 | 13542 | 15014 | 16568 |
| 419 | 1589 | 3163 | 4578 | 6141 | 7896 | 9489 | 10903 | 12203 | 13543 | 15015 | 16569 |
| 420 | 1590 | 3164 | 4579 | 6142 | 7897 | 9490 | 10904 | 12204 | 13544 | 15016 | 16570 |
| 421 | 1591 | 3165 | 4580 | 6143 | 7898 | 9491 | 10905 | 12205 | 13545 | 15017 | 16571 |
| 422 | 1592 | 3170 | 4581 | 6144 | 7899 | 9492 | 10906 | 12206 | 13582 | 15018 | 16572 |
| 423 | 1593 | 3171 | 4582 | 6145 | 7974 | 9493 | 10907 | 12207 | 13583 | 15019 | 16573 |
| 424 | 1598 | 3172 | 4583 | 6146 | 7975 | 9494 | 10908 | 12208 | 13584 | 15020 | 16574 |
| 425 | 1599 | 3173 | 4584 | 6163 | 7976 | 9495 | 10909 | 12209 | 13585 | 15021 | 16575 |
| 426 | 1600 | 3174 | 4585 | 6164 | 7977 | 9496 | 10916 | 12210 | 13586 | 15022 | 16576 |
| 427 | 1601 | 3189 | 4586 | 6165 | 7978 | 9497 | 10917 | 12232 | 13598 | 15023 | 16598 |
| 428 | 1602 | 3190 | 4587 | 6168 | 7979 | 9498 | 10918 | 12233 | 13599 | 15024 | 16599 |
| 429 | 1603 | 3191 | 4588 | 6169 | 7980 | 9499 | 10925 | 12234 | 13600 | 15025 | 16600 |
| 430 | 1604 | 3192 | 4589 | 6170 | 7981 | 9500 | 10926 | 12235 | 13606 | 15040 | 16601 |
| 463 | 1605 | 3193 | 4617 | 6171 | 7982 | 9501 | 10927 | 12236 | 13607 | 15041 | 16603 |
| 464 | 1606 | 3194 | 4618 | 6172 | 8020 | 9502 | 10928 | 12237 | 13608 | 15042 | 16604 |
| 465 | 1607 | 3195 | 4619 | 6173 | 8021 | 9503 | 10960 | 12238 | 13609 | 15043 | 16605 |
| 466 | 1608 | 3201 | 4620 | 6185 | 8022 | 9504 | 10961 | 12239 | 13610 | 15044 | 16606 |
| 467 | 1609 | 3202 | 4621 | 6186 | 8029 | 9505 | 10962 | 12240 | 13611 | 15045 | 16624 |
| 468 | 1610 | 3203 | 4622 | 6187 | 8030 | 9547 | 10963 | 12241 | 13612 | 15046 | 16625 |
| 469 | 1611 | 3204 | 4623 | 6188 | 8031 | 9548 | 10964 | 12242 | 13613 | 15047 | 16626 |
| 470 | 1612 | 3205 | 4639 | 6189 | 8032 | 9549 | 10965 | 12247 | 13614 | 15048 | 16627 |
| 471 | 1613 | 3206 | 4640 | 6190 | 8033 | 9550 | 10966 | 12248 | 13620 | 15091 | 16628 |
| 472 | 1614 | 3207 | 4641 | 6191 | 8034 | 9557 | 10967 | 12249 | 13621 | 15092 | 16629 |
| 473 | 1619 | 3223 | 4642 | 6192 | 8035 | 9558 | 10968 | 12250 | 13622 | 15093 | 16630 |
| 474 | 1620 | 3224 | 4643 | 6193 | 8060 | 9559 | 10969 | 12251 | 13623 | 15094 | 16642 |
| 475 | 1621 | 3225 | 4644 | 6194 | 8061 | 9560 | 10970 | 12252 | 13624 | 15095 | 16643 |
| 476 | 1622 | 3226 | 4645 | 6241 | 8062 | 9561 | 10971 | 12253 | 13625 | 15096 | 16644 |
| 477 | 1623 | 3227 | 4646 | 6298 | 8067 | 9562 | 10972 | 12254 | 13630 | 15097 | 16645 |
| 484 | 1624 | 3228 | 4647 | 6299 | 8068 | 9563 | 10973 | 12255 | 13631 | 15098 | 16646 |
| 485 | 1625 | 3229 | 4648 | 6300 | 8069 | 9564 | 10974 | 12256 | 13632 | 15099 | 16647 |
| 486 | 1626 | 3230 | 4649 | 6301 | 8070 | 9565 | 10975 | 12257 | 13643 | 15100 | 16648 |
| 487 | 1627 | 3231 | 4650 | 6302 | 8071 | 9566 | 10977 | 12258 | 13644 | 15101 | 16649 |
| 488 | 1628 | 3232 | 4651 | 6303 | 8072 | 9567 | 10978 | 12259 | 13645 | 15102 | 16650 |
| 489 | 1629 | 3233 | 4652 | 6304 | 8073 | 9580 | 10979 | 12260 | 13646 | 15103 | 16651 |
| 506 | 1637 | 3234 | 4653 | 6305 | 8074 | 9581 | 10980 | 12261 | 13647 | 15104 | 16652 |
| 507 | 1638 | 3235 | 4659 | 6306 | 8075 | 9582 | 11008 | 12262 | 13682 | 15105 | 16653 |
| 508 | 1639 | 3236 | 4660 | 6310 | 8076 | 9583 | 11009 | 12263 | 13683 | 15106 | 16654 |
| 509 | 1640 | 3238 | 4661 | 6311 | 8077 | 9584 | 11010 | 12264 | 13684 | 15107 | 16655 |
| 510 | 1641 | 3239 | 4662 | 6312 | 8078 | 9585 | 11011 | 12265 | 13685 | 15108 | 16656 |
| 511 | 1642 | 3240 | 4663 | 6313 | 8079 | 9586 | 11012 | 12266 | 13686 | 15109 | 16657 |
| 512 | 1647 | 3247 | 4664 | 6314 | 8080 | 9587 | 11013 | 12267 | 13687 | 15110 | 16658 |
| 513 | 1648 | 3248 | 4665 | 6315 | 8081 | 9588 | 11014 | 12268 | 13688 | 15111 | 16659 |
| 523 | 1649 | 3249 | 4666 | 6316 | 8082 | 9589 | 11022 | 12289 | 13689 | 15112 | 16660 |
| 524 | 1650 | 3250 | 4667 | 6317 | 8083 | 9590 | 11023 | 12303 | 13690 | 15113 | 16677 |
| 525 | 1651 | 3251 | 4668 | 6318 | 8084 | 9591 | 11024 | 12304 | 13706 | 15114 | 16678 |
| 526 | 1652 | 3260 | 4669 | 6319 | 8085 | 9592 | 11025 | 12305 | 13707 | 15115 | 16679 |
| 527 | 1653 | 3261 | 4670 | 6320 | 8086 | 9593 | 11026 | 12306 | 13708 | 15116 | 16680 |
| 528 | 1654 | 3262 | 4671 | 6321 | 8087 | 9594 | 11027 | 12307 | 13709 | 15117 | 16681 |
| 529 | 1655 | 3263 | 4672 | 6322 | 8088 | 9595 | 11028 | 12308 | 13710 | 15118 | 16682 |
| 530 | 1656 | 3264 | 4673 | 6328 | 8089 | 9596 | 11029 | 12309 | 13711 | 15119 | 16683 |
| 531 | 1657 | 3265 | 4686 | 6329 | 8090 | 9597 | 11030 | 12310 | 13719 | 15120 | 16684 |
| 532 | 1658 | 3266 | 4687 | 6330 | 8091 | 9598 | 11031 | 12311 | 13720 | 15121 | 16685 |
| 533 | 1659 | 3267 | 4688 | 6331 | 8092 | 9599 | 11032 | 12312 | 13721 | 15122 | 16686 |
| 560 | 1660 | 3268 | 4689 | 6332 | 8116 | 9600 | 11033 | 12313 | 13722 | 15123 | 16687 |
| 561 | 1661 | 3269 | 4690 | 6333 | 8117 | 9622 | 11034 | 12314 | 13723 | 15124 | 16688 |
| 562 | 1662 | 3270 | 4707 | 6334 | 8118 | 9623 | 11035 | 12315 | 13724 | 15125 | 16689 |
| 563 | 1663 | 3271 | 4708 | 6335 | 8119 | 9624 | 11036 | 12316 | 13725 | 15126 | 16690 |
| 564 | 1664 | 3272 | 4709 | 6336 | 8120 | 9625 | 11037 | 12317 | 13726 | 15127 | 16691 |
| 565 | 1665 | 3273 | 4710 | 6337 | 8121 | 9626 | 11038 | 12336 | 13727 | 15128 | 16692 |
| 566 | 1666 | 3274 | 4711 | 6338 | 8122 | 9627 | 11039 | 12337 | 13728 | 15129 | 16693 |
| 567 | 1667 | 3275 | 4712 | 6359 | 8123 | 9628 | 11040 | 12338 | 13729 | 15130 | 16705 |
| 568 | 1668 | 3276 | 4713 | 6360 | 8139 | 9629 | 11041 | 12339 | 13730 | 15131 | 16706 |
| 569 | 1669 | 3277 | 4714 | 6366 | 8140 | 9630 | 11042 | 12340 | 13731 | 15152 | 16707 |
| 570 | 1670 | 3278 | 4715 | 6379 | 8141 | 9631 | 11043 | 12345 | 13732 | 15153 | 16708 |
| 571 | 1671 | 3279 | 4716 | 6380 | 8142 | 9658 | 11044 | 12346 | 13733 | 15154 | 16709 |
| 572 | 1677 | 3280 | 4717 | 6381 | 8150 | 9659 | 11045 | 12347 | 13734 | 15155 | 16710 |
| 581 | 1678 | 3281 | 4718 | 6382 | 8151 | 9667 | 11046 | 12348 | 13735 | 15156 | 16711 |
| 582 | 1679 | 3282 | 4719 | 6383 | 8152 | 9668 | 11047 | 12349 | 13736 | 15200 | 16712 |
| 583 | 1680 | 3283 | 4720 | 6384 | 8153 | 9669 | 11048 | 12350 | 13737 | 15201 | 16713 |
| 584 | 1681 | 3284 | 4728 | 6385 | 8154 | 9670 | 11049 | 12351 | 13738 | 15202 | 16714 |
| 585 | 1687 | 3285 | 4729 | 6386 | 8155 | 9671 | 11060 | 12352 | 13739 | 15203 | 16715 |
| 586 | 1688 | 3286 | 4730 | 6387 | 8156 | 9672 | 11061 | 12353 | 13740 | 15204 | 16716 |
| 587 | 1690 | 3287 | 4731 | 6388 | 8157 | 9673 | 11062 | 12354 | 13741 | 15205 | 16717 |
| 588 | 1700 | 3288 | 4732 | 6389 | 8158 | 9674 | 11063 | 12355 | 13742 | 15206 | 16718 |
| 589 | 1701 | 3289 | 4733 | 6390 | 8159 | 9675 | 11064 | 12356 | 13743 | 15237 | 16720 |
| 602 | 1702 | 3290 | 4734 | 6391 | 8160 | 9676 | 11065 | 12357 | 13744 | 15241 | 16721 |
| 603 | 1703 | 3291 | 4735 | 6392 | 8161 | 9677 | 11066 | 12358 | 13745 | 15242 | 16722 |
| 604 | 1704 | 3292 | 4736 | 6393 | 8162 | 9678 | 11067 | 12359 | 13746 | 15243 | 16723 |
| 605 | 1705 | 3311 | 4737 | 6394 | 8184 | 9679 | 11068 | 12360 | 13747 | 15244 | 16724 |
| 606 | 1706 | 3312 | 4763 | 6395 | 8185 | 9680 | 11069 | 12361 | 13748 | 15245 | 16725 |
| 607 | 1707 | 3313 | 4764 | 6396 | 8186 | 9720 | 11070 | 12362 | 13749 | 15247 | 16745 |
| 608 | 1708 | 3314 | 4765 | 6409 | 8235 | 9721 | 11071 | 12363 | 13750 | 15248 | 16746 |
| 609 | 1709 | 3315 | 4766 | 6410 | 8236 | 9722 | 11072 | 12364 | 13751 | 15249 | 16747 |
| 610 | 1710 | 3316 | 4767 | 6411 | 8237 | 9723 | 11073 | 12365 | 13782 | 15250 | 16748 |
| 611 | 1711 | 3317 | 4810 | 6447 | 8238 | 9724 | 11074 | 12366 | 13783 | 15251 | 16749 |
| 612 | 1712 | 3318 | 4811 | 6448 | 8239 | 9725 | 11075 | 12367 | 13784 | 15252 | 16750 |
| 613 | 1713 | 3319 | 4812 | 6449 | 8240 | 9726 | 11100 | 12368 | 13785 | 15253 | 16751 |
| 620 | 1714 | 3320 | 4813 | 6450 | 8242 | 9727 | 11101 | 12369 | 13786 | 15254 | 16752 |
| 621 | 1715 | 3345 | 4814 | 6451 | 8243 | 9728 | 11102 | 12394 | 13787 | 15259 | 16753 |
| 622 | 1716 | 3346 | 4815 | 6452 | 8244 | 9729 | 11103 | 12395 | 13788 | 15260 | 16754 |
| 623 | 1717 | 3347 | 4816 | 6453 | 8245 | 9730 | 11104 | 12396 | 13789 | 15261 | 16755 |
| 624 | 1718 | 3348 | 4817 | 6454 | 8246 | 9731 | 11105 | 12397 | 13790 | 15262 | 16756 |
| 625 | 1719 | 3349 | 4818 | 6455 | 8247 | 9732 | 11135 | 12398 | 13791 | 15263 | 16757 |
| 626 | 1722 | 3350 | 4838 | 6456 | 8248 | 9733 | 11136 | 12399 | 13792 | 15264 | 16758 |
| 627 | 1723 | 3351 | 4839 | 6457 | 8263 | 9734 | 11153 | 12400 | 13793 | 15265 | 16759 |
| 628 | 1724 | 3352 | 4840 | 6458 | 8264 | 9735 | 11154 | 12401 | 13794 | 15266 | 16760 |
| 629 | 1725 | 3353 | 4841 | 6459 | 8265 | 9736 | 11155 | 12402 | 13795 | 15267 | 16761 |
| 630 | 1726 | 3409 | 4842 | 6460 | 8266 | 9737 | 11156 | 12403 | 13796 | 15268 | 16781 |
| 631 | 1727 | 3425 | 4843 | 6461 | 8267 | 9738 | 11157 | 12404 | 13797 | 15269 | 16790 |
| 632 | 1728 | 3426 | 4844 | 6462 | 8268 | 9739 | 11175 | 12405 | 13835 | 15301 | 16791 |
| 633 | 1729 | 3427 | 4845 | 6463 | 8269 | 9740 | 11176 | 12406 | 13836 | 15302 | 16792 |
| 634 | 1730 | 3428 | 4846 | 6464 | 8270 | 9741 | 11177 | 12407 | 13840 | 15303 | 16793 |
| 635 | 1731 | 3429 | 4847 | 6465 | 8271 | 9742 | 11178 | 12408 | 13841 | 15304 | 16794 |
| 636 | 1732 | 3430 | 4848 | 6466 | 8272 | 9743 | 11179 | 12427 | 13842 | 15305 | 16795 |
| 637 | 1733 | 3431 | 4849 | 6467 | 8273 | 9744 | 11180 | 12428 | 13877 | 15318 | 16806 |
| 638 | 1757 | 3432 | 4850 | 6468 | 8274 | 9745 | 11212 | 12429 | 13878 | 15319 | 16807 |
| 639 | 1758 | 3433 | 4851 | 6469 | 8275 | 9746 | 11213 | 12430 | 13879 | 15320 | 16808 |
| 675 | 1759 | 3434 | 4852 | 6470 | 8276 | 9747 | 11214 | 12431 | 13880 | 15321 | 16809 |
| 676 | 1760 | 3435 | 4853 | 6471 | 8277 | 9748 | 11215 | 12432 | 13881 | 15322 | 16810 |
| 679 | 1761 | 3436 | 4854 | 6472 | 8307 | 9749 | 11216 | 12433 | 13882 | 15323 | 16811 |
| 680 | 1762 | 3437 | 4855 | 6473 | 8308 | 9750 | 11217 | 12434 | 13883 | 15353 | 16812 |
| 681 | 1770 | 3438 | 4856 | 6474 | 8309 | 9751 | 11218 | 12435 | 13884 | 15354 | 16813 |
| 694 | 1771 | 3457 | 4857 | 6475 | 8310 | 9752 | 11219 | 12436 | 13885 | 15355 | 16814 |
| 695 | 1772 | 3458 | 4858 | 6476 | 8311 | 9753 | 11220 | 12437 | 13886 | 15404 | 16816 |
| 696 | 1773 | 3459 | 4859 | 6477 | 8312 | 9754 | 11221 | 12483 | 13887 | 15405 | 16817 |
| 697 | 1774 | 3472 | 4860 | 6478 | 8313 | 9755 | 11223 | 12484 | 13888 | 15406 | 16818 |
| 698 | 1775 | 3473 | 4861 | 6483 | 8314 | 9756 | 11224 | 12485 | 13889 | 15407 | 16819 |
| 699 | 1776 | 3474 | 4862 | 6484 | 8315 | 9757 | 11225 | 12489 | 13890 | 15408 | 16820 |
| 700 | 1777 | 3475 | 4863 | 6485 | 8316 | 9758 | 11226 | 12490 | 13931 | 15409 | 16821 |
| 701 | 1778 | 3476 | 4868 | 6486 | 8317 | 9759 | 11227 | 12491 | 13932 | 15410 | 16822 |
| 702 | 1779 | 3477 | 4869 | 6487 | 8318 | 9760 | 11251 | 12492 | 13933 | 15411 | 16823 |
| 706 | 1780 | 3478 | 4870 | 6488 | 8319 | 9761 | 11252 | 12493 | 13939 | 15412 | 16824 |
| 707 | 1781 | 3479 | 4871 | 6489 | 8320 | 9768 | 11253 | 12494 | 13940 | 15413 | 16825 |
| 708 | 1782 | 3486 | 4872 | 6490 | 8321 | 9769 | 11254 | 12495 | 13941 | 15421 | 16826 |
| 709 | 1783 | 3487 | 4910 | 6491 | 8322 | 9770 | 11255 | 12496 | 13942 | 15422 | 16827 |
| 710 | 1784 | 3488 | 4911 | 6492 | 8323 | 9771 | 11256 | 12497 | 13943 | 15423 | 16828 |
| 711 | 1785 | 3489 | 4912 | 6493 | 8324 | 9775 | 11257 | 12498 | 13944 | 15424 | 16829 |
| 712 | 1786 | 3490 | 4913 | 6494 | 8325 | 9776 | 11258 | 12499 | 13945 | 15425 | 16830 |
| 713 | 1787 | 3491 | 4914 | 6495 | 8326 | 9777 | 11259 | 12500 | 13946 | 15426 | 16831 |
| 714 | 1788 | 3492 | 4915 | 6496 | 8327 | 9778 | 11260 | 12501 | 13947 | 15427 | 16832 |
| 715 | 1789 | 3493 | 4916 | 6503 | 8328 | 9779 | 11261 | 12502 | 13948 | 15428 | 16833 |
| 716 | 1790 | 3494 | 4917 | 6504 | 8329 | 9780 | 11262 | 12503 | 13949 | 15429 | 16834 |
| 717 | 1791 | 3495 | 4918 | 6505 | 8330 | 9781 | 11263 | 12504 | 13950 | 15430 | 16835 |
| 718 | 1792 | 3496 | 4919 | 6506 | 8333 | 9782 | 11264 | 12505 | 13951 | 15431 | 16836 |
| 719 | 1793 | 3515 | 4920 | 6507 | 8334 | 9783 | 11265 | 12506 | 13952 | 15432 | 16837 |
| 720 | 1794 | 3516 | 4921 | 6508 | 8335 | 9784 | 11290 | 12507 | 13953 | 15433 | 16838 |
| 721 | 1795 | 3517 | 4922 | 6509 | 8338 | 9785 | 11291 | 12508 | 13954 | 15434 | 16839 |
| 722 | 1796 | 3522 | 4923 | 6510 | 8339 | 9786 | 11292 | 12509 | 13955 | 15435 | 16866 |
| 723 | 1797 | 3523 | 4924 | 6511 | 8340 | 9787 | 11293 | 12510 | 13956 | 15436 | 16867 |
| 729 | 1818 | 3532 | 4925 | 6512 | 8341 | 9788 | 11294 | 12511 | 13959 | 15437 | 16868 |
| 730 | 1819 | 3533 | 4926 | 6513 | 8342 | 9814 | 11295 | 12512 | 13960 | 15438 | 16869 |
| 731 | 1820 | 3534 | 4927 | 6514 | 8343 | 9815 | 11296 | 12536 | 13961 | 15439 | 16870 |
| 732 | 1821 | 3535 | 4945 | 6523 | 8344 | 9816 | 11297 | 12537 | 13988 | 15440 | 16871 |
| 733 | 1829 | 3536 | 4946 | 6524 | 8345 | 9817 | 11298 | 12538 | 13989 | 15441 | 16872 |
| 734 | 1856 | 3537 | 4947 | 6525 | 8346 | 9818 | 11299 | 12539 | 13990 | 15442 | 16873 |
| 741 | 1857 | 3538 | 4948 | 6526 | 8347 | 9819 | 11300 | 12540 | 13991 | 15443 | 16874 |
| 742 | 1858 | 3539 | 4949 | 6527 | 8348 | 9833 | 11301 | 12541 | 13992 | 15497 | 16881 |
| 743 | 1859 | 3540 | 4950 | 6528 | 8349 | 9834 | 11302 | 12542 | 13993 | 15498 | 16920 |
| 744 | 1860 | 3541 | 4978 | 6529 | 8350 | 9835 | 11303 | 12543 | 14002 | 15499 | 16921 |
| 745 | 1861 | 3542 | 4995 | 6530 | 8355 | 9836 | 11304 | 12544 | 14003 | 15500 | 16924 |
| 746 | 1862 | 3543 | 4996 | 6531 | 8356 | 9837 | 11305 | 12545 | 14004 | 15501 | 16925 |
| 747 | 1863 | 3544 | 4997 | 6532 | 8357 | 9838 | 11306 | 12546 | 14005 | 15518 | 16926 |
| 748 | 1864 | 3545 | 4998 | 6533 | 8363 | 9839 | 11307 | 12547 | 14006 | 15519 | 16927 |
| 749 | 1882 | 3546 | 5018 | 6534 | 8364 | 9840 | 11308 | 12548 | 14007 | 15520 | 16928 |
| 750 | 1883 | 3547 | 5019 | 6535 | 8365 | 9841 | 11309 | 12549 | 14008 | 15521 | 16929 |
| 751 | 1884 | 3548 | 5020 | 6536 | 8392 | 9849 | 11310 | 12550 | 14009 | 15522 | 16930 |
| 752 | 1885 | 3549 | 5021 | 6537 | 8393 | 9850 | 11311 | 12551 | 14010 | 15523 | 16931 |
| 753 | 1886 | 3550 | 5022 | 6538 | 8394 | 9851 | 11312 | 12552 | 14011 | 15524 | 16932 |
| 754 | 1887 | 3551 | 5023 | 6539 | 8395 | 9852 | 11313 | 12553 | 14012 | 15525 | 16933 |
| 755 | 1888 | 3552 | 5024 | 6540 | 8396 | 9853 | 11314 | 12585 | 14013 | 15526 | 16938 |
| 756 | 1889 | 3554 | 5025 | 6541 | 8397 | 9854 | 11315 | 12586 | 14014 | 15527 | 16939 |
| 757 | 1890 | 3555 | 5026 | 6542 | 8398 | 9855 | 11316 | 12587 | 14015 | 15528 | 16940 |
| 758 | 1891 | 3556 | 5027 | 6543 | 8399 | 9856 | 11317 | 12588 | 14016 | 15529 | 16941 |
| 759 | 1892 | 3557 | 5028 | 6544 | 8400 | 9857 | 11318 | 12589 | 14017 | 15530 | 16942 |
| 760 | 1893 | 3560 | 5029 | 6545 | 8401 | 9858 | 11319 | 12590 | 14018 | 15531 | 16960 |
| 761 | 1894 | 3561 | 5030 | 6546 | 8412 | 9859 | 11320 | 12591 | 14019 | 15574 | 16961 |
| 762 | 1895 | 3562 | 5038 | 6547 | 8413 | 9860 | 11321 | 12595 | 14020 | 15601 | 16962 |
| 763 | 1896 | 3571 | 5039 | 6548 | 8414 | 9861 | 11322 | 12596 | 14021 | 15622 | 16963 |
| 764 | 1897 | 3572 | 5043 | 6549 | 8415 | 9865 | 11323 | 12597 | 14031 | 15623 | 16964 |
| 765 | 1898 | 3573 | 5044 | 6550 | 8416 | 9866 | 11324 | 12598 | 14032 | 15624 | 16965 |
| 766 | 1899 | 3574 | 5045 | 6551 | 8417 | 9867 | 11325 | 12599 | 14033 | 15625 | 16966 |
| 767 | 1900 | 3575 | 5046 | 6560 | 8418 | 9868 | 11326 | 12600 | 14034 | 15626 | 16967 |
| 768 | 1901 | 3576 | 5047 | 6561 | 8419 | 9869 | 11327 | 12601 | 14038 | 15627 | 16968 |
| 769 | 1902 | 3577 | 5066 | 6569 | 8420 | 9905 | 11328 | 12602 | 14039 | 15628 | 16969 |
| 770 | 1903 | 3578 | 5067 | 6570 | 8421 | 9906 | 11329 | 12603 | 14041 | 15629 | 16999 |
| 771 | 1904 | 3579 | 5068 | 6571 | 8422 | 9907 | 11330 | 12604 | 14042 | 15630 | 17000 |
| 772 | 1905 | 3580 | 5069 | 6572 | 8423 | 9908 | 11331 | 12605 | 14043 | 15631 | 17001 |
| 773 | 1906 | 3634 | 5070 | 6573 | 8424 | 9909 | 11332 | 12606 | 14052 | 15632 | 17002 |
| 774 | 1907 | 3635 | 5071 | 6574 | 8425 | 9910 | 11333 | 12607 | 14053 | 15633 | 17003 |
| 775 | 1908 | 3636 | 5072 | 6575 | 8426 | 9911 | 11334 | 12608 | 14054 | 15634 | 17004 |
| 797 | 1909 | 3637 | 5073 | 6576 | 8427 | 9912 | 11335 | 12609 | 14055 | 15635 | 17005 |
| 798 | 1910 | 3645 | 5074 | 6577 | 8428 | 9913 | 11336 | 12610 | 14065 | 15636 | 17006 |
| 799 | 1911 | 3646 | 5075 | 6578 | 8429 | 9930 | 11337 | 12611 | 14066 | 15637 | 17007 |
| 800 | 1912 | 3647 | 5076 | 6579 | 8456 | 9948 | 11338 | 12612 | 14067 | 15638 | 17032 |
| 801 | 1913 | 3648 | 5077 | 6580 | 8457 | 9949 | 11339 | 12613 | 14068 | 15639 | 17033 |
| 802 | 1914 | 3649 | 5078 | 6581 | 8458 | 9950 | 11340 | 12623 | 14069 | 15640 | 17034 |
| 803 | 1915 | 3650 | 5079 | 6582 | 8459 | 9953 | 11341 | 12624 | 14070 | 15641 | 17035 |
| 804 | 1916 | 3651 | 5080 | 6583 | 8460 | 9954 | 11342 | 12625 | 14071 | 15642 | 17036 |
| 805 | 1917 | 3652 | 5087 | 6584 | 8461 | 9955 | 11343 | 12626 | 14072 | 15643 | 17042 |
| 806 | 1918 | 3653 | 5091 | 6585 | 8462 | 9956 | 11344 | 12627 | 14073 | 15644 | 17043 |
| 807 | 1919 | 3654 | 5092 | 6586 | 8463 | 9957 | 11345 | 12628 | 14074 | 15645 | 17044 |
| 808 | 1959 | 3655 | 5093 | 6587 | 8464 | 9958 | 11346 | 12629 | 14075 | 15646 | 17045 |
| 809 | 1960 | 3656 | 5094 | 6588 | 8465 | 9959 | 11347 | 12632 | 14076 | 15647 | 17046 |
| 820 | 1961 | 3657 | 5095 | 6589 | 8466 | 9960 | 11348 | 12633 | 14077 | 15648 | 17047 |
| 821 | 1962 | 3658 | 5096 | 6590 | 8467 | 9961 | 11349 | 12634 | 14078 | 15649 | 17048 |
| 822 | 1963 | 3659 | 5097 | 6591 | 8468 | 9962 | 11350 | 12635 | 14079 | 15650 | 17049 |
| 823 | 1964 | 3660 | 5098 | 6645 | 8514 | 9963 | 11351 | 12636 | 14080 | 15651 | 17050 |
| 824 | 1965 | 3661 | 5099 | 6646 | 8515 | 9964 | 11352 | 12637 | 14090 | 15652 | 17051 |
| 825 | 1966 | 3662 | 5100 | 6647 | 8516 | 9980 | 11353 | 12669 | 14100 | 15653 | 17052 |
| 826 | 1967 | 3663 | 5101 | 6648 | 8517 | 9981 | 11354 | 12670 | 14101 | 15654 | 17053 |
| 827 | 1968 | 3664 | 5102 | 6664 | 8518 | 9982 | 11355 | 12671 | 14102 | 15655 | 17054 |
| 828 | 1969 | 3665 | 5103 | 6665 | 8519 | 9983 | 11356 | 12672 | 14103 | 15656 | 17055 |
| 829 | 1970 | 3666 | 5104 | 6666 | 8520 | 9984 | 11357 | 12673 | 14104 | 15657 | 17056 |
| 830 | 1971 | 3667 | 5136 | 6667 | 8521 | 9985 | 11358 | 12674 | 14105 | 15658 | 17057 |
| 831 | 1972 | 3668 | 5137 | 6668 | 8522 | 9986 | 11359 | 12675 | 14106 | 15659 | 17058 |
| 832 | 1973 | 3698 | 5138 | 6698 | 8523 | 9987 | 11360 | 12676 | 14107 | 15676 | 17059 |
| 833 | 1974 | 3699 | 5139 | 6699 | 8524 | 9988 | 11370 | 12677 | 14108 | 15677 | 17060 |
| 834 | 1975 | 3700 | 5140 | 6700 | 8525 | 9989 | 11371 | 12678 | 14109 | 15678 | 17061 |
| 835 | 1976 | 3734 | 5141 | 6701 | 8526 | 9990 | 11372 | 12679 | 14110 | 15679 | 17062 |
| 836 | 1977 | 3735 | 5142 | 6702 | 8527 | 9999 | 11373 | 12680 | 14111 | 15680 | 17063 |
| 837 | 1978 | 3736 | 5143 | 6703 | 8528 | 10000 | 11374 | 12681 | 14112 | 15681 | 17064 |
| 857 | 1979 | 3737 | 5146 | 6704 | 8529 | 10001 | 11375 | 12682 | 14113 | 15682 | 17065 |
| 870 | 1980 | 3738 | 5147 | 6705 | 8530 | 10002 | 11376 | 12683 | 14114 | 15683 | 17066 |
| 871 | 1981 | 3755 | 5161 | 6706 | 8531 | 10003 | 11377 | 12684 | 14115 | 15684 | 17067 |
| 872 | 1982 | 3756 | 5162 | 6707 | 8566 | 10004 | 11378 | 12689 | 14116 | 15685 | 17068 |
| 873 | 1983 | 3757 | 5163 | 6708 | 8567 | 10005 | 11379 | 12690 | 14117 | 15686 | 17069 |
| 874 | 1984 | 3758 | 5164 | 6709 | 8568 | 10006 | 11380 | 12691 | 14118 | 15687 | 17070 |
| 875 | 1985 | 3759 | 5165 | 6710 | 8569 | 10037 | 11392 | 12707 | 14119 | 15688 | 17071 |
| 879 | 1986 | 3760 | 5166 | 6711 | 8570 | 10038 | 11393 | 12708 | 14120 | 15689 | 17072 |
| 880 | 1987 | 3761 | 5167 | 6712 | 8571 | 10039 | 11394 | 12709 | 14121 | 15705 | 17073 |
| 881 | 1988 | 3762 | 5168 | 6713 | 8572 | 10040 | 11395 | 12710 | 14122 | 15706 | 17074 |
| 890 | 1989 | 3763 | 5169 | 6714 | 8573 | 10041 | 11396 | 12711 | 14123 | 15707 | 17075 |
| 891 | 2016 | 3764 | 5170 | 6715 | 8574 | 10042 | 11397 | 12712 | 14124 | 15708 | 17076 |
| 892 | 2017 | 3765 | 5171 | 6716 | 8575 | 10043 | 11398 | 12713 | 14133 | 15709 | 17077 |
| 893 | 2018 | 3766 | 5172 | 6717 | 8576 | 10044 | 11399 | 12714 | 14134 | 15710 | 17078 |
| 894 | 2045 | 3767 | 5178 | 6718 | 8577 | 10045 | 11400 | 12715 | 14139 | 15711 | 17079 |
| 906 | 2046 | 3768 | 5179 | 6719 | 8578 | 10047 | 11401 | 12716 | 14140 | 15712 | 17080 |
| 907 | 2047 | 3769 | 5180 | 6720 | 8579 | 10048 | 11402 | 12717 | 14141 | 15713 | 17081 |
| 908 | 2048 | 3770 | 5181 | 6726 | 8580 | 10049 | 11403 | 12718 | 14142 | 15714 | 17082 |
| 909 | 2049 | 3797 | 5182 | 6727 | 8581 | 10050 | 11405 | 12719 | 14143 | 15715 | 17083 |
| 910 | 2050 | 3798 | 5183 | 6731 | 8582 | 10051 | 11406 | 12720 | 14144 | 15716 | 17084 |
| 928 | 2051 | 3799 | 5184 | 6732 | 8583 | 10052 | 11407 | 12721 | 14145 | 15717 | 17085 |
| 929 | 2052 | 3800 | 5185 | 6733 | 8584 | 10053 | 11408 | 12722 | 14146 | 15718 | 17086 |
| 930 | 2053 | 3801 | 5186 | 6734 | 8585 | 10054 | 11409 | 12723 | 14147 | 15719 | 17087 |
| 931 | 2054 | 3802 | 5187 | 6735 | 8586 | 10055 | 11410 | 12724 | 14148 | 15720 | 17095 |
| 932 | 2062 | 3803 | 5188 | 6736 | 8587 | 10056 | 11411 | 12725 | 14149 | 15721 | 17096 |
| 933 | 2063 | 3804 | 5189 | 6737 | 8588 | 10057 | 11412 | 12726 | 14150 | 15722 | 17097 |
| 934 | 2064 | 3805 | 5190 | 6738 | 8589 | 10058 | 11413 | 12727 | 14151 | 15723 | 17098 |
| 935 | 2065 | 3806 | 5191 | 6750 | 8590 | 10059 | 11414 | 12728 | 14152 | 15724 | 17116 |
| 936 | 2066 | 3807 | 5192 | 6751 | 8591 | 10060 | 11415 | 12729 | 14153 | 15725 | 17117 |
| 937 | 2067 | 3808 | 5193 | 6752 | 8592 | 10061 | 11416 | 12730 | 14154 | 15726 | 17118 |
| 938 | 2073 | 3809 | 5194 | 6753 | 8593 | 10062 | 11417 | 12731 | 14155 | 15727 | 17119 |
| 939 | 2074 | 3810 | 5195 | 6754 | 8594 | 10063 | 11418 | 12732 | 14156 | 15728 | 17120 |
| 940 | 2096 | 3811 | 5196 | 6755 | 8595 | 10064 | 11419 | 12733 | 14157 | 15729 | 17121 |
| 943 | 2097 | 3812 | 5197 | 6756 | 8596 | 10065 | 11420 | 12734 | 14171 | 15730 | 17122 |
| 944 | 2098 | 3813 | 5198 | 6757 | 8610 | 10066 | 11421 | 12735 | 14172 | 15731 | 17123 |
| 945 | 2099 | 3814 | 5199 | 6758 | 8611 | 10067 | 11422 | 12736 | 14173 | 15732 | 17124 |
| 946 | 2100 | 3815 | 5200 | 6759 | 8612 | 10068 | 11423 | 12737 | 14174 | 15733 | 17125 |
| 947 | 2101 | 3816 | 5208 | 6760 | 8613 | 10069 | 11424 | 12738 | 14175 | 15734 | 17126 |
| 948 | 2102 | 3817 | 5209 | 6761 | 8614 | 10070 | 11425 | 12744 | 14176 | 15735 | 17127 |
| 949 | 2103 | 3818 | 5210 | 6762 | 8615 | 10071 | 11426 | 12745 | 14177 | 15736 | 17128 |
| 950 | 2104 | 3819 | 5211 | 6763 | 8616 | 10072 | 11427 | 12746 | 14178 | 15737 | 17129 |
| 951 | 2129 | 3820 | 5212 | 6764 | 8618 | 10073 | 11434 | 12747 | 14179 | 15738 | 17130 |
| 952 | 2130 | 3821 | 5213 | 6765 | 8619 | 10074 | 11435 | 12748 | 14180 | 15739 | 17131 |
| 960 | 2131 | 3844 | 5214 | 6766 | 8620 | 10075 | 11436 | 12749 | 14181 | 15740 | 17132 |
| 961 | 2132 | 3845 | 5215 | 6767 | 8621 | 10076 | 11437 | 12750 | 14182 | 15741 | 17133 |
| 962 | 2133 | 3846 | 5216 | 6768 | 8622 | 10077 | 11438 | 12751 | 14183 | 15742 | 17156 |
| 963 | 2134 | 3857 | 5217 | 6769 | 8623 | 10078 | 11439 | 12752 | 14184 | 15743 | 17157 |
| 964 | 2135 | 3858 | 5218 | 6770 | 8624 | 10079 | 11440 | 12758 | 14185 | 15778 | 17158 |
| 965 | 2136 | 3859 | 5219 | 6771 | 8625 | 10080 | 11441 | 12759 | 14186 | 15779 | 17159 |
| 966 | 2137 | 3860 | 5220 | 6772 | 8626 | 10081 | 11457 | 12760 | 14187 | 15780 | 17160 |
| 967 | 2138 | 3861 | 5221 | 6773 | 8627 | 10082 | 11458 | 12761 | 14188 | 15781 | 17169 |
| 968 | 2139 | 3862 | 5222 | 6774 | 8628 | 10083 | 11459 | 12762 | 14189 | 15782 | 17170 |
| 969 | 2140 | 3863 | 5223 | 6775 | 8629 | 10084 | 11468 | 12763 | 14190 | 15783 | 17171 |
| 970 | 2141 | 3864 | 5224 | 6776 | 8630 | 10085 | 11469 | 12764 | 14191 | 15784 | 17172 |
| 971 | 2142 | 3865 | 5225 | 6777 | 8631 | 10086 | 11470 | 12765 | 14192 | 15785 | 17173 |
| 972 | 2143 | 3866 | 5226 | 6778 | 8654 | 10087 | 11471 | 12766 | 14193 | 15786 | 17174 |
| 973 | 2144 | 3867 | 5231 | 6779 | 8655 | 10088 | 11474 | 12767 | 14194 | 15787 | 17175 |
| 974 | 2145 | 3868 | 5232 | 6780 | 8656 | 10102 | 11475 | 12771 | 14210 | 15788 | 17176 |
| 975 | 2146 | 3869 | 5233 | 6781 | 8657 | 10103 | 11476 | 12823 | 14211 | 15789 | 17177 |
| 976 | 2147 | 3870 | 5234 | 6782 | 8658 | 10123 | 11477 | 12824 | 14212 | 15790 | 17178 |
| 977 | 2148 | 3871 | 5235 | 6783 | 8659 | 10124 | 11489 | 12825 | 14213 | 15791 | 17179 |
| 978 | 2168 | 3872 | 5236 | 6784 | 8660 | 10125 | 11490 | 12826 | 14215 | 15792 | 17180 |
| 979 | 2169 | 3873 | 5237 | 6785 | 8661 | 10126 | 11491 | 12827 | 14216 | 15793 | 17187 |
| 980 | 2170 | 3874 | 5238 | 6786 | 8662 | 10127 | 11492 | 12828 | 14217 | 15804 | 17198 |
| 981 | 2171 | 3875 | 5239 | 6787 | 8663 | 10128 | 11493 | 12829 | 14218 | 15805 | 17199 |
| 982 | 2172 | 3876 | 5240 | 6788 | 8664 | 10129 | 11494 | 12830 | 14219 | 15806 | 17200 |
| 983 | 2173 | 3877 | 5241 | 6789 | 8665 | 10130 | 11495 | 12831 | 14220 | 15807 | 17201 |
| 984 | 2174 | 3878 | 5242 | 6801 | 8700 | 10131 | 11496 | 12832 | 14221 | 15808 | 17202 |
| 985 | 2175 | 3879 | 5243 | 6802 | 8701 | 10132 | 11497 | 12833 | 14222 | 15809 | 17220 |
| 986 | 2176 | 3880 | 5244 | 6803 | 8702 | 10133 | 11498 | 12834 | 14223 | 15810 | 17221 |
| 987 | 2177 | 3881 | 5245 | 6804 | 8703 | 10134 | 11529 | 12835 | 14224 | 15811 | 17222 |
| 988 | 2178 | 3882 | 5246 | 6805 | 8704 | 10135 | 11530 | 12836 | 14225 | 15812 | 17223 |
| 989 | 2179 | 3883 | 5247 | 6806 | 8705 | 10136 | 11551 | 12837 | 14226 | 15813 | 17224 |
| 990 | 2180 | 3884 | 5248 | 6807 | 8721 | 10137 | 11552 | 12838 | 14227 | 15814 | 17225 |
| 992 | 2181 | 3885 | 5251 | 6808 | 8722 | 10138 | 11553 | 12839 | 14228 | 15815 | 17226 |
| 993 | 2182 | 3886 | 5276 | 6809 | 8723 | 10139 | 11554 | 12840 | 14229 | 15816 | 17227 |
| 994 | 2183 | 3887 | 5277 | 6816 | 8724 | 10140 | 11555 | 12855 | 14230 | 15817 | 17230 |
| 995 | 2184 | 3888 | 5278 | 6817 | 8726 | 10141 | 11556 | 12856 | 14238 | 15818 | 17231 |
| 996 | 2185 | 3889 | 5279 | 6818 | 8727 | 10161 | 11557 | 12857 | 14239 | 15819 | 17232 |
| 997 | 2186 | 3890 | 5280 | 6819 | 8728 | 10162 | 11558 | 12858 | 14240 | 15820 | 17233 |
| 998 | 2218 | 3891 | 5281 | 6820 | 8729 | 10163 | 11593 | 12859 | 14241 | 15821 | 17234 |
| 1003 | 2219 | 3892 | 5282 | 6821 | 8730 | 10164 | 11594 | 12860 | 14242 | 15822 | 17244 |
| 1004 | 2220 | 3893 | 5283 | 6822 | 8731 | 10165 | 11595 | 12861 | 14243 | 15823 | 17245 |
| 1005 | 2221 | 3894 | 5284 | 6846 | 8732 | 10166 | 11596 | 12875 | 14244 | 15824 | 17246 |
| 1006 | 2228 | 3895 | 5285 | 6847 | 8743 | 10167 | 11597 | 12876 | 14249 | 15825 | 17247 |
| 1007 | 2229 | 3896 | 5295 | 6848 | 8744 | 10168 | 11598 | 12877 | 14250 | 15826 | 17253 |
| 1008 | 2230 | 3897 | 5296 | 6849 | 8745 | 10182 | 11599 | 12878 | 14256 | 15827 | 17254 |
| 1009 | 2231 | 3898 | 5297 | 6850 | 8746 | 10183 | 11600 | 12879 | 14257 | 15828 | 17255 |
| 1010 | 2232 | 3899 | 5298 | 6851 | 8747 | 10184 | 11601 | 12880 | 14258 | 15829 | 17256 |
| 1011 | 2233 | 3900 | 5299 | 6852 | 8748 | 10185 | 11602 | 12895 | 14259 | 15830 | 17257 |
| 1012 | 2234 | 3901 | 5300 | 6853 | 8749 | 10186 | 11603 | 12915 | 14260 | 15831 | 17258 |
| 1013 | 2235 | 3902 | 5301 | 6854 | 8750 | 10187 | 11608 | 12920 | 14261 | 15832 | 17259 |
| 1014 | 2236 | 3903 | 5302 | 6855 | 8756 | 10188 | 11609 | 12921 | 14262 | 15833 | 17260 |
| 1015 | 2237 | 3904 | 5303 | 6856 | 8757 | 10189 | 11610 | 12922 | 14263 | 15834 | 17261 |
| 1016 | 2238 | 3905 | 5304 | 6857 | 8758 | 10190 | 11611 | 12923 | 14264 | 15835 | 17262 |
| 1017 | 2251 | 3906 | 5305 | 6858 | 8759 | 10191 | 11612 | 12924 | 14265 | 15836 | 17263 |
| 1018 | 2252 | 3907 | 5306 | 6870 | 8760 | 10194 | 11613 | 12925 | 14266 | 15837 | 17264 |
| 1019 | 2266 | 3908 | 5307 | 6914 | 8761 | 10195 | 11614 | 12926 | 14267 | 15838 | 17265 |
| 1020 | 2267 | 3909 | 5308 | 6930 | 8762 | 10196 | 11615 | 12927 | 14268 | 15839 | 17266 |
| 1021 | 2268 | 3910 | 5317 | 6931 | 8763 | 10197 | 11616 | 12928 | 14269 | 15840 | 17267 |
| 1022 | 2269 | 3929 | 5332 | 6932 | 8764 | 10198 | 11617 | 12929 | 14270 | 15841 | 17289 |
| 1023 | 2270 | 3930 | 5333 | 6938 | 8765 | 10199 | 11618 | 12930 | 14271 | 15842 | 17290 |
| 1024 | 2271 | 3931 | 5334 | 6939 | 8766 | 10222 | 11619 | 12931 | 14272 | 15843 | 17291 |
| 1025 | 2280 | 3932 | 5335 | 6940 | 8767 | 10223 | 11620 | 12932 | 14273 | 15844 | 17292 |
| 1026 | 2281 | 3933 | 5343 | 6941 | 8787 | 10224 | 11621 | 12933 | 14274 | 15845 | 17293 |
| 1027 | 2301 | 3934 | 5344 | 6945 | 8817 | 10225 | 11622 | 12949 | 14275 | 15846 | 17294 |
| 1028 | 2302 | 3935 | 5345 | 6946 | 8822 | 10226 | 11623 | 12950 | 14276 | 15847 | 17295 |
| 1029 | 2303 | 3936 | 5346 | 6947 | 8823 | 10227 | 11624 | 12951 | 14284 | 15848 | 17296 |
| 1030 | 2304 | 3937 | 5402 | 6948 | 8824 | 10228 | 11625 | 12952 | 14285 | 15849 | 17297 |
| 1031 | 2305 | 3938 | 5403 | 6949 | 8825 | 10229 | 11626 | 12953 | 14286 | 15850 | 17298 |
| 1032 | 2306 | 3939 | 5404 | 6976 | 8826 | 10230 | 11627 | 12954 | 14287 | 15851 | 17299 |
| 1033 | 2307 | 3940 | 5405 | 6978 | 8827 | 10241 | 11628 | 12955 | 14288 | 15852 | 17300 |
| 1034 | 2308 | 3957 | 5406 | 6979 | 8828 | 10242 | 11636 | 12956 | 14289 | 15853 | 17301 |
| 1042 | 2309 | 3958 | 5407 | 6980 | 8829 | 10243 | 11637 | 12957 | 14290 | 15854 | 17302 |
| 1043 | 2310 | 3959 | 5408 | 6981 | 8830 | 10244 | 11642 | 12958 | 14291 | 15860 | 17303 |
| 1044 | 2311 | 3960 | 5409 | 7000 | 8831 | 10245 | 11643 | 12959 | 14292 | 15861 | 17304 |
| 1045 | 2312 | 3961 | 5410 | 7001 | 8833 | 10246 | 11667 | 12960 | 14293 | 15862 | 17305 |
| 1046 | 2313 | 3962 | 5411 | 7002 | 8834 | 10247 | 11668 | 12961 | 14294 | 15863 | 17306 |
| 1047 | 2314 | 3963 | 5412 | 7003 | 8856 | 10248 | 11669 | 12962 | 14319 | 15864 | 17307 |
| 1048 | 2315 | 3964 | 5413 | 7004 | 8857 | 10249 | 11670 | 12980 | 14320 | 15865 | 17308 |
| 1049 | 2316 | 3965 | 5414 | 7011 | 8858 | 10250 | 11671 | 12981 | 14321 | 15866 | 17309 |
| 1050 | 2317 | 3966 | 5415 | 7012 | 8859 | 10251 | 11672 | 12982 | 14322 | 15867 | 17310 |
| 1051 | 2318 | 3967 | 5416 | 7013 | 8860 | 10252 | 11673 | 12983 | 14323 | 15868 | 17311 |
| 1052 | 2319 | 3968 | 5417 | 7014 | 8861 | 10262 | 11674 | 12984 | 14324 | 15869 | 17312 |
| 1053 | 2320 | 3969 | 5444 | 7015 | 8862 | 10263 | 11675 | 12985 | 14325 | 15876 | 17313 |
| 1054 | 2321 | 3970 | 5445 | 7016 | 8863 | 10264 | 11676 | 12986 | 14332 | 15877 | 17314 |
| 1055 | 2322 | 4000 | 5446 | 7017 | 8864 | 10279 | 11677 | 12987 | 14333 | 15878 | 17315 |
| 1056 | 2323 | 4001 | 5447 | 7018 | 8865 | 10280 | 11678 | 12988 | 14342 | 15879 | 17316 |
| 1057 | 2324 | 4002 | 5448 | 7019 | 8866 | 10281 | 11679 | 12989 | 14343 | 15880 | 17317 |
| 1058 | 2325 | 4003 | 5467 | 7042 | 8867 | 10282 | 11680 | 12990 | 14371 | 15881 | 17318 |
| 1059 | 2326 | 4004 | 5468 | 7043 | 8868 | 10284 | 11681 | 12991 | 14372 | 15882 | 17319 |
| 1060 | 2327 | 4005 | 5469 | 7044 | 8869 | 10285 | 11682 | 12992 | 14373 | 15883 | 17320 |
| 1061 | 2328 | 4009 | 5470 | 7045 | 8870 | 10286 | 11683 | 12993 | 14374 | 15884 | 17321 |
| 1062 | 2354 | 4010 | 5480 | 7046 | 8871 | 10287 | 11684 | 12994 | 14375 | 15885 | 17322 |
| 1063 | 2355 | 4011 | 5481 | 7047 | 8872 | 10288 | 11685 | 12995 | 14376 | 15886 | 17323 |
| 1064 | 2356 | 4012 | 5482 | 7048 | 8873 | 10289 | 11686 | 13016 | 14377 | 15887 | 17336 |
| 1065 | 2357 | 4013 | 5483 | 7049 | 8874 | 10290 | 11687 | 13017 | 14378 | 15905 | 17337 |
| 1066 | 2358 | 4014 | 5484 | 7050 | 8875 | 10291 | 11688 | 13018 | 14379 | 15906 | 17338 |
| 1067 | 2367 | 4015 | 5485 | 7051 | 8876 | 10298 | 11689 | 13019 | 14380 | 15907 | 17339 |
| 1068 | 2368 | 4016 | 5503 | 7077 | 8877 | 10299 | 11690 | 13020 | 14381 | 15908 | 17340 |
| 1097 | 2369 | 4017 | 5504 | 7084 | 8878 | 10300 | 11691 | 13021 | 14382 | 15909 | 17341 |
| 1098 | 2370 | 4018 | 5505 | 7085 | 8879 | 10301 | 11692 | 13022 | 14383 | 15910 | 17342 |
| 1099 | 2371 | 4019 | 5506 | 7086 | 8880 | 10302 | 11693 | 13023 | 14384 | 15911 | 17343 |
| 1100 | 2372 | 4020 | 5507 | 7087 | 8907 | 10303 | 11694 | 13024 | 14385 | 15912 | 17344 |
| 1101 | 2373 | 4045 | 5508 | 7088 | 8908 | 10304 | 11695 | 13025 | 14386 | 15913 | 17346 |
| 1102 | 2374 | 4046 | 5509 | 7093 | 8909 | 10327 | 11696 | 13026 | 14387 | 15914 | 17347 |
| 1103 | 2375 | 4047 | 5510 | 7094 | 8910 | 10328 | 11697 | 13027 | 14388 | 15915 | 17348 |
| 1112 | 2376 | 4048 | 5511 | 7095 | 8911 | 10336 | 11698 | 13028 | 14389 | 15916 | 17349 |
| 1113 | 2377 | 4049 | 5512 | 7096 | 8912 | 10337 | 11699 | 13029 | 14390 | 15917 | 17350 |
| 1114 | 2378 | 4050 | 5513 | 7103 | 8913 | 10338 | 11700 | 13049 | 14403 | 15924 | 17351 |
| 1115 | 2379 | 4051 | 5514 | 7104 | 8921 | 10339 | 11701 | 13050 | 14404 | 15925 | 17352 |
| 1116 | 2380 | 4052 | 5515 | 7105 | 8922 | 10340 | 11724 | 13051 | 14405 | 15959 | 17365 |
| 1117 | 2381 | 4053 | 5516 | 7106 | 8923 | 10341 | 11725 | 13052 | 14406 | 15960 | 17366 |
| 1118 | 2382 | 4054 | 5517 | 7107 | 8924 | 10342 | 11726 | 13053 | 14407 | 15961 | 17367 |
| 1119 | 2383 | 4055 | 5518 | 7108 | 8925 | 10343 | 11727 | 13054 | 14408 | 15962 | 17368 |
| 1120 | 2384 | 4056 | 5539 | 7109 | 8937 | 10344 | 11728 | 13055 | 14409 | 15963 | 17369 |
| 1121 | 2385 | 4057 | 5540 | 7110 | 8938 | 10345 | 11729 | 13056 | 14410 | 15964 | 17370 |
| 1122 | 2386 | 4058 | 5541 | 7111 | 8939 | 10346 | 11730 | 13057 | 14411 | 15965 | 17371 |
| 1123 | 2387 | 4059 | 5542 | 7112 | 8940 | 10347 | 11731 | 13058 | 14412 | 15966 | 17372 |
| 1124 | 2388 | 4060 | 5543 | 7113 | 8941 | 10348 | 11732 | 13059 | 14413 | 15967 | 17373 |
| 1125 | 2434 | 4061 | 5544 | 7114 | 8942 | 10349 | 11733 | 13060 | 14414 | 15968 | 17374 |
| 1126 | 2435 | 4062 | 5545 | 7115 | 8943 | 10417 | 11734 | 13061 | 14415 | 15969 | 17375 |
| 1127 | 2436 | 4063 | 5546 | 7116 | 8944 | 10418 | 11735 | 13062 | 14416 | 15970 | 17376 |
| 1128 | 2437 | 4064 | 5547 | 7117 | 8945 | 10419 | 11736 | 13063 | 14417 | 15971 | 17377 |
| 1129 | 2438 | 4065 | 5548 | 7118 | 8946 | 10430 | 11737 | 13064 | 14418 | 15972 | 17383 |
| 1130 | 2461 | 4066 | 5549 | 7119 | 8947 | 10431 | 11792 | 13065 | 14419 | 15973 | 17384 |
| 1131 | 2462 | 4067 | 5550 | 7120 | 8948 | 10432 | 11793 | 13074 | 14420 | 15974 | 17385 |
| 1132 | 2463 | 4068 | 5551 | 7165 | 8962 | 10433 | 11794 | 13075 | 14421 | 15975 | 17386 |
| 1157 | 2464 | 4069 | 5552 | 7166 | 8963 | 10434 | 11795 | 13076 | 14422 | 15976 | 17387 |
| 1158 | 2465 | 4070 | 5553 | 7167 | 8964 | 10435 | 11796 | 13077 | 14423 | 15977 | 17388 |
| 1159 | 2466 | 4071 | 5554 | 7168 | 8965 | 10436 | 11797 | 13078 | 14424 | 16008 | 17389 |
| 1160 | 2467 | 4072 | 5555 | 7169 | 8966 | 10437 | 11798 | 13079 | 14425 | 16009 | 17390 |
| 1161 | 2468 | 4073 | 5556 | 7170 | 8967 | 10438 | 11799 | 13080 | 14426 | 16010 | 17424 |
| 1162 | 2469 | 4074 | 5557 | 7171 | 8968 | 10439 | 11800 | 13081 | 14427 | 16011 | 17429 |
| 1163 | 2470 | 4075 | 5558 | 7186 | 8969 | 10440 | 11801 | 13082 | 14428 | 16012 | 17430 |
| 1164 | 2471 | 4076 | 5559 | 7187 | 8970 | 10441 | 11805 | 13095 | 14429 | 16013 | 17431 |
| 1165 | 2472 | 4077 | 5560 | 7188 | 8971 | 10442 | 11806 | 13096 | 14430 | 16016 | 17432 |
| 1166 | 2474 | 4078 | 5569 | 7189 | 8973 | 10443 | 11807 | 13097 | 14431 | 16017 | 17433 |
| 1167 | 2475 | 4079 | 5570 | 7190 | 8974 | 10449 | 11808 | 13098 | 14432 | 16018 | 17434 |
| 1168 | 2476 | 4080 | 5586 | 7191 | 8975 | 10450 | 11809 | 13099 | 14433 | 16019 | 17435 |
| 1169 | 2477 | 4081 | 5587 | 7192 | 8976 | 10451 | 11810 | 13100 | 14434 | 16020 | 17436 |
| 1170 | 2478 | 4082 | 5588 | 7193 | 8977 | 10452 | 11827 | 13101 | 14435 | 16038 | 17437 |
| 1171 | 2479 | 4083 | 5589 | 7194 | 8978 | 10453 | 11828 | 13102 | 14436 | 16039 | 17438 |
| 1172 | 2480 | 4084 | 5590 | 7195 | 8984 | 10454 | 11845 | 13103 | 14437 | 16040 | 17439 |
| 1173 | 2481 | 4085 | 5603 | 7196 | 8985 | 10495 | 11846 | 13104 | 14438 | 16041 | 17440 |
| 1174 | 2482 | 4086 | 5604 | 7197 | 8986 | 10496 | 11847 | 13105 | 14439 | 16042 | 17441 |
| 1175 | 2483 | 4087 | 5605 | 7198 | 8987 | 10497 | 11848 | 13106 | 14440 | 16043 | 17442 |
| 1176 | 2484 | 4088 | 5606 | 7199 | 8988 | 10498 | 11849 | 13107 | 14441 | 16044 | 17443 |
| 1177 | 2485 | 4089 | 5607 | 7200 | 8989 | 10499 | 11850 | 13108 | 14442 | 16079 | 17444 |
| 1178 | 2486 | 4090 | 5609 | 7201 | 9000 | 10500 | 11856 | 13109 | 14443 | 16080 | 17445 |
| 1179 | 2487 | 4091 | 5610 | 7202 | 9001 | 10501 | 11857 | 13110 | 14444 | 16081 | 17446 |
| 1196 | 2488 | 4092 | 5611 | 7203 | 9002 | 10514 | 11858 | 13111 | 14445 | 16082 | 17447 |
| 1197 | 2489 | 4093 | 5612 | 7204 | 9003 | 10515 | 11859 | 13112 | 14446 | 16083 | 17448 |
| 1198 | 2490 | 4094 | 5613 | 7205 | 9004 | 10516 | 11860 | 13113 | 14447 | 16121 | 17449 |
| 1199 | 2491 | 4095 | 5614 | 7206 | 9005 | 10517 | 11861 | 13114 | 14448 | 16122 | 17450 |
| 1200 | 2492 | 4096 | 5615 | 7207 | 9006 | 10529 | 11862 | 13115 | 14449 | 16129 | 17451 |
| 1201 | 2493 | 4097 | 5616 | 7208 | 9007 | 10530 | 11895 | 13116 | 14450 | 16130 | 17452 |
| 1202 | 2523 | 4098 | 5617 | 7209 | 9008 | 10531 | 11896 | 13117 | 14451 | 16131 | 17453 |
| 1203 | 2524 | 4099 | 5618 | 7210 | 9009 | 10532 | 11897 | 13118 | 14452 | 16132 | 17454 |
| 1204 | 2525 | 4100 | 5619 | 7211 | 9010 | 10533 | 11898 | 13119 | 14453 | 16133 | 17455 |
| 1205 | 2526 | 4101 | 5620 | 7212 | 9011 | 10534 | 11899 | 13120 | 14454 | 16134 | 17456 |
| 1206 | 2527 | 4102 | 5621 | 7213 | 9012 | 10553 | 11905 | 13121 | 14455 | 16135 | 17457 |
| 1207 | 2548 | 4103 | 5622 | 7214 | 9013 | 10554 | 11906 | 13122 | 14456 | 16136 | 17458 |
| 1208 | 2549 | 4118 | 5623 | 7215 | 9014 | 10555 | 11907 | 13123 | 14457 | 16137 | 17459 |
| 1209 | 2555 | 4119 | 5624 | 7216 | 9015 | 10556 | 11908 | 13124 | 14458 | 16138 | 17460 |
| 1210 | 2556 | 4138 | 5625 | 7217 | 9016 | 10565 | 11909 | 13125 | 14466 | 16139 | 17461 |
| 1211 | 2557 | 4139 | 5627 | 7218 | 9017 | 10566 | 11910 | 13126 | 14467 | 16140 | 17462 |
| 1227 | 2558 | 4140 | 5628 | 7238 | 9020 | 10567 | 11911 | 13127 | 14468 | 16141 | 17463 |
| 1228 | 2559 | 4141 | 5629 | 7239 | 9021 | 10568 | 11912 | 13128 | 14469 | 16142 | 17464 |
| 1229 | 2560 | 4142 | 5630 | 7240 | 9022 | 10569 | 11913 | 13129 | 14470 | 16143 | 17465 |
| 1230 | 2561 | 4143 | 5631 | 7241 | 9023 | 10570 | 11914 | 13130 | 14471 | 16144 | 17466 |
| 1231 | 2562 | 4144 | 5632 | 7242 | 9024 | 10571 | 11915 | 13174 | 14472 | 16145 | 17467 |
| 1232 | 2563 | 4145 | 5633 | 7243 | 9025 | 10572 | 11916 | 13175 | 14473 | 16146 | 17468 |
| 1233 | 2564 | 4146 | 5634 | 7244 | 9027 | 10573 | 11917 | 13176 | 14474 | 16147 | 17469 |
| 1234 | 2565 | 4147 | 5635 | 7245 | 9028 | 10576 | 11918 | 13177 | 14476 | 16148 | 17470 |
| 1235 | 2566 | 4148 | 5636 | 7261 | 9029 | 10577 | 11919 | 13178 | 14477 | 16149 | 17471 |
| 1236 | 2567 | 4149 | 5637 | 7262 | 9030 | 10578 | 11920 | 13179 | 14478 | 16150 | 17472 |
| 1237 | 2568 | 4150 | 5648 | 7263 | 9031 | 10579 | 11921 | 13180 | 14479 | 16151 | 17473 |
| 1238 | 2569 | 4151 | 5649 | 7264 | 9032 | 10580 | 11922 | 13181 | 14480 | 16152 | 17474 |
| 1239 | 2570 | 4152 | 5650 | 7265 | 9033 | 10581 | 11923 | 13182 | 14481 | 16153 | 17475 |
| 1240 | 2571 | 4153 | 5651 | 7282 | 9034 | 10582 | 11924 | 13183 | 14482 | 16154 | 17476 |
| 1241 | 2572 | 4154 | 5652 | 7283 | 9035 | 10583 | 11925 | 13184 | 14483 | 16155 | 17477 |
| 1242 | 2573 | 4155 | 5653 | 7284 | 9038 | 10584 | 11926 | 13185 | 14484 | 16156 | 17478 |
| 1243 | 2574 | 4156 | 5654 | 7285 | 9039 | 10585 | 11927 | 13186 | 14485 | 16157 | 17481 |
| 1244 | 2575 | 4157 | 5655 | 7286 | 9040 | 10586 | 11928 | 13187 | 14486 | 16158 | 17484 |
| 1245 | 2576 | 4158 | 5656 | 7287 | 9041 | 10587 | 11929 | 13188 | 14487 | 16159 | 17485 |
| 1246 | 2577 | 4159 | 5657 | 7288 | 9042 | 10588 | 11930 | 13189 | 14488 | 16183 | 17486 |
| 1247 | 2578 | 4160 | 5658 | 7338 | 9046 | 10589 | 11931 | 13190 | 14489 | 16184 | 17487 |
| 1248 | 2579 | 4179 | 5659 | 7339 | 9047 | 10590 | 11932 | 13191 | 14490 | 16185 | 17488 |
| 1249 | 2580 | 4180 | 5660 | 7340 | 9048 | 10591 | 11933 | 13192 | 14491 | 16186 | 17489 |
| 1250 | 2602 | 4181 | 5661 | 7341 | 9049 | 10592 | 11934 | 13193 | 14492 | 16187 | 17490 |
| 1251 | 2603 | 4189 | 5662 | 7342 | 9050 | 10593 | 11935 | 13194 | 14493 | 16188 | 17491 |
| 1252 | 2604 | 4190 | 5663 | 7343 | 9051 | 10594 | 11936 | 13195 | 14494 | 16194 | 17492 |
| 1253 | 2605 | 4207 | 5664 | 7344 | 9052 | 10595 | 11937 | 13196 | 14495 | 16195 | 17493 |
| 1257 | 2606 | 4208 | 5665 | 7345 | 9053 | 10596 | 11938 | 13197 | 14496 | 16196 | 17494 |
| | | | | | | | | | | | 17495 |

**Table 4 - medium list**

| **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** |
|---|---|---|---|---|---|---|---|---|---|---|---|
| 8 | 1356 | 2611 | 4455 | 5605 | 7170 | 8960 | 10192 | 11712 | 13255 | 14539 | 16166 |
| 9 | 1357 | 2612 | 4456 | 5606 | 7171 | 8961 | 10193 | 11713 | 13256 | 14540 | 16167 |
| 19 | 1358 | 2613 | 4457 | 5607 | 7175 | 8984 | 10200 | 11714 | 13263 | 14541 | 16168 |
| 20 | 1359 | 2614 | 4458 | 5612 | 7176 | 8985 | 10201 | 11715 | 13264 | 14542 | 16169 |
| 21 | 1360 | 2615 | 4488 | 5613 | 7177 | 8986 | 10202 | 11716 | 13265 | 14543 | 16170 |
| 48 | 1361 | 2616 | 4489 | 5614 | 7178 | 8987 | 10237 | 11746 | 13266 | 14544 | 16171 |
| 57 | 1362 | 2645 | 4490 | 5615 | 7179 | 8988 | 10238 | 11747 | 13267 | 14545 | 16172 |
| 58 | 1363 | 2646 | 4513 | 5616 | 7219 | 8989 | 10239 | 11748 | 13268 | 14561 | 16173 |
| 59 | 1418 | 2647 | 4514 | 5617 | 7220 | 9000 | 10240 | 11749 | 13269 | 14562 | 16174 |
| 60 | 1419 | 2648 | 4515 | 5618 | 7221 | 9001 | 10260 | 11750 | 13270 | 14563 | 16175 |
| 61 | 1420 | 2649 | 4516 | 5619 | 7222 | 9002 | 10261 | 11751 | 13271 | 14564 | 16176 |
| 62 | 1421 | 2650 | 4517 | 5620 | 7223 | 9003 | 10265 | 11752 | 13272 | 14565 | 16177 |
| 63 | 1422 | 2651 | 4518 | 5621 | 7224 | 9004 | 10266 | 11753 | 13273 | 14566 | 16178 |
| 64 | 1423 | 2652 | 4519 | 5622 | 7225 | 9005 | 10267 | 11754 | 13274 | 14571 | 16179 |
| 65 | 1424 | 2653 | 4520 | 5623 | 7226 | 9006 | 10268 | 11755 | 13275 | 14572 | 16180 |
| 66 | 1425 | 2654 | 4521 | 5624 | 7227 | 9007 | 10269 | 11756 | 13276 | 14573 | 16320 |
| 67 | 1426 | 2655 | 4522 | 5625 | 7228 | 9008 | 10270 | 11757 | 13277 | 14574 | 16321 |
| 68 | 1427 | 2656 | 4523 | 5626 | 7266 | 9009 | 10271 | 11758 | 13278 | 14575 | 16322 |
| 69 | 1428 | 2686 | 4524 | 5641 | 7267 | 9010 | 10272 | 11759 | 13279 | 14576 | 16323 |
| 70 | 1429 | 2699 | 4525 | 5642 | 7268 | 9011 | 10273 | 11760 | 13280 | 14577 | 16324 |
| 71 | 1430 | 2700 | 4526 | 5643 | 7269 | 9018 | 10274 | 11761 | 13281 | 14578 | 16325 |
| 72 | 1431 | 2701 | 4527 | 5644 | 7270 | 9019 | 10298 | 11762 | 13339 | 14579 | 16326 |
| 73 | 1432 | 2702 | 4528 | 5668 | 7271 | 9027 | 10299 | 11763 | 13340 | 14580 | 16327 |
| 74 | 1433 | 2703 | 4529 | 5669 | 7272 | 9038 | 10315 | 11764 | 13341 | 14581 | 16328 |
| 75 | 1491 | 2826 | 4530 | 5693 | 7273 | 9039 | 10316 | 11765 | 13342 | 14582 | 16329 |
| 76 | 1492 | 2827 | 4531 | 5694 | 7274 | 9040 | 10317 | 11766 | 13343 | 14583 | 16337 |
| 77 | 1493 | 2828 | 4532 | 5695 | 7275 | 9041 | 10327 | 11767 | 13344 | 14584 | 16338 |
| 78 | 1494 | 2829 | 4533 | 5696 | 7276 | 9042 | 10328 | 11768 | 13395 | 14585 | 16339 |
| 79 | 1495 | 2830 | 4534 | 5712 | 7282 | 9060 | 10401 | 11769 | 13396 | 14616 | 16340 |
| 80 | 1496 | 2860 | 4535 | 5713 | 7283 | 9061 | 10402 | 11770 | 13397 | 14617 | 16341 |
| 81 | 1497 | 2864 | 4536 | 5714 | 7284 | 9062 | 10403 | 11771 | 13398 | 14618 | 16342 |
| 82 | 1498 | 2865 | 4572 | 5715 | 7285 | 9063 | 10404 | 11829 | 13399 | 14619 | 16343 |
| 83 | 1499 | 2866 | 4573 | 5716 | 7286 | 9064 | 10405 | 11830 | 13400 | 14620 | 16344 |
| 84 | 1500 | 2867 | 4624 | 5717 | 7302 | 9065 | 10406 | 11831 | 13401 | 14621 | 16345 |
| 85 | 1501 | 2904 | 4625 | 5718 | 7303 | 9066 | 10407 | 11832 | 13402 | 14622 | 16346 |
| 88 | 1502 | 2905 | 4626 | 5725 | 7304 | 9067 | 10408 | 11833 | 13403 | 14635 | 16347 |
| 94 | 1547 | 2906 | 4627 | 5726 | 7305 | 9068 | 10409 | 11834 | 13461 | 14636 | 16348 |
| 95 | 1548 | 2907 | 4628 | 5727 | 7306 | 9069 | 10410 | 11835 | 13462 | 14637 | 16349 |
| 96 | 1549 | 2908 | 4629 | 5728 | 7307 | 9070 | 10411 | 11836 | 13463 | 14638 | 16350 |
| 97 | 1550 | 2909 | 4630 | 5729 | 7308 | 9071 | 10412 | 11837 | 13464 | 14639 | 16351 |
| 98 | 1551 | 2910 | 4631 | 5730 | 7309 | 9072 | 10413 | 11838 | 13465 | 14640 | 16352 |
| 99 | 1552 | 2911 | 4632 | 5825 | 7377 | 9091 | 10414 | 11868 | 13466 | 14641 | 16353 |
| 100 | 1553 | 2912 | 4633 | 5837 | 7378 | 9092 | 10415 | 11869 | 13468 | 14727 | 16354 |
| 101 | 1554 | 2913 | 4634 | 5838 | 7379 | 9093 | 10416 | 11870 | 13469 | 14728 | 16355 |
| 102 | 1555 | 2914 | 4635 | 5839 | 7417 | 9097 | 10417 | 11871 | 13470 | 14729 | 16356 |
| 103 | 1556 | 2915 | 4636 | 5840 | 7418 | 9098 | 10418 | 11872 | 13471 | 14778 | 16357 |
| 117 | 1557 | 2916 | 4637 | 5841 | 7419 | 9099 | 10419 | 11873 | 13472 | 14779 | 16358 |
| 118 | 1558 | 2917 | 4638 | 5842 | 7420 | 9100 | 10420 | 11874 | 13473 | 14780 | 16359 |
| 143 | 1559 | 2918 | 4659 | 5843 | 7421 | 9101 | 10421 | 11875 | 13474 | 14781 | 16360 |
| 144 | 1560 | 2919 | 4660 | 5844 | 7422 | 9102 | 10422 | 11876 | 13475 | 14782 | 16389 |
| 145 | 1561 | 2920 | 4694 | 5865 | 7423 | 9103 | 10423 | 11877 | 13476 | 14783 | 16390 |
| 146 | 1562 | 2921 | 4695 | 5866 | 7424 | 9104 | 10424 | 11900 | 13477 | 14784 | 16391 |
| 147 | 1563 | 2929 | 4696 | 5867 | 7425 | 9105 | 10436 | 11901 | 13478 | 14857 | 16392 |
| 148 | 1564 | 2930 | 4697 | 5868 | 7426 | 9106 | 10437 | 11902 | 13479 | 14858 | 16393 |
| 149 | 1565 | 2931 | 4698 | 5869 | 7473 | 9107 | 10438 | 11903 | 13480 | 14859 | 16394 |
| 150 | 1566 | 2932 | 4699 | 5870 | 7474 | 9108 | 10439 | 11939 | 13481 | 14860 | 16395 |
| 151 | 1567 | 2933 | 4707 | 5871 | 7475 | 9151 | 10460 | 11940 | 13482 | 14861 | 16396 |
| 152 | 1568 | 2934 | 4708 | 5872 | 7476 | 9152 | 10461 | 11941 | 13483 | 14862 | 16408 |
| 166 | 1569 | 2946 | 4709 | 5873 | 7477 | 9153 | 10462 | 11942 | 13484 | 14863 | 16409 |
| 167 | 1574 | 2971 | 4710 | 5907 | 7478 | 9154 | 10463 | 11943 | 13485 | 14864 | 16420 |
| 179 | 1575 | 2972 | 4711 | 5908 | 7479 | 9155 | 10514 | 11944 | 13486 | 14865 | 16421 |
| 180 | 1576 | 2973 | 4712 | 5909 | 7480 | 9156 | 10565 | 11945 | 13487 | 14866 | 16422 |
| 181 | 1581 | 2974 | 4713 | 5910 | 7497 | 9157 | 10566 | 11946 | 13488 | 14867 | 16423 |
| 182 | 1582 | 2975 | 4714 | 5911 | 7498 | 9158 | 10567 | 11947 | 13489 | 14968 | 16424 |
| 195 | 1583 | 2976 | 4715 | 5946 | 7499 | 9159 | 10568 | 11948 | 13490 | 14969 | 16425 |
| 196 | 1584 | 2977 | 4716 | 5947 | 7500 | 9160 | 10569 | 11949 | 13491 | 14970 | 16426 |
| 197 | 1585 | 2978 | 4717 | 5948 | 7501 | 9161 | 10570 | 11950 | 13506 | 14971 | 16427 |
| 198 | 1637 | 2979 | 4718 | 5949 | 7502 | 9193 | 10571 | 11951 | 13507 | 14972 | 16428 |
| 199 | 1638 | 2980 | 4719 | 5950 | 7503 | 9194 | 10572 | 11952 | 13511 | 14973 | 16429 |
| 200 | 1639 | 2981 | 4720 | 5951 | 7504 | 9195 | 10573 | 11953 | 13512 | 14974 | 16430 |
| 201 | 1640 | 2982 | 4721 | 5952 | 7574 | 9196 | 10574 | 11954 | 13513 | 14975 | 16472 |
| 202 | 1641 | 2983 | 4728 | 5953 | 7575 | 9197 | 10575 | 11955 | 13514 | 14976 | 16473 |
| 203 | 1642 | 2984 | 4729 | 5954 | 7576 | 9204 | 10618 | 11956 | 13515 | 14977 | 16474 |
| 204 | 1643 | 2985 | 4730 | 5974 | 7577 | 9205 | 10639 | 11957 | 13516 | 14978 | 16475 |
| 205 | 1644 | 3024 | 4731 | 5975 | 7578 | 9206 | 10640 | 11958 | 13517 | 14979 | 16476 |
| 206 | 1645 | 3025 | 4732 | 5976 | 7579 | 9207 | 10641 | 11959 | 13518 | 14980 | 16482 |
| 207 | 1646 | 3026 | 4733 | 5977 | 7580 | 9208 | 10642 | 11960 | 13519 | 14981 | 16483 |
| 208 | 1657 | 3027 | 4734 | 5978 | 7581 | 9209 | 10643 | 11961 | 13520 | 15006 | 16484 |
| 209 | 1658 | 3028 | 4735 | 5979 | 7582 | 9210 | 10644 | 11962 | 13521 | 15007 | 16485 |
| 210 | 1659 | 3029 | 4768 | 5980 | 7597 | 9211 | 10645 | 11963 | 13522 | 15008 | 16486 |
| 211 | 1660 | 3030 | 4769 | 5999 | 7598 | 9212 | 10646 | 11964 | 13523 | 15009 | 16487 |
| 212 | 1661 | 3031 | 4819 | 6000 | 7599 | 9213 | 10647 | 11965 | 13524 | 15010 | 16488 |
| 213 | 1662 | 3032 | 4820 | 6001 | 7600 | 9214 | 10655 | 11966 | 13525 | 15011 | 16489 |
| 214 | 1663 | 3033 | 4821 | 6035 | 7601 | 9215 | 10656 | 11967 | 13526 | 15012 | 16490 |
| 215 | 1664 | 3034 | 4822 | 6036 | 7602 | 9216 | 10657 | 11968 | 13527 | 15038 | 16518 |
| 216 | 1665 | 3073 | 4823 | 6037 | 7603 | 9217 | 10658 | 11969 | 13528 | 15039 | 16519 |
| 217 | 1666 | 3074 | 4824 | 6064 | 7604 | 9218 | 10659 | 11970 | 13529 | 15052 | 16520 |
| 218 | 1667 | 3075 | 4837 | 6065 | 7605 | 9219 | 10660 | 12005 | 13530 | 15053 | 16521 |
| 266 | 1668 | 3076 | 4838 | 6066 | 7606 | 9220 | 10661 | 12006 | 13556 | 15054 | 16525 |
| 267 | 1669 | 3077 | 4839 | 6067 | 7636 | 9221 | 10662 | 12013 | 13557 | 15055 | 16526 |
| 268 | 1670 | 3078 | 4840 | 6068 | 7637 | 9222 | 10663 | 12014 | 13558 | 15056 | 16527 |
| 269 | 1671 | 3079 | 4841 | 6069 | 7638 | 9223 | 10664 | 12015 | 13559 | 15057 | 16528 |
| 270 | 1672 | 3080 | 4842 | 6070 | 7639 | 9224 | 10665 | 12016 | 13560 | 15058 | 16529 |
| 271 | 1673 | 3081 | 4843 | 6071 | 7640 | 9225 | 10666 | 12017 | 13561 | 15059 | 16530 |
| 272 | 1674 | 3082 | 4844 | 6080 | 7641 | 9226 | 10667 | 12018 | 13562 | 15060 | 16531 |
| 273 | 1675 | 3083 | 4845 | 6081 | 7643 | 9227 | 10668 | 12034 | 13563 | 15061 | 16532 |
| 274 | 1676 | 3084 | 4846 | 6108 | 7644 | 9228 | 10669 | 12064 | 13564 | 15062 | 16533 |
| 275 | 1682 | 3085 | 4847 | 6109 | 7645 | 9229 | 10670 | 12065 | 13565 | 15063 | 16537 |
| 276 | 1683 | 3086 | 4864 | 6110 | 7646 | 9230 | 10672 | 12066 | 13566 | 15064 | 16538 |
| 277 | 1684 | 3087 | 4865 | 6111 | 7647 | 9231 | 10673 | 12067 | 13567 | 15065 | 16539 |
| 278 | 1685 | 3088 | 4866 | 6137 | 7648 | 9232 | 10674 | 12068 | 13582 | 15066 | 16540 |
| 279 | 1686 | 3089 | 4867 | 6138 | 7649 | 9233 | 10675 | 12069 | 13583 | 15067 | 16541 |
| 280 | 1690 | 3090 | 4887 | 6139 | 7650 | 9234 | 10676 | 12070 | 13584 | 15068 | 16542 |
| 295 | 1700 | 3091 | 4888 | 6140 | 7651 | 9235 | 10677 | 12071 | 13585 | 15069 | 16543 |
| 296 | 1701 | 3092 | 4889 | 6141 | 7652 | 9236 | 10678 | 12072 | 13586 | 15070 | 16577 |
| 297 | 1713 | 3093 | 4890 | 6142 | 7653 | 9330 | 10679 | 12073 | 13587 | 15071 | 16578 |
| 298 | 1714 | 3094 | 4891 | 6143 | 7688 | 9331 | 10680 | 12074 | 13588 | 15072 | 16579 |
| 299 | 1715 | 3095 | 4892 | 6144 | 7689 | 9332 | 10681 | 12075 | 13589 | 15073 | 16580 |
| 300 | 1716 | 3096 | 4893 | 6145 | 7690 | 9333 | 10682 | 12076 | 13590 | 15074 | 16581 |
| 301 | 1717 | 3097 | 4894 | 6146 | 7696 | 9334 | 10683 | 12077 | 13591 | 15075 | 16582 |
| 302 | 1718 | 3117 | 4895 | 6163 | 7697 | 9335 | 10700 | 12078 | 13592 | 15076 | 16583 |
| 303 | 1719 | 3118 | 4896 | 6164 | 7698 | 9336 | 10701 | 12079 | 13606 | 15077 | 16584 |
| 334 | 1720 | 3119 | 4897 | 6165 | 7699 | 9337 | 10702 | 12080 | 13607 | 15078 | 16585 |
| 335 | 1721 | 3120 | 4898 | 6185 | 7700 | 9338 | 10703 | 12081 | 13608 | 15079 | 16586 |
| 336 | 1726 | 3121 | 4899 | 6186 | 7701 | 9339 | 10704 | 12082 | 13609 | 15080 | 16587 |
| 337 | 1727 | 3122 | 4900 | 6187 | 7702 | 9340 | 10705 | 12083 | 13610 | 15081 | 16588 |
| 338 | 1728 | 3123 | 4901 | 6188 | 7703 | 9341 | 10716 | 12084 | 13611 | 15082 | 16589 |
| 339 | 1729 | 3124 | 4902 | 6189 | 7704 | 9342 | 10717 | 12089 | 13612 | 15083 | 16590 |
| 340 | 1730 | 3151 | 4903 | 6190 | 7705 | 9343 | 10718 | 12090 | 13613 | 15084 | 16591 |
| 341 | 1731 | 3152 | 4904 | 6191 | 7706 | 9344 | 10719 | 12091 | 13614 | 15117 | 16592 |
| 342 | 1732 | 3153 | 4905 | 6192 | 7714 | 9345 | 10720 | 12092 | 13630 | 15118 | 16593 |
| 431 | 1733 | 3154 | 4906 | 6193 | 7715 | 9346 | 10721 | 12093 | 13631 | 15119 | 16624 |
| 432 | 1734 | 3155 | 4907 | 6194 | 7716 | 9347 | 10722 | 12094 | 13632 | 15120 | 16625 |
| 433 | 1735 | 3156 | 4908 | 6199 | 7717 | 9348 | 10773 | 12095 | 13681 | 15121 | 16626 |
| 434 | 1736 | 3170 | 4909 | 6200 | 7718 | 9349 | 10808 | 12096 | 13682 | 15122 | 16627 |
| 435 | 1737 | 3171 | 4910 | 6201 | 7719 | 9350 | 10809 | 12098 | 13683 | 15123 | 16655 |
| 436 | 1738 | 3172 | 4911 | 6202 | 7720 | 9351 | 10810 | 12099 | 13684 | 15124 | 16656 |
| 437 | 1739 | 3173 | 4912 | 6203 | 7721 | 9352 | 10811 | 12100 | 13685 | 15125 | 16657 |
| 438 | 1740 | 3174 | 4913 | 6204 | 7722 | 9353 | 10812 | 12101 | 13686 | 15126 | 16658 |
| 439 | 1741 | 3196 | 4914 | 6205 | 7723 | 9354 | 10813 | 12123 | 13687 | 15127 | 16659 |
| 440 | 1742 | 3197 | 4918 | 6206 | 7724 | 9355 | 10814 | 12124 | 13688 | 15128 | 16660 |
| 495 | 1743 | 3198 | 4919 | 6207 | 7725 | 9356 | 10815 | 12195 | 13689 | 15129 | 16677 |
| 538 | 1744 | 3199 | 4920 | 6208 | 7726 | 9357 | 10816 | 12204 | 13690 | 15130 | 16678 |
| 539 | 1745 | 3200 | 4921 | 6209 | 7727 | 9468 | 10817 | 12205 | 13697 | 15131 | 16679 |
| 540 | 1746 | 3247 | 4922 | 6210 | 7728 | 9469 | 10818 | 12206 | 13698 | 15137 | 16680 |
| 541 | 1747 | 3248 | 4923 | 6211 | 7729 | 9470 | 10819 | 12207 | 13699 | 15138 | 16681 |
| 542 | 1748 | 3249 | 4924 | 6212 | 7730 | 9471 | 10820 | 12208 | 13700 | 15139 | 16682 |
| 543 | 1749 | 3250 | 4925 | 6213 | 7731 | 9472 | 10821 | 12209 | 13701 | 15140 | 16683 |
| 544 | 1750 | 3251 | 4926 | 6214 | 7732 | 9473 | 10822 | 12210 | 13702 | 15141 | 16684 |
| 545 | 1751 | 3267 | 4927 | 6215 | 7733 | 9474 | 10823 | 12232 | 13703 | 15142 | 16685 |
| 560 | 1757 | 3268 | 4995 | 6216 | 7734 | 9475 | 10824 | 12233 | 13704 | 15143 | 16686 |
| 561 | 1758 | 3269 | 4996 | 6217 | 7735 | 9536 | 10825 | 12234 | 13705 | 15144 | 16687 |
| 562 | 1759 | 3270 | 4997 | 6218 | 7736 | 9537 | 10826 | 12235 | 13712 | 15145 | 16688 |
| 563 | 1760 | 3271 | 4998 | 6219 | 7737 | 9547 | 10827 | 12236 | 13713 | 15146 | 16689 |
| 564 | 1763 | 3272 | 5024 | 6220 | 7738 | 9548 | 10828 | 12237 | 13719 | 15147 | 16690 |
| 565 | 1764 | 3273 | 5025 | 6221 | 7739 | 9549 | 10829 | 12238 | 13720 | 15148 | 16691 |
| 566 | 1765 | 3274 | 5026 | 6222 | 7740 | 9550 | 10869 | 12256 | 13721 | 15149 | 16692 |
| 573 | 1766 | 3275 | 5027 | 6223 | 7741 | 9553 | 10870 | 12257 | 13722 | 15150 | 16693 |
| 574 | 1767 | 3276 | 5028 | 6224 | 7742 | 9554 | 10871 | 12258 | 13723 | 15151 | 16730 |
| 575 | 1768 | 3277 | 5029 | 6225 | 7743 | 9555 | 10872 | 12259 | 13724 | 15152 | 16731 |
| 576 | 1769 | 3278 | 5030 | 6241 | 7744 | 9556 | 10873 | 12260 | 13725 | 15160 | 16732 |
| 577 | 1772 | 3279 | 5048 | 6297 | 7745 | 9557 | 10874 | 12261 | 13726 | 15161 | 16733 |
| 578 | 1773 | 3280 | 5049 | 6307 | 7746 | 9558 | 10875 | 12262 | 13727 | 15162 | 16734 |
| 579 | 1774 | 3281 | 5061 | 6308 | 7747 | 9559 | 10876 | 12263 | 13747 | 15163 | 16735 |
| 580 | 1775 | 3282 | 5062 | 6309 | 7748 | 9560 | 10877 | 12264 | 13748 | 15164 | 16736 |
| 625 | 1776 | 3283 | 5063 | 6328 | 7749 | 9561 | 10925 | 12265 | 13749 | 15165 | 16737 |
| 626 | 1777 | 3284 | 5064 | 6329 | 7750 | 9562 | 10926 | 12266 | 13750 | 15166 | 16738 |
| 627 | 1778 | 3285 | 5065 | 6330 | 7751 | 9568 | 10927 | 12267 | 13751 | 15167 | 16739 |
| 628 | 1779 | 3286 | 5066 | 6331 | 7752 | 9569 | 10928 | 12268 | 13786 | 15168 | 16740 |
| 629 | 1780 | 3287 | 5067 | 6385 | 7753 | 9570 | 10942 | 12273 | 13787 | 15173 | 16750 |
| 630 | 1781 | 3288 | 5068 | 6386 | 7754 | 9571 | 10943 | 12274 | 13788 | 15174 | 16751 |
| 631 | 1782 | 3289 | 5069 | 6387 | 7755 | 9572 | 10944 | 12275 | 13789 | 15175 | 16752 |
| 632 | 1783 | 3290 | 5070 | 6388 | 7756 | 9573 | 10945 | 12276 | 13790 | 15176 | 16753 |
| 633 | 1784 | 3291 | 5071 | 6389 | 7757 | 9579 | 10946 | 12277 | 13791 | 15177 | 16754 |
| 634 | 1785 | 3292 | 5072 | 6390 | 7758 | 9597 | 10947 | 12278 | 13792 | 15205 | 16755 |
| 640 | 1786 | 3418 | 5073 | 6391 | 7759 | 9598 | 10948 | 12279 | 13793 | 15206 | 16756 |
| 641 | 1787 | 3419 | 5074 | 6392 | 7760 | 9599 | 10949 | 12280 | 13794 | 15211 | 16757 |
| 642 | 1788 | 3420 | 5075 | 6393 | 7761 | 9600 | 10950 | 12281 | 13795 | 15212 | 16758 |
| 643 | 1789 | 3421 | 5076 | 6394 | 7762 | 9615 | 10951 | 12282 | 13843 | 15213 | 16759 |
| 644 | 1790 | 3422 | 5077 | 6395 | 7763 | 9616 | 10952 | 12283 | 13844 | 15214 | 16760 |
| 645 | 1791 | 3423 | 5078 | 6396 | 7764 | 9617 | 10953 | 12284 | 13845 | 15215 | 16761 |
| 646 | 1792 | 3424 | 5079 | 6397 | 7765 | 9618 | 10976 | 12285 | 13846 | 15216 | 16781 |
| 647 | 1793 | 3439 | 5080 | 6398 | 7766 | 9619 | 11002 | 12286 | 13847 | 15237 | 16782 |
| 704 | 1794 | 3440 | 5081 | 6399 | 7767 | 9620 | 11003 | 12311 | 13848 | 15249 | 16783 |
| 705 | 1795 | 3441 | 5082 | 6400 | 7768 | 9621 | 11004 | 12312 | 13849 | 15250 | 16784 |
| 735 | 1799 | 3442 | 5083 | 6401 | 7769 | 9632 | 11005 | 12313 | 13850 | 15251 | 16815 |
| 736 | 1800 | 3443 | 5084 | 6402 | 7770 | 9633 | 11006 | 12314 | 13851 | 15252 | 16891 |
| 737 | 1801 | 3444 | 5085 | 6403 | 7771 | 9634 | 11007 | 12315 | 13852 | 15253 | 16892 |
| 738 | 1802 | 3445 | 5086 | 6404 | 7797 | 9635 | 11008 | 12316 | 13853 | 15254 | 16893 |
| 739 | 1803 | 3446 | 5120 | 6405 | 7798 | 9636 | 11009 | 12317 | 13854 | 15287 | 16894 |
| 740 | 1804 | 3447 | 5121 | 6406 | 7799 | 9637 | 11010 | 12363 | 13855 | 15288 | 16895 |
| 741 | 1805 | 3448 | 5122 | 6407 | 7934 | 9638 | 11011 | 12364 | 13856 | 15289 | 16896 |
| 742 | 1806 | 3449 | 5123 | 6408 | 7935 | 9640 | 11012 | 12365 | 13899 | 15290 | 16897 |
| 743 | 1807 | 3450 | 5124 | 6420 | 7936 | 9641 | 11013 | 12366 | 13900 | 15291 | 16898 |
| 744 | 1829 | 3451 | 5125 | 6421 | 7983 | 9642 | 11014 | 12367 | 13901 | 15292 | 16899 |
| 745 | 1865 | 3452 | 5126 | 6523 | 7984 | 9689 | 11015 | 12368 | 13902 | 15293 | 16924 |
| 746 | 1866 | 3453 | 5127 | 6524 | 7985 | 9690 | 11016 | 12369 | 13903 | 15294 | 16925 |
| 747 | 1867 | 3454 | 5128 | 6525 | 7986 | 9691 | 11017 | 12409 | 13904 | 15303 | 16926 |
| 748 | 1868 | 3457 | 5129 | 6526 | 7987 | 9692 | 11018 | 12410 | 13905 | 15304 | 16927 |
| 749 | 1869 | 3458 | 5130 | 6527 | 7988 | 9693 | 11019 | 12411 | 13906 | 15305 | 16928 |
| 750 | 1870 | 3459 | 5131 | 6528 | 7989 | 9694 | 11020 | 12412 | 13907 | 15421 | 16929 |
| 751 | 1871 | 3480 | 5132 | 6592 | 7990 | 9695 | 11021 | 12413 | 13908 | 15422 | 16930 |
| 752 | 1872 | 3485 | 5133 | 6593 | 7991 | 9696 | 11026 | 12414 | 13909 | 15423 | 17034 |
| 753 | 1873 | 3508 | 5134 | 6594 | 7992 | 9768 | 11027 | 12415 | 13957 | 15424 | 17035 |
| 754 | 1874 | 3509 | 5135 | 6595 | 7993 | 9769 | 11028 | 12416 | 13958 | 15425 | 17036 |
| 755 | 1980 | 3522 | 5146 | 6634 | 7994 | 9770 | 11029 | 12427 | 13994 | 15426 | 17042 |
| 756 | 1981 | 3523 | 5147 | 6635 | 8037 | 9771 | 11030 | 12428 | 13995 | 15427 | 17043 |
| 820 | 1982 | 3553 | 5148 | 6636 | 8038 | 9833 | 11031 | 12429 | 14002 | 15428 | 17044 |
| 821 | 1983 | 3634 | 5149 | 6637 | 8043 | 9834 | 11032 | 12430 | 14003 | 15429 | 17045 |
| 822 | 1984 | 3635 | 5150 | 6638 | 8044 | 9835 | 11033 | 12431 | 14004 | 15430 | 17046 |
| 823 | 1985 | 3636 | 5151 | 6669 | 8045 | 9836 | 11034 | 12432 | 14005 | 15431 | 17047 |
| 824 | 1986 | 3637 | 5152 | 6676 | 8046 | 9837 | 11035 | 12433 | 14006 | 15432 | 17048 |
| 825 | 1987 | 3638 | 5153 | 6677 | 8047 | 9838 | 11036 | 12434 | 14007 | 15433 | 17049 |
| 826 | 1988 | 3639 | 5154 | 6678 | 8048 | 9839 | 11037 | 12435 | 14008 | 15434 | 17050 |
| 827 | 1989 | 3640 | 5155 | 6679 | 8049 | 9840 | 11038 | 12436 | 14009 | 15435 | 17051 |
| 828 | 2016 | 3641 | 5156 | 6680 | 8050 | 9841 | 11039 | 12437 | 14010 | 15436 | 17052 |
| 829 | 2017 | 3642 | 5157 | 6681 | 8051 | 9862 | 11040 | 12445 | 14025 | 15437 | 17053 |
| 830 | 2018 | 3680 | 5158 | 6682 | 8052 | 9863 | 11041 | 12505 | 14026 | 15438 | 17054 |
| 876 | 2034 | 3681 | 5159 | 6683 | 8063 | 9864 | 11042 | 12506 | 14027 | 15439 | 17055 |
| 877 | 2035 | 3682 | 5160 | 6684 | 8064 | 9870 | 11043 | 12507 | 14028 | 15440 | 17056 |
| 888 | 2036 | 3683 | 5173 | 6685 | 8065 | 9871 | 11044 | 12508 | 14029 | 15441 | 17057 |
| 889 | 2037 | 3694 | 5174 | 6686 | 8066 | 9872 | 11045 | 12509 | 14030 | 15442 | 17058 |
| 895 | 2038 | 3695 | 5175 | 6687 | 8139 | 9873 | 11046 | 12576 | 14041 | 15443 | 17059 |
| 896 | 2039 | 3696 | 5176 | 6688 | 8140 | 9874 | 11047 | 12577 | 14042 | 15444 | 17060 |
| 897 | 2040 | 3697 | 5177 | 6689 | 8141 | 9875 | 11048 | 12578 | 14043 | 15518 | 17061 |
| 898 | 2041 | 3701 | 5265 | 6690 | 8142 | 9876 | 11049 | 12579 | 14065 | 15519 | 17062 |
| 899 | 2042 | 3702 | 5266 | 6691 | 8143 | 9877 | 11109 | 12580 | 14066 | 15520 | 17063 |
| 992 | 2043 | 3703 | 5267 | 6692 | 8144 | 9878 | 11126 | 12581 | 14067 | 15521 | 17073 |
| 993 | 2044 | 3704 | 5268 | 6693 | 8145 | 9879 | 11127 | 12582 | 14068 | 15522 | 17074 |
| 994 | 2111 | 3705 | 5327 | 6698 | 8146 | 9880 | 11128 | 12583 | 14069 | 15523 | 17075 |
| 995 | 2112 | 3706 | 5328 | 6699 | 8147 | 9881 | 11144 | 12584 | 14070 | 15524 | 17076 |
| 996 | 2113 | 3707 | 5329 | 6700 | 8148 | 9882 | 11145 | 12627 | 14071 | 15525 | 17077 |
| 997 | 2114 | 3708 | 5330 | 6701 | 8149 | 9883 | 11146 | 12628 | 14072 | 15526 | 17078 |
| 1003 | 2115 | 3755 | 5331 | 6702 | 8212 | 9884 | 11147 | 12629 | 14073 | 15527 | 17079 |
| 1004 | 2127 | 3756 | 5336 | 6703 | 8213 | 9885 | 11148 | 12651 | 14074 | 15528 | 17080 |
| 1005 | 2128 | 3757 | 5337 | 6704 | 8214 | 9886 | 11175 | 12652 | 14075 | 15529 | 17081 |
| 1006 | 2129 | 3758 | 5338 | 6705 | 8215 | 9887 | 11176 | 12653 | 14076 | 15530 | 17082 |
| 1007 | 2130 | 3797 | 5339 | 6706 | 8216 | 9888 | 11177 | 12654 | 14100 | 15531 | 17083 |
| 1008 | 2131 | 3798 | 5340 | 6707 | 8242 | 9889 | 11178 | 12655 | 14101 | 15537 | 17084 |
| 1009 | 2132 | 3799 | 5347 | 6708 | 8243 | 9890 | 11179 | 12656 | 14102 | 15559 | 17085 |
| 1010 | 2133 | 3800 | 5348 | 6709 | 8244 | 9891 | 11180 | 12657 | 14103 | 15560 | 17086 |
| 1011 | 2134 | 3801 | 5349 | 6710 | 8245 | 9892 | 11223 | 12658 | 14147 | 15561 | 17087 |
| 1012 | 2135 | 3802 | 5350 | 6711 | 8246 | 9893 | 11224 | 12659 | 14148 | 15562 | 17116 |
| 1013 | 2136 | 3803 | 5351 | 6712 | 8247 | 9894 | 11225 | 12660 | 14149 | 15563 | 17117 |
| 1014 | 2163 | 3804 | 5352 | 6713 | 8248 | 9895 | 11226 | 12661 | 14150 | 15574 | 17118 |
| 1015 | 2164 | 3805 | 5353 | 6714 | 8254 | 9896 | 11227 | 12662 | 14151 | 15636 | 17119 |
| 1016 | 2165 | 3806 | 5354 | 6715 | 8255 | 9897 | 11288 | 12663 | 14152 | 15637 | 17120 |
| 1017 | 2166 | 3807 | 5355 | 6716 | 8256 | 9898 | 11289 | 12664 | 14153 | 15638 | 17124 |
| 1018 | 2167 | 3900 | 5356 | 6717 | 8257 | 9899 | 11354 | 12703 | 14154 | 15639 | 17125 |
| 1019 | 2205 | 3901 | 5357 | 6718 | 8258 | 9900 | 11355 | 12704 | 14155 | 15663 | 17126 |
| 1020 | 2206 | 3902 | 5358 | 6719 | 8259 | 9901 | 11356 | 12705 | 14156 | 15664 | 17127 |
| 1021 | 2207 | 3911 | 5359 | 6720 | 8260 | 9902 | 11357 | 12707 | 14157 | 15665 | 17128 |
| 1022 | 2208 | 3929 | 5360 | 6731 | 8261 | 9903 | 11361 | 12708 | 14214 | 15666 | 17129 |
| 1023 | 2209 | 3930 | 5361 | 6732 | 8262 | 9904 | 11370 | 12709 | 14238 | 15667 | 17130 |
| 1024 | 2210 | 3931 | 5362 | 6733 | 8278 | 9948 | 11371 | 12710 | 14239 | 15668 | 17131 |
| 1025 | 2211 | 3932 | 5363 | 6734 | 8279 | 9949 | 11372 | 12711 | 14240 | 15669 | 17132 |
| 1026 | 2212 | 3933 | 5364 | 6735 | 8280 | 9950 | 11373 | 12712 | 14241 | 15676 | 17133 |
| 1027 | 2222 | 3934 | 5365 | 6736 | 8281 | 9956 | 11374 | 12713 | 14242 | 15677 | 17134 |
| 1028 | 2223 | 3935 | 5366 | 6737 | 8282 | 9957 | 11375 | 12714 | 14256 | 15678 | 17135 |
| 1029 | 2224 | 3936 | 5367 | 6738 | 8283 | 9958 | 11376 | 12715 | 14257 | 15679 | 17136 |
| 1030 | 2225 | 3937 | 5369 | 6739 | 8284 | 9959 | 11377 | 12716 | 14277 | 15680 | 17137 |
| 1031 | 2226 | 3938 | 5370 | 6740 | 8285 | 9960 | 11378 | 12717 | 14278 | 15681 | 17138 |
| 1032 | 2227 | 3939 | 5371 | 6741 | 8286 | 9961 | 11379 | 12718 | 14279 | 15682 | 17139 |
| 1033 | 2239 | 3944 | 5386 | 6742 | 8287 | 9962 | 11380 | 12719 | 14280 | 15683 | 17140 |
| 1034 | 2240 | 3945 | 5387 | 6743 | 8362 | 9963 | 11383 | 12720 | 14281 | 15684 | 17141 |
| 1097 | 2241 | 3946 | 5388 | 6744 | 8392 | 9964 | 11384 | 12721 | 14282 | 15685 | 17142 |
| 1098 | 2242 | 3947 | 5389 | 6745 | 8393 | 9972 | 11385 | 12722 | 14284 | 15686 | 17143 |
| 1099 | 2243 | 3948 | 5390 | 6746 | 8457 | 9973 | 11386 | 12723 | 14285 | 15687 | 17144 |
| 1100 | 2251 | 3949 | 5391 | 6747 | 8458 | 9974 | 11392 | 12724 | 14286 | 15688 | 17145 |
| 1101 | 2252 | 3950 | 5392 | 6748 | 8485 | 9975 | 11393 | 12725 | 14287 | 15689 | 17156 |
| 1102 | 2280 | 3971 | 5425 | 6749 | 8486 | 9980 | 11394 | 12726 | 14288 | 15701 | 17157 |
| 1103 | 2281 | 3972 | 5426 | 6756 | 8487 | 9981 | 11401 | 12727 | 14289 | 15702 | 17158 |
| 1104 | 2290 | 3973 | 5427 | 6757 | 8488 | 9982 | 11402 | 12728 | 14290 | 15703 | 17159 |
| 1105 | 2291 | 3974 | 5428 | 6758 | 8489 | 9983 | 11403 | 12729 | 14291 | 15704 | 17160 |
| 1106 | 2292 | 3979 | 5429 | 6759 | 8490 | 9984 | 11428 | 12730 | 14292 | 15732 | 17181 |
| 1107 | 2293 | 3980 | 5430 | 6760 | 8491 | 9985 | 11429 | 12739 | 14293 | 15733 | 17182 |
| 1108 | 2294 | 3981 | 5431 | 6761 | 8492 | 9986 | 11430 | 12740 | 14294 | 15734 | 17183 |
| 1109 | 2295 | 3982 | 5432 | 6762 | 8493 | 9987 | 11431 | 12741 | 14295 | 15735 | 17184 |
| 1110 | 2296 | 3983 | 5433 | 6763 | 8494 | 9988 | 11432 | 12742 | 14296 | 15736 | 17185 |
| 1111 | 2354 | 3984 | 5434 | 6764 | 8495 | 9989 | 11433 | 12743 | 14297 | 15737 | 17186 |
| 1133 | 2355 | 3985 | 5435 | 6765 | 8496 | 9990 | 11434 | 12803 | 14298 | 15738 | 17216 |
| 1134 | 2356 | 4000 | 5436 | 6766 | 8497 | 10005 | 11435 | 12804 | 14299 | 15739 | 17217 |
| 1135 | 2357 | 4001 | 5437 | 6767 | 8498 | 10006 | 11436 | 12805 | 14326 | 15740 | 17218 |
| 1136 | 2358 | 4002 | 5438 | 6768 | 8499 | 10047 | 11437 | 12806 | 14327 | 15741 | 17235 |
| 1137 | 2382 | 4003 | 5439 | 6769 | 8500 | 10048 | 11438 | 12807 | 14328 | 15742 | 17236 |
| 1138 | 2383 | 4004 | 5440 | 6770 | 8501 | 10049 | 11439 | 12808 | 14329 | 15743 | 17237 |
| 1139 | 2384 | 4005 | 5441 | 6771 | 8591 | 10050 | 11440 | 12841 | 14330 | 15847 | 17238 |
| 1140 | 2385 | 4009 | 5442 | 6772 | 8592 | 10051 | 11441 | 12842 | 14331 | 15848 | 17239 |
| 1141 | 2386 | 4010 | 5443 | 6773 | 8593 | 10052 | 11444 | 12843 | 14332 | 15849 | 17240 |
| 1142 | 2387 | 4011 | 5449 | 6774 | 8594 | 10053 | 11445 | 12844 | 14333 | 15850 | 17241 |
| 1148 | 2388 | 4012 | 5450 | 6775 | 8595 | 10054 | 11463 | 12845 | 14354 | 15851 | 17242 |
| 1149 | 2417 | 4013 | 5451 | 6776 | 8596 | 10055 | 11464 | 12846 | 14355 | 15852 | 17243 |
| 1227 | 2418 | 4014 | 5452 | 6777 | 8610 | 10056 | 11465 | 12915 | 14356 | 15853 | 17253 |
| 1228 | 2419 | 4015 | 5453 | 6778 | 8611 | 10057 | 11466 | 12951 | 14357 | 15854 | 17254 |
| 1229 | 2420 | 4016 | 5454 | 6779 | 8612 | 10058 | 11467 | 12952 | 14358 | 15958 | 17255 |
| 1230 | 2421 | 4017 | 5455 | 6810 | 8613 | 10059 | 11481 | 12980 | 14359 | 15985 | 17256 |
| 1231 | 2422 | 4018 | 5456 | 6823 | 8617 | 10089 | 11482 | 12981 | 14400 | 15986 | 17257 |
| 1232 | 2423 | 4019 | 5457 | 6824 | 8706 | 10090 | 11503 | 12982 | 14401 | 15987 | 17258 |
| 1233 | 2424 | 4020 | 5458 | 6825 | 8707 | 10091 | 11504 | 12983 | 14402 | 15993 | 17259 |
| 1234 | 2425 | 4021 | 5459 | 6826 | 8708 | 10092 | 11505 | 12984 | 14403 | 15994 | 17260 |
| 1235 | 2426 | 4022 | 5465 | 6827 | 8709 | 10093 | 11506 | 12985 | 14404 | 15995 | 17261 |
| 1236 | 2427 | 4104 | 5466 | 6890 | 8710 | 10094 | 11507 | 12986 | 14405 | 15996 | 17262 |
| 1237 | 2428 | 4120 | 5486 | 6891 | 8711 | 10095 | 11508 | 12987 | 14406 | 15997 | 17263 |
| 1238 | 2429 | 4121 | 5487 | 6933 | 8712 | 10096 | 11509 | 12988 | 14407 | 15998 | 17264 |
| 1239 | 2430 | 4122 | 5488 | 6934 | 8713 | 10097 | 11510 | 12989 | 14408 | 15999 | 17265 |
| 1240 | 2431 | 4123 | 5489 | 6935 | 8714 | 10098 | 11511 | 12990 | 14409 | 16000 | 17266 |
| 1251 | 2432 | 4124 | 5490 | 6936 | 8715 | 10099 | 11512 | 13141 | 14410 | 16001 | 17267 |
| 1252 | 2433 | 4125 | 5491 | 6937 | 8716 | 10100 | 11593 | 13142 | 14411 | 16002 | 17278 |
| 1253 | 2549 | 4126 | 5492 | 6938 | 8717 | 10101 | 11594 | 13143 | 14412 | 16003 | 17279 |
| 1272 | 2563 | 4127 | 5493 | 6939 | 8718 | 10104 | 11595 | 13144 | 14413 | 16004 | 17280 |
| 1273 | 2564 | 4128 | 5494 | 6940 | 8719 | 10105 | 11596 | 13145 | 14414 | 16005 | 17281 |
| 1274 | 2565 | 4129 | 5495 | 6941 | 8720 | 10106 | 11597 | 13146 | 14415 | 16006 | 17282 |
| 1275 | 2566 | 4130 | 5496 | 6942 | 8721 | 10107 | 11598 | 13147 | 14416 | 16007 | 17283 |
| 1276 | 2567 | 4131 | 5497 | 6943 | 8722 | 10108 | 11599 | 13148 | 14417 | 16008 | 17315 |
| 1277 | 2568 | 4132 | 5498 | 6944 | 8723 | 10109 | 11600 | 13149 | 14466 | 16009 | 17316 |
| 1278 | 2569 | 4133 | 5499 | 6950 | 8724 | 10110 | 11601 | 13150 | 14467 | 16010 | 17317 |
| 1279 | 2570 | 4134 | 5500 | 6951 | 8725 | 10116 | 11602 | 13151 | 14468 | 16011 | 17318 |
| 1280 | 2585 | 4135 | 5501 | 6952 | 8726 | 10117 | 11603 | 13152 | 14469 | 16012 | 17319 |
| 1323 | 2586 | 4182 | 5502 | 6953 | 8833 | 10118 | 11653 | 13153 | 14470 | 16013 | 17320 |
| 1328 | 2587 | 4183 | 5539 | 6954 | 8834 | 10119 | 11654 | 13154 | 14471 | 16045 | 17321 |
| 1329 | 2588 | 4184 | 5540 | 6955 | 8878 | 10142 | 11655 | 13155 | 14472 | 16046 | 17322 |
| 1330 | 2589 | 4185 | 5541 | 6997 | 8879 | 10143 | 11686 | 13156 | 14473 | 16047 | 17323 |
| 1331 | 2590 | 4187 | 5542 | 6998 | 8880 | 10144 | 11687 | 13157 | 14474 | 16048 | 17396 |
| 1332 | 2591 | 4188 | 5543 | 6999 | 8881 | 10152 | 11688 | 13158 | 14512 | 16049 | 17397 |
| 1338 | 2592 | 4295 | 5544 | 7077 | 8935 | 10153 | 11689 | 13159 | 14513 | 16050 | 17398 |
| 1339 | 2593 | 4339 | 5553 | 7084 | 8936 | 10154 | 11702 | 13160 | 14529 | 16051 | 17399 |
| 1347 | 2602 | 4340 | 5554 | 7085 | 8951 | 10155 | 11703 | 13161 | 14530 | 16052 | 17400 |
| 1348 | 2603 | 4341 | 5555 | 7086 | 8952 | 10177 | 11704 | 13168 | 14531 | 16053 | 17401 |
| 1349 | 2604 | 4342 | 5556 | 7087 | 8953 | 10178 | 11705 | 13248 | 14532 | 16054 | 17402 |
| 1350 | 2605 | 4343 | 5557 | 7088 | 8954 | 10179 | 11706 | 13249 | 14533 | 16160 | |
| 1351 | 2606 | 4344 | 5558 | 7165 | 8955 | 10180 | 11707 | 13250 | 14534 | 16161 | |
| 1352 | 2607 | 4345 | 5559 | 7166 | 8956 | 10181 | 11708 | 13251 | 14535 | 16162 | |
| 1353 | 2608 | 4377 | 5560 | 7167 | 8957 | 10182 | 11709 | 13252 | 14536 | 16163 | |
| 1354 | 2609 | 4378 | 5603 | 7168 | 8958 | 10183 | 11710 | 13253 | 14537 | 16164 | |
| 1355 | 2610 | 4386 | 5604 | 7169 | 8959 | 10184 | 11711 | 13254 | 14538 | 16165 | |

**Table 5 - small list**

| **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** | **SEQ ID NO** |
|---|---|---|---|---|---|---|---|---|---|---|---|
| 8 | 1773 | 3024 | 4521 | 5488 | 6952 | 8726 | 9980 | 11509 | 12727 | 14533 | 16349 |
| 9 | 1774 | 3025 | 4522 | 5489 | 6953 | 8881 | 9981 | 11510 | 12728 | 14534 | 16350 |
| 19 | 1775 | 3026 | 4523 | 5490 | 6997 | 8984 | 9982 | 11511 | 12729 | 14535 | 16351 |
| 20 | 1776 | 3027 | 4524 | 5491 | 6998 | 8985 | 9983 | 11512 | 12730 | 14536 | 16352 |
| 21 | 1777 | 3073 | 4572 | 5492 | 6999 | 8986 | 9984 | 11653 | 12803 | 14537 | 16353 |
| 48 | 1778 | 3074 | 4573 | 5493 | 7077 | 8987 | 9985 | 11654 | 12804 | 14538 | 16354 |
| 88 | 1779 | 3075 | 4721 | 5494 | 7084 | 8988 | 9986 | 11655 | 12805 | 14539 | 16355 |
| 94 | 1780 | 3076 | 4728 | 5495 | 7085 | 8989 | 9987 | 11702 | 12806 | 14540 | 16356 |
| 95 | 1781 | 3077 | 4729 | 5496 | 7086 | 9003 | 9988 | 11703 | 12807 | 14541 | 16357 |
| 96 | 1782 | 3078 | 4730 | 5497 | 7087 | 9004 | 9989 | 11704 | 12808 | 14542 | 16358 |
| 97 | 1783 | 3079 | 4731 | 5498 | 7088 | 9005 | 9990 | 11705 | 12842 | 14543 | 16359 |
| 98 | 1784 | 3080 | 4732 | 5499 | 7165 | 9006 | 10005 | 11706 | 12843 | 14544 | 16360 |
| 99 | 1785 | 3081 | 4733 | 5500 | 7166 | 9007 | 10006 | 11707 | 12844 | 14545 | 16389 |
| 100 | 1786 | 3082 | 4734 | 5501 | 7167 | 9008 | 10089 | 11708 | 12845 | 14564 | 16390 |
| 117 | 1787 | 3083 | 4735 | 5502 | 7168 | 9009 | 10090 | 11709 | 12846 | 14565 | 16391 |
| 118 | 1788 | 3084 | 4768 | 5626 | 7169 | 9010 | 10091 | 11710 | 12915 | 14566 | 16392 |
| 143 | 1789 | 3085 | 4769 | 5643 | 7170 | 9011 | 10092 | 11711 | 12980 | 14571 | 16393 |
| 144 | 1790 | 3170 | 4819 | 5644 | 7171 | 9018 | 10093 | 11712 | 12981 | 14572 | 16394 |
| 145 | 1791 | 3171 | 4820 | 5712 | 7175 | 9019 | 10094 | 11713 | 12982 | 14573 | 16395 |
| 146 | 1792 | 3267 | 4821 | 5713 | 7176 | 9151 | 10095 | 11714 | 12983 | 14574 | 16396 |
| 147 | 1793 | 3268 | 4822 | 5725 | 7177 | 9152 | 10096 | 11715 | 12984 | 14575 | 16472 |
| 148 | 1794 | 3269 | 4823 | 5726 | 7178 | 9153 | 10097 | 11716 | 13168 | 14576 | 16473 |
| 149 | 1795 | 3270 | 4824 | 5727 | 7179 | 9154 | 10098 | 11868 | 13248 | 14577 | 16474 |
| 150 | 1799 | 3271 | 4837 | 5728 | 7266 | 9155 | 10099 | 11869 | 13249 | 14578 | 16475 |
| 151 | 1800 | 3272 | 4887 | 5729 | 7267 | 9156 | 10100 | 11870 | 13250 | 14579 | 16476 |
| 152 | 1801 | 3273 | 4888 | 5730 | 7268 | 9157 | 10101 | 11871 | 13251 | 14580 | 16482 |
| 166 | 1802 | 3274 | 4889 | 5825 | 7269 | 9158 | 10104 | 11872 | 13252 | 14581 | 16483 |
| 167 | 1865 | 3275 | 4890 | 5865 | 7270 | 9159 | 10105 | 11873 | 13253 | 14582 | 16484 |
| 204 | 1866 | 3276 | 4891 | 5866 | 7271 | 9160 | 10106 | 11874 | 13254 | 14583 | 16485 |
| 205 | 1867 | 3277 | 4892 | 5867 | 7272 | 9161 | 10142 | 11875 | 13255 | 14584 | 16486 |
| 206 | 1868 | 3278 | 4893 | 5868 | 7273 | 9193 | 10143 | 11876 | 13256 | 14585 | 16487 |
| 207 | 1869 | 3279 | 4894 | 5869 | 7274 | 9194 | 10144 | 11877 | 13263 | 14616 | 16488 |
| 208 | 1870 | 3280 | 4895 | 5870 | 7275 | 9195 | 10152 | 11900 | 13264 | 14617 | 16489 |
| 209 | 1871 | 3281 | 4896 | 5871 | 7276 | 9196 | 10153 | 11901 | 13265 | 14618 | 16490 |
| 210 | 1872 | 3282 | 4897 | 5872 | 7377 | 9197 | 10154 | 11902 | 13266 | 14619 | 16518 |
| 295 | 1873 | 3283 | 4898 | 5873 | 7378 | 9330 | 10155 | 11903 | 13267 | 14620 | 16519 |
| 296 | 1874 | 3284 | 4899 | 5999 | 7379 | 9331 | 10177 | 11939 | 13268 | 14621 | 16520 |
| 297 | 1986 | 3285 | 4900 | 6000 | 7475 | 9332 | 10178 | 11940 | 13269 | 14622 | 16521 |
| 298 | 1987 | 3286 | 4901 | 6001 | 7476 | 9333 | 10179 | 11941 | 13270 | 14635 | 16537 |
| 299 | 1988 | 3287 | 4902 | 6035 | 7477 | 9334 | 10180 | 11942 | 13271 | 14636 | 16538 |
| 300 | 1989 | 3288 | 4903 | 6036 | 7497 | 9335 | 10181 | 11943 | 13272 | 14637 | 16539 |
| 301 | 2111 | 3289 | 4904 | 6037 | 7498 | 9336 | 10200 | 11944 | 13395 | 14638 | 16540 |
| 302 | 2112 | 3290 | 4905 | 6108 | 7499 | 9337 | 10201 | 11945 | 13396 | 14639 | 16541 |
| 303 | 2113 | 3291 | 4906 | 6109 | 7500 | 9338 | 10202 | 11946 | 13397 | 14640 | 16542 |
| 431 | 2114 | 3292 | 4907 | 6110 | 7501 | 9339 | 10265 | 11947 | 13398 | 14641 | 16543 |
| 432 | 2115 | 3418 | 4908 | 6111 | 7502 | 9340 | 10266 | 11948 | 13399 | 14780 | 16577 |
| 433 | 2129 | 3419 | 4909 | 6163 | 7503 | 9341 | 10267 | 11949 | 13400 | 14968 | 16578 |
| 434 | 2130 | 3420 | 4995 | 6164 | 7504 | 9342 | 10268 | 11950 | 13401 | 14969 | 16579 |
| 435 | 2131 | 3421 | 4996 | 6165 | 7574 | 9343 | 10269 | 11951 | 13402 | 14970 | 16580 |
| 436 | 2132 | 3422 | 4997 | 6185 | 7575 | 9344 | 10270 | 11952 | 13403 | 14971 | 16581 |
| 437 | 2133 | 3423 | 4998 | 6186 | 7576 | 9345 | 10401 | 11953 | 13461 | 14972 | 16582 |
| 438 | 2134 | 3424 | 5048 | 6187 | 7577 | 9346 | 10402 | 11954 | 13462 | 14973 | 16583 |
| 439 | 2135 | 3439 | 5049 | 6188 | 7578 | 9347 | 10403 | 11955 | 13463 | 14974 | 16584 |
| 440 | 2136 | 3440 | 5080 | 6189 | 7579 | 9348 | 10404 | 11956 | 13464 | 14975 | 16585 |
| 495 | 2163 | 3441 | 5126 | 6190 | 7580 | 9349 | 10405 | 11957 | 13465 | 14976 | 16586 |
| 538 | 2164 | 3442 | 5127 | 6191 | 7581 | 9350 | 10406 | 11958 | 13466 | 14977 | 16587 |
| 539 | 2165 | 3443 | 5128 | 6192 | 7582 | 9351 | 10407 | 11959 | 13468 | 14978 | 16588 |
| 540 | 2166 | 3444 | 5129 | 6193 | 7597 | 9352 | 10408 | 11960 | 13469 | 14979 | 16589 |
| 541 | 2167 | 3445 | 5130 | 6194 | 7598 | 9353 | 10409 | 11961 | 13470 | 14980 | 16590 |
| 542 | 2205 | 3446 | 5131 | 6199 | 7599 | 9354 | 10410 | 11962 | 13471 | 14981 | 16591 |
| 543 | 2206 | 3447 | 5132 | 6200 | 7600 | 9355 | 10411 | 11963 | 13472 | 15006 | 16592 |
| 544 | 2207 | 3448 | 5133 | 6201 | 7601 | 9356 | 10412 | 11964 | 13473 | 15007 | 16593 |
| 545 | 2208 | 3449 | 5134 | 6202 | 7602 | 9536 | 10413 | 11965 | 13474 | 15008 | 16677 |
| 573 | 2209 | 3450 | 5135 | 6203 | 7603 | 9537 | 10414 | 11966 | 13475 | 15009 | 16678 |
| 574 | 2210 | 3451 | 5146 | 6204 | 7604 | 9547 | 10415 | 11967 | 13476 | 15010 | 16679 |
| 575 | 2211 | 3452 | 5147 | 6205 | 7605 | 9548 | 10416 | 11968 | 13477 | 15011 | 16680 |
| 576 | 2212 | 3453 | 5154 | 6206 | 7606 | 9549 | 10420 | 11969 | 13478 | 15012 | 16681 |
| 577 | 2222 | 3454 | 5155 | 6207 | 7636 | 9550 | 10421 | 11970 | 13479 | 15173 | 16682 |
| 578 | 2223 | 3457 | 5156 | 6208 | 7637 | 9553 | 10422 | 12013 | 13480 | 15174 | 16683 |
| 579 | 2224 | 3458 | 5157 | 6209 | 7638 | 9554 | 10423 | 12014 | 13481 | 15175 | 16684 |
| 580 | 2225 | 3459 | 5158 | 6210 | 7639 | 9555 | 10424 | 12015 | 13482 | 15176 | 16685 |
| 640 | 2226 | 3480 | 5159 | 6211 | 7640 | 9556 | 10436 | 12016 | 13483 | 15177 | 16686 |
| 641 | 2227 | 3481 | 5160 | 6212 | 7641 | 9557 | 10437 | 12017 | 13484 | 15211 | 16687 |
| 642 | 2239 | 3482 | 5173 | 6213 | 7643 | 9558 | 10438 | 12034 | 13485 | 15212 | 16688 |
| 643 | 2240 | 3483 | 5174 | 6214 | 7644 | 9559 | 10439 | 12064 | 13486 | 15213 | 16689 |
| 644 | 2241 | 3484 | 5175 | 6215 | 7645 | 9560 | 10460 | 12065 | 13487 | 15214 | 16690 |
| 645 | 2242 | 3485 | 5176 | 6216 | 7646 | 9561 | 10461 | 12066 | 13488 | 15215 | 16750 |
| 646 | 2243 | 3508 | 5177 | 6217 | 7647 | 9562 | 10462 | 12067 | 13489 | 15216 | 16751 |
| 647 | 2354 | 3509 | 5327 | 6297 | 7648 | 9579 | 10463 | 12068 | 13490 | 15287 | 16752 |
| 704 | 2355 | 3522 | 5328 | 6387 | 7649 | 9615 | 10574 | 12069 | 13491 | 15288 | 16753 |
| 705 | 2356 | 3523 | 5329 | 6388 | 7650 | 9616 | 10575 | 12070 | 13506 | 15289 | 16754 |
| 735 | 2357 | 3553 | 5330 | 6389 | 7651 | 9617 | 10618 | 12071 | 13507 | 15290 | 16755 |
| 736 | 2358 | 3634 | 5331 | 6390 | 7652 | 9618 | 10660 | 12072 | 13587 | 15291 | 16756 |
| 737 | 2417 | 3635 | 5336 | 6391 | 7653 | 9619 | 10661 | 12073 | 13588 | 15292 | 16757 |
| 738 | 2418 | 3636 | 5337 | 6392 | 7688 | 9620 | 10662 | 12074 | 13589 | 15293 | 16758 |
| 739 | 2419 | 3637 | 5338 | 6393 | 7689 | 9621 | 10663 | 12075 | 13590 | 15294 | 16759 |
| 740 | 2420 | 3638 | 5339 | 6394 | 7690 | 9632 | 10664 | 12076 | 13591 | 15444 | 16760 |
| 876 | 2421 | 3639 | 5340 | 6395 | 7702 | 9633 | 10665 | 12077 | 13592 | 15518 | 16761 |
| 877 | 2422 | 3640 | 5347 | 6396 | 7703 | 9634 | 10666 | 12078 | 13697 | 15519 | 16781 |
| 888 | 2423 | 3641 | 5348 | 6397 | 7704 | 9635 | 10667 | 12079 | 13698 | 15520 | 16782 |
| 889 | 2424 | 3642 | 5349 | 6398 | 7705 | 9636 | 10668 | 12080 | 13699 | 15521 | 16783 |
| 895 | 2425 | 3680 | 5350 | 6399 | 7706 | 9637 | 10669 | 12081 | 13700 | 15522 | 16784 |
| 896 | 2426 | 3681 | 5351 | 6400 | 7934 | 9638 | 10670 | 12098 | 13701 | 15523 | 16891 |
| 897 | 2427 | 3682 | 5352 | 6401 | 7935 | 9640 | 10716 | 12099 | 13702 | 15524 | 16892 |
| 898 | 2428 | 3683 | 5353 | 6592 | 7936 | 9641 | 10717 | 12195 | 13703 | 15525 | 16893 |
| 899 | 2429 | 3694 | 5354 | 6593 | 7983 | 9642 | 10718 | 12204 | 13704 | 15526 | 16894 |
| 1104 | 2430 | 3695 | 5355 | 6594 | 7984 | 9689 | 10719 | 12205 | 13705 | 15527 | 16895 |
| 1105 | 2431 | 3696 | 5356 | 6595 | 7985 | 9690 | 10720 | 12206 | 13712 | 15528 | 16896 |
| 1106 | 2432 | 3697 | 5357 | 6634 | 7986 | 9691 | 10721 | 12207 | 13713 | 15529 | 16897 |
| 1107 | 2433 | 3701 | 5358 | 6635 | 7987 | 9692 | 10722 | 12208 | 13719 | 15530 | 16898 |
| 1148 | 2563 | 3702 | 5359 | 6636 | 7988 | 9693 | 10773 | 12209 | 13720 | 15531 | 16899 |
| 1149 | 2564 | 3703 | 5360 | 6637 | 7989 | 9694 | 10942 | 12210 | 13721 | 15663 | 17042 |
| 1227 | 2565 | 3704 | 5361 | 6638 | 7990 | 9695 | 10943 | 12256 | 13722 | 15664 | 17043 |
| 1228 | 2566 | 3705 | 5362 | 6669 | 7991 | 9696 | 10944 | 12257 | 13723 | 15665 | 17044 |
| 1229 | 2567 | 3706 | 5363 | 6714 | 7992 | 9768 | 10945 | 12258 | 13724 | 15666 | 17045 |
| 1230 | 2568 | 3707 | 5364 | 6715 | 7993 | 9769 | 10946 | 12259 | 13725 | 15667 | 17046 |
| 1231 | 2569 | 3708 | 5365 | 6716 | 7994 | 9770 | 10947 | 12260 | 13726 | 15668 | 17047 |
| 1232 | 2570 | 3911 | 5366 | 6717 | 8063 | 9771 | 10948 | 12261 | 13727 | 15669 | 17048 |
| 1233 | 2686 | 3929 | 5367 | 6718 | 8064 | 9862 | 10949 | 12262 | 13957 | 15701 | 17049 |
| 1234 | 2860 | 3930 | 5369 | 6719 | 8065 | 9863 | 10950 | 12263 | 13958 | 15702 | 17050 |
| 1235 | 2864 | 3931 | 5370 | 6720 | 8066 | 9864 | 10951 | 12264 | 13994 | 15703 | 17051 |
| 1236 | 2865 | 3932 | 5371 | 6731 | 8139 | 9870 | 10952 | 12311 | 13995 | 15704 | 17052 |
| 1237 | 2866 | 3933 | 5386 | 6732 | 8140 | 9871 | 10953 | 12312 | 14002 | 15958 | 17053 |
| 1238 | 2867 | 3934 | 5387 | 6733 | 8141 | 9872 | 10976 | 12313 | 14003 | 15985 | 17054 |
| 1239 | 2904 | 3935 | 5388 | 6734 | 8142 | 9873 | 11015 | 12314 | 14004 | 15986 | 17055 |
| 1240 | 2905 | 3936 | 5389 | 6735 | 8242 | 9874 | 11016 | 12315 | 14005 | 15987 | 17056 |
| 1323 | 2906 | 3937 | 5390 | 6736 | 8243 | 9875 | 11017 | 12316 | 14006 | 15993 | 17057 |
| 1330 | 2907 | 3938 | 5391 | 6737 | 8362 | 9876 | 11033 | 12445 | 14007 | 15994 | 17058 |
| 1331 | 2908 | 3939 | 5392 | 6738 | 8392 | 9877 | 11034 | 12576 | 14008 | 15995 | 17059 |
| 1332 | 2909 | 3944 | 5425 | 6756 | 8393 | 9878 | 11035 | 12577 | 14009 | 15996 | 17060 |
| 1338 | 2910 | 3945 | 5426 | 6757 | 8457 | 9879 | 11036 | 12578 | 14010 | 15997 | 17061 |
| 1339 | 2911 | 3946 | 5427 | 6758 | 8458 | 9880 | 11037 | 12579 | 14147 | 15998 | 17062 |
| 1500 | 2912 | 3947 | 5428 | 6759 | 8485 | 9881 | 11038 | 12580 | 14148 | 15999 | 17063 |
| 1501 | 2913 | 3948 | 5429 | 6760 | 8486 | 9882 | 11126 | 12581 | 14149 | 16000 | 17124 |
| 1502 | 2914 | 3949 | 5430 | 6761 | 8487 | 9883 | 11127 | 12582 | 14150 | 16001 | 17125 |
| 1574 | 2915 | 3950 | 5431 | 6762 | 8488 | 9884 | 11128 | 12583 | 14151 | 16002 | 17126 |
| 1575 | 2916 | 4009 | 5432 | 6763 | 8489 | 9885 | 11144 | 12584 | 14152 | 16003 | 17127 |
| 1576 | 2917 | 4010 | 5433 | 6764 | 8490 | 9886 | 11145 | 12627 | 14153 | 16004 | 17128 |
| 1581 | 2918 | 4011 | 5434 | 6765 | 8491 | 9887 | 11146 | 12628 | 14154 | 16005 | 17129 |
| 1582 | 2919 | 4012 | 5435 | 6766 | 8492 | 9888 | 11147 | 12629 | 14155 | 16006 | 17130 |
| 1583 | 2920 | 4013 | 5436 | 6767 | 8493 | 9889 | 11148 | 12703 | 14156 | 16007 | 17131 |
| 1584 | 2921 | 4014 | 5437 | 6768 | 8494 | 9890 | 11175 | 12704 | 14157 | 16320 | 17132 |
| 1585 | 2929 | 4015 | 5438 | 6769 | 8495 | 9891 | 11176 | 12705 | 14277 | 16321 | 17133 |
| 1637 | 2930 | 4016 | 5439 | 6770 | 8496 | 9892 | 11361 | 12707 | 14278 | 16322 | 17278 |
| 1638 | 2931 | 4017 | 5440 | 6771 | 8497 | 9893 | 11434 | 12708 | 14279 | 16323 | 17279 |
| 1639 | 2932 | 4018 | 5441 | 6772 | 8498 | 9894 | 11435 | 12709 | 14280 | 16324 | 17280 |
| 1640 | 2933 | 4019 | 5442 | 6773 | 8499 | 9895 | 11436 | 12710 | 14281 | 16325 | 17281 |
| 1641 | 2934 | 4020 | 5443 | 6774 | 8500 | 9896 | 11437 | 12711 | 14282 | 16326 | 17282 |
| 1642 | 2971 | 4104 | 5449 | 6775 | 8501 | 9897 | 11438 | 12712 | 14326 | 16327 | 17283 |
| 1643 | 2972 | 4182 | 5450 | 6776 | 8610 | 9898 | 11439 | 12713 | 14327 | 16328 | 17396 |
| 1644 | 2973 | 4183 | 5451 | 6777 | 8611 | 9899 | 11440 | 12714 | 14328 | 16329 | 17397 |
| 1645 | 2974 | 4184 | 5452 | 6778 | 8612 | 9900 | 11441 | 12715 | 14329 | 16337 | 17398 |
| 1646 | 2975 | 4185 | 5453 | 6779 | 8613 | 9901 | 11463 | 12716 | 14330 | 16338 | 17399 |
| 1690 | 2976 | 4295 | 5454 | 6810 | 8711 | 9902 | 11464 | 12717 | 14331 | 16339 | 17400 |
| 1726 | 2977 | 4386 | 5455 | 6890 | 8712 | 9903 | 11465 | 12718 | 14332 | 16340 | 17401 |
| 1727 | 2978 | 4513 | 5456 | 6891 | 8713 | 9904 | 11466 | 12719 | 14333 | 16341 | 17402 |
| 1728 | 2979 | 4514 | 5457 | 6938 | 8714 | 9948 | 11467 | 12720 | 14400 | 16342 | |
| 1729 | 2980 | 4515 | 5458 | 6939 | 8715 | 9949 | 11503 | 12721 | 14401 | 16343 | |
| 1730 | 2981 | 4516 | 5459 | 6940 | 8716 | 9950 | 11504 | 12722 | 14402 | 16344 | |
| 1731 | 2982 | 4517 | 5465 | 6941 | 8717 | 9972 | 11505 | 12723 | 14512 | 16345 | |
| 1732 | 2983 | 4518 | 5466 | 6944 | 8718 | 9973 | 11506 | 12724 | 14513 | 16346 | |
| 1733 | 2984 | 4519 | 5486 | 6950 | 8719 | 9974 | 11507 | 12725 | 14531 | 16347 | |
| 1772 | 2985 | 4520 | 5487 | 6951 | 8720 | 9975 | 11508 | 12726 | 14532 | 16348 | |

In a specific embodiment, the set of markers consists of spliceosomal proteins. The invention provides a method of using the above markers to distinguish between patients with good or poor prognosis, i.e., between patients belonging to groups classified as AML high-risk or low-risk.

The sets of markers listed in Tables 4-5 partially overlap; in other words, some markers are present in multiple sets, while other markers are unique to a set.

### Sets of protein markers:

In the clinic the selected protein marker may differ from patient to patient, as not all protein markers are identified in all patients. As exemplified in Example 2, protein markers used in a prospective diagnosis may be any protein markers that are identified from one or more samples from one or more patients, that are also provided herein, i.e., in Tables 1, 4 and/or 5 or which sequence is any of SEQ ID NOs 1-17500. As described in Example 1, in the data analysis Protein groups were defined based on gene symbols to obtain a gene symbol centric quantification, i.e., the SEQ ID NOs 1-17500 provided in the sequence listing can be defined in groups based on the gene symbols provided in Table 1. Thus, in a sample from a patient, it is common that of the Gene Symbols listed in Table 1, only a subset is identified in the patient, and are used for the classification. Due to the difference in protein expression between individual patients, providing at minimum of protein markers from the list that should be present is preferred. For instance, in a nonlimiting example, a sample could e.g., contain 2005 of the 2519 protein markers identified by their Gene Symbols listed in Table 1, of the 2005 protein markers, only 1038 of the protein markers are also correctly upregulated or downregulated, as they share their sequence with the sequences of the SEQ ID NOs as provided in Tables 2 and 3, thus the classification is done on the basis of the 1038 protein markers out of the total 2519 protein markers.

In that regard, all proteins that are found to be usable for quantitatively determining the proteomic risk profile of an AML patient are protein markers with a sequence listed in the sequence listing, i.e., SEQ ID NOs: 1-17500. By identifying abnormal protein levels of at least 5 proteins selected from the list of proteins with SEQ ID NOs: 1-17500 as listed in the sequence listing, patients can be stratified to belong to high-risk or low risk groups or to high-risk, intermediate-risk or low-risk groups. Tables 4-5 list presently preferred protein markers for determining abnormal levels of which allows stratifying patients to belong to high-risk or low-risk groups.

In the list of proteins with SEQ ID NOs: 1-17500, protein expression levels are considered abnormal when the level of any one of the proteins with a SEQ ID NO as provided in Table 3 are downregulated to <95% of the median expression level for the intermediate samples of the cohort, or any one of the proteins with a SEQ ID NO as provided in Table 2 are up-regulated >5% above the median expression level of the cohort.

In a preferred embodiment, the method for stratifying an AML patient comprises determining that at least 10 proteins selected from the list of proteins with SEQ ID NOs: 1-17500 have an abnormal expression level. In a preferred embodiment, the method for stratifying an AML patient comprises determining that at least 50 proteins selected from the list of proteins with SEQ ID NOs: 1-17500 have an abnormal expression level. In a preferred embodiment, the method for stratifying an AML patient comprises determining that at least 100 proteins selected from the list of proteins with SEQ ID NOs: 1-17500 have an abnormal expression level. In a preferred embodiment, the method for stratifying an AML patient comprises determining that at least 1000 proteins selected from the list of proteins with SEQ ID NOs: 1-17500 have an abnormal expression level. In a preferred embodiment, the method for stratifying an AML patient comprises determining that at least 10000 proteins selected from the list of proteins with SEQ ID NOs: 1-17500 have an abnormal expression level. In a preferred embodiment, the method for stratifying an AML patient comprises determining that at least 15000 proteins selected from the list of proteins with SEQ ID NOs: 1-17500 have an abnormal expression level.

In a preferred embodiment, the method for stratifying an AML patient comprises determining that at least 10, such as at least 100, 1000, 10000 or 15000 proteins selected from the list of proteins with SEQ ID NOs: 1-17500 have an abnormal expression level that correspond to the direction of abnormal expression as provided in Table 2, which specifies upregulated proteins or as provided in Table 3 which specifies downregulated proteins.

In a preferred embodiment, the method for stratifying an AML patient comprises determining that at least 50 % of the proteins selected from the list of proteins with SEQ ID NO: 1-17500 have an abnormal level. In a preferred embodiment, the method for stratifying an AML patient as belonging to a high-risk group comprises determining that at least 50 % of the proteins selected from the list of proteins with SEQ ID NO: 1-17500 have an abnormal level. In a preferred embodiment, the method for stratifying an AML patient as belonging to a low-risk group comprises determining that less than 50 % of the proteins selected from the list of proteins with SEQ ID NO: 1-17500 have an abnormal level.

In a preferred embodiment, the method for stratifying an AML patient as belonging to a intermediate-risk group comprises determining that more than 25 % but less than 50 % of the proteins selected from the list of proteins with SEQ ID NO: 1-17500 have an abnormal level.

In a preferred embodiment, the method for stratifying an AML patient as belonging to a low-risk group comprises determining that less than 25 % of the proteins selected from the list of proteins with SEQ ID NO: 1-17500 have an abnormal level.

In a preferred embodiment, the method for stratifying an AML patient comprises determining that at least 50 % of the proteins selected from the list of protein markers identified by their Gene Symbol as provided in Table 1 have an abnormal level. In a preferred embodiment, the method for stratifying an AML patient as belonging to a high-risk group comprises determining that at least 50 % of the proteins selected from the list of protein markers identified by their Gene Symbol as provided in Table 1 have an abnormal level. In a preferred embodiment, the method for stratifying an AML patient as belonging to a low-risk group comprises determining that less than 50 % of the proteins selected from the list of protein markers identified by their Gene Symbol as provided in Table 1 have an abnormal level.

In a preferred embodiment, the method for stratifying an AML patient as belonging to an intermediate-risk group comprises determining that more than 25 % but less than 50 % of the proteins selected from the list of protein markers identified by their Gene Symbol as provided in Table 1 have an abnormal level.

In a preferred embodiment, the method for stratifying an AML patient as belonging to a low-risk group comprises determining that less than 25 % of the proteins selected from the list of protein markers identified by their Gene Symbol as provided in Table 1 have an abnormal level.

The herein presented method is a tool for stratifying an AML patient into a high-risk or a low-risk group, wherein the proteomic risk profile of said patient is done by identifying abnormal protein levels of at least 5 proteins selected from the list of proteins with SEQ ID NOs as provided in table 4, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

The herein presented method is a tool for stratifying an AML patient into a high-risk or a low-risk group, wherein the proteomic risk profile of said patient is done by identifying abnormal protein levels of at least 5 proteins selected from the list of proteins with SEQ ID NOs as provided in table 5, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

### Clustering patients

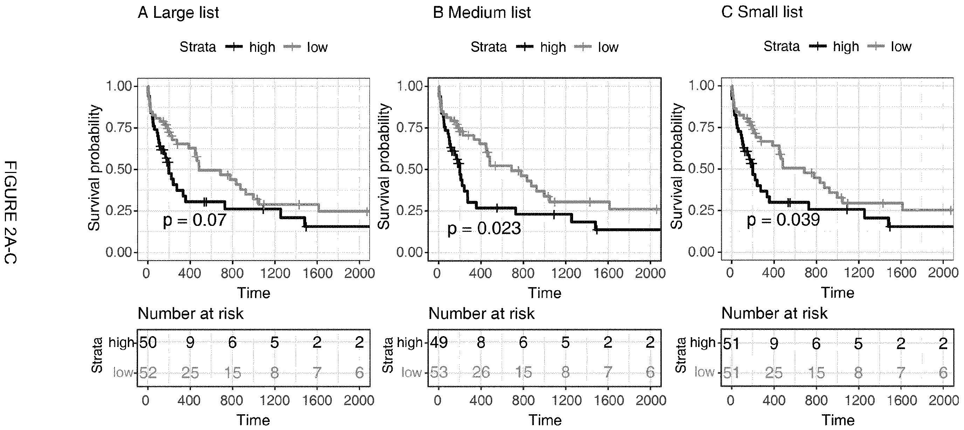

As shown in Example 1, AML patients were stratified into three groups based on clustering of spliceosomal proteins (Fig 2,3 and 6A) and investigated whether there is an association between outcome and spliceosome proteins. It was found that higher spliceosome levels were significantly associated with shorter overall survival (Fig 2,3 and 6B) and that lower levels were associated to a higher rate of complete remission.

In conclusion, it is possible to cluster proteins of SEQ ID NO: 1-17500 into sub-groups, wherein one or more of the sub-groups is/are used to stratify patients into at least a high-risk and a low-risk group. One such sub-group is provided in Table 4, another such sub-group is provided in Table 5.

Thus, the present invention also relates to a method for stratifying an AML patient into a high-risk or a low-risk group, wherein the proteomic risk profile of said patient is done by identifying abnormal protein levels of at least 5 proteins selected from the list of proteins with SEQ ID NOs as provided in Table 4, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

Also, the present invention also relates to a method for stratifying an AML patient into a high-risk or a low-risk group, wherein the proteomic risk profile of said patient is done by identifying abnormal protein levels of at least 5 proteins selected from the list of proteins with SEQ ID NOs as provided in Table 5, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

Furthermore, the present invention also relates to a method for stratifying an AML patient into a high-risk, intermediate-risk or a low-risk group, wherein the proteomic risk profile of said patient is done by identifying abnormal protein levels of at least 5 proteins selected from the list of proteins with SEQ ID NOs as provided in Table 4, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

Additionally, the present invention also relates to a method for stratifying an AML patient into a high-risk, intermediate-risk or a low-risk group, wherein the proteomic risk profile of said patient is done by identifying abnormal protein levels of at least 5 proteins selected from the list of proteins with SEQ ID NOs as provided in Table 5, wherein the protein level is considered abnormal when said protein level is downregulated or upregulated compared to the median expression level of the cohort, wherein downregulated refers to a protein level that is less than 95% of the median expression level of said protein for the intermediate samples of the cohort, upregulated refers to a protein level that is at least 5% above the median expression level of said protein for the intermediate samples of the cohort, proteins that are downregulated are proteins with a SEQ ID NO provided in Table 2, and proteins that are upregulated are proteins with a SEQ ID NO provided in Table 3.

### Algorithms

Clustering algorithm(s) used in the presented method is/are one or more algorithms selected from the group consisting of Random Forest, k-Top Scoring Pairs, k-Nearest Neighbor, Support Vector Machines, oPLS-DA, PLS-DA, t-SNE, UMAP, PCA, lasso, Decision Trees, Naïve Bayes and Logistic Regression.

In one or more embodiments, the present disclosure relates to a method for clustering a cohort of AML patients into risk groups, wherein the clustering method comprises the use of one or more of the algorithms selected from this group consisting of Random Forest, k-Top Scoring Pairs, k-Nearest Neighbor, Support Vector Machines, oPLS-DA, PLS-DA, t-SNE, UMAP, PCA, lasso, Decision Trees, Naïve Bayes and Logistic Regression.

In one or more embodiments, the present disclosure relates to a method for clustering a cohort of AML patients into risk groups, wherein the clustering method comprises the use of the k-Top Scoring Pairs, Support Vector Machines and/or Random Forest, and wherein the data is obtained using DIA-MS, DDA-MS, PRM-MS and/or ELISA.

In one or more embodiments, the present disclosure relates to a method for clustering a cohort of AML patients into risk groups, wherein the clustering method comprises the use of Random Forest, k-Top Scoring Pairs, k-Nearest Neighbor and/or Support Vector Machines.

In one or more embodiments, the present invention relates to a method for generating a proteomic profile of a patient suspected of suffering from high-risk Acute Myeloid Leukemia ("AML"), and/or for stratifying an AML patient into a high-risk or a low-risk group or into a high-risk, intermediate risk and low risk group in relation to overall survival and/or complete remission, based on proteomic profiling according to the present invention, wherein the stratification method comprises the use of one or more of the algorithms selected from this group consisting of Random Forest, k-Top Scoring Pairs, k-Nearest Neighbor, Support Vector Machines, oPLS-DA, PLS-DA, t-SNE, UMAP, PCA, lasso, Decision Trees, Naïve Bayes and Logistic Regression.

In one or more embodiments, the present invention relates to a method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic profiling according to the present invention, wherein the stratification method comprises the use Random Forest, k-Top Scoring Pairs, k-Nearest Neighbor and/or Support Vector Machines.

### Proteogenomic method

In one embodiment, a method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic profiling according to the present invention in addition comprises processing the patient's sample and extracting the patients DNA and analyzing the extracted DNA.

Thus, the present invention also relates to a method for stratifying an AML patient into a high-risk or a low-risk group as described herein, wherein the method further comprises, extracting chromosomal DNA from said sample, and analyzing the extracted DNA and determining genetic risk factors based on genetic variants in one or more genes and providing a patient stratification into groups based on their clinical and molecular profile.

Said sample can e.g., be obtained from bone marrow, peripheral blood, isolated cells from blood, tumour and normal tissue (mouth swab, skin biopsy etc.,) often required for genetic analysis (WGS/WES, for example for ELN2017).

### Extracting DNA

Extracting DNA is a routine procedure in molecular biology. For the chemical method, there are many different commercially available kits used for extraction.

### Analyzing the extracted DNA

Quantitation of nucleic acids is commonly performed to determine the average concentrations of DNA or RNA present in a mixture, as well as their purity. To date, there are two main approaches used by the person skilled in the art to quantitate, or establish the concentration, of nucleic acids (such as DNA or RNA) in a solution. These are spectrophotometric quantification and UV fluorescence tagging in presence of a DNA dye.

The extracted DNA can be analysed using DNA hybridization and sequencing, and/or quantitative mass spectrometry analysis and/or ELISA or similar methodologies known to the skilled artesan.

The extracted DNA can e.g., be analysed by Short tandem repeat (STR) analysis which builds upon restriction fragment length polymorphism (RFLP) and Amplified fragment-length polymorphism (AmpFLP) used in the past by shrinking the size of the repeat units, to 2 to 6 base pairs, and by combining multiple different loci into one PCR reaction. Alternatively, next-generation sequencing, such as, but not limited to massively parallel sequencing (MPS) can be employed.

For identification of e.g., single nucleotide polymorphism or other DNA sequences variations Dynamic allele-specific hybridization (DASH), molecular beacons or microarrays e.g., high-density oligonucleotide arrays can be employed.

### Genetic standards

In one embodiment, a method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic-genomic profiling according to the present invention comprises, extracting chromosomal DNA from said patient, analyzing the extracted DNA and determining genetic risk factors based on genetic variants in one or more genes listed in a genetic standard, selected from the group consisting of ELN2017 and NCCN Guidelines and providing a patient stratification into groups based on their Overall, complete, combined, clinical and molecular profile.

### ELN Guidelines

The ELN guidelines for stratification of AML patients are updated regularly and comprises a number of mutated or otherwise abnormal genes. In general AML patients are stratified into three groups, Favourable, Intermediate and Adverse, thus aiming at predicting the expected clinical outcome for a patient.

The ELN2017 guidelines to stratify patients into different risk groups comprise the following abnormal genes:
Favourable:
   t(8;21)(q22;q22.1); RUNX1-RUNX1T1
   inv(16)(p13.1;q22) or t(16;16)(p13.1;q22); CBFB-MYH11
   Mutated NPM1 without FLT3-YTD or with FLT3-ITD^{low}
   Biallelic mutated CEBPA
Intermediate:
   Mutated NPM1 and FLT3-ITD^{high}
   Wild-type NPM1 without FLT3-YTD or with FLT3- ITD^{low} (without adverse-risk genetic lesions) t(9;11)(p21.3;q23.3); MLLT3-KMT2A
   Cytogenetic abnormalities not classified as favourable or adverse
Adverse:
   t(6;9)(p23;q34.1); DEK-NUP214
   t(v;11q23.3); KMT2A rearranged
   t(9;22)(q34.1;q11.2); BCR-ABL1
   inv(3)(q21.3;q26.2) or t(3;3)(q21.3;q26.2); GATA2, MECOM(EVII)
   - 5 or del(5q); - 7; -17/abn(17p)
   Complex karyotype,c monosomal karyotyped
   Wild-type NPM1 and FL73-ITD^{high}
   Mutated RUNXI
   Mutated ASXLI
   Mutated TP53

### NCCN Guidelines

The NCCN guidelines for stratification of AML patients are updated regularly and comprises a number of mutated or otherwise abnormal genes. In general AML patients are stratified into three groups, Favourable, Intermediate, and Intermediate and Poor or Adverse, thus aiming at predicting the expected clinical outcome for a patient.

The NCCN 2020 guidelines to stratify patients into different risk groups comprise the following abnormal genes:
Favourable:
   t(8;21)(q22;q22.1); RUNX1-RUNX1T1
   inv(16)(p13.1q22) or t(16;16)(p13.1q22); CBFB-MYH11
   Biallelic mutated CEBPA
   Mutated NPM1 without FLT3-ITD
Intermediate:
   Mutated NPM1 and FLT3-ITD^{high}
   Wild-type NPM1 without FLT3-ITD or with FL73-ITD^{low} (without adverse-risk genetic lesions)
   t(9;11)(p21.3;q23.3); MLLT3-KMT2A
   Cytogenetic abnormalities not classified as favourable or adverse
Poor or Adverse:
   t(6;9)(p23;q34.1); DEK-NUP214
   t(v;11q23.3); KMT2A rearranged
   t(9;22)(q34.1;q11.2); BCR-ABL1
   inv(3)(q21.3q26.2) or t(3;3)(q21,3;q26.2); GATA2,MECOME(EVI1)
   -5 or del(5q);-7;-17/abn(17p)
   Complex karyotype, monosomal karyotype
   • Wild-type NPM1 and FLT3-ITD^{high}
   Mutated RUNX1
   Mutated ASXL1
   Mutated TP53

In embodiments, the invention relates to a method for stratifying an AML patient into a high-risk or a low-risk group in relation to overall survival after 1 year, wherein genetic risk factors are determined based on genetic variants in at least 5 genes listed in a genetic standard, such as but not limited to selected from the group consisting of ELN2017 and NCCN Guidelines and providing a patient stratification into groups based on their clinical and molecular profile.

A method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic-genomic profiling according to the present invention comprises, comprises determining genetic risk factors based on genetic variants in at least 10 genes listed in a genetic standard, selected from the group consisting of ELN2017 and NCCN Guidelines and providing a patient stratification into groups based on their clinical and molecular profile.

A method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic-genomic profiling according to the present invention comprises of determining genetic risk factors based on genetic variants in at least 50 genes listed in a genetic standard, selected from the group consisting of ELN2017 and NCCN Guidelines and providing a patient stratification into groups based on their clinical and molecular profile.

A method for generating a proteomic profile of a patient suspected of suffering from high-risk Acute Myeloid Leukemia ("AML"), and/or for stratifying an AML patient into a high-risk or a low-risk group in relation to overall survival and/or complete remission, based on proteomic-genomic profiling according to the present invention comprises of determining genetic risk factors based on genetic variants in at least 100 genes listed in a genetic standard, selected from the group consisting of ELN2017 and NCCN guidelines and providing a patient stratification into groups based on their clinical and molecular profile.

### Put to practice in different ways

The present invention can be put to practice in different ways. First, decreased and/or increased expression of the proteins may be used for diagnosing high-risk AML. As used herein, the term "diagnosing" refers, without limitation, to a process aimed at determining whether or not a subject is afflicted with high-risk AML. This is also meant to include instances where the presence or a stage of AML is not finally determined but that further diagnostic testing is warranted. In such embodiments, the method is not by itself determinative of the presence or absence of AML, or the stage of AML in the subject but can indicate that further diagnostic testing is needed or would be beneficial. Therefore, the present method may be combined with one or more other diagnostic methods for the final determination of the presence or absence of AML and/or high-risk AML in the subject. Such other diagnostic methods are well known to those skilled in the art.

Mass spectrometry may be used to detect changes in the absolute or relative expression level of a protein or set of proteins (or their peptide products produced by digestion). This is to be taken to include changes in alternative splicing of proteins, their Post-Translational Modifications or changes in the structure of the post-translational modifications themselves (e.g., changes in glycan structures of O- or N-linked glycosylated peptides).

Importantly, the present invention enables early detection or diagnosis of high-risk AML. Early diagnosis of high-risk AML could allow early treatment and, thus, delay progression. Moreover, early diagnosis would enable the subject to take appropriate measures aiming at delaying progression or alleviate symptoms.

In some embodiments, the present method may be used to define a subgroup of AML, i.e., to stratify a patient into an AML subgroup, such as, but not limited to, belonging to a high-risk or low-risk group. High-risk and low-risk groups are in some embodiments defined in relation to overall survival after 1 year, in relation to overall survival, complete remission and/or event-free remission.

### Overall survival, overall survival after 1 year

In the current context, patient overall survival is used to describe if the patient is still alive for a given period of time after diagnosis. It is a method of describing prognosis in certain disease conditions. Overall survival rate can be used as yardstick for the assessment of standards of therapy. The survival period is usually reckoned from date of diagnosis or start of treatment. Survival rates are important for prognosis and may be different depending on treatments as well as the overall general health of the patient. In general, it is known in the field to use mean overall survival rates to estimate the patient's prognosis. This is often expressed over standard time periods, like one, five, and ten years. For example, a patient with a higher one-year overall survival has a better prognosis. Sometimes the overall survival is reported as a death rate (%) without specifying the period the % applies to (possibly one year) or the period it is averaged over (possibly 5 years). Disease-specific survival rate refers to "the percentage of people in a study or treatment group who have not died from a specific disease in a defined period of time. The time period usually begins at the time of diagnosis or at the start of treatment and ends at the time of death. Patients who died from causes other than the disease being studied are not counted in this measurement.

In some embodiments of the current context, the herein described stratification method will allow to stratify the patient into a high-risk or low-risk group in relation to overall AML specific survival, such as over a standard time period, such as over one, five or ten years. A patient belonging in a high-risk group in relation to overall survival will have a low expectation of overall survival. E.g., a patient belonging in a high-risk group in relation to overall survival after 1, 5 or 10 year(s) will have a low expectation of overall survival after 1, 5 or 10 year(s), respectively.

Median survival, or "median overall survival" is also commonly used to express survival rates. This is the amount of time after which 50% of the patients have died and 50% have survived. In some embodiments of the current context, the herein described stratification method will allow to stratify the patient into a high-risk or low-risk group in relation to median AML specific survival. A patient belonging in a high-risk group in relation to median survival will have a low expectation of survival after the amount of time after which 50% of the patients have died.

In cancer research, various types of survival rates can be relevant, depending on the cancer type and stage. These include the disease-free survival (DFS) (the period after curative treatment [disease eliminated] when no disease can be detected), the progression-free survival (PFS) (the period after treatment when disease [which could not be eliminated] remains stable, that is, does not progress), and the metastasis-free survival (MFS) or distant metastasis-free survival (DMFS) (the period until metastasis is detected). Progression can be categorized as local progression, regional progression, locoregional progression, and metastatic progression. In some embodiments of the current context, the herein described stratification method will further allow to stratify the patient into a high-risk or low-risk group in relation to disease-free survival (DFS), progression-free survival (PFS), metastasis-free survival (MFS) or distant metastasis-free survival (DMFS).

The comparison is usually made through the Kaplan-Meier estimator approach.

### Complete remission and/or event-free survival

In the current context, the term "Complete remission" is used interchangeably with the term "permanently cured". The survival at any given time is equal to those that are cured plus those that are not cured, but who have not yet died or, in the case of diseases that feature asymptomatic remissions, have not yet re-developed signs and symptoms of the disease. When all of the non-cured people have died or re-developed the disease, only the permanently cured members of the population will remain, and the DFS curve will be perfectly flat. The earliest point in time that the curve goes flat is the point at which all remaining disease-free survivors are declared to be permanently cured. If the curve never goes flat, then the disease is formally considered incurable (with the existing treatments).

Cure rate curves can be determined through an analysis of the data. The analysis allows the statistician to determine the proportion of people that are permanently cured by a given treatment, and also how long after treatment it is necessary to wait before declaring an asymptomatic individual to be cured.

Several cure rate models exist, such as the expectation-maximization algorithm and Markov chain Monte Carlo model. It is possible to use cure rate models to compare the efficacy of different treatments. Generally, the survival curves are adjusted for the effects of normal aging on mortality, especially when diseases of older people are being studied.

AML has been proven to have multiple plateaus, so that what was once hailed as a "cure" results unexpectedly in very late relapses. The goal of treatment for acute myeloid leukemia (AML) is to put the leukemia into complete remission (the bone marrow and blood cell counts return to normal), preferably a complete molecular remission (no signs of leukemia in the bone marrow, even using sensitive lab tests), and to keep it that way. If the patient remains in complete remission for at least 3 years, such as at least 5 years or more, the patient is considered to be cured.

In the current context, the term remission (complete remission) is defined as having no evidence of leukemia after treatment. This means the bone marrow contains fewer than 5% blast cells, the blood cell counts are within normal limits, and there are no signs or symptoms of the disease.

In the present context, the term "event-free remission" is used interchangeably with the term "event-free survival (EFS)". Event-free survival (EFS) may be the preferred endpoint, where the investigator wants the endpoint to reflect the primary treatment, and not subsequent treatments that are given where the study drug fails, and not subsequent treatments that are given if relapse occurs. Where the study drug fails, subsequent treatments are often not controlled by the investigator. In contrast to the endpoint of EFS, the endpoint of overall survival takes into account second-line treatments that are given where the study drug fails, or where the study drug is unacceptably toxic.

In some clinical trials, EFS may be the preferred endpoint, where the cancer in question can be reliably treated by existing drugs. In this situation, use of overall survival as the endpoint would not make much sense, as this particular endpoint would be triggered by so few study subjects. Recent advances for treating childhood acute lymphocytic leukemia (ALL) allow the "vast majority" of patients to achieve complete remission and then to be cured as opposed to in adult oncology. Event-free survival has been defined in a number of ways in clinical trials for the various leukemias. For AML, EFS is defined as as as failure to achieve remission, resistant leukemia, relapse, second malignancy, or death of any cause, as failure to achieve CR, relapse, or death as a result of any cause. CR means complete remission (recovery of morphologically normal BM and blood counts (i.e. neutrophils≥1,500/µL and platelets≥100,000/µL), and no circulating leukemic blasts or evidence of extramedullary leukemia).

Regarding the endpoint of time-to-event, or event-free-survival, this sort of composite endpoint should not be configured too broadly. Where the endpoint of time-to-event is defined, for example as "progression of major symptoms," and "death," the value of this particular endpoint would be reduced by using a broader composite that also includes the event "discontinuation of treatment" or "exposure to rescue treatment."

In some embodiments of the current context, the herein described stratification method will allow to stratify the patient into a high-risk or low-risk group in relation to complete remission or event-free remission. A patient belonging in a high-risk group in relation to complete remission or event-free remission will have a less than average expectation compared to an AML patient cohort of AML patients to achieve complete remission or event-free remission.

### Sub-categories and combination of risk assessment

The method of the present invention for the first time provides a tool for generating a proteomic profile of a patient suspected of suffering from high-risk Acute Myeloid Leukemia ("AML"), and/or for stratifying an AML patient into a high-risk or a low-risk group or into a high-risk, intermediate-risk or low risk group in relation to overall survival event free survival, overall survival and/or reaching complete remission.

Thus, the patient stratification is as a basis used to determine if the patient belongs to one of two predefined groups of AML patients, i.e., those with high-risk or low-risk in relation to a median in a given patient cohort.

Alternatively, the patient stratification is as a basis used to determine if the patient belongs to one of three predefined groups of AML patients, i.e., those with high-risk, intermediate-risk or low-risk in relation to a median in a given patient cohort.

In some embodiment, the stratification from the proteomic and genomic methods are combined to further generate additional groups. In some embodiment, the stratification from the proteomic and genomic methods are combined to further generate the categories, adverse-high, adverse-low, intermediate-high, intermediate-low, favorable-high, favorable-low. Thus, in some embodiment, the proteomics based stratification or the genomic based stratification is used as a secondary risk stratification that further divides the proteomic stratification and/or genetic stratification into sub-groups, such as two subgroups for each group, resulting in six subgroups in total.

In an embodiment, subgroups are employed selectively to divide certain genetic categories. In another embodiment, subgroups are employed selectively to divide certain proteomic categories.

In an embodiment the result of the proteomic stratification is used to subgroup the groups of the genetic stratification. In another embodiment the genetic stratification is used to subgroup the groups of the proteomic stratification. In an embodiment the proteomic stratification of a patient into a high-risk or low risk group is used to subdivide the intermediate group of a genetic standard such as ELN and/or NCCN, into Intermediate-high-risk, Intermediate-low-risk.

In another embodiment the sub-groups Intermediate-high-risk, Intermediate-low-risk are combined with the groups of the genetic standard to form three combined groups, comprising Highest combined risk (Adverse High, Intermediate-high), Intermediate combined risk (Adverse-low, Intermediate-low) and Favorable (Favorable-high, Favorable -low).

Alternatively, the categories can be constructed as: Highest combined risk (Adverse High, Intermediate-high), Intermediate combined risk (Adverse-low, Favorable-high) and Lowest combined risk (Intermediate-low, Favourable-low).

The proteomic risk stratification comprising high-risk, intermediate-risk and low-risk can also be used as stated above i.e., as a secondary measure to further subdivide patients within the genetic categories providing in total 9 categories when combined with the genetic standard.

In the present disclosure "guidelines" and "standard" are used interchangeably.

### Scoring matrix

Alternatively, a scoring matrix can be used where patients are scored 3 for the highest risk, 2 for intermediate and 1 for low risk/ favourable in the genetic risk stratification and the proteomics risk stratification respectively, as shown in Table 6.

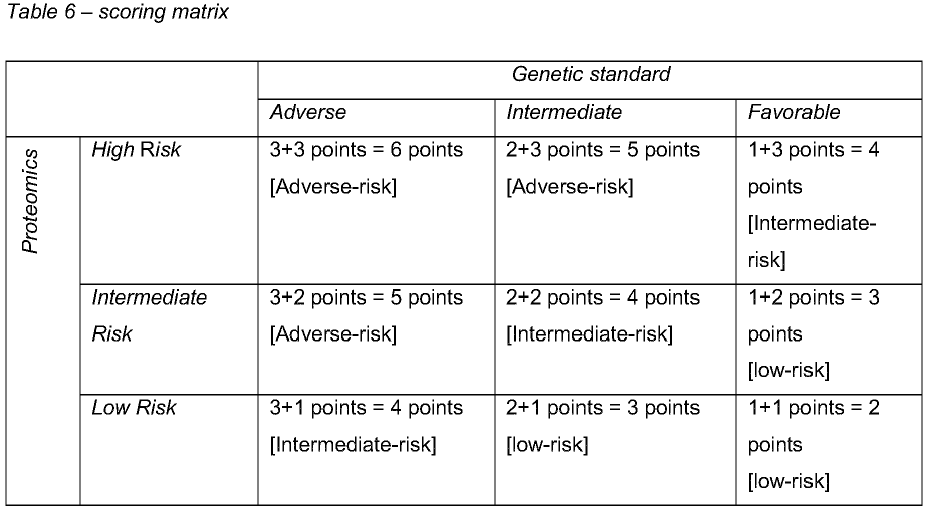

Thus, in an embodiment, the proteomic and genetic risk stratification are combined using a scoring matrix, providing the groups Adverse-risk, Intermediate-risk and Low-risk.

In an embodiment, the proteomic and genetic risk stratification are combined using a scoring matrix, as shown in Table 6.

Accordingly, the present invention may also be used for stratifying participants for clinical studies. An example here could be the choice between using Oxford Biomedica's gene therapy called ProSavin (OXB101) that is most effective in treating early-stage patients or using their higher dosage drug, OXB102 which is targeted at patients in an advanced disease state.

### Overall, complete, combined, clinical and molecular profile

The patient to be stratified with the method described herein is a human either suspected of having AML, or having been diagnosed with AML, such as high-risk AML. Methods for identifying subjects suspected of having AML may include physical examination, subject's family medical history, subject's medical history, biopsy, or a number of imaging technologies such as ultrasonography, computed tomography, magnetic resonance imaging, magnetic resonance spectroscopy, or positron emission tomography. Diagnostic methods for AML and the clinical delineation of AML diagnoses are well known to those of skill in the medical arts.

The present invention relates to the application of recently developed proteomic methods for identifying expression levels of AML-related proteins selected to correlate with AML progression and predicting disease outcome useful for diagnosis and prognosis. As is well known in the field, a patients response to treatment and survival or remission is always highly dependent on the patient's overall, complete, combined, clinical and molecular profile. Thus, the presented method can in the clinic be combined with assessing age, gender, pre-treatment, other complications, co-administrations etc.

Thus, a method for stratifying an AML patient into a high-risk or a low-risk group according to the present invention in one embodiment further comprises, stratifying AML patients belonging to the following patient groups selected from the group consisting of age, gender, pre-treatment, AML-etiology, ECOG status, other complications and co-administrations.

The proteomics based risk stratification can be combined with genetics based risk assessment (e.g. ELN2017) in several straightforward ways.

### Drug screening

Furthermore, the present invention may be utilized in drug screening for identifying drugs suitable for restoring normal translation in a cell. For instance, compound libraries may be screened for modulators of decreased overall translation by applying a candidate compound over a cell culture showing decreased or increased protein expression of any one of the proteins listed in Table 1, 4, or 5, such as in a cell culture obtained from a subject with AML, and analyzing the compound for any change in the level of translation by any standard technique known in the art. Normalized translation by pharmacological means would be expected to prevent, cure, ameliorate or alleviate AML.

### Predicting response to apoptosis modulating drugs

The present invention relates to the application of recently developed proteomic methods for identifying expression levels of AML-related proteins selected to correlate with AML progression and predicting disease outcome useful for diagnosis and prognosis. Furthermore, said method can be used for predicting response to apoptosis modulating drugs (including but not limited to Venetoclax, Navitoclax and Triciribine or combinations thereof) in an AML patient, wherein the patient is stratified as respondent when said patient is found to belong to the high-risk group as defined by proteomic risk profiling.

### Chemotherapy

Furthermore, the herein disclosed method can be used for predicting response to standard chemotherapy wherein the patient is stratified as respondent when said patient is found to belong to the low-risk group. AS shown in the experimental section, patients belonging to the low-risk group defined by proteomic risk profiling are significantly better responders (a higher proportion reach complete remission defined as <5% blasts in Bone Marrow).

### Monitoring

In some embodiments, biological samples may be obtained from the subject at various time points before, during, or after treatment. The level of translation in the biological sample is then determined and compared with that in a biological sample obtained from the same subject at a different time point, or with a control level obtained, for example, from a reference sample derived from an individual whose AML state is known and/or who has not been exposed to said treatment. In some embodiments, predetermined reference values obtained from a pool of apparently healthy individuals may be used as control levels in said comparisons.

Accordingly, the present invention may be used not only for diagnostic purposes but also for monitoring AML. As used herein, the term "monitoring" includes monitoring a subject's disease state or progression of AML over time, as well as monitoring any possible remission or relapse of the disease, or response to treatment. Said monitoring may be carried out by continuously assessing the level of translation and/or performing a medical test repeatedly. In some embodiments of the present invention, a subject's disease state is monitored by obtaining samples repeatedly, assaying the samples for decreased overall translation, and comparing assay results with one another and with a reference value to identify any change in the subject's disease state. Any of these aspects of the invention may be used in combination with other diagnostic tests.

In some embodiments, any presently known or future preventative and/or treatment regimen may be prescribed and/or administered to subjects diagnosed with AML by the present method. Methods are thus provided for identifying a subject with high-risk or low-risk AML, and then prescribing a preventative or therapeutic regimen to said subject. Thus, some embodiments may be directed to methods of reducing risk of AML or treating AML in a subject. In some further embodiments, such methods may be carried out in the context of a clinical study.

### Therapeutic target

In some aspects, the specific AML associated proteins identified herein may be utilized as a therapeutic target. These proteins can be targeted by specific reagents designed to interfere with their functions and or expression. For example, many of these proteins have specific receptors and therapeutic agents can be used to block the interactions of proteins with their receptors or with other AML associated proteins in order to treat AML. Additionally, some of the proteins are enzymes. Therapeutics may be used to interfere with the enzymatic activities of these proteins. Additionally, the expression of these proteins can be inhibited using inhibitory RNA. A therapeutic agent useful for blocking a protein-receptor or a protein-protein interaction is any type of reagent that binds to one or both of the proteins (receptor or ligand) and blocks the proteins from interacting. The reagent may be a protein, small molecule, nucleic acid or any other type of molecule which binds to and blocks the interaction, such as a receptor antagonist. For example, the reagent may be (using antibodies, antibody fragments, peptides or peptidomimetics. Integrins as therapeutic reagents are described in, for example, Goodman and Picard, TIPS, 968, p. 1 (2012). Additionally anti-integrins, such as anti-integrin antibodies may be used as therapeutic reagents.

A therapeutic agent useful for blocking enzyme function is any reagent that interrupts the interaction or activity of the enzyme with its substrate. For example, the reagent may directly interfere with the interaction. For instance, a structural antagonist of the substrate may compete for binding to the enzyme and block the interaction between the enzyme and substrate. Additionally, the regent may indirectly interfere with the interaction by causing a conformational change or stability change in the enzyme which results in a loss of the enzymes ability to bind to the substrate or act on the substrate.

As used herein, the term treat, treated, or treating when used with respect to a disorder refers to a prophylactic treatment which increases the resistance of a subject to development of the disease or, in other words, decreases the likelihood that the subject will develop the disease as well as a treatment after the subject has developed the disease in order to fight the disease, prevent the disease from becoming worse, or slow the progression of the disease compared to in the absence of the therapy.

### Kit for use in diagnosing or stratifying an AML patient

The present invention also provides a kit for use in the herein disclosed method for stratifying an AML patient. Said kit may comprise any reagents or test agents necessary for assessing protein translation as disclosed herein. Those skilled in the art can easily determine the reagents to be included depending on the specifics of the embodiment in question and a desired technique for carrying out said assessment. In some embodiments, an appropriate control sample or a threshold value may be comprised in the kit. The kit may also comprise a computer readable medium, comprising computer-executable instructions for performing any of the methods of the present disclosure.

### Machine learning

In addition, the present invention also presents a method for determining proteomic marker profiles for stratifying AML patients into at least a high-risk or a low-risk group, based on comparing the proteomic profiles of an AML patient cohort of AML patients, wherein the method comprises,
a. isolating blood and/or tissue samples from said patients,
b. processing said sample(s), wherein the processing comprises
   i. extracting expressed proteins from said sample,
c. analyzing the extracted proteins,
d. determining a median expression level of said extracted proteins for the intermediate samples of the cohort, and
e. quantitatively determining the proteomic risk profile of said patients by identifying abnormal protein levels of at least 5 proteins selected from the list of proteins with SEQ ID NOs: 1-17500, wherein the protein level is considered abnormal when the level of any one of the proteins with a SEQ ID NO as provided in Table 3 are <95% of the median expression level for the intermediate samples of the cohort, or any one of the proteins with a SEQ ID NO as provided in Table 2 are up-regulated by at least >5% above the median expression level of the cohort,
f. feeding said proteomic profiles into a machine learning algorithm to determine which protein profiles are high-risk or low-risk determinants,
g. validating the outcome of step f) in a sample group of patients with known disease outcome.

A machine learning algorithm to determine which protein profiles are high-risk or low-risk determinants in step f. can e.g., be either Hidden Markov Model (HMM) or unsupervised machine learning (ML) or supervised machine learning (ML) or a combination thereof.

As is apparent to a skilled person, any embodiments, details, advantages, etc. of the disclosed methods apply accordingly to other aspects of the present invention, including a kit for use in said methods, and vice versa.

It will be obvious to a person skilled in the art that, as technology advances, the inventive concept can be implemented in various ways. The invention and its embodiments are not limited to the examples described above but may vary within the scope of the claims.

### EXAMPLES

The following examples are put forth so as to provide those of ordinary skill in the art with a complete disclosure and description of how to make and use the subject invention and are not intended to limit the scope of what is regarded as the invention. Efforts have been made to ensure accuracy with respect to the numbers used (e.g., amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

### Example 1 Proteogenomic Analysis of treatment naïve Acute Myeloid Leukemia cohort

The present example relates to the prognostic analysis using the proteomic analysis of the present disclosure, in brief, the cohort consisted of 274 patients form the Clinseq-AML cohort that was treated in Sweden between February 1997 and August 2014.

### Materials and Methods

### Patients

Bone marrow or peripheral blood samples were obtained at the time of diagnosis from 274 AML patients of the Clinseq-AML cohort treated in Sweden between February 1997 and August 2014. Samples were separated for mononuclear cells and stored in isothermal liquid nitrogen freezers at -180 °C. All patients were treated with intensive induction regimens, including anthracyclines and cytosine arabinoside, according to national guidelines. Clinical data was retrieved from the Swedish Adult Acute Leukemia Registry or from patient records. For the proteomics study 118 patient samples from the Clinseq-AML cohort, which had sufficient material and were representative of the greater cohort were selected.

### Data dependent acquisition Mass Spectrometry using TMT10 plex labelling

Cell pellets were dissolved in Lysis buffer (4% SDS, 50 mM HEPES pH 7,6, 1 mM DTT), heated to 95°C and sonicated. The total protein amount was estimated (Bio-Rad DC). Samples were then prepared for mass spectrometry analysis using a modified version of the SP3 protein clean-up and a digestion protocol [Moggridge, S., et al., J Proteome Res, 2018. 17(4): p. 1730-1740; Hughes, C.S., et al., Mol Syst Biol, 2014. 10: p. 757.], where proteins were digested by LysC and trypsin (sequencing grade modified, Pierce). In brief, up to 250 µg protein from each sample was alkylated with 4 mM Chloroacetamide. Sera-Mag SP3 bead mix (20 µl) was transferred into the protein sample together with 100% Acetonitrile to a final concentration of 70 %. The mix was incubated under rotation at room temperature for 18 min. The mix was placed on the magnetic rack and the supernatant was discarded, followed by two washes with 70 % ethanol and one with 100 % acetonitrile. The beads-protein mixture was reconstituted in 100 µl LysC buffer (0.5 M Urea, 50 mM HEPES pH: 7.6 and 1:50 enzyme (LysC) to protein ratio) and incubated overnight. Finally, trypsin was added in 1:50 enzyme to protein ratio in 100 µl 50 mM HEPES pH 7.6 and incubated overnight. The peptides were eluted from the mixture after placing the mixture on a magnetic rack, followed by peptide concentration measurement (Bio-Rad DC Assay). The samples were then pH adjusted using TEAB pH 8.5 (100 mM final conc.), 65 µg of peptides from each sample were labelled with isobaric TMT-tags (TMT10plex reagent) according to the manufacturer's protocol (Thermo Scientific). Each set consisted of 9 individual patient samples and the tenth channel contained the same sample pool in each set, consisting of a mixture of patient samples. Sample pools were used as denominators when calculating TMT-ratios and thus served to link the 8 sets together. The tryptic peptides for each set were separated by immobilized pH gradient - isoelectric focusing (IPG-IEF) on 3-10 strips.

Of note, the labelling efficiency was determined by LC-MS/MS before pooling of the samples. For the sample clean-up step, a solid phase extraction (SPE strata-X-C, Phenomenex) was performed and purified samples were dried in a SpeedVac. An aliquot of approximately 10 µg was suspended in LC mobile phase A and 1 µg was injected on the LC-MS/MS system.

Online LC-MS was performed using a Dionex UltiMate^{™} 3000 RSLCnano System coupled to a Q-Exactive-HF mass spectrometer (Thermo Scientific). Each of the 72 plate wells was dissolved in 20ul solvent A and 10ul were injected. Samples were trapped on a C18 guard-desalting column (Acclaim PepMap 100, 75µm x 2 cm, nanoViper, C18, 5 µm, 100A), and separated on a 50 cm long C18 column (Easy spray PepMap RSLC, C18, 2 µm, 100A, 75 µm x 50 cm). The nano capillary solvent A was 95% water, 5%DMSO, 0.1% formic acid; and solvent B was 5% water, 5% DMSO, 95% acetonitrile, 0.1% formic acid. At a constant flow of 0.25 µl min-1, the curved gradient went from 6-8% B up to 40% B in each fraction in a dynamic range of gradient length followed by a steep increase to 100% B in 5 min. FTMS master scans with 60,000 resolution (and mass range 300-1500 m/z) were followed by data-dependent MS/MS (30 000 resolution) on the top 5 ions using higher energy collision dissociation (HCD) at 30% normalized collision energy. Precursors were isolated with a 2 m/z window. Automatic gain control (AGC) targets were 1e6 for MS1 and 1e5 for MS2. Maximum injection times were 100 ms for MS1 and 100 ms for MS2. The entire duty cycle lasted -2.5 s. Dynamic exclusion was used with 30 s duration. Precursors with unassigned charge state or charge state 1 were excluded. An underfill ratio of 1% was used.

Protein and peptide identification and quantification was carried out as previously described [Branca, R.M., et al., Nat Methods, 2014. 11(1):p. 59-62; Johansson, H., et al., Nat Commun, 2019]. Briefly, Orbitrap raw MS/MS files were converted to mzML format using msConvert from the ProteoWizard tool suite. Spectra were then searched using MSGF+ (v10072) and Percolator (v2.08), where search results from 8 subsequent fraction were grouped for Percolator target/decoy analysis. All searches were done against the human protein subset of Ensembl 92 in the Galaxy platform. MSGF+ settings included precursor mass tolerance of 10 ppm, fully-tryptic peptides, maximum peptide length of 50 amino acids and a maximum charge of 6. Fixed modifications were TMT-10plex on lysines and peptide N-termini, and carbamidomethylation on cysteine residues, a variable modification was used for oxidation on methionine residues. Quantification of TMT-10plex reporter ions was done using OpenMS project's IsobaricAnalyzer (v2.0). PSMs found at 1% FDR (false discovery rate) were used to infer gene identities.

Protein quantification by TMT10plex reporter ions was calculated using TMT PSM ratios to the entire sample set (all 10 TMT-channels) and normalized to the sample median. The median PSM TMT reporter ratio from peptides unique to a gene symbol was used for quantification. Protein false discovery rates were calculated using the picked-FDR method using gene symbols as protein groups and limited to 1% FDR.

### Data independent acquisition Mass Spectrometry - label free

Cell pellets were dissolved in Lysis buffer (4% SDS, 50 mM HEPES pH 7,6, 1 mM DTT), heated to 95°C and sonicated. The total protein amount was estimated (Bio-Rad DC). Samples were then prepared for mass spectrometry analysis using a modified version of the SP3 protein clean-up and a digestion protocol, where proteins were digested by LysC and trypsin (sequencing grade modified, Pierce). In brief, up to 250 µg protein from each sample was alkylated with 4 mM Chloroacetamide. Sera-Mag SP3 bead mix (20 µl) was transferred into the protein sample together with 100% Acetonitrile to a final concentration of 70 %. The mix was incubated under rotation at room temperature for 18 min. The mix was placed on the magnetic rack and the supernatant was discarded, followed by two washes with 70 % ethanol and one with 100 % acetonitrile. The beads-protein mixture was reconstituted in 100 µl LysC buffer (0.5 M Urea, 50 mM HEPES pH: 7.6 and 1:50 enzyme (LysC) to protein ratio) and incubated overnight. Finally, trypsin was added in 1:50 enzyme to protein ratio in 100 µl 50 mM HEPES pH 7.6 and incubated overnight. The peptides were eluted from the mixture after placing the mixture on a magnetic rack, followed by peptide concentration measurement (Bio-Rad DC Assay).

Thereafter, a peptide clean-up was performed for all samples using the SP3 method. Briefly, fresh SP3 beads suspension (10 µg/µl, 1:10 bead to sample volume) and ACN (final concentration of 95%) were added to 50-200 µg of peptides and incubated under rotation at RT for 30 min. The tubes were then placed on a magnetic rack, the supernatant was discarded, and the beads were washed twice with 200 µl of ACN. The beads were briefly air-dried, after which the peptides were eluted with 100 µl of 3% ACN/0.1% FA and transferred to a new tube. The peptide concentration was measured using the Bio-Rad DC protein assay. The required quantities for further LC-MS analysis were aliquoted and dried in a SpeedVac.

### MS data acquisition

Peptides were separated using a FAIMS system coupled to a Q Exactive Exploris (Thermo Fischer Scientific, San Jose, CA, USA). Samples were trapped on an Acclaim PepMap nanotrap column (C18, 3 mm, 100 A, 75 µm x 20 mm, Thermo Scientific), and separated on an Acclaim PepMap RSLC column (C18, 2 µm bead size, 100 A, 75 µm x 50 cm, Thermo Scientific). Peptides were separated using a gradient of mobile phase A (5% DMSO, 0.1% FA) and B (90% ACN, 5% DMSO, 0.1% FA), ranging from 6% to 30% B in 180 min with a flow of 250 nl/min.

For the DIA-based analysis of the individual tumor samples, the samples were dissolved in phase A (5% DMSO, 0.1% FA) and 5 µg of peptides were injected into the LC-MS system. The data was acquired using a variable window strategy. The survey scan was performed at 120,000 resolution from 400-1200 m/z, with a max injection time of 200 ms and target of 1 × 106 ions. For generation of HCD fragmentation spectra, maximum ion injection time was set as auto and AGC of 2 x 105 were used before fragmentation at 25% normalized collision energy, 30,000 resolution. The sizes of the precursor ion selection windows were optimized to have similar density of precursors m/z based on identified peptides from the spectral library. The median size of windows was 18.3 m/z with a range of 15-88 m/z covering the scan range of 400-1200 m/z. Neighbor windows had 2 m/z overlap.

### DIA-based peptide and protein identification and quantification

Peptide and protein identification and quantification were performed using Spectronaut. All parameters were set as default and for each peptide, the best 3 to 6 fragments were used. Results were filtered at all the precursor, peptide, and protein levels with 1% False discovery rate (FDR). For protein identification and quantification, all DIA raw files were analyzed by Spectronaut software package (version 13.10) from Biognosys. Files were searched against ENSEMBL protein database (GRCh38.92.pep.all.fasta). All parameters were kept as default for protein identification. Briefly, runs were recalibrated using iRT (retention time normalization) standard peptides in a local and non-linear regression. Precursors, peptides and proteins were filtered with FDR 1%. The decoy database was created by mutation method. For quantification, only peptides unique to a protein group were used. Protein groups were defined based on gene symbols to obtain a gene symbol centric quantification. Stripped peptide quantification was defined as the top precursor quantity. Protein group quantification was calculated by the median value of up to 3 most abundant peptides. Normalization was performed at the MS2 level and quantification at the MS2 level based on the peak area. The data filtering was set as Q value for each sample. Some identifications did not have true quantifications at the MS1 level and the instrument's software automatically imputed these with 1, thus, these values of 1 were treated as NAs for further quantitative analysis.

From the identified protein groups, a list of 2519 protein groups identified by their gene symbol were identified as relevant for the classification of the patients into different groups, the groups being high-risk and low-risk groups or high-risk, intermediate-risk and low-risk groups, with relation to the overall survival after 1 (one) year, event free survival, overall survival and/or reaching complete remission.

### Classifying patients into groups

The identified gene symbol lists, protein groups, and/or proteins can be utilized in several ways to stratify patients into 2 or 3 groups.

### Data preparation

Using Labelfree LC-MS/MS data generated by a Data Independent Analysis, as described above, following steps were done after the data was obtained from the mass spectrometer(s):
a) Data normalization using Spectronaut (automated).
b) MS2-level value quantification (described above).
c) Samples with less than 4700 protein groups with corresponding gene symbols identified (as described above) were excluded due to too poor coverage, in the present example this excluded 16 samples.
d) All markers identified and quantified in all samples were utilized
   I. For the full protein group set comprising 2519 protein groups/gene symbols/markers n = 1028/2519 (Protein sequences provided in sequence listing, SEQ ID NOs 1-17500)
   II. For the medium protein group set comprising 537 protein groups/gene symbols/markers (Protein sequences provided in Table 4) n = 332/537
   III. For the small protein group set comprising 249 protein groups/gene symbols/markers (Protein sequences provided in Table 5) n= 178/249.
e) Markers seen as upregulated in the high-risk group ("up") were considered upregulated if levels were >5% above the median level for intermediate samples. Similarly, markers seen as downregulated in the high-risk group were considered downregulated if they were <95% of the median level for the intermediate samples.
f) The number of markers having the correct regulation (up or down) were summed for each sample.

### Patient classification

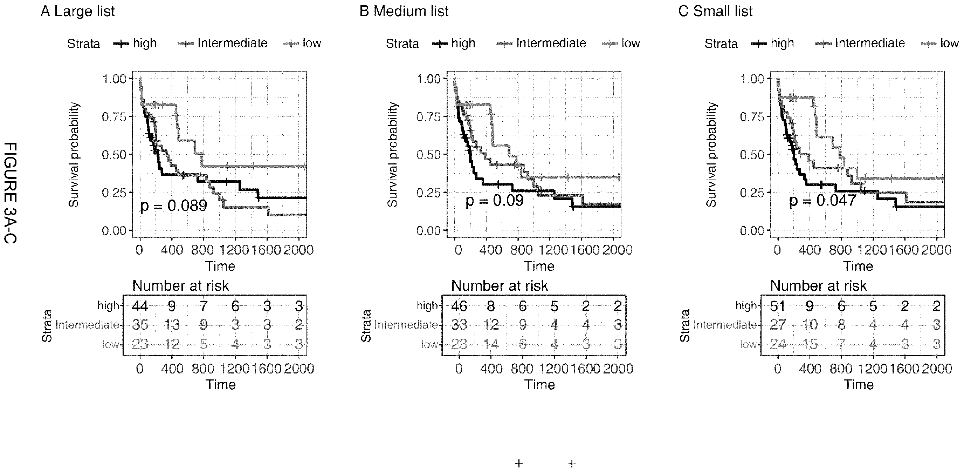

The data that was prepared for each sample could now be classified into different risk-groups in different ways. In the present example, two groups or three were initially used for the classification, as described in the following steps using a simple algorithm:
a) Samples were classified using the following cutoffs:
   I. 2 groups (a high-risk and a low-risk group)
      i. If more than 50% of identified markers were up or down regulated in the correct direction (marker positivity) the sample/patient was considered high-risk.
   II. 3 groups (high risk - intermediate risk - low risk)
      i. If less than 35% of identified markers were up or down regulated in the correct direction see tables 2 and 3the sample/patient was considered low-risk.
      ii. If >50-58% of identified markers were up or down regulated in the correct direction (markers listed in table1 >55%, table 4> 54%, table 5 >50) the sample/patient was considered high-risk.
      iii. Remaining patients were considered intermediate risk.
b) Kaplan-Meier curves were calculated for each classification as is shown in Figures 2 and 3 for the marker lists of different sizes.

The classification of the patients is also exemplified in Examples 1 and 2 of the present disclosure.

Similar approaches could also be employed, if the samples are analysed by DDA (Data Dependent Analysis) and/or isobaric labelling approaches the relationship to an internal standard could be utilized. For Parallel Reaction Monitoring (PRM) based approaches relationships to spiked in labelled peptides could be used.

Additionally, the protein markers could be quantified using affinity-based approaches and compared to a reference or standard. Possible technologies include Western blot, ELISA, Proximity Ligation Assays, Proximity Extension Assays, aptamer-, affimer or bead-based technologies (e.g. Luminex), Reversed phase protein arrays or similar technologies known to the skilled person.

Classification of patients based on the gene lists could be done as specified above (with potential method or reference modifications) or could utilize alternative approaches based on clustering or machine learning. This would include but not be limited to the following either alone or in combination: Random Forest, k-Top Scoring Pairs, k-Nearest Neighbor, Support Vector Machines, oPLS-DA, PLS-DA, t-SNE, UMAP, PCA, lasso, Decision Trees, Naïve Bayes, Logistic Regression.

Output from classification can be utilized in combination with existing genomic based stratification methods to improve patient risk assessment.

### Results

Patients were characterized by mass-spectrometry based proteomics as well as RNA-sequencing, DNA-panel sequencing, Epic array and ex-vivo drug screening at baseline (Fig 5A). Longitudinal follow-up, survival outcome and clinical characteristics as well as treatment response was recorded for all patients. Mutational frequencies of commonly mutated genes were comparable to previous studies.

### The proteomic landscape of AML

To better understand how the phenotypes of the proteome landscape of AML and how they relate to survival and treatment outcomes, a previously developed method was applied, in-depth HiRIEF-LC-MS/MS [22], which has been utilized previously to perform in-depth characterizations of clinical ALL [23], Breast [24] and Lung cancer [Lehtiö et al. in revision] samples. The workflow identified and quantified more than 12000 protein products (gene centric, FDR<1%) across the entire cohort, with a full overlap of 8632 proteins (Fig 5B).

Using hierarchical clustering 9 clusters were identified in the cohort (Fig 5C,). Survival outcomes differed between the distinct clusters (Fig 5D,), with cluster 4 and 1 exhibiting poorer survival and patients in cluster 2 and 7 exhibiting longer survival. The experimental part also discloses observed treatment response, measured as whether patients achieved complete remission following induction therapy, and genetic risk classification (ELN2017) varied considerably between the clusters (not shown). Patients grouped in cluster 1 showed an average treatment response (60% CR, compared to 64% for the entire cohort), they also exhibited an overall intermediate risk classification (ELN2017, not shown). Patients in cluster 7 exhibited better treatment response (83% CR) as well as overall favorable ELN2017-scores while patients in clusters 2 and 3 had improved CR-rates (80% and 70%, respectively), but more adverse ELN-scores. For clusters 4-6 the remission rates were close to 50% in general (not shown).

To investigate the relationship between mutational patterns and proteome phenotypes the panel sequencing data [Wang, M., et al., Validation of risk stratification models in acute myeloid leukemia using sequencing-based molecular profiling. Leukemia, 2017. 31(10): p. 2029-2036] was utilized to investigate if there were enrichment in the clusters for specific genetic aberrations. It was found that cluster 1 was enriched for FLT3 internal tandem duplications (ITD) (adj. pval = 0.0006), NPM1 mutations (adj.pval = 0.0022) and biallelic CEBPA mutations (adj. pval = 0.030) while cluster 4 showed enrichment for mutations in splice factors SF3B1 and U2AF1 (adj.pvals = 0.005 and 0.089, respectively, figure 5D and F). Cluster 7 consisted entirely of patients with inv(16) (enrichment adj. pval < 3.1E-9), which is in line with previous reports from transcriptomic studies of AML where the expression profile of inv(16) is clearly distinguishable from other AML subtypes [Gutierrez, N.C., et al., Gene expression profile reveals deregulation of genes with relevant functions in the different subclasses of acute myeloid leukemia. Leukemia, 2005. 19(3): p. 402-9]. By applying the mutation-based classifications from Papaemmanuil et al. [Papaemmanuil, E., H. Dohner, and P.J. Campbell, Genomic Classification in Acute Myeloid Leukemia. N Engl J Med, 2016. 375(9): p. 900-1] their classification was also compared to the proteomic clusters; again inv(16) overlapped with cluster 7, and it was found that NPM1 mutated AML accounted for the majority of cases in Cluster 1, 2 and 6. Biallelic CEBPA was predominantly found in cluster 1, but MLL-rearranged cases divided evenly between cluster 1 and 2. The p53-Complex Karyotype subtype was also evenly divided between clusters 1 and 3 while the spliceosome-chromosomal modifier subtype was spread out across several clusters. Cluster 4 was almost entirely made up of this subtype however, reflecting the enrichment for splice factor mutations observed in that cluster (Fig 5F).

Conversely, it was also investigated which mutations and genetic aberrations had the largest impact on protein levels and found that NPM1, FLT3^{ITD} led to the most prominent changes on the proteome level closely followed by transcription related changes due to biallelic CEBPA mutation and inv(16) (not shown). For 117 of the patients matching RNAseq and proteomics data were obtained, and it was compared the correlation of the overlap (n=8971) on the gene symbol level. A median spearman correlation of 0.36 was found, which is within the range of what has been reported in previous studies of solid tumors [Johansson, H., et al., Breast cancer quantitative proteome and proteogenomic landscape. Nat Commun, 2019. In Press; Mertins, P., et al., Proteogenomics connects somatic mutations to signalling in breast cancer. Nature, 2016. 534(7605): p. 55-62; Zhang, H., et al., Integrated Proteogenomic Characterization of Human High-Grade Serous Ovarian Cancer. Cell, 2016. 166(3): p. 755-765.], notably higher and a greater proportion of significant correlations was found (66%, adjusted P-value < 0.01) compared to what was found previously in acute lymphoblastic leukemia [Yang, M., et al., Proteogenomics and Hi-C reveal transcriptional dysregulation in high hyperdiploid childhood acute lymphoblastic leukemia. Nat Commun, 2019. 10(1): p. 1519.] (not shown). Correlations of complex members was also significantly higher on the protein level compared to the mRNA level in line with previous reports [Johansson, H., et al., Breast cancer quantitative proteome and proteogenomic landscape. Nat Commun, 2019. In Press; Yang, M., et al., Proteogenomics and Hi-C reveal transcriptional dysregulation in high hyperdiploid childhood acute lymphoblastic leukemia. Nat Commun, 2019. 10(1): p. 1519] (not shown).

It was also noted that the proteome level clusters also differed in terms of FAB-classification [Bennett, J.M., et al., Proposals for the classification of the acute leukaemias. French-American-British (FAB) co-operative group. Br J Haematol, 1976. 33(4): p. 451-8] with stem-like or granulocytic differentiated subtypes (M0-M3) being more prevalent in cluster 1,4 and 6 while monocytic subtypes (M4-M5) were dominant in cluster 2 (not shown). In line with this, monocytic markers (CD14, and CD36) were elevated in samples from patients from cluster 2 and to a degree also in cluster 7 (inv(16)) (not shown). Patients in cluster 4 also exhibited increased levels of stemness markers CD34 [Krause, D.S., et al., CD34: structure, biology, and clinical utility. Blood, 1996. 87(1): p. 1-13] and CD133 [Wuchter, C., et al., Impact of CD133 (AC133) and CD90 expression analysis for acute leukemia immunophenotyping. Haematologica, 2001. 86(2): p. 154-61.]. Additionally, it was also explored how signature gene sets for AML subtype differentiation derived from single cell RNAseq van Galen, P., et al., Single-Cell RNA-Seq Reveals AML Hierarchies Relevant to Disease Progression and Immunity. Cell, 2019. 176(6): p. 1265-1281 e24. differed between the presently presented clusters and found that protein levels of genes associated to a HSC-like phenotype were up in cluster 4 and 5, clusters 1, 6 and 8 had a more progenitor /GMP like phenotype while cluster 2, 3 and to a degree 7 exhibited a promono/monocytic phenotype (Fig 5H).

To more broadly characterize the proteomic phenotypes the individual clusters were compared, and enrichment analysis was employed to investigate up and down regulated pathways (Fig 51 -). It was observed that monocyte related genes as well as genes associated to inflammation, TNF-signaling and IL10-signaling were upregulated in cluster 2. In clusters 3 and 5 increased levels of integrin signalling, and integrin interaction related proteins was found. The inv(16) related cluster 7 exhibited decreased oxidative phosphorylation as well as increased chromosome maintenance. Clusters 1 and 4 exhibited increased levels of proteins related to transcription, mRNA processing and splicing. Specifically, spliceosome proteins were generally upregulated in these two clusters (Fig 6A).

### Clustering of spliceosomal proteins

The patients were stratified into three groups based on clustering of spliceosomal proteins (Fig 6A) and investigated whether there is an association between outcome and spliceosome proteins. It was found that higher spliceosome levels were significantly associated with shorter overall survival (Fig 6B) and that lower levels were associated to a higher rate of complete remission (Chi-square High vs Low p-value = 0.02; Intermediate vs Low p-value = 0.05). Spliceosomal proteins were overall well correlated (not shown) while transcripts exhibited a lower level of correlation (Wilcoxon pval <1 E-27). Additionally, the mRNA-protein correlations for the spliceosomal gene products were overall poor and the same phenotype could not be observed using the RNA sequencing data (fig 6C). No gender differences were observed between the groups, but patients with low levels tended to be older. Also, patients with low or intermediate spliceosome levels had lower percentages of blasts both in the bone marrow and lower absolute levels in peripheral circulation compared to patients with high levels. Patients with a high spliceosome phenotype also had lower thrombocyte levels (median TPK: 54); low thrombocyte levels have previously been linked to improved OS in AML [Zhang, Y., et al., Low Platelet Counts at Diagnosis Predict Better Survival for Patients with Intermediate-Risk Acute Myeloid Leukemia. Acta Haematol, 2020. 143(1): p. 9-18.] (not shown).

There were no obvious differences in FAB classification between the three groups but using the scRNAseq derived gene sets described above it was observed that low spliceosome levels were more associated to a promono- or monocyte-like subtype and that the patients with high levels of spliceosome proteins also had increased levels of proteins associated to a progenitor-like state (Fig 6D).

There was no found association (x2-test, p = 0.62), between ELN2017 classification and spliceosome levels indicating that they are independent risk factors (Fig. 6E-F). Univariate cox-regression confirmed that both Adverse ELN-classification as well as high spliceosome levels were related to survival and both metrics retained their significant in a multivariable model indicating independent contributions (not shown). Indeed, combining the two metrics lead to improved stratification of the patient cohort in relation to overall survival (Fig 6G). To better elucidate the genetic contribution to the high spliceosome phenotype it was further investigated which mutations were found more frequently in the high and low spliceosome groups respectively (Fig 6H).

Markers for cell proliferation (MKI67 (Spearman cor = -0.158, p >0.05), PCNA (Spearman cor = 0.180, p >0.05), TOP2A (Spearman cor = -0.222, p <0.05)) did not display significant positive correlation to spliceosome levels. Comparing the three groups it was found that in addition to spliceosome and RNA processing genes being upregulated, genes involved in chromatin organization, sumoylation and DNA repair were also upregulated. Gene sets related to fatty acid metabolism, GPCR and chemokine signalling, hematopoietic lineage markers and cell adhesion/integrins/focal adhesion were comparatively downregulated (Fig 61).

### Example 2 - prospective analysis of patients

### Introduction and Results

To evaluate the prognostic power of the method of the present disclosure, a small set of diagnosis samples was analysed prospectively via DIA-LC-MS/MS. 15 patients who were diagnosed with AML at a single center in the Stockholm County region and who after sampling received as induction treatment for their AML only a combination of Cytarabine and Danorubicin, or patients who received no treatment.

Of the 15 patient samples analysed, 10 of the samples had robust quantification (>4700 gene products quantified, selection criteria as set in Example 1). These samples were classified in the same manner as the retrospective, discovery cohort of Example 1.

When stratifying the patients into two groups (High and low risk respectively) 7 patients were classified as low risk and 3 as high risk. Of the 7 high-risk patients, 3 patients had very high marker positivity (>65%; range: 67% - 76%) on average for the three lists (table 7).

**Table 7 - Classification output of 10 prospective samples.**

| **Marker Positivity*** | | | | **Classification (2 groups)** | | |
|---|---|---|---|---|---|---|
| Patient | Large list | Medium list | Small list | Large list | Medium list | Small list |
| Patient 3 | 74% | 78% | 75% | High | High | High |
| Patient 10 | 63% | 69% | 68% | High | High | High |
| Patient 1 | 73% | 64% | 63% | High | High | High |
| Patient 9 | 61% | 61% | 57% | High | High | High |
| Patient 8 | 51% | 60% | 60% | High | High | High |
| Patient 5 | 59% | 59% | 60% | High | High | High |
| Patient 2 | 64% | 57% | 61% | High | High | High |
| Patient 4 | 42% | 48% | 46% | Low | Low | Low |
| Patient 6 | 44% | 40% | 43% | Low | Low | Low |
| Patient 7 | 24% | 26% | 27% | Low | Low | Low |

In Table 7, the marker positivity was assessed in all patients, the percentages indicate what proportion of detected markers (n = 935 for the Large, n = 312 for the Medium and n = 168 for the Small list) had abundance levels in the correct direction relative to threshold values. Classification was based on the same percentage cutoffs used for classifying the prospective DIA samples.

Next, the marker positivity percentages were employed to stratify the patients into 3 groups (High, Intermediate and Low risk) using the same cut-offs as employed for classifying the retrospective DIA-samples in Example 1.

**Table 8 - Classification output of 10 prospective samples into 3 groups.**

| | **Marker Positivity*** | | | **Classification (3 groups)** | | |
|---|---|---|---|---|---|---|
| Patient | Large | Medium | Small | Large | Medium | Small |
| Patient 3 | 74% | 78% | 75% | High | High | High |
| Patient 10 | 63% | 69% | 68% | High | High | High |
| Patient 1 | 73% | 64% | 63% | High | High | High |
| Patient 9 | 61% | 61% | 57% | High | High | High |
| Patient 8 | 51% | 60% | 60% | Intermediate | High | High |
| Patient 5 | 59% | 59% | 60% | High | High | High |
| Patient 2 | 64% | 57% | 61% | High | High Intermedi ate | High Intermedi ate |
| Patient 4 | 42% | 48% | 46% | Intermediate | | |
| | | | | | Intermedi ate | Intermedi ate |
| Patient 6 | 44% | 40% | 43% | Intermediate | | |
| Patient 7 | 24% | 26% | 27% | Low | Low | Low |

In table 8, the marker positivity was assessed in all patients, the percentages indicate what proportion of detected markers (n = 935 for the Large, n = 312 for the Medium and n = 168 for the Small list) had abundance levels in the correct direction relative to threshold values. Classification was based on the same percentage cut-offs used for classifying the prospective DIA samples into 3 groups.

To assess concordance with existing genetic risk-stratification, standard ELN2017 was estimated from the existing clinical data, which is shown in Table 9.

**Table 9 - Estimation of ELN2017 risk in 10 prospective patients.**

| Patient | **ELN estimate** | **ELN-related changes** |
|---|---|---|
| Patient 3 | Adverse | RUNX1 mutation |
| Patient 10 | Favorable | NPM1, no FLT3 mutation, normal karyotype |
| Patient 1 | Favorable | Biallelic-CEBPA mutation |
| Patient 9 | Favorable | NPM1, no FLT3 mutation, normal karyotype |
| Patient 8 | Intermediate | normal karyotype |
| Patient 5 | Intermediate | normal karyotype |
| Patient 2 | Adverse | ASXL1 Mutation |
| Patient 4 | Favorable | inv(16) |
| Patient 6 | Adverse | KMT2A-rearr (not 9;11) |
| Patient 7 | Intermediate | None |

In Table 9, the ELN2017 risk-level was estimated based on existing clinical and genetic data using Table 5 in [Döhner H, Estey E, Grimwade D, et al. Blood. 2017;129(4):424-447].

To evaluate the two-risk stratification, methods information from clinical records was tabulated (Table 10).

**Table 10 - Patient outcomes in 10 prospective patients.**

| | Treatment Outcome | Status | EFS | Survival (cens allo) | Overall Survival |
|---|---|---|---|---|---|
| Patient 3 | CR | Allo | 150* | 175* | 273 |
| Patient 10 | CR | Death | 156 | 185 | 185 |
| Patient 1 | CR2 (venetoclax) | Allo | 71* | 121* | 416 |
| Patient 9 | CR | Allo | 224* | 252* | 341 |
| Patient 8 | no CR | Death | 0*** | 44 | 44 |
| Patient 5 | no CR (untreated) | Death | 0*** | 6 | 6 |
| Patient 2 | CR | Death | 65 | 93 | 93 |
| Patient 4 | CR | Lives, no allo | 157** | 182 | 182 |
| Patient 6 | CR | Lives, no allo | 137** | 163 | 163 |
| Patient 7 | CR | Allo | 140* | 166* | 451 |

In Table 10, the following patient outcomes are tabulated: Treatment outcome, CR = Complete Remission, CR2 = Complete Remission after second treatment (second treatment in parentheses), no CR = Did not reach CR, untreated = Patient died before treatment could begin. Status, Allo = Patient received Allogenic stem cell transplantation, Death = patient died, Lives, no allo = Patient is still alive at end of follow up and did not receive an allogenic HSCT. EFS = Event free survival, calculated from date of CR until any of the following events: * = allogenic HSCT, ** = End of follow up, *** = did not reach CR and 0 is imputed. Survival (cens allo) = Overall Survival time for individual patients from date of diagnosis until either death, end of follow up or date for allogenic HSCT. Overall Survival = Overall Survival time for individual patients from date of diagnosis until either death or end of follow up.

Most of the patients reached CR, one patient (patient 5) died before treatment could begin, one patient did not reach CR (patient 8) and one patient only reached CR after a second round of treatment with Venetoclax (Patient 1).

To evaluate the method, the outcome for patients in the different groups were compared. For the classification of patients into 2 groups (high and low, see table 7) the seven patients classified as high risk had an initial treatment response of 57% (4 patients reached CR1), in the low-risk group all 3 patients reached CR1. In the high-risk group four of the patients died (median survival 69 days) and the remaining 3 received allogenic HSCT (median survival time before transplantation: 175 days). In the low-risk group, one patient received a transplant 166 days after diagnosis and two patients were still alive after end of follow-up despite no transplantation (median survival: 173 days). In the 3-group classification, all 7 patients classified as high-risk group remained in that group while two of the patients classified as low-risk previously, were classified as intermediate risk (Table 8). Comparatively, the ELN2017 estimation into 3 groups (Adverse, Intermediate and Favorable; table 9) grouped three patients as Adverse risk. All three patients reached CR1 and had a median EFS of 137 days (Table 9 and 10). One patient died, one received an allogenic HSCT and one was still alive at end of follow-up. 3 patients were assessed to belong to the intermediate risk group, one of them reached CR1 while the other two patients died, and both died within 44 days or less of diagnosis. The remaining 4 patients were assessed as favorable by ELN2017 criteria, of these patients, 3 reached CR1 and one reached CR after a second round of treatment with Venetoclax (Table 9 and 10). Two patients received allogenic HSCT (median survival until transplant: 149 days), one patient died 185 days after diagnosis and one patient was still alive at end of follow-up (182 days after diagnosis).

To evaluate if combining the two risk assessment methods could improve the patient stratification the ELN2017 assessments were combined with the proteomic classifications (Table 11).

**Table 11 - Combined scoring of prospective patients based on ELN2017 assessment and proteomic risk groups.**

| | ELN estimate | Proteomic risk (2 groups) | Proteomic risk (3 groups) | Combined score 1 | Combined score 2 |
|---|---|---|---|---|---|
| Patient 3 | Adverse | High | High | 6 | 6 |
| Patient 10 | Favorable | High | High | 4 | 4 |
| Patient 1 | Favorable | High | High | 4 | 4 |
| Patient 9 | Favorable | High | High | 4 | 4 |
| Patient 8 | Intermediate | High | High | 5 | 5 |
| Patient 5 | Intermediate | High | High | 5 | 5 |
| Patient 2 | Adverse | High | High | 6 | 6 |
| Patient 4 | Favorable | Low | Intermediate | 2 | 3 |
| Patient 6 | Adverse | Low | Intermediate | 4 | 5 |
| Patient 7 | Intermediate | Low | Low | 3 | 3 |

In Table 11, the classifications of patients into 2 groups (Table 7) and 3 groups (Table 8) were summarized and the median classification was used here (Patient 8 was classified as borderline high/intermediate in one list and high in the other two). Adverse gives 3 points, Intermediate 2 points and Favorable 1 point. For the proteomic risk groups High gives 3 points, Intermediate 2 points and Low 1 point. For each patient, the ELN score was summed with the score from the 2 and 3 group classifications into Combined score 1 and 2, respectively.

**Table 12 - Patient outcomes and Combined score 1.**

| | Combined score 1 | Treatment Outcome | Status | EFS | Survival (cens allo) | Overall Survival |
|---|---|---|---|---|---|---|
| Patient 3 | 6 | CR | Allo | 150* | 175* | 273 |
| Patient 2 | 6 | CR | Death | 65 | 93 | 93 |
| Patient 8 | 5 | no CR | Death | 0** | 44 | 44 |
| Patient 5 | 5 | no CR (no treatment) | Death | 0** | 6 | 6 |
| Patient 10 | 4 | CR | Death | 156 | 185 | 185 |
| Patient 1 | 4 | CR2 (venetoclax) | Allo | 71* | 121* | 416 |
| Patient 9 | 4 | CR | Allo | 224* | 252* | 341 |
| Patient 6 | 4 | CR | Lives, no allo | 137** * | 163 | 163 |
| Patient 7 | 3 | CR | Allo | 140* | 166* | 451 |
| Patient 4 | 2 | CR | Lives, no allo | 157** * | 182 | 182 |

In Table 12, patients are sorted by Combined score 1 and combined with patient outcomes from Table 10.

**Table 13 - Patient outcomes and Combined score 2.**

| | Combined score 2 | Treatment Outcome | Status | EFS | Survival (cens allo) | Overall Survival |
|---|---|---|---|---|---|---|
| Patient 3 | 6 | CR | Allo | 150* | 175* | 273 |
| Patient 2 | 6 | CR | Death | 65 | 93 | 93 |
| Patient 8 | 5 | no CR | Death | 0** | 44 | 44 |
| Patient 5 | 5 | no CR (no treatment) | Death | 0** | 6 | 6 |
| Patient 6 | 5 | CR | Lives, no allo | 137* ** | 163 | 163 |
| Patient 10 | 4 | CR | Death | 156 | 185 | 185 |
| Patient 1 | 4 | CR2 (venetoclax) | Allo | 71* | 121* | 416 |
| Patient 9 | 4 | CR | Allo | 224* | 252* | 341 |
| Patient 4 | 3 | CR | Lives, no allo | 157* ** | 182 | 182 |
| Patient 7 | 3 | CR | Allo | 140* | 166* | 451 |

In Table 13, patients sorted by Combined score 2 and combined with patient outcomes from table 10.

Four patients had a combined score 1 of 5 or 6, the median EFS for the group was 33 days (0-150 days), median survival (cens_allo) was 69 days (6-175) and median Overall survival 69 days (6-273 days). 2 out of 4 patients reached CR in this group and 3 out of 4 patients died. The remaining patient received allogenic HSCT (Table 12) 150 days after reaching CR. Four patients had a Combined score 1 of 4, the median EFS for the group was 147 days (71-224 days), median survival (cens allo) was 174 days (121-252) and median Overall survival 263 days (163-451 days). 3 out of 4 patients reached CR1 in this group and 1 out of 4 patients died. Two of the remaining patients received allogenic HSCT (Table 6) 71-224 days after reaching CR. For the two patients with a combined score 1 of 3 or less both reached CR1, one was transplanted and the other patient was still alive at the end of the follow-up. Combined score 2 produced as noted very similar results for all patients except for patient 6 who went from 4 (combined score 1) to 5 (combined score 2). Patient 6 was classified as low risk (2 group classification) and intermediate risk (3 group classification). Patient 6 was also assessed to have an adverse ELN2017 risk due to a KMT2A-rearrangement (table 9). Despite this Patient 6 reached CR1 and was still alive at the end of the follow up, 137 days after reaching CR without any transplantation.

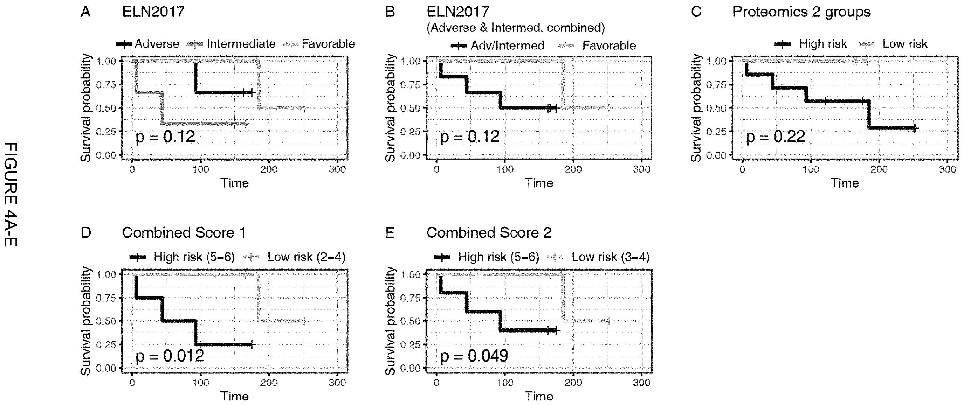

Survival curves were plotted for patients stratified by ELN, proteomics or a combination of the two and significance testing was performed to evaluate the differences between groups using the log-rank test (Figure 4). Stratifying the 10 patients by the estimated ELN2017-risk produced no significant differences (Figure 4A), however, contrasting Adverse+Intermediate risk against Favorable risk showed a trend of better survival in the Favorable group (Fig 4B), at 100 days after diagnosis all Favorable patients were still alive while 50% of the remaining Adverse/Intermediate group patients had succumb to the disease. Similarly, contrasting the 7 patients classified as high risk by our proteomic assessment against the 3 classified as low risk did not reveal any significant differences between the groups but produced the expected trend where the high-risk group exhibited shorter survival (Figure 4C) and at 100 days post diagnosis all low-risk patients were alive while 43% of high-risk patients had died. Surprisingly, when utilizing the combined scores that incorporates both the proteomic risk and the ELN2017-risk (Figure 4D and E) it was observed significant differences despite the very limited number of patients and the short follow-up period.

### Materials and Methods

### Patient samples

Bone marrow or peripheral blood samples were obtained at the time of diagnosis from 15 AML patients of treated in Sweden between 2018 and 2020. Samples were separated for mononuclear cells, cells were washed in PBS and pelleted by centrifugation. Cell pellets were snap-frozen and stored at -80 °C. All patients were treated with intensive induction regimens, including anthracyclines and cytosine arabinoside, according to national guidelines. Patients treated with other induction regimens , such as Hydrea, Venetoclax or Azacytidine, or who had their initial course of cytarabine and danorubicine supplemented with additional targeted treatments, such as Mylotar or Midostaurin, were not included. Clinical data was retrieved from the Swedish Adult Acute Leukemia Registry or from patient records.

### Mass Spectrometry - DIA based proteomics

Each sample was dissolved in 200 µl lysis buffer (25 mM HEPES pH 7.6, 4 % SDS, 1 mM DTT), heated at 90° C for 5 min and sonicated for 1 min. The total protein amount was estimated (Bio-Rad DC). Samples were then prepared for mass spectrometry analysis using a modified version of the SP3 protein clean-up and a digestion protocol (as described earlier in Example 1), where proteins were digested by LycC and trypsin (sequencing grade modified, Pierce). In brief, 200 µg (or the entire amount if <200 µg protein was available) from each sample was alkylated with 4 mM Chloroacetamide. Sera-Mag SP3 (GE Healthcare products 45152105050250 and 65152105050250, distributed by Thermo Fisher) bead mix (20 µl) was transferred into the protein sample together with 100% Acetonitrile to a final concentration of 70%. The mix was incubated under rotation at room temperature for 20 min. The mix was placed on the magnetic rack and the supernatant was discarded, followed by two washes with 70 % ethanol and one with 100 % acetonitrile. The beads-protein mixture was reconstituted in 100 µl Lys-C buffer (0.5 M Urea, 50 mM HEPES pH: 7.6 and 1:50 enzyme (Lys-C) to protein ratio) and incubated overnight. Finally, trypsin was added in 1:50 enzyme to protein ratio in 100 µl 50 mM HEPES pH 7.6 and incubated overnight. Peptide concentration was measured using Bio-Rad DC.

50 µg of peptides from each sample were cleaned by SP3 beads. For that, peptides were dried by SpeedVac, and dissolved in 20 µl water. 10 µl beads were added to each tube and mixed by short vortex. 570 µl acetonitrile was added to each sample to reach 95 % ACN. The mixture was incubated for 30 minutes at room temperature. To remove the buffer, the tube was placed on a magnetic rack and incubated for 2 minutes at room temperature. Supernatant was discarded. Magnetic beads were washed by addition of 250 µl of acetonitrile and incubated for 30 seconds on the magnetic stand. Supernatant was discarded and the beads air-dried. Tryptic peptides were detached from the beads by addition of 100 µl of 3 % ACN, 0.1 % FA and transferred to a new tube.

5 µg of peptides from each sample were injected and separated using an Ultimate 3000 RSLCnano system coupled to an Exploris 480 (Thermo Fischer Scientific, San Jose, CA, USA). Samples were trapped on an Acclaim PepMap nanotrap column (C18, 3 mm, 100 Å, 75µm × 20 mm, Thermo Scientific), and separated on an Aurora Series UHPLC emitter column (25 cm × 75 µm ID, 1.6 µm C18, lonoptiks). Peptides were separated using a gradient of mobile phase A (5 % DMSO, 0.1 % FA) and B (90 % ACN, 5 % DMSO, 0.1 % FA), ranging from 6 % to 30 % B in 120 min with a flow of 0.400 ml/min.

For data independent acquisition (DIA), data was acquired using a FAIMS devise with two CVs: -45 and -65 V. The DIA windows size was 25 m/z in a mass range from 375 to1175 m/z, neighbor windows had an overlap of 1 m/z. For each of the CVs, the survey scan was performed at 120,000 resolution from 375-1175 m/z, with a max injection time set to auto and target of 3e6 ions. For generation of HCD fragmentation spectra, max ion injection time was set as auto and AGC of 2e5 were used before fragmentation at 28 % normalized collision energy, 15,000 resolution. For protein identification and quantification, all raw files analyzed by Spectronaut using the Direct-DIA option without the use of a spectral library, files were searched against ENSEMBL protein database (GRCh38.98.pep.all.fasta). All parameters were kept as default for protein identification. Briefly, runs were recalibrated using iRT standard peptides in a local and non-linear regression. Precursors, peptides and proteins were filtered with FDR 1 %. The decoy database was created by mutation method. For quantification, only peptides unique to a protein group were used. Protein groups were defined based on gene symbols to obtain a gene symbol centric quantification. Stripped peptide quantification was defined as the top precursor quantity. Protein group quantification was calculated by the median value of the top 3 most abundant peptides. Quantification was performed at the MS2 level based on the peak area. The quantitative values were filtered using the qvalue for each sample. Imputation was not performed at any stage of the quantification data generation.

### Patient classification

Patient samples with sufficiently deep proteomics data (>4700 gene products quantified) were classified using the same approach as for the retrospective cohort. Proteins fully quantified across all samples (both the retrospective, discovery cohort and the new prospective samples) were retained. Proteins/gene products overlapping with the 3 lists were utilized for classification yielding: Large list: n = 935/2500 (1028/2500 for the previous retrospective-only DIA classification), Medium: n = 31/537 (332/537 previously), Small: n = 168/249, (178/249 previously). Markers seen as upregulated in the high-risk group ("up") were considered upregulated if levels were >5% above the median level for intermediate samples (in the retrospective group based on the HiRIEF data). Similarly, markers seen as downregulated in the high-risk group were considered downregulated if they were <95% of the median level for the intermediate samples (again, as defined by the HiRIEF data).

For every sample the results were tallied and the percentage of markers trending in the correct direction was calculated.

### Discussion

The proteomic classification method was assessed by applying it to diagnostic samples from 10 prospectively collected AML patients. It was found that 7 out of 10 patients were classified as high risk and 3 as Intermediate/low risk. Treatment response was worse in the High-risk group (57% reaching CR1) and 4 of 7 patients died. In the low-risk group, all patients responded to treatment and no deaths were recorded.

It was also evaluated the combination of our method with an existing genetic risk stratification method (ELN2017) and found that they could be combined to produce improved stratification outputs compared to only using the genetic method for the patients here evaluated. As the number of patients was limited, finding statistically significant differences in survival was not expected, but nonetheless the combination of both methods stratified patients into 2 groups with significantly different survival outcomes.

Additionally, the method was designed based on data from a retrospective cohort where patient samples were viably frozen, then thawed and washed before LC-MS/MS analysis. Here the method using prospectively collected samples which were not freeze-thawed was evaluated and thus demonstrate that the method is compatible with this sample collection technique as well.

## Claims

1. A method for stratifying an AML patient into a high-risk or a low-risk group in relation to overall survival and/or complete remission, based on proteomic profiling of said patient, compared to an AML patient cohort, wherein the method comprises
a. isolating a blood and/or tissue sample from said patient,
b. processing said sample, wherein the processing comprises extracting expressed proteins from said sample,
c. analyzing the extracted proteins, including quantitatively determining their expression level,
d. determining a median expression level of at least 5 proteins of said extracted proteins, selected from the list of proteins with SEQ ID NOs: 1-17500 listed in table 1 for the intermediate samples of the cohort,
e. quantitatively determining the proteomic risk profile of said patient by identifying abnormal protein expression levels of at least 5 proteins selected from the list of proteins with SEQ ID NOs: 1-17500 listed in table 1, wherein the protein expression level is considered abnormal when
i. the protein expression level of a protein with a SEQ ID NO listed in Table 2 is downregulated in such a way that the protein expression level of said protein is less than 95% of the median expression level of said protein for the intermediate samples of the cohort, or
ii. the protein expression level of a protein with a SEQ ID NO listed in Table 3 is upregulated in such a way that the protein expression level of said protein is at least 5% above the median expression level of said protein for the intermediate samples of the cohort, and
f. wherein said AML patient is stratified as belonging to a high-risk group when abnormal protein levels are detected in step e).

2. The method for stratifying an AML patient into a high-risk or a low-risk group according to claim 1, wherein the median expression level of the at least 5, 10, 50 or 100 proteins selected from the list of proteins with SEQ ID NOs: 1-17500 listed in table 1 for the intermediate samples of the cohort is predetermined.

3. A method for stratifying an AML patient into a high-risk or a low-risk group according to any of the preceding claims, wherein said patient is classified as belonging to a high-risk group, when said patient's proteomic profile shows abnormal protein levels in at least 50% of the selected proteins.

4. A method for stratifying an AML patient into a high-risk or a low-risk group according to any of the preceding claims, wherein one or more clustering algorithm(s) is/are used to cluster patients into at least a high-risk and a low-risk group based on abnormal protein levels of at least 5 proteins selected from the list of proteins with SEQ ID NOs: 1-17500.

5. A method for stratifying an AML patient into a high-risk or a low-risk group according to claim 4, wherein the clustering algorithm(s) is/are one or more algorithms selected from the group consisting of Random Forest, k-Top Scoring Pairs, k-Nearest Neighbor, Support Vector Machines, oPLS-DA, PLS-DA, t-SNE, UMAP, PCA, lasso, Decision Trees, Naive Bayes and Logistic Regression.

6. A method for stratifying an AML patient into a high-risk or a low-risk group according to any of the preceding claims, wherein less than 1000, 500, 100 or 50 protein sequences of any of SEQ ID NO: 1-17500, are used for stratifying patients into at least a high-risk and a low-risk group.

7. A method for stratifying an AML patient into a high-risk or a low-risk group according to any of the preceding claims, wherein
a. a patient with a proteomic profile, where at least 50 % of the proteins selected from the list of proteins with SEQ ID NO: 1-17500 have an abnormal level, is considered a high-risk patient and
b. a patient with a proteomic profile, where less than 50 % of the proteins selected from the list of proteins with SEQ ID NO: 1-17500, have an abnormal level, is considered a low-risk patient.

8. A method for stratifying an AML patient into a high-risk or a low-risk group according to any of the preceding claims, wherein the proteomic risk profile of said patient is done by identifying abnormal protein levels of at least 5 proteins selected from the list of proteins with a SEQ ID NO provided in Table 4 or in Table 5.

9. A method for stratifying an AML patient into a high-risk or a low-risk group according to any of the preceding claims, wherein the method further comprises, extracting chromosomal DNA from said sample in step b. and analyzing the extracted DNA and determining genetic risk factors based on genetic variants in one or more genes listed in a genetic standard.

10. A method according to claim 15, wherein the genetic standard is selected from the group consisting of ELN2017 and NCCN guidelines.

11. A method for stratifying an AML patient into a high-risk or a low-risk group according to any one of claims 9-10, wherein genetic risk factors are determined based on genetic variants in at least 5, 10, 50 or 100 genes listed in a genetic standard and providing a patient stratification into groups based on their clinical and molecular profile.

12. A method for stratifying an AML patient into a high-risk or a low-risk group according to any one of claims 15-20, comprising providing a patient stratification into groups based on their overall, complete, combined, clinical and molecular profile.

13. A method for stratifying an AML patient into a high-risk or a low-risk group according to any of the preceding claims, wherein the method further comprises stratifying AML patients belonging to the following patient groups age, gender, pre-treatment, other complications, co-administrations, AML-etiology, ECOG status.

14. A method for predicting response to one or more apoptosis modulating drug(s), such as to a drug selected from the group consisting of venetoclax, navitoclax and triciribine, or to chemotherapy, in an AML patient based on a method according to any one of claims 1-13, wherein the patient is stratified as respondent when said patient is found to belong to the high-risk group.

15. A method of predicting response to therapy in a patient with acute myeloid leukemia (AML) comprising:
a. performing mass spectrometry on a plasma sample from a patient to generate a protein-spectra comprising protein peaks;
b. identifying a protein peak or group of protein peaks in the protein spectra corresponding to one or more proteins selected from the list of proteins with SEQ ID NOs: 1-17500, and
c. predicting the patient's response to therapy based on the identification of one or more of the protein peaks.
